(19) Europäisches Patentamt
European Patent Office
Office européen des brevets

(11) EP 3 269 740 A1

(12) **EUROPEAN PATENT APPLICATION**

(43) Date of publication:
**17.01.2018 Bulletin 2018/03**

(21) Application number: **17181249.8**

(22) Date of filing: **13.07.2017**

(51) Int Cl.:
***C07K 16/40*** *(2006.01)* ***A61K 39/00*** *(2006.01)*
***C07K 14/725*** *(2006.01)* ***C07K 16/30*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **13.07.2016 EP 16179337**

(71) Applicant: **Mabimmune Diagnostics AG**
**8952 Schlieren (CH)**

(72) Inventors:
- **COMBALUZIER, Benoit**
  **8902 Urdorf (CH)**
- **BROKOPP, Chad**
  **8952 Schlieren (CH)**
- **GRIMM, Jan**
  **8600 Dübendorf (CH)**
- **GERSBACHER, Manuel Tobias**
  **8047 Zurich (CH)**

(74) Representative: **Steinecke, Peter**
**Müller Fottner Steinecke**
**Rechtsanwalts- und Patentanwaltspartnerschaft mbB**
**P.O. Box 1140**
**52412 Jülich (DE)**

(54) **NOVEL ANTI-FIBROBLAST ACTIVATION PROTEIN (FAP) BINDING AGENTS AND USES THEREOF**

(57) Provided are novel human-derived antibodies specific for Fibroblast Activation Protein (FAP), preferably capable of selectively inhibiting the enzymatic activity of FAP, and chimeric antigen receptors (CARs) directed against the human FAP antigen as well as methods related thereto. In addition, methods of diagnosing and/or monitoring diseases and treatments thereof which are associated with FAP are provided. Assays and kits related to antibodies specific for FAP are also disclosed. The novel anti-FAP antibodies can be used in pharmaceutical and diagnostic compositions for FAP-targeted immunotherapy and diagnostics.

EP 3 269 740 A1

SEQUENCE LISTING

<110> Mabimmune Diagnostics AG

<120> Novel anti-Fibroblast Activation Protein (FAP) binding agents and uses thereof

<130> MA84A05/P-EP/EP

<150> EP 16 179 337.7
<151> 2016-07-13

<160> 65

<170> PatentIn version 3.5

<210> 1
<211> 363
<212> DNA
<213> Homo sapiens

<220>
<221> CDS
<222> (1)..(363)
<223> NI-206.82C2 variable heavy chain (VH) sequence (not PIMC)

<220>
<221> V_region
<222> (91)..(111)
<223> complementarity determining region (CDR) VH-CDR1

<220>
<221> V_region
<222> (154)..(207)
<223> complementarity determining region (CDR) VH-CDR2

<220>
<221> V_region
<222> (304)..(330)
<223> complementarity determining region (CDR) VH-CDR3

<400> 1
```
cag gtg cag ctg cag gag tcg ggt cca gga ctg gtg aag ccc tcg cag         48
Gln Val Gln Leu Gln Glu Ser Gly Pro Gly Leu Val Lys Pro Ser Gln
1               5                   10                  15

acc ctc tca ctc acc tgt gcc atc tcc ggg gac agt gtc tct agc aac         96
Thr Leu Ser Leu Thr Cys Ala Ile Ser Gly Asp Ser Val Ser Ser Asn
            20                  25                  30

agt gtt act tgg aac tgg atc agg cag tcc cca tcg aga ggc ctt gag        144
Ser Val Thr Trp Asn Trp Ile Arg Gln Ser Pro Ser Arg Gly Leu Glu
        35                  40                  45

tgg ctg gga agg aca tac tac agg tcc aag tgg tat aat gat tat gca        192
Trp Leu Gly Arg Thr Tyr Tyr Arg Ser Lys Trp Tyr Asn Asp Tyr Ala
    50                  55                  60

gta tct gtg aaa ggt cga ata acc atc aat cca gac act tcc aag aac        240
Val Ser Val Lys Gly Arg Ile Thr Ile Asn Pro Asp Thr Ser Lys Asn
65                  70                  75                  80
```

```
cag ttc tac ctg cag ttg aaa tct gtg act ccc gag gat gcg gct gtc         288
Gln Phe Tyr Leu Gln Leu Lys Ser Val Thr Pro Glu Asp Ala Ala Val
                85                  90                  95

tat tat tgt gca aga gat agt agc atc tta tat ggg gac tac tgg ggc         336
Tyr Tyr Cys Ala Arg Asp Ser Ser Ile Leu Tyr Gly Asp Tyr Trp Gly
            100                 105                 110

cag gga acc ctg gtc acc gtc tcc tcg                                    363
Gln Gly Thr Leu Val Thr Val Ser Ser
        115                 120
```

<210> 2
<211> 121
<212> PRT
<213> Homo sapiens

<400> 2

```
Gln Val Gln Leu Gln Glu Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Val Thr Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Gly Arg Ile Thr Ile Asn Pro Asp Thr Ser Lys Asn
65                  70                  75                  80

Gln Phe Tyr Leu Gln Leu Lys Ser Val Thr Pro Glu Asp Ala Ala Val
                85                  90                  95

Tyr Tyr Cys Ala Arg Asp Ser Ser Ile Leu Tyr Gly Asp Tyr Trp Gly
            100                 105                 110

Gln Gly Thr Leu Val Thr Val Ser Ser
        115                 120
```

<210> 3
<211> 363
<212> DNA
<213> Homo sapiens

<220>
<221> CDS
<222> (1)..(363)
<223> NI-206.82C2-PIMC variable heavy chain (VH) sequence after correction of primer induced mutations

```
<220>
<221>  V_region
<222>  (91)..(111)
<223>  complementarity determining region (CDR) VH-CDR1

<220>
<221>  V_region
<222>  (154)..(207)
<223>  complementarity determining region (CDR) VH-CDR2

<220>
<221>  V_region
<222>  (304)..(330)
<223>  complementarity determining region (CDR) VH-CDR3

<400>  3
cag gta cag ctg cag cag tca ggt cca gga ctg gtg aag ccc tcg cag        48
Gln Val Gln Leu Gln Gln Ser Gly Pro Gly Leu Val Lys Pro Ser Gln
1               5                   10                  15

acc ctc tca ctc acc tgt gcc atc tcc ggg gac agt gtc tct agc aac        96
Thr Leu Ser Leu Thr Cys Ala Ile Ser Gly Asp Ser Val Ser Ser Asn
            20                  25                  30

agt gtt act tgg aac tgg atc agg cag tcc cca tcg aga ggc ctt gag       144
Ser Val Thr Trp Asn Trp Ile Arg Gln Ser Pro Ser Arg Gly Leu Glu
        35                  40                  45

tgg ctg gga agg aca tac tac agg tcc aag tgg tat aat gat tat gca       192
Trp Leu Gly Arg Thr Tyr Tyr Arg Ser Lys Trp Tyr Asn Asp Tyr Ala
    50                  55                  60

gta tct gtg aaa ggt cga ata acc atc aat cca gac act tcc aag aac       240
Val Ser Val Lys Gly Arg Ile Thr Ile Asn Pro Asp Thr Ser Lys Asn
65                  70                  75                  80

cag ttc tac ctg cag ttg aaa tct gtg act ccc gag gat gcg gct gtc       288
Gln Phe Tyr Leu Gln Leu Lys Ser Val Thr Pro Glu Asp Ala Ala Val
                85                  90                  95

tat tat tgt gca aga gat agt agc atc tta tat ggg gac tac tgg ggc       336
Tyr Tyr Cys Ala Arg Asp Ser Ser Ile Leu Tyr Gly Asp Tyr Trp Gly
            100                 105                 110

cag gga acc ctg gtc acc gtc tcc tcg                                   363
Gln Gly Thr Leu Val Thr Val Ser Ser
        115                 120


<210>  4
<211>  121
<212>  PRT
<213>  Homo sapiens

<400>  4

Gln Val Gln Leu Gln Gln Ser Gly Pro Gly Leu Val Lys Pro Ser Gln
1               5                   10                  15


Thr Leu Ser Leu Thr Cys Ala Ile Ser Gly Asp Ser Val Ser Ser Asn
            20                  25                  30
```

Ser Val Thr Trp Asn Trp Ile Arg Gln Ser Pro Ser Arg Gly Leu Glu
35 40 45

Trp Leu Gly Arg Thr Tyr Tyr Arg Ser Lys Trp Tyr Asn Asp Tyr Ala
50 55 60

Val Ser Val Lys Gly Arg Ile Thr Ile Asn Pro Asp Thr Ser Lys Asn
65 70 75 80

Gln Phe Tyr Leu Gln Leu Lys Ser Val Thr Pro Glu Asp Ala Ala Val
85 90 95

Tyr Tyr Cys Ala Arg Asp Ser Ser Ile Leu Tyr Gly Asp Tyr Trp Gly
100 105 110

Gln Gly Thr Leu Val Thr Val Ser Ser
115 120

<210> 5
<211> 345
<212> DNA
<213> Homo sapiens

<220>
<221> CDS
<222> (1)..(345)
<223> NI-206.82C2 variable lambda light chain (VL) sequence (PIMC by default)

<220>
<221> V_region
<222> (67)..(108)
<223> complementarity determining region (CDR) VL-CDR1

<220>
<221> V_region
<222> (157)..(177)
<223> complementarity determining region (CDR) VL-CDR2

<220>
<221> V_region
<222> (289)..(315)
<223> complementarity determining region (CDR) VL-CDR3

<400> 5
cag gct gtg ctg act cag ccg tct tcc ctc tct gca tct cct gga gca 48
Gln Ala Val Leu Thr Gln Pro Ser Ser Leu Ser Ala Ser Pro Gly Ala
1 5 10 15

tca gcc agt ctc acc tgc acc ttg ccc agt ggc atc aat gtt ggt acc 96
Ser Ala Ser Leu Thr Cys Thr Leu Pro Ser Gly Ile Asn Val Gly Thr
20 25 30

tac agg ata ttc tgg ttc cag cag aag cca ggg agt cct ccc cag tat 144

```
Tyr Arg Ile Phe Trp Phe Gln Gln Lys Pro Gly Ser Pro Pro Gln Tyr
        35                  40                  45

ctc ctg agt tac aaa tca gac tca gat aat cac cag ggc tct gga gtc        192
Leu Leu Ser Tyr Lys Ser Asp Ser Asp Asn His Gln Gly Ser Gly Val
    50                  55                  60

ccc agc cgc ttc tct gga tcc aaa gat gct tcg gcc aat gca ggg att        240
Pro Ser Arg Phe Ser Gly Ser Lys Asp Ala Ser Ala Asn Ala Gly Ile
65                  70                  75                  80

tta ctc atc tct ggg ctc cag tct gag gat gag gct gac tat tac tgt        288
Leu Leu Ile Ser Gly Leu Gln Ser Glu Asp Glu Ala Asp Tyr Tyr Cys
                85                  90                  95

atg att tgg cac agc agc gct tgg gtg ttc ggc gga ggg acc aag ctg        336
Met Ile Trp His Ser Ser Ala Trp Val Phe Gly Gly Gly Thr Lys Leu
            100                 105                 110

acc gtc cta                                                            345
Thr Val Leu
        115


<210>  6
<211>  115
<212>  PRT
<213>  Homo sapiens

<400>  6

Gln Ala Val Leu Thr Gln Pro Ser Ser Leu Ser Ala Ser Pro Gly Ala
1               5                   10                  15


Ser Ala Ser Leu Thr Cys Thr Leu Pro Ser Gly Ile Asn Val Gly Thr
            20                  25                  30


Tyr Arg Ile Phe Trp Phe Gln Gln Lys Pro Gly Ser Pro Pro Gln Tyr
        35                  40                  45


Leu Leu Ser Tyr Lys Ser Asp Ser Asp Asn His Gln Gly Ser Gly Val
    50                  55                  60


Pro Ser Arg Phe Ser Gly Ser Lys Asp Ala Ser Ala Asn Ala Gly Ile
65                  70                  75                  80


Leu Leu Ile Ser Gly Leu Gln Ser Glu Asp Glu Ala Asp Tyr Tyr Cys
                85                  90                  95


Met Ile Trp His Ser Ser Ala Trp Val Phe Gly Gly Gly Thr Lys Leu
            100                 105                 110


Thr Val Leu
        115
```

```
<210>  7
<211>  366
<212>  DNA
<213>  Homo sapiens


<220>
<221>  CDS
<222>  (1)..(366)
<223>  NI-206.59B4 variable heavy chain (VH) sequence (PIMC)

<220>
<221>  V_region
<222>  (91)..(105)
<223>  complementarity determining region (CDR) VH-CDR1

<220>
<221>  V_region
<222>  (148)..(198)
<223>  complementarity determining region (CDR) VH-CDR2

<220>
<221>  V_region
<222>  (295)..(333)
<223>  complementarity determining region (CDR) VH-CDR3

<400>  7
cag gta cag ctg gtg caa tct ggg gct gag gtg aag aag cct ggg gcc        48
Gln Val Gln Leu Val Gln Ser Gly Ala Glu Val Lys Lys Pro Gly Ala
1               5                   10                  15

tca gtg aag gtc tcc tgc aag act tct gga tac acc ttc acc gac tac        96
Ser Val Lys Val Ser Cys Lys Thr Ser Gly Tyr Thr Phe Thr Asp Tyr
            20                  25                  30

tat ata cac tgg gtg cga cag gcc cct gga caa ggg ctt gaa tgg atg       144
Tyr Ile His Trp Val Arg Gln Ala Pro Gly Gln Gly Leu Glu Trp Met
        35                  40                  45

gga tgg atc aac cct aac aga ggt ggc aca aac tat gca caa aaa ttt       192
Gly Trp Ile Asn Pro Asn Arg Gly Gly Thr Asn Tyr Ala Gln Lys Phe
    50                  55                  60

cag ggc agg gtc acc atg acc agg gac acc tcc atc gct aca gcc tac       240
Gln Gly Arg Val Thr Met Thr Arg Asp Thr Ser Ile Ala Thr Ala Tyr
65                  70                  75                  80

atg gag ttg agt aga ctg aga tct gac gac acg gcc gtg tat tac tgt       288
Met Glu Leu Ser Arg Leu Arg Ser Asp Asp Thr Ala Val Tyr Tyr Cys
                85                  90                  95

gcg act gcg tcg cta aaa ata gca gca gtt ggt aca ttt gac tgc tgg       336
Ala Thr Ala Ser Leu Lys Ile Ala Ala Val Gly Thr Phe Asp Cys Trp
            100                 105                 110

ggc cag ggc acc ctg gtc acc gtc tcc tcg                               366
Gly Gln Gly Thr Leu Val Thr Val Ser Ser
        115                 120


<210>  8
<211>  122
<212>  PRT
```

<213> Homo sapiens

<400> 8

Gln Val Gln Leu Val Gln Ser Gly Ala Glu Val Lys Lys Pro Gly Ala
1               5                   10                  15

Ser Val Lys Val Ser Cys Lys Thr Ser Gly Tyr Thr Phe Thr Asp Tyr
            20                  25                  30

Tyr Ile His Trp Val Arg Gln Ala Pro Gly Gln Gly Leu Glu Trp Met
        35                  40                  45

Gly Trp Ile Asn Pro Asn Arg Gly Gly Thr Asn Tyr Ala Gln Lys Phe
    50                  55                  60

Gln Gly Arg Val Thr Met Thr Arg Asp Thr Ser Ile Ala Thr Ala Tyr
65                  70                  75                  80

Met Glu Leu Ser Arg Leu Arg Ser Asp Asp Thr Ala Val Tyr Tyr Cys
                85                  90                  95

Ala Thr Ala Ser Leu Lys Ile Ala Ala Val Gly Thr Phe Asp Cys Trp
            100                 105                 110

Gly Gln Gly Thr Leu Val Thr Val Ser Ser
        115                 120

<210> 9
<211> 321
<212> DNA
<213> Homo sapiens

<220>
<221> CDS
<222> (1)..(321)
<223> NI-206.59B4 variable lambda light chain (VL) sequence (PIMC)

<220>
<221> V_region
<222> (67)..(99)
<223> complementarity determining region (CDR) VL-CDR1

<220>
<221> V_region
<222> (145)..(165)
<223> complementarity determining region (CDR) VL-CDR2

<220>
<221> V_region
<222> (262)..(291)
<223> complementarity determining region (CDR) VL-CDR3

<400> 9

```
tcc tat gag ctg act cag cca ccc tcg gtg tca gtg tcc cca gga cag         48
Ser Tyr Glu Leu Thr Gln Pro Pro Ser Val Ser Val Ser Pro Gly Gln
1               5                   10                  15

acg gcc agg atc acc tgc tct gga gat gca ttg tca aag caa tat gct         96
Thr Ala Arg Ile Thr Cys Ser Gly Asp Ala Leu Ser Lys Gln Tyr Ala
            20                  25                  30

ttt tgg ttc cag cag aag cca ggc cag gcc cct ata ttg gtg ata tat        144
Phe Trp Phe Gln Gln Lys Pro Gly Gln Ala Pro Ile Leu Val Ile Tyr
        35                  40                  45

caa gac act aag agg ccc tca ggg atc cct ggg cga ttc tct ggc tcc        192
Gln Asp Thr Lys Arg Pro Ser Gly Ile Pro Gly Arg Phe Ser Gly Ser
    50                  55                  60

agc tca ggg aca aca gtc acg ttg acc atc agt gga gcc cag gca gac        240
Ser Ser Gly Thr Thr Val Thr Leu Thr Ile Ser Gly Ala Gln Ala Asp
65                  70                  75                  80

gac gag gct gac tat tat tgt caa tca gca gac agc agt ggt act tat        288
Asp Glu Ala Asp Tyr Tyr Cys Gln Ser Ala Asp Ser Ser Gly Thr Tyr
                85                  90                  95

gtc ttc gga act ggg acc aag gtc acc gtc cta                            321
Val Phe Gly Thr Gly Thr Lys Val Thr Val Leu
            100                 105


<210>  10
<211>  107
<212>  PRT
<213>  Homo sapiens

<400>  10

Ser Tyr Glu Leu Thr Gln Pro Pro Ser Val Ser Val Ser Pro Gly Gln
1               5                   10                  15


Thr Ala Arg Ile Thr Cys Ser Gly Asp Ala Leu Ser Lys Gln Tyr Ala
            20                  25                  30


Phe Trp Phe Gln Gln Lys Pro Gly Gln Ala Pro Ile Leu Val Ile Tyr
        35                  40                  45


Gln Asp Thr Lys Arg Pro Ser Gly Ile Pro Gly Arg Phe Ser Gly Ser
    50                  55                  60


Ser Ser Gly Thr Thr Val Thr Leu Thr Ile Ser Gly Ala Gln Ala Asp
65                  70                  75                  80


Asp Glu Ala Asp Tyr Tyr Cys Gln Ser Ala Asp Ser Ser Gly Thr Tyr
                85                  90                  95


Val Phe Gly Thr Gly Thr Lys Val Thr Val Leu
            100                 105
```

<210> 11
<211> 348
<212> DNA
<213> Homo sapiens

<220>
<221> CDS
<222> (1)..(348)
<223> NI-206.22F7 variable heavy chain (VH) sequence (not PIMC)

<220>
<221> V_region
<222> (91)..(105)
<223> complementarity determining region (CDR) VH-CDR1

<220>
<221> V_region
<222> (148)..(198)
<223> complementarity determining region (CDR) VH-CDR2

<220>
<221> V_region
<222> (295)..(315)
<223> complementarity determining region (CDR) VH-CDR3

<400> 11
```
gag gtg cag ctg gtg gag act ggg gga ggc gtg gtc cag cct ggg agg       48
Glu Val Gln Leu Val Glu Thr Gly Gly Gly Val Val Gln Pro Gly Arg
1               5                   10                  15

tcc ctg aga ctc tcc tgt gca gcc tct gga ttc agc ttc agt acc cat       96
Ser Leu Arg Leu Ser Cys Ala Ala Ser Gly Phe Ser Phe Ser Thr His
            20                  25                  30

ggc atg tac tgg gtc cgc cag cct cca ggc aag ggg ctg gag tgg gtg      144
Gly Met Tyr Trp Val Arg Gln Pro Pro Gly Lys Gly Leu Glu Trp Val
        35                  40                  45

gca gtt ata tca tat gat gga agt gat aaa aag tat gca gac tcc gtg      192
Ala Val Ile Ser Tyr Asp Gly Ser Asp Lys Lys Tyr Ala Asp Ser Val
    50                  55                  60

aag ggc cgg ttc acc atc tcc aga gac aat tcc aag aac acg gtg tat      240
Lys Gly Arg Phe Thr Ile Ser Arg Asp Asn Ser Lys Asn Thr Val Tyr
65                  70                  75                  80

ttg gaa atg agc agc gtg aga gct gag gac acg gct cta tat tac tgt      288
Leu Glu Met Ser Ser Val Arg Ala Glu Asp Thr Ala Leu Tyr Tyr Cys
                85                  90                  95

ttc tgc cgc cgg gat gct ttt gat ctc tgg ggc caa ggg aca atg gtc      336
Phe Cys Arg Arg Asp Ala Phe Asp Leu Trp Gly Gln Gly Thr Met Val
            100                 105                 110

acc gtc tct tcg                                                      348
Thr Val Ser Ser
        115
```

<210> 12
<211> 116

<212> PRT
<213> Homo sapiens

<400> 12

Glu Val Gln Leu Val Glu Thr Gly Gly Gly Val Val Gln Pro Gly Arg
1               5                   10                  15

Ser Leu Arg Leu Ser Cys Ala Ala Ser Gly Phe Ser Phe Ser Thr His
            20                  25                  30

Gly Met Tyr Trp Val Arg Gln Pro Pro Gly Lys Gly Leu Glu Trp Val
        35                  40                  45

Ala Val Ile Ser Tyr Asp Gly Ser Asp Lys Lys Tyr Ala Asp Ser Val
    50                  55                  60

Lys Gly Arg Phe Thr Ile Ser Arg Asp Asn Ser Lys Asn Thr Val Tyr
65                  70                  75                  80

Leu Glu Met Ser Ser Val Arg Ala Glu Asp Thr Ala Leu Tyr Tyr Cys
                85                  90                  95

Phe Cys Arg Arg Asp Ala Phe Asp Leu Trp Gly Gln Gly Thr Met Val
            100                 105                 110

Thr Val Ser Ser
        115

<210> 13
<211> 324
<212> DNA
<213> Homo sapiens

<220>
<221> CDS
<222> (1)..(324)
<223> NI-206.22F7 variable lambda light chain (VL) sequence (not PIMC)

<220>
<221> V_region
<222> (67)..(99)
<223> complementarity determining region (CDR) VL-CDR1

<220>
<221> V_region
<222> (145)..(165)
<223> complementarity determining region (CDR) VL-CDR2

<220>
<221> V_region
<222> (262)..(294)
<223> complementarity determining region (CDR) VL-CDR3

<400> 13
```
tcc tat gtg ctg act cag cca ccc tcg gtg tca gtg tcc cca gga caa        48
Ser Tyr Val Leu Thr Gln Pro Pro Ser Val Ser Val Ser Pro Gly Gln
1               5                   10                  15

acg gcc agg atc acc tgc tct gga gat gca ttg cca aaa aag tat gct        96
Thr Ala Arg Ile Thr Cys Ser Gly Asp Ala Leu Pro Lys Lys Tyr Ala
            20                  25                  30

tat tgg tac cag cag aag tca ggc cag gcc cct gtg ctg gtc atc tat       144
Tyr Trp Tyr Gln Gln Lys Ser Gly Gln Ala Pro Val Leu Val Ile Tyr
        35                  40                  45

gag gac acc aaa cga ccc tcc ggg atc cct gag aga ttc tct ggc tcc       192
Glu Asp Thr Lys Arg Pro Ser Gly Ile Pro Glu Arg Phe Ser Gly Ser
    50                  55                  60

agc tca ggg aca atg gcc acc ttg act atc agt ggg gcc cag gtg gag       240
Ser Ser Gly Thr Met Ala Thr Leu Thr Ile Ser Gly Ala Gln Val Glu
65                  70                  75                  80

gat gaa gct gac tat tac tgt tac tca aca gac agc agc ggt aat tat       288
Asp Glu Ala Asp Tyr Tyr Cys Tyr Ser Thr Asp Ser Ser Gly Asn Tyr
                85                  90                  95

tgg gta ttc ggc gga ggg acc gag gtg acc gtc cta                       324
Trp Val Phe Gly Gly Gly Thr Glu Val Thr Val Leu
            100                 105
```

<210> 14
<211> 108
<212> PRT
<213> Homo sapiens

<400> 14

```
Ser Tyr Val Leu Thr Gln Pro Pro Ser Val Ser Val Ser Pro Gly Gln
1               5                   10                  15

Thr Ala Arg Ile Thr Cys Ser Gly Asp Ala Leu Pro Lys Lys Tyr Ala
            20                  25                  30

Tyr Trp Tyr Gln Gln Lys Ser Gly Gln Ala Pro Val Leu Val Ile Tyr
        35                  40                  45

Glu Asp Thr Lys Arg Pro Ser Gly Ile Pro Glu Arg Phe Ser Gly Ser
    50                  55                  60

Ser Ser Gly Thr Met Ala Thr Leu Thr Ile Ser Gly Ala Gln Val Glu
65                  70                  75                  80

Asp Glu Ala Asp Tyr Tyr Cys Tyr Ser Thr Asp Ser Ser Gly Asn Tyr
                85                  90                  95

Trp Val Phe Gly Gly Gly Thr Glu Val Thr Val Leu
            100                 105
```

<210> 15
<211> 372
<212> DNA
<213> Homo sapiens

<220>
<221> CDS
<222> (1)..(372)
<223> NI-206.27E8 variable heavy chain (VH) sequence (PIMC by default)

<220>
<221> V_region
<222> (91)..(105)
<223> complementarity determining region (CDR) VH-CDR1

<220>
<221> V_region
<222> (148)..(204)
<223> complementarity determining region (CDR) VH-CDR2

<220>
<221> V_region
<222> (304)..(339)
<223> complementarity determining region (CDR) VH-CDR3

<400> 15
```
gag gtg cag ctg gtg gag tct ggg gga ggc ttg gtt gag cct ggg ggg       48
Glu Val Gln Leu Val Glu Ser Gly Gly Gly Leu Val Glu Pro Gly Gly
1               5                   10                  15

tcc cta aga ctc tcc tgt gca gcc tct ggt ttc act ttc agt gat gcc       96
Ser Leu Arg Leu Ser Cys Ala Ala Ser Gly Phe Thr Phe Ser Asp Ala
            20                  25                  30

tgg atg aac tgg gtc cgc cag gct cca ggg aag ggg ctg gag tgg gtc      144
Trp Met Asn Trp Val Arg Gln Ala Pro Gly Lys Gly Leu Glu Trp Val
        35                  40                  45

ggg cgt att aaa acg aaa agc gat ggt ggg aca aca gac tac gct gca      192
Gly Arg Ile Lys Thr Lys Ser Asp Gly Gly Thr Thr Asp Tyr Ala Ala
    50                  55                  60

ccc gtg aga ggc aga ttt tcc atc tca aga gat gat tca aaa aac aca      240
Pro Val Arg Gly Arg Phe Ser Ile Ser Arg Asp Asp Ser Lys Asn Thr
65                  70                  75                  80

ctg ttt ctg gaa atg aac agc ctg aag acc gag gac aca gcc ata tat      288
Leu Phe Leu Glu Met Asn Ser Leu Lys Thr Glu Asp Thr Ala Ile Tyr
                85                  90                  95

tat tgt ttt att act gtc ata gta gta tcc tcc gaa tct cca ctt gac      336
Tyr Cys Phe Ile Thr Val Ile Val Val Ser Ser Glu Ser Pro Leu Asp
            100                 105                 110

cac tgg ggc cag gga acc ctg gtc acc gtc tcc tcg                      372
His Trp Gly Gln Gly Thr Leu Val Thr Val Ser Ser
        115                 120
```

<210> 16

<211> 124
<212> PRT
<213> Homo sapiens

<400> 16

Glu Val Gln Leu Val Glu Ser Gly Gly Gly Leu Val Glu Pro Gly Gly
1 5 10 15

Ser Leu Arg Leu Ser Cys Ala Ala Ser Gly Phe Thr Phe Ser Asp Ala
20 25 30

Trp Met Asn Trp Val Arg Gln Ala Pro Gly Lys Gly Leu Glu Trp Val
35 40 45

Gly Arg Ile Lys Thr Lys Ser Asp Gly Gly Thr Thr Asp Tyr Ala Ala
50 55 60

Pro Val Arg Gly Arg Phe Ser Ile Ser Arg Asp Asp Ser Lys Asn Thr
65 70 75 80

Leu Phe Leu Glu Met Asn Ser Leu Lys Thr Glu Asp Thr Ala Ile Tyr
85 90 95

Tyr Cys Phe Ile Thr Val Ile Val Val Ser Ser Glu Ser Pro Leu Asp
100 105 110

His Trp Gly Gln Gly Thr Leu Val Thr Val Ser Ser
115 120

<210> 17
<211> 324
<212> DNA
<213> Homo sapiens

<220>
<221> CDS
<222> (1)..(324)
<223> NI-206.27E8 variable lambda light chain (VL) sequence (PIMC by default)

<220>
<221> V_region
<222> (67)..(99)
<223> complementarity determining region (CDR) VL-CDR1

<220>
<221> V_region
<222> (145)..(165)
<223> complementarity determining region (CDR) VL-CDR2

<220>
<221> V_region
<222> (262)..(294)

<223>  complementarity determining region (CDR) VL-CDR3

<400>  17
```
tcc tat gag ctg act cag cca ccc tcg gtg tca gtg tcc cca gga cag          48
Ser Tyr Glu Leu Thr Gln Pro Pro Ser Val Ser Val Ser Pro Gly Gln
1               5                   10                  15

acg gcc agg atc acc tgc tct gga gac gaa ctg cca aaa caa tat gct          96
Thr Ala Arg Ile Thr Cys Ser Gly Asp Glu Leu Pro Lys Gln Tyr Ala
            20                  25                  30

tat tgg tac cag cag aag cca ggc cag gcc cct gtg ttg gtg ata tat         144
Tyr Trp Tyr Gln Gln Lys Pro Gly Gln Ala Pro Val Leu Val Ile Tyr
        35                  40                  45

aag gac aga cag agg ccc tca ggg atc cct gag cga ttc tct ggc tcc         192
Lys Asp Arg Gln Arg Pro Ser Gly Ile Pro Glu Arg Phe Ser Gly Ser
    50                  55                  60

agc tca ggg aca aca gtc acg ttg acc atc agt gga gtc cag gca gaa         240
Ser Ser Gly Thr Thr Val Thr Leu Thr Ile Ser Gly Val Gln Ala Glu
65                  70                  75                  80

gac gag gct gac tat tac tgt caa tca gca tac agc att aat act tat         288
Asp Glu Ala Asp Tyr Tyr Cys Gln Ser Ala Tyr Ser Ile Asn Thr Tyr
                85                  90                  95

gtg att ttc ggc gga ggg acc aag ctg acc gtc cta                         324
Val Ile Phe Gly Gly Gly Thr Lys Leu Thr Val Leu
            100                 105
```

<210>  18
<211>  108
<212>  PRT
<213>  Homo sapiens

<400>  18

```
Ser Tyr Glu Leu Thr Gln Pro Pro Ser Val Ser Val Ser Pro Gly Gln
1               5                   10                  15


Thr Ala Arg Ile Thr Cys Ser Gly Asp Glu Leu Pro Lys Gln Tyr Ala
            20                  25                  30


Tyr Trp Tyr Gln Gln Lys Pro Gly Gln Ala Pro Val Leu Val Ile Tyr
        35                  40                  45


Lys Asp Arg Gln Arg Pro Ser Gly Ile Pro Glu Arg Phe Ser Gly Ser
    50                  55                  60


Ser Ser Gly Thr Thr Val Thr Leu Thr Ile Ser Gly Val Gln Ala Glu
65                  70                  75                  80


Asp Glu Ala Asp Tyr Tyr Cys Gln Ser Ala Tyr Ser Ile Asn Thr Tyr
                85                  90                  95
```

```
Val Ile Phe Gly Gly Gly Thr Lys Leu Thr Val Leu
            100                 105


<210>  19
<211>  375
<212>  DNA
<213>  Homo sapiens


<220>
<221>  CDS
<222>  (1)..(375)
<223>  NI-206.12G4 variable heavy chain (VH) sequence (not PIMC)

<220>
<221>  V_region
<222>  (91)..(105)
<223>  complementarity determining region (CDR) VH-CDR1

<220>
<221>  V_region
<222>  (148)..(198)
<223>  complementarity determining region (CDR) VH-CDR2

<220>
<221>  V_region
<222>  (295)..(342)
<223>  complementarity determining region (CDR) VH-CDR3

<400>  19
gag gtg cag ctg gtg gag tct ggg gga ggc ttg gtc aag cct gga ggg       48
Glu Val Gln Leu Val Glu Ser Gly Gly Gly Leu Val Lys Pro Gly Gly
1               5                   10                  15

tcc ctg aga ctc tcc tgt gca gcc tct gga ttc acc ttc agt gac tac       96
Ser Leu Arg Leu Ser Cys Ala Ala Ser Gly Phe Thr Phe Ser Asp Tyr
            20                  25                  30

tac atg agc tgg atc cgc cag gct cca ggg aag ggg ctg gaa tgg att      144
Tyr Met Ser Trp Ile Arg Gln Ala Pro Gly Lys Gly Leu Glu Trp Ile
        35                  40                  45

tct tat att agt agt ggt agt agt tac aca aac tat gca gac tct gtg      192
Ser Tyr Ile Ser Ser Gly Ser Ser Tyr Thr Asn Tyr Ala Asp Ser Val
    50                  55                  60

aag ggc cga ttc acc atc tcc aga gac aac gcc aag aag tca gtg tat      240
Lys Gly Arg Phe Thr Ile Ser Arg Asp Asn Ala Lys Lys Ser Val Tyr
65                  70                  75                  80

ctg gaa gtc aac ggc ctg aca gtc gag gac acg gct gtg tat tac tgt      288
Leu Glu Val Asn Gly Leu Thr Val Glu Asp Thr Ala Val Tyr Tyr Cys
                85                  90                  95

gcg aga gtt cga tat ggg gac cgg gag atg gca aca atc gga gga ttt      336
Ala Arg Val Arg Tyr Gly Asp Arg Glu Met Ala Thr Ile Gly Gly Phe
            100                 105                 110

gat ttc tgg ggc cag gga acc ctg gtc acc gtc tcc tcg                  375
Asp Phe Trp Gly Gln Gly Thr Leu Val Thr Val Ser Ser
        115                 120                 125
```

<210> 20
<211> 125
<212> PRT
<213> Homo sapiens

<400> 20

Glu Val Gln Leu Val Glu Ser Gly Gly Gly Leu Val Lys Pro Gly Gly
1 5 10 15

Ser Leu Arg Leu Ser Cys Ala Ala Ser Gly Phe Thr Phe Ser Asp Tyr
20 25 30

Tyr Met Ser Trp Ile Arg Gln Ala Pro Gly Lys Gly Leu Glu Trp Ile
35 40 45

Ser Tyr Ile Ser Ser Gly Ser Ser Tyr Thr Asn Tyr Ala Asp Ser Val
50 55 60

Lys Gly Arg Phe Thr Ile Ser Arg Asp Asn Ala Lys Lys Ser Val Tyr
65 70 75 80

Leu Glu Val Asn Gly Leu Thr Val Glu Asp Thr Ala Val Tyr Tyr Cys
85 90 95

Ala Arg Val Arg Tyr Gly Asp Arg Glu Met Ala Thr Ile Gly Gly Phe
100 105 110

Asp Phe Trp Gly Gln Gly Thr Leu Val Thr Val Ser Ser
115 120 125

<210> 21
<211> 324
<212> DNA
<213> Homo sapiens

<220>
<221> CDS
<222> (1)..(324)
<223> NI-206.12G4 variable lambda light chain (VL) sequence (PIMC by default)

<220>
<221> V_region
<222> (67)..(99)
<223> complementarity determining region (CDR) VL-CDR1

<220>
<221> V_region
<222> (145)..(165)
<223> complementarity determining region (CDR) VL-CDR2

<220>

```
<221>  V_region
<222>  (262)..(294)
<223>  complementarity determining region (CDR) VL-CDR3

<400>  21
tcc tat gag ctg act cag cca ccc tcg gtg tca gtg tcc cca gga cag       48
Ser Tyr Glu Leu Thr Gln Pro Pro Ser Val Ser Val Ser Pro Gly Gln
1               5                   10                  15

acg gcc agg atc acc tgc tct gga gat gca ctg cca aag caa tat gct       96
Thr Ala Arg Ile Thr Cys Ser Gly Asp Ala Leu Pro Lys Gln Tyr Ala
            20                  25                  30

tat tgg tat caa cag agc cca ggc cag gcc cct gtg ttg gtg ata tat      144
Tyr Trp Tyr Gln Gln Ser Pro Gly Gln Ala Pro Val Leu Val Ile Tyr
        35                  40                  45

aaa gac agt gag agg ccc tca ggg atc cct gag cga ttc tct ggc tcc      192
Lys Asp Ser Glu Arg Pro Ser Gly Ile Pro Glu Arg Phe Ser Gly Ser
    50                  55                  60

agc tca ggg aca aca gtc acg ttg acc atc agt gga gtc cag gca gaa      240
Ser Ser Gly Thr Thr Val Thr Leu Thr Ile Ser Gly Val Gln Ala Glu
65                  70                  75                  80

gac gag gct gac tat tac tgt caa tca gca gac agc ggt ggt act tct      288
Asp Glu Ala Asp Tyr Tyr Cys Gln Ser Ala Asp Ser Gly Gly Thr Ser
                85                  90                  95

agg ata ttc ggc gga ggg acc aag ttg acc gtc ctg                      324
Arg Ile Phe Gly Gly Gly Thr Lys Leu Thr Val Leu
            100                 105


<210>  22
<211>  108
<212>  PRT
<213>  Homo sapiens

<400>  22

Ser Tyr Glu Leu Thr Gln Pro Pro Ser Val Ser Val Ser Pro Gly Gln
1               5                   10                  15


Thr Ala Arg Ile Thr Cys Ser Gly Asp Ala Leu Pro Lys Gln Tyr Ala
            20                  25                  30


Tyr Trp Tyr Gln Gln Ser Pro Gly Gln Ala Pro Val Leu Val Ile Tyr
        35                  40                  45


Lys Asp Ser Glu Arg Pro Ser Gly Ile Pro Glu Arg Phe Ser Gly Ser
    50                  55                  60


Ser Ser Gly Thr Thr Val Thr Leu Thr Ile Ser Gly Val Gln Ala Glu
65                  70                  75                  80


Asp Glu Ala Asp Tyr Tyr Cys Gln Ser Ala Asp Ser Gly Gly Thr Ser
                85                  90                  95
```

```
Arg Ile Phe Gly Gly Gly Thr Lys Leu Thr Val Leu
            100                 105


<210>  23
<211>  372
<212>  DNA
<213>  Homo sapiens


<220>
<221>  CDS
<222>  (1)..(372)
<223>  NI-206.17A6 variable heavy chain (VH) sequence (PIMC by default)

<220>
<221>  V_region
<222>  (91)..(105)
<223>  complementarity determining region (CDR) VH-CDR1

<220>
<221>  V_region
<222>  (148)..(195)
<223>  complementarity determining region (CDR) VH-CDR2

<220>
<221>  V_region
<222>  (292)..(339)
<223>  complementarity determining region (CDR) VH-CDR3

<400>  23
cag gtg cag ctg cag gag tcg ggc cca gga ctg gtg agg tct acg gag        48
Gln Val Gln Leu Gln Glu Ser Gly Pro Gly Leu Val Arg Ser Thr Glu
1               5                   10                  15

acc ctg tcc ctc acc tgc ctt gtc tct ggt gac tcc atc aac agt cac        96
Thr Leu Ser Leu Thr Cys Leu Val Ser Gly Asp Ser Ile Asn Ser His
            20                  25                  30

tac tgg agt tgg ctc cgg cag tcc cca ggg agg ggc ctg gaa tgg att       144
Tyr Trp Ser Trp Leu Arg Gln Ser Pro Gly Arg Gly Leu Glu Trp Ile
        35                  40                  45

ggg tac att tac tac act ggg ccc acc aac tac aat ccc tcc ctc aag       192
Gly Tyr Ile Tyr Tyr Thr Gly Pro Thr Asn Tyr Asn Pro Ser Leu Lys
    50                  55                  60

agt cga gtc tcc ata tca ctg ggc acg tcc aag gac cag ttc tcc ctg       240
Ser Arg Val Ser Ile Ser Leu Gly Thr Ser Lys Asp Gln Phe Ser Leu
65                  70                  75                  80

aag ctg agt tct gtg acc gct gcg gac acg gcc aga tat tac tgt gcg       288
Lys Leu Ser Ser Val Thr Ala Ala Asp Thr Ala Arg Tyr Tyr Cys Ala
                85                  90                  95

aga aat aag gtc ttt tgg cgt ggt tct gac ttc tac tac tac atg gac       336
Arg Asn Lys Val Phe Trp Arg Gly Ser Asp Phe Tyr Tyr Tyr Met Asp
            100                 105                 110

gtc tgg ggc aaa ggg acc acg gtc acc gtc tcc tcg                       372
Val Trp Gly Lys Gly Thr Thr Val Thr Val Ser Ser
```

115 120

<210> 24
<211> 124
<212> PRT
<213> Homo sapiens

<400> 24

Gln Val Gln Leu Gln Glu Ser Gly Pro Gly Leu Val Arg Ser Thr Glu
1 5 10 15

Thr Leu Ser Leu Thr Cys Leu Val Ser Gly Asp Ser Ile Asn Ser His
20 25 30

Tyr Trp Ser Trp Leu Arg Gln Ser Pro Gly Arg Gly Leu Glu Trp Ile
35 40 45

Gly Tyr Ile Tyr Tyr Thr Gly Pro Thr Asn Tyr Asn Pro Ser Leu Lys
50 55 60

Ser Arg Val Ser Ile Ser Leu Gly Thr Ser Lys Asp Gln Phe Ser Leu
65 70 75 80

Lys Leu Ser Ser Val Thr Ala Ala Asp Thr Ala Arg Tyr Tyr Cys Ala
85 90 95

Arg Asn Lys Val Phe Trp Arg Gly Ser Asp Phe Tyr Tyr Tyr Met Asp
100 105 110

Val Trp Gly Lys Gly Thr Thr Val Thr Val Ser Ser
115 120

<210> 25
<211> 330
<212> DNA
<213> Homo sapiens

<220>
<221> CDS
<222> (1)..(330)
<223> NI-206.17A6 variable kappa light chain (VK) sequence (not PIMC)

<220>
<221> V_region
<222> (70)..(105)
<223> complementarity determining region (CDR) VK-CDR1

<220>
<221> V_region
<222> (151)..(171)
<223> complementarity determining region (CDR) VK-CDR2

```
<220>
<221>  V_region
<222>  (268)..(300)
<223>  complementarity determining region (CDR) VK-CDR3

<400>  25
gaa att gtg ttg aca cag tct cca ggc acc ctg tct ttg tct cta ggg        48
Glu Ile Val Leu Thr Gln Ser Pro Gly Thr Leu Ser Leu Ser Leu Gly
1               5                   10                  15

gaa aga gcc acc ctc tcc tgc agg gcc agt cag agt ctt gcc aac aac        96
Glu Arg Ala Thr Leu Ser Cys Arg Ala Ser Gln Ser Leu Ala Asn Asn
            20                  25                  30

tac tta gcc tgg tac cag cag aaa cct ggc cag gct ccc agg ctc ctc       144
Tyr Leu Ala Trp Tyr Gln Gln Lys Pro Gly Gln Ala Pro Arg Leu Leu
        35                  40                  45

atg tat gac gca tcc acc agg gcc act ggc atc cct gac agg ttc agt       192
Met Tyr Asp Ala Ser Thr Arg Ala Thr Gly Ile Pro Asp Arg Phe Ser
    50                  55                  60

ggc agt ggg tct ggg aca gac ttc act ctc acc atc agc aga ctg gag       240
Gly Ser Gly Ser Gly Thr Asp Phe Thr Leu Thr Ile Ser Arg Leu Glu
65                  70                  75                  80

cct gaa gat ttt gca gtg tat tac tgc cag caa ttt gtt acc tca cac       288
Pro Glu Asp Phe Ala Val Tyr Tyr Cys Gln Gln Phe Val Thr Ser His
                85                  90                  95

cac atg tac att ttt ggc cag ggg acc aag gtg gaa atc aaa               330
His Met Tyr Ile Phe Gly Gln Gly Thr Lys Val Glu Ile Lys
            100                 105                 110


<210>  26
<211>  110
<212>  PRT
<213>  Homo sapiens

<400>  26

Glu Ile Val Leu Thr Gln Ser Pro Gly Thr Leu Ser Leu Ser Leu Gly
1               5                   10                  15


Glu Arg Ala Thr Leu Ser Cys Arg Ala Ser Gln Ser Leu Ala Asn Asn
            20                  25                  30


Tyr Leu Ala Trp Tyr Gln Gln Lys Pro Gly Gln Ala Pro Arg Leu Leu
        35                  40                  45


Met Tyr Asp Ala Ser Thr Arg Ala Thr Gly Ile Pro Asp Arg Phe Ser
    50                  55                  60


Gly Ser Gly Ser Gly Thr Asp Phe Thr Leu Thr Ile Ser Arg Leu Glu
65                  70                  75                  80


Pro Glu Asp Phe Ala Val Tyr Tyr Cys Gln Gln Phe Val Thr Ser His
```

85 90 95

His Met Tyr Ile Phe Gly Gln Gly Thr Lys Val Glu Ile Lys
100 105 110

<210> 27
<211> 15
<212> PRT
<213> Artificial Sequence

<220>
<223> FAP-specific peptide in ELISA; amino acids 378 to 392

<400> 27

His Tyr Ile Lys Asp Thr Val Glu Asn Ala Ile Gln Ile Thr Ser
1 5 10 15

<210> 28
<211> 15
<212> PRT
<213> Artificial Sequence

<220>
<223> FAP-specific peptide in ELISA; amino acids 622 to 636

<400> 28

Gly Trp Ser Tyr Gly Gly Tyr Val Ser Ser Leu Ala Leu Ala Ser
1 5 10 15

<210> 29
<211> 16
<212> PRT
<213> Artificial Sequence

<220>
<223> FAP-specific peptide in ELISA; amino acids 721 to 736

<400> 29

Gln Val Asp Phe Gln Ala Met Trp Tyr Ser Asp Gln Asn His Gly Leu
1 5 10 15

<210> 30
<211> 15
<212> PRT
<213> Artificial Sequence

<220>
<223> Peptide 131 (amino acids 521 to 535) of pepscan image of NI-206.82C2

<400> 30

Lys Met Ile Leu Pro Pro Gln Phe Asp Arg Ser Lys Lys Tyr Pro
1 5 10 15

<210> 31
<211> 15
<212> PRT
<213> Artificial Sequence

<220>
<223> Peptide 132 (amino acids 525 to 539) in pepscan image of NI-206.82.C2

<400> 31

Pro Pro Gln Phe Asp Arg Ser Lys Lys Tyr Pro Leu Leu Ile Gln
1 5 10 15

<210> 32
<211> 11
<212> PRT
<213> Artificial Sequence

<220>
<223> Epitope of NI-206.82C2 antibody

<400> 32

Pro Pro Gln Phe Asp Arg Ser Lys Lys Tyr Pro
1 5 10

<210> 33
<211> 7
<212> PRT
<213> Artificial Sequence

<220>
<223> Epitope of NI-206.59B4 antibody

<400> 33

Ser Tyr Lys Thr Phe Phe Pro
1 5

<210> 34
<211> 11
<212> PRT
<213> Artificial Sequence

<220>
<223> Epitope of NI-206.22F7 antibody

<400> 34

Lys Asp Thr Val Glu Asn Ala Ile Gln Ile Thr
1 5 10

<210> 35
<211> 7
<212> PRT
<213> Artificial Sequence

<220>
<223> Epitope of NI-206.27E8 antibody

<400> 35

Asn Ile Tyr Leu Lys Gln Arg
1 5

<210> 36
<211> 15
<212> PRT
<213> Artificial Sequence

<220>
<223> Epitope of NI-206.12G4 antibody

<400> 36

Thr Asp Gln Glu Ile Lys Ile Leu Glu Glu Asn Lys Glu Leu Glu
1 5 10 15

<210> 37
<211> 11
<212> PRT
<213> Artificial Sequence

<220>
<223> Epitope of NI-206.17A6 antibody

<400> 37

Val Leu Tyr Asn Ile Glu Thr Gly Gln Ser Tyr
1 5 10

<210> 38
<211> 19
<212> PRT
<213> Artificial Sequence

<220>
<223> FAP fragment comprising epitope of antibody NI-206.82C2

<400> 38

Lys Met Ile Leu Pro Pro Gln Phe Asp Arg Ser Lys Lys Tyr Pro Leu
1 5 10 15

Leu Ile Gln

<210> 39
<211> 5
<212> PRT
<213> Artificial Sequence

<220>
<223> Minimum epitope region of FAP fragment comprising amino acids 521 to 539 recognized by antibody NI-206.82C2

<400> 39

Phe Asp Arg Ser Lys
1 5

<210> 40
<211> 363
<212> DNA
<213> Homo sapiens

<220>
<221> CDS
<222> (1)..(363)
<223> NI-206.18H2 variable heavy chain (VH) sequence (not PIMC)

<220>
<221> V_region
<222> (91)..(108)
<223> complementarity determining region (CDR) VH-CDR1

<220>
<221> V_region
<222> (151)..(198)
<223> complementarity determining region (CDR) VH-CDR2

<220>
<221> V_region
<222> (295)..(330)
<223> complementarity determining region (CDR) VH-CDR3

<400> 40
cag gtg cag ctg cag gag tcg ggc cca cga ctg gtg aag cct tcg gag 48
Gln Val Gln Leu Gln Glu Ser Gly Pro Arg Leu Val Lys Pro Ser Glu
1 5 10 15

acc ctg tct ctc acc tgc act gtc tct ggt tac tcc att agt aat ggt 96
Thr Leu Ser Leu Thr Cys Thr Val Ser Gly Tyr Ser Ile Ser Asn Gly
20 25 30

tac tac tgg ggc tgg atc cga cag ccc cca ggg cag ggg ctg gag tgg 144
Tyr Tyr Trp Gly Trp Ile Arg Gln Pro Pro Gly Gln Gly Leu Glu Trp
35 40 45

att gcg agt gtc tgg cat agt ggg aac acc tac cac aac ccg tcc ctc 192
Ile Ala Ser Val Trp His Ser Gly Asn Thr Tyr His Asn Pro Ser Leu
50 55 60

aaa agt cga gtc acc att tta gtg gac acg ttg aag aac caa ttt tcc 240
Lys Ser Arg Val Thr Ile Leu Val Asp Thr Leu Lys Asn Gln Phe Ser
65 70 75 80

ctg aac ctg aag tct gtg acc gcc gca gac acg gcc tta tat tac tgt 288
Leu Asn Leu Lys Ser Val Thr Ala Ala Asp Thr Ala Leu Tyr Tyr Cys
85 90 95

gcg aga tca tat aca cga aac gac gtg ggc gct ttt gag acg tgg ggc 336
Ala Arg Ser Tyr Thr Arg Asn Asp Val Gly Ala Phe Glu Thr Trp Gly
100 105 110

caa ggg aca atg gtc acc gtc tct tcg 363

Gln Gly Thr Met Val Thr Val Ser Ser
115 120

<210> 41
<211> 121
<212> PRT
<213> Homo sapiens

<400> 41

Gln Val Gln Leu Gln Glu Ser Gly Pro Arg Leu Val Lys Pro Ser Glu
1 5 10 15

Thr Leu Ser Leu Thr Cys Thr Val Ser Gly Tyr Ser Ile Ser Asn Gly
20 25 30

Tyr Tyr Trp Gly Trp Ile Arg Gln Pro Pro Gly Gln Gly Leu Glu Trp
35 40 45

Ile Ala Ser Val Trp His Ser Gly Asn Thr Tyr His Asn Pro Ser Leu
50 55 60

Lys Ser Arg Val Thr Ile Leu Val Asp Thr Leu Lys Asn Gln Phe Ser
65 70 75 80

Leu Asn Leu Lys Ser Val Thr Ala Ala Asp Thr Ala Leu Tyr Tyr Cys
85 90 95

Ala Arg Ser Tyr Thr Arg Asn Asp Val Gly Ala Phe Glu Thr Trp Gly
100 105 110

Gln Gly Thr Met Val Thr Val Ser Ser
115 120

<210> 42
<211> 327
<212> DNA
<213> Homo sapiens

<220>
<221> CDS
<222> (1)..(327)
<223> NI-206.18H2 variable lambda light chain (VL) sequence (PIMC by default)

<220>
<221> V_region
<222> (67)..(99)
<223> complementarity determining region (CDR) VL-CDR1

<220>
<221> V_region
<222> (145)..(165)

<223> complementarity determining region (CDR) VL-CDR2

<220>
<221> V_region
<222> (262)..(297)
<223> complementarity determining region (CDR) VL-CDR3

<400> 42
```
tcc tat gag ctg act cag cca ccc tcg gtg tct gtg tcc cca gga caa      48
Ser Tyr Glu Leu Thr Gln Pro Pro Ser Val Ser Val Ser Pro Gly Gln
1               5                   10                  15

acg gcc agg atc gcc tgc tct gga gat gaa ttg cca aaa aga tat gct      96
Thr Ala Arg Ile Ala Cys Ser Gly Asp Glu Leu Pro Lys Arg Tyr Ala
            20                  25                  30

tat tgg tac cag cag aag tca ggc cag gcc cct gta ctg gtc atg tat      144
Tyr Trp Tyr Gln Gln Lys Ser Gly Gln Ala Pro Val Leu Val Met Tyr
        35                  40                  45

gag gac acc aag cga ccc tcc ggg att cct gag aga ttg tct ggc tcc      192
Glu Asp Thr Lys Arg Pro Ser Gly Ile Pro Glu Arg Leu Ser Gly Ser
    50                  55                  60

tcc tca ggg aca gtg acc act ctg act att agt ggg gcc cag gtg gag      240
Ser Ser Gly Thr Val Thr Thr Leu Thr Ile Ser Gly Ala Gln Val Glu
65                  70                  75                  80

gat gag gct gac tac tac tgt tac tca aca gcc ccc agt ggc aat cac      288
Asp Glu Ala Asp Tyr Tyr Cys Tyr Ser Thr Ala Pro Ser Gly Asn His
                85                  90                  95

act ttt gtc ttc gga act ggg acc agg gtc acc gtc ctt                  327
Thr Phe Val Phe Gly Thr Gly Thr Arg Val Thr Val Leu
            100                 105
```

<210> 43
<211> 109
<212> PRT
<213> Homo sapiens

<400> 43
```
Ser Tyr Glu Leu Thr Gln Pro Pro Ser Val Ser Val Ser Pro Gly Gln
1               5                   10                  15

Thr Ala Arg Ile Ala Cys Ser Gly Asp Glu Leu Pro Lys Arg Tyr Ala
            20                  25                  30

Tyr Trp Tyr Gln Gln Lys Ser Gly Gln Ala Pro Val Leu Val Met Tyr
        35                  40                  45

Glu Asp Thr Lys Arg Pro Ser Gly Ile Pro Glu Arg Leu Ser Gly Ser
    50                  55                  60

Ser Ser Gly Thr Val Thr Thr Leu Thr Ile Ser Gly Ala Gln Val Glu
65                  70                  75                  80
```

Asp Glu Ala Asp Tyr Tyr Cys Tyr Ser Thr Ala Pro Ser Gly Asn His
85 90 95

Thr Phe Val Phe Gly Thr Gly Thr Arg Val Thr Val Leu
100 105

<210> 44
<211> 354
<212> DNA
<213> Homo sapiens

<220>
<221> CDS
<222> (1)..(354)
<223> NI-206.20A8 variable heavy chain (VH) sequence (PIMC by default)

<220>
<221> V_region
<222> (91)..(105)
<223> complementarity determining region (CDR) VH-CDR1

<220>
<221> V_region
<222> (148)..(204)
<223> complementarity determining region (CDR) VH-CDR2

<220>
<221> V_region
<222> (301)..(321)
<223> complementarity determining region (CDR) VH-CDR3

<400> 44
gag gtg cag ctg gtg gag tcc ggg gga ggc ttg gtc cag ccg ggg ggg 48
Glu Val Gln Leu Val Glu Ser Gly Gly Gly Leu Val Gln Pro Gly Gly
1 5 10 15

tcc ctc aaa ctc tcc tgt gca ggc tct ggc tta gac ctc agt gcc tct 96
Ser Leu Lys Leu Ser Cys Ala Gly Ser Gly Leu Asp Leu Ser Ala Ser
20 25 30

gct gtg cac tgg gtc cgc cag gcc tcc ggg aaa ggg ctg gag tgg att 144
Ala Val His Trp Val Arg Gln Ala Ser Gly Lys Gly Leu Glu Trp Ile
35 40 45

ggc cgc atc aga agc aag cct aat cac tat gcg aca aca tat ctg gcg 192
Gly Arg Ile Arg Ser Lys Pro Asn His Tyr Ala Thr Thr Tyr Leu Ala
50 55 60

tcg gtg aga ggc aga ttc atc ctc tcc aga gat gat tca gag aac acg 240
Ser Val Arg Gly Arg Phe Ile Leu Ser Arg Asp Asp Ser Glu Asn Thr
65 70 75 80

gcc tat ctc caa atg aac agc ctg aga acc gag gac aca gcc gta tat 288
Ala Tyr Leu Gln Met Asn Ser Leu Arg Thr Glu Asp Thr Ala Val Tyr
85 90 95

tac tgc aga att gga att ctg aat tct gac cac tgg ggc cgg gga acc 336
Tyr Cys Arg Ile Gly Ile Leu Asn Ser Asp His Trp Gly Arg Gly Thr
100 105 110

ctg gtc acc gtc tcc tcg 354
Leu Val Thr Val Ser Ser
115

<210> 45
<211> 118
<212> PRT
<213> Homo sapiens

<400> 45

Glu Val Gln Leu Val Glu Ser Gly Gly Gly Leu Val Gln Pro Gly Gly
1 5 10 15

Ser Leu Lys Leu Ser Cys Ala Gly Ser Gly Leu Asp Leu Ser Ala Ser
20 25 30

Ala Val His Trp Val Arg Gln Ala Ser Gly Lys Gly Leu Glu Trp Ile
35 40 45

Gly Arg Ile Arg Ser Lys Pro Asn His Tyr Ala Thr Thr Tyr Leu Ala
50 55 60

Ser Val Arg Gly Arg Phe Ile Leu Ser Arg Asp Asp Ser Glu Asn Thr
65 70 75 80

Ala Tyr Leu Gln Met Asn Ser Leu Arg Thr Glu Asp Thr Ala Val Tyr
85 90 95

Tyr Cys Arg Ile Gly Ile Leu Asn Ser Asp His Trp Gly Arg Gly Thr
100 105 110

Leu Val Thr Val Ser Ser
115

<210> 46
<211> 330
<212> DNA
<213> Homo sapiens

<220>
<221> CDS
<222> (1)..(330)
<223> NI-206-20A8 variable lambda light chain (VL) sequence (PIMC by default)

<220>
<221> V_region
<222> (67)..(105)
<223> complementarity determining region (CDR) VL-CDR1

<220>

```
<221>  V_region
<222>  (151)..(171)
<223>  complementarity determining region (CDR) VL-CDR2

<220>
<221>  V_region
<222>  (268)..(300)
<223>  complementarity determining region (CDR) VL-CDR3

<400>  46
cag tct gtg ctg act cag cca ccc tca gcg tct ggg acc ccc ggg cag         48
Gln Ser Val Leu Thr Gln Pro Pro Ser Ala Ser Gly Thr Pro Gly Gln
1               5                   10                  15

acg gtc atc atc tct tgc tct gga agc agc tcc aat ctc gga agg aaa         96
Thr Val Ile Ile Ser Cys Ser Gly Ser Ser Ser Asn Leu Gly Arg Lys
            20                  25                  30

act cta aac tgg tac cag caa ctc cca gga gcg gcc ccc aaa ctc ctc        144
Thr Leu Asn Trp Tyr Gln Gln Leu Pro Gly Ala Ala Pro Lys Leu Leu
        35                  40                  45

att ttt aaa aat gat cag cgg gcc tca ggg gtc cct gac cga ttc tct        192
Ile Phe Lys Asn Asp Gln Arg Ala Ser Gly Val Pro Asp Arg Phe Ser
    50                  55                  60

gcc tcc aag tct ggc acc tca gcc tcc ctg acc atc agt gga ctc cag        240
Ala Ser Lys Ser Gly Thr Ser Ala Ser Leu Thr Ile Ser Gly Leu Gln
65                  70                  75                  80

tct gac gat gag gct gat tat tac tgt gga aca tgg gat gac agc ctg        288
Ser Asp Asp Glu Ala Asp Tyr Tyr Cys Gly Thr Trp Asp Asp Ser Leu
                85                  90                  95

gat aat tgg gtg ttc ggc gga ggg acc aag gtg acc gtc cta                330
Asp Asn Trp Val Phe Gly Gly Gly Thr Lys Val Thr Val Leu
            100                 105                 110


<210>  47
<211>  110
<212>  PRT
<213>  Homo sapiens

<400>  47

Gln Ser Val Leu Thr Gln Pro Pro Ser Ala Ser Gly Thr Pro Gly Gln
1               5                   10                  15


Thr Val Ile Ile Ser Cys Ser Gly Ser Ser Ser Asn Leu Gly Arg Lys
            20                  25                  30


Thr Leu Asn Trp Tyr Gln Gln Leu Pro Gly Ala Ala Pro Lys Leu Leu
        35                  40                  45


Ile Phe Lys Asn Asp Gln Arg Ala Ser Gly Val Pro Asp Arg Phe Ser
    50                  55                  60


Ala Ser Lys Ser Gly Thr Ser Ala Ser Leu Thr Ile Ser Gly Leu Gln
```

```
65                  70                  75                  80

Ser Asp Asp Glu Ala Asp Tyr Tyr Cys Gly Thr Trp Asp Asp Ser Leu
                85                  90                  95

Asp Asn Trp Val Phe Gly Gly Gly Thr Lys Val Thr Val Leu
            100                 105                 110


<210>  48
<211>  384
<212>  DNA
<213>  Homo sapiens


<220>
<221>  CDS
<222>  (1)..(384)
<223>  NI-206.6D3 variable heavy chain (VH) sequence (PIMC by default)

<220>
<221>  V_region
<222>  (91)..(105)
<223>  complementarity determining region (CDR) VH-CDR1

<220>
<221>  V_region
<222>  (148)..(204)
<223>  complementarity determining region (CDR) VH-CDR2

<220>
<221>  V_region
<222>  (301)..(351)
<223>  complementarity determining region (CDR) VH-CDR3

<400>  48
gag gtg cag ctg gtg gag tct ggg gga ggc ttg gta aag cct ggg gag        48
Glu Val Gln Leu Val Glu Ser Gly Gly Gly Leu Val Lys Pro Gly Glu
1               5                   10                  15

tcc ctt aga ctc tcc tgt gca gcc tct gga ttc act ttc agt aag gcc        96
Ser Leu Arg Leu Ser Cys Ala Ala Ser Gly Phe Thr Phe Ser Lys Ala
            20                  25                  30

tgg atg aac tgg gtc cgc cag ggt cca ggg aag ggg ctg gag tgg gtt       144
Trp Met Asn Trp Val Arg Gln Gly Pro Gly Lys Gly Leu Glu Trp Val
        35                  40                  45

ggc cgt gtt aaa agc aaa act gat ggt ggg aca aca gac tac gct gca       192
Gly Arg Val Lys Ser Lys Thr Asp Gly Gly Thr Thr Asp Tyr Ala Ala
    50                  55                  60

ccc gtg aaa ggc aga ttc acc atc tca aga gac gat tca aaa gac aca       240
Pro Val Lys Gly Arg Phe Thr Ile Ser Arg Asp Asp Ser Lys Asp Thr
65                  70                  75                  80

gtg ttt ctg caa atg aac agc ctg aaa acc gaa gat aca gcc gta tat       288
Val Phe Leu Gln Met Asn Ser Leu Lys Thr Glu Asp Thr Ala Val Tyr
                85                  90                  95

tac tgt tcc atc cag ttt att gta gta ggt gat agg ggc cga gac gac       336
```

Tyr Cys Ser Ile Gln Phe Ile Val Val Gly Asp Arg Gly Arg Asp Asp
100 105 110

cag tac atg gac gtc tgg ggc aaa ggg acc acg gtc acc gtc tcc tcg 384
Gln Tyr Met Asp Val Trp Gly Lys Gly Thr Thr Val Thr Val Ser Ser
115 120 125

<210> 49
<211> 128
<212> PRT
<213> Homo sapiens

<400> 49

Glu Val Gln Leu Val Glu Ser Gly Gly Gly Leu Val Lys Pro Gly Glu
1 5 10 15

Ser Leu Arg Leu Ser Cys Ala Ala Ser Gly Phe Thr Phe Ser Lys Ala
20 25 30

Trp Met Asn Trp Val Arg Gln Gly Pro Gly Lys Gly Leu Glu Trp Val
35 40 45

Gly Arg Val Lys Ser Lys Thr Asp Gly Gly Thr Thr Asp Tyr Ala Ala
50 55 60

Pro Val Lys Gly Arg Phe Thr Ile Ser Arg Asp Asp Ser Lys Asp Thr
65 70 75 80

Val Phe Leu Gln Met Asn Ser Leu Lys Thr Glu Asp Thr Ala Val Tyr
85 90 95

Tyr Cys Ser Ile Gln Phe Ile Val Val Gly Asp Arg Gly Arg Asp Asp
100 105 110

Gln Tyr Met Asp Val Trp Gly Lys Gly Thr Thr Val Thr Val Ser Ser
115 120 125

<210> 50
<211> 324
<212> DNA
<213> Homo sapiens

<220>
<221> CDS
<222> (1)..(324)
<223> NI-206.6D3 variable lambda light chain (VL) sequence (not PIMC)

<220>
<221> V_region
<222> (67)..(99)
<223> complementarity determining region (CDR) VL-CDR1

<220>
<221> V_region
<222> (145)..(165)
<223> complementarity determining region (CDR) VL-CDR2

<220>
<221> V_region
<222> (262)..(294)
<223> complementarity determining region (CDR) VL-CDR3

<400> 50
tcc tat gtg ctg act cag cca ccc tcg gtg tca gtg tcc cca gga cag 48
Ser Tyr Val Leu Thr Gln Pro Pro Ser Val Ser Val Ser Pro Gly Gln
1 5 10 15

acg gcc agg atc acc tgc tct gga gat gca ttg cca aag caa tat gct 96
Thr Ala Arg Ile Thr Cys Ser Gly Asp Ala Leu Pro Lys Gln Tyr Ala
20 25 30

ttt tgg tac cag cag aag cca ggc cag gcc cct gta att atg ata tat 144
Phe Trp Tyr Gln Gln Lys Pro Gly Gln Ala Pro Val Ile Met Ile Tyr
35 40 45

aaa gac aat cag agg ccc tca ggg atc cct gag cga ttc tct ggc tcc 192
Lys Asp Asn Gln Arg Pro Ser Gly Ile Pro Glu Arg Phe Ser Gly Ser
50 55 60

agc tca ggg aca aca gtc acg ttg acc atc agt gga gtc cag aca gaa 240
Ser Ser Gly Thr Thr Val Thr Leu Thr Ile Ser Gly Val Gln Thr Glu
65 70 75 80

gac gag gct gac tat tac tgt caa tca gca gac agc act aat act cat 288
Asp Glu Ala Asp Tyr Tyr Cys Gln Ser Ala Asp Ser Thr Asn Thr His
85 90 95

gtg gta ttc ggc gga ggg acc aag ctg acc gtc cta 324
Val Val Phe Gly Gly Gly Thr Lys Leu Thr Val Leu
100 105

<210> 51
<211> 108
<212> PRT
<213> Homo sapiens

<400> 51

Ser Tyr Val Leu Thr Gln Pro Pro Ser Val Ser Val Ser Pro Gly Gln
1 5 10 15

Thr Ala Arg Ile Thr Cys Ser Gly Asp Ala Leu Pro Lys Gln Tyr Ala
20 25 30

Phe Trp Tyr Gln Gln Lys Pro Gly Gln Ala Pro Val Ile Met Ile Tyr
35 40 45

Lys Asp Asn Gln Arg Pro Ser Gly Ile Pro Glu Arg Phe Ser Gly Ser
50 55 60

```
Ser Ser Gly Thr Thr Val Thr Leu Thr Ile Ser Gly Val Gln Thr Glu
65                  70                  75                  80

Asp Glu Ala Asp Tyr Tyr Cys Gln Ser Ala Asp Ser Thr Asn Thr His
                85                  90                  95

Val Val Phe Gly Gly Gly Thr Lys Leu Thr Val Leu
            100                 105
```

<210> 52
<211> 369
<212> DNA
<213> Homo sapiens

<220>
<221> CDS
<222> (1)..(369)
<223> NI-206.14C5 variable heavy chain (VH) sequence (PIMC by default)

<220>
<221> V_region
<222> (91)..(105)
<223> complementarity determining region (CDR) VH-CDR1

<220>
<221> V_region
<222> (148)..(195)
<223> complementarity determining region (CDR) VH-CDR2

<220>
<221> V_region
<222> (292)..(336)
<223> complementarity determining region (CDR) VH-CDR3

<400> 52

```
cag gtg cag ctg cag gag tcg ggc cca gga ctg gtg aag cct tcg gag      48
Gln Val Gln Leu Gln Glu Ser Gly Pro Gly Leu Val Lys Pro Ser Glu
1               5                   10                  15

acc ctg tcc ctc act tgc act gtc tct aat ggc tcc atc aat aat aac      96
Thr Leu Ser Leu Thr Cys Thr Val Ser Asn Gly Ser Ile Asn Asn Asn
            20                  25                  30

tac tgg agc tgg ctc cgg cag ccc ccc ggg aag ggg ctg caa tgg att      144
Tyr Trp Ser Trp Leu Arg Gln Pro Pro Gly Lys Gly Leu Gln Trp Ile
        35                  40                  45

ggt tat gtc tat tac agt ggg agc gca aag tat aac ccc tcc ctc cag      192
Gly Tyr Val Tyr Tyr Ser Gly Ser Ala Lys Tyr Asn Pro Ser Leu Gln
    50                  55                  60

agt cga gtc tct ctt tca gta gac aga tcc aag aac caa ttc tcc ctg      240
Ser Arg Val Ser Leu Ser Val Asp Arg Ser Lys Asn Gln Phe Ser Leu
65                  70                  75                  80

gag ctg agc tct gtc acc gct gcg gac acg gcc gtc tat tac tgt gcg      288
Glu Leu Ser Ser Val Thr Ala Ala Asp Thr Ala Val Tyr Tyr Cys Ala
                85                  90                  95
```

```
agg acc tat tgt agt ggt cgt gac act tgt ttc tat ttc ttt gac aac        336
Arg Thr Tyr Cys Ser Gly Arg Asp Thr Cys Phe Tyr Phe Phe Asp Asn
            100                 105                 110

tgg ggc cag gga acc ctg gtc acc gtc tcc tcg                            369
Trp Gly Gln Gly Thr Leu Val Thr Val Ser Ser
        115                 120


<210>  53
<211>  123
<212>  PRT
<213>  Homo sapiens

<400>  53

Gln Val Gln Leu Gln Glu Ser Gly Pro Gly Leu Val Lys Pro Ser Glu
1               5                   10                  15


Thr Leu Ser Leu Thr Cys Thr Val Ser Asn Gly Ser Ile Asn Asn Asn
            20                  25                  30


Tyr Trp Ser Trp Leu Arg Gln Pro Pro Gly Lys Gly Leu Gln Trp Ile
        35                  40                  45


Gly Tyr Val Tyr Tyr Ser Gly Ser Ala Lys Tyr Asn Pro Ser Leu Gln
    50                  55                  60


Ser Arg Val Ser Leu Ser Val Asp Arg Ser Lys Asn Gln Phe Ser Leu
65                  70                  75                  80


Glu Leu Ser Ser Val Thr Ala Ala Asp Thr Ala Val Tyr Tyr Cys Ala
                85                  90                  95


Arg Thr Tyr Cys Ser Gly Arg Asp Thr Cys Phe Tyr Phe Phe Asp Asn
            100                 105                 110


Trp Gly Gln Gly Thr Leu Val Thr Val Ser Ser
        115                 120


<210>  54
<211>  330
<212>  DNA
<213>  Homo sapiens


<220>
<221>  CDS
<222>  (1)..(330)
<223>  NI-206.14C5 variable lambda light chain (VL) sequence (PIMC by
       default)

<220>
<221>  V_region
<222>  (67)..(108)
```

<223> complementarity determining region (CDR) VL-CDR1

<220>
<221> V_region
<222> (154)..(174)
<223> complementarity determining region (CDR) VL-CDR2

<220>
<221> V_region
<222> (271)..(300)
<223> complementarity determining region (CDR) VL-CDR3

<400> 54
```
cag tct gcc ctg act cag cct gcc tcc gtg tct ggg tct cct aga cag        48
Gln Ser Ala Leu Thr Gln Pro Ala Ser Val Ser Gly Ser Pro Arg Gln
1               5                   10                  15

tcg ctc acc atc tcc tgc act gga acc atg agt gat gtt ggg agg tat        96
Ser Leu Thr Ile Ser Cys Thr Gly Thr Met Ser Asp Val Gly Arg Tyr
            20                  25                  30

gac ctt gtc tca tgg tac caa caa cac cct ggc aaa gcc ccc aac gtc       144
Asp Leu Val Ser Trp Tyr Gln Gln His Pro Gly Lys Ala Pro Asn Val
        35                  40                  45

atc att tat gcc gtc act aag cgg ccc tca ggg gtt tct gat cgc ttc       192
Ile Ile Tyr Ala Val Thr Lys Arg Pro Ser Gly Val Ser Asp Arg Phe
    50                  55                  60

tct ggc tcc aag tct ggc acc acg gcc tcc ctg aca atc tct ggg ctc       240
Ser Gly Ser Lys Ser Gly Thr Thr Ala Ser Leu Thr Ile Ser Gly Leu
65                  70                  75                  80

cag gct gag gac gag gct tat tat tac tgc tgt tca tat gca act gtt       288
Gln Ala Glu Asp Glu Ala Tyr Tyr Tyr Cys Cys Ser Tyr Ala Thr Val
                85                  90                  95

aac agt tgg cta ttc ggc gga ggg acc aag gtg acc gtc cta               330
Asn Ser Trp Leu Phe Gly Gly Gly Thr Lys Val Thr Val Leu
            100                 105                 110
```

<210> 55
<211> 110
<212> PRT
<213> Homo sapiens

<400> 55
```
Gln Ser Ala Leu Thr Gln Pro Ala Ser Val Ser Gly Ser Pro Arg Gln
1               5                   10                  15

Ser Leu Thr Ile Ser Cys Thr Gly Thr Met Ser Asp Val Gly Arg Tyr
            20                  25                  30

Asp Leu Val Ser Trp Tyr Gln Gln His Pro Gly Lys Ala Pro Asn Val
        35                  40                  45

Ile Ile Tyr Ala Val Thr Lys Arg Pro Ser Gly Val Ser Asp Arg Phe
    50                  55                  60
```

```
Ser Gly Ser Lys Ser Gly Thr Thr Ala Ser Leu Thr Ile Ser Gly Leu
65                  70                  75                  80

Gln Ala Glu Asp Glu Ala Tyr Tyr Tyr Cys Cys Ser Tyr Ala Thr Val
                85                  90                  95

Asn Ser Trp Leu Phe Gly Gly Gly Thr Lys Val Thr Val Leu
            100                 105                 110


<210>  56
<211>  351
<212>  DNA
<213>  Homo sapiens


<220>
<221>  CDS
<222>  (1)..(351)
<223>  NI-206.34C11 variable heavy chain (VH) sequence (not PIMC)

<220>
<221>  V_region
<222>  (91)..(105)
<223>  complementarity determining region (CDR) VH-CDR1

<220>
<221>  V_region
<222>  (148)..(198)
<223>  complementarity determining region (CDR) VH-CDR2

<220>
<221>  V_region
<222>  (295)..(318)
<223>  complementarity determining region (CDR) VH-CDR3

<400>  56
gag gtg cag ctg gtg gag act ggg gga ggc ttg gta cag cct ggg ggg          48
Glu Val Gln Leu Val Glu Thr Gly Gly Gly Leu Val Gln Pro Gly Gly
1               5                   10                  15

tcc ctg aga ctc gcc tgt gca gcc tct gga ttc acc ttt agc agc tat          96
Ser Leu Arg Leu Ala Cys Ala Ala Ser Gly Phe Thr Phe Ser Ser Tyr
            20                  25                  30

gcc atg agc tgg gtc cgc cag gct cca ggg aag ggg ctg gag tgg gtc         144
Ala Met Ser Trp Val Arg Gln Ala Pro Gly Lys Gly Leu Glu Trp Val
        35                  40                  45

tca ggt att agt gat agt ggt ggt agc aca tac tac gct gac gcc gtg         192
Ser Gly Ile Ser Asp Ser Gly Gly Ser Thr Tyr Tyr Ala Asp Ala Val
    50                  55                  60

aag ggc cgg ttc acc att tcc aaa gac aat tcc aag aac acc ctg tat         240
Lys Gly Arg Phe Thr Ile Ser Lys Asp Asn Ser Lys Asn Thr Leu Tyr
65                  70                  75                  80

ctg caa atg aac agc ctg aga gcc gag gac acg gcc gta tat tac tgt         288
Leu Gln Met Asn Ser Leu Arg Ala Glu Asp Thr Ala Val Tyr Tyr Cys
```

```
                    85                  90                  95

gcg aga gtt gac tct aat agt atg gac gtc tgg ggc caa ggg acc acg         336
Ala Arg Val Asp Ser Asn Ser Met Asp Val Trp Gly Gln Gly Thr Thr
            100                 105                 110

gtc acc gtc tcc tcg                                                     351
Val Thr Val Ser Ser
        115


<210>  57
<211>  117
<212>  PRT
<213>  Homo sapiens

<400>  57

Glu Val Gln Leu Val Glu Thr Gly Gly Gly Leu Val Gln Pro Gly Gly
1               5                   10                  15


Ser Leu Arg Leu Ala Cys Ala Ala Ser Gly Phe Thr Phe Ser Ser Tyr
            20                  25                  30


Ala Met Ser Trp Val Arg Gln Ala Pro Gly Lys Gly Leu Glu Trp Val
        35                  40                  45


Ser Gly Ile Ser Asp Ser Gly Gly Ser Thr Tyr Tyr Ala Asp Ala Val
    50                  55                  60


Lys Gly Arg Phe Thr Ile Ser Lys Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Val Asp Ser Asn Ser Met Asp Val Trp Gly Gln Gly Thr Thr
            100                 105                 110


Val Thr Val Ser Ser
        115


<210>  58
<211>  330
<212>  DNA
<213>  Homo sapiens


<220>
<221>  CDS
<222>  (1)..(330)
<223>  NI-206.34C11 variable lambda light chain (VL) sequence (PIMC by
       default)

<220>
```

<221> V_region
<222> (67)..(105)
<223> complementarity determining region (CDR) VL-CDR1

<220>
<221> V_region
<222> (151)..(171)
<223> complementarity determining region (CDR) VL-CDR2

<220>
<221> V_region
<222> (268)..(300)
<223> complementarity determining region (CDR) VL-CDR3

<400> 58
```
cag tct gtg ttg acg cag ccg ccc tca ctg tct gcg gcc cca gga cag         48
Gln Ser Val Leu Thr Gln Pro Pro Ser Leu Ser Ala Ala Pro Gly Gln
1               5                   10                  15

aag gtc acc atc tcc tgc tct gga agc agc tcc aac att ggg aat aat         96
Lys Val Thr Ile Ser Cys Ser Gly Ser Ser Ser Asn Ile Gly Asn Asn
            20                  25                  30

tat gta tcc tgg tac cag caa ctc cca gga aca gcc ccc aaa ctc ctc        144
Tyr Val Ser Trp Tyr Gln Gln Leu Pro Gly Thr Ala Pro Lys Leu Leu
        35                  40                  45

att tat aac aat gat aag cgg ccc tca ggg att tct gac cga ttc tct        192
Ile Tyr Asn Asn Asp Lys Arg Pro Ser Gly Ile Ser Asp Arg Phe Ser
    50                  55                  60

ggc tcc aag tct ggc acg tca gcc acc ctg ggc atc acc gga ctc cag        240
Gly Ser Lys Ser Gly Thr Ser Ala Thr Leu Gly Ile Thr Gly Leu Gln
65                  70                  75                  80

act ggg gac gag gcc gat tat tac tgc gga aca tgg gat agg agc ctg        288
Thr Gly Asp Glu Ala Asp Tyr Tyr Cys Gly Thr Trp Asp Arg Ser Leu
                85                  90                  95

agt ggt agg gtg ttc ggc gga ggg acc aag ctg acc gtc cta                330
Ser Gly Arg Val Phe Gly Gly Gly Thr Lys Leu Thr Val Leu
            100                 105                 110
```

<210> 59
<211> 110
<212> PRT
<213> Homo sapiens

<400> 59

```
Gln Ser Val Leu Thr Gln Pro Pro Ser Leu Ser Ala Ala Pro Gly Gln
1               5                   10                  15

Lys Val Thr Ile Ser Cys Ser Gly Ser Ser Ser Asn Ile Gly Asn Asn
            20                  25                  30

Tyr Val Ser Trp Tyr Gln Gln Leu Pro Gly Thr Ala Pro Lys Leu Leu
        35                  40                  45
```

Ile Tyr Asn Asn Asp Lys Arg Pro Ser Gly Ile Ser Asp Arg Phe Ser
    50                  55                  60

Gly Ser Lys Ser Gly Thr Ser Ala Thr Leu Gly Ile Thr Gly Leu Gln
65                  70                  75                  80

Thr Gly Asp Glu Ala Asp Tyr Tyr Cys Gly Thr Trp Asp Arg Ser Leu
                85                  90                  95

Ser Gly Arg Val Phe Gly Gly Gly Thr Lys Leu Thr Val Leu
            100                 105                 110

<210> 60
<211> 11
<212> PRT
<213> Artificial Sequence

<220>
<223> Epitope of NI-206-18H2 antibody

<400> 60

Ile Gln Leu Pro Lys Glu Glu Ile Lys Lys Leu
1               5                   10

<210> 61
<211> 11
<212> PRT
<213> Artificial Sequence

<220>
<223> Epitope of NI-206.82C2

<400> 61

Tyr Leu Glu Ser Asp Tyr Ser Lys Leu Trp Arg
1               5                   10

<210> 62
<211> 15
<212> PRT
<213> Artificial Sequence

<220>
<223> Peptide 28 (amino acids 109 to 123) of pepscan image of NI-206.82C2

<400> 62

Arg Gln Phe Val Tyr Leu Glu Ser Asp Tyr Ser Lys Leu Trp Arg
1               5                   10                  15

<210> 63
<211> 15
<212> PRT
<213> Artificial Sequence

```
<220>
<223>  Peptide 29 (amino acids 113 to 127) of pepscan image of
       NI-206.82C2

<400>  63

Tyr Leu Glu Ser Asp Tyr Ser Lys Leu Trp Arg Tyr Ser Tyr Thr
1               5                   10                  15


<210>  64
<211>  15
<212>  PRT
<213>  Artificial Sequence

<220>
<223>  Peptide 125 (amino acids 497 to 511) of pepscan image of
       NI-206.18H2

<400>  64

Ala Leu Lys Asn Ile Gln Leu Pro Lys Glu Glu Ile Lys Lys Leu
1               5                   10                  15


<210>  65
<211>  15
<212>  PRT
<213>  Artificial Sequence

<220>
<223>  Peptide 126 (amino acids 501 to 515) of pepscan image of
       NI-206.18H2

<400>  65

Ile Gln Leu Pro Lys Glu Glu Ile Lys Lys Leu Glu Val Asp Glu
1               5                   10                  15
```

## Description

FIELD OF THE INVENTION

The present invention generally relates to antibody-based therapy and diagnosis of diseases associated with Fibroblast Activation Protein (FAP). In particular, the present invention relates to novel molecules specifically binding to human FAP and epitopes thereof, particularly human-derived recombinant antibodies as well as fragments, biotechnological and synthetic derivatives thereof, chimeric antigen receptors (CARs) directed against human FAP and equivalent FAP-binding agents, which are useful in the treatment of diseases and conditions induced by FAP and especially in T cell mediated treatment of FAP induced tumor associated diseases. Furthermore, the present invention relates to FAP-binding molecules which exhibit an increased avidity to transmembrane FAP in the acidic pH environment found in tumors, compared to lower avidity at the neutral pH environment in healthy tissue. In a particular aspect, a selective and potent FAP inhibitory agent is provided. In addition, the present invention relates to pharmaceutical and diagnostic compositions comprising said antibodies and agents valuable both as a diagnostic tool to identify diseases associated with FAP and also to passive vaccination strategy as well as active vaccination with antigens comprising the novel epitopes of the antibodies of the present invention for treating diseases associated with FAP such as various cancers, inflammatory and cardiovascular diseases and blood clotting disorders.

Furthermore, the present invention relates to a method of diagnosing a disease or condition induced by enhanced FAP activity, in particular protease activity for example in tumor tissue, which in accordance with the present invention is reflected by an increased level of FAP and a specific epitope of FAP, respectively, in a body fluid, in particular blood of the subject affected with the disease or condition. This finding also let to the development of a novel method of monitoring the treatment of the FAP induced disease with a therapeutic agent or determining the therapeutic utility of a candidate agent, preferably an anti-FAP antibody comprising determining the level of FAP in a sample derived from a body fluid, preferably blood of the subject following administration of the agent to the subject, wherein the absence or a reduced level of FAP in the sample of the subject compared to a control indicates progress in the treatment and therapeutic utility of the agent, respectively, wherein the method is characterized in that the level of FAP is determined by way of detecting a particular epitope of FAP.

BACKGROUND OF THE INVENTION

Human Fibroblast Activation Protein (FAP; GenBank Accession Number AAC51668; NCBI Reference Sequence: NM_004460.3), also known as Seprase, is a 170 kDa integral membrane serine peptidase (EC 3.4.21.B28). Together with dipeptidyl peptidase IV (DPPIV, also known as CD26; GenBank Accession Number P27487), a closely related cell-surface enzyme, and other peptidases, FAP belongs to the dipeptidyl peptidase IV family (Yu et al., FEBS J. 277 (2010), 1126-1144). It is a homodimer containing two N-glycosylated subunits with a large C-terminal extracellular domain, in which the enzyme's catalytic domain is located (Scanlan et al., Proc. Natl. Acad. Sci. USA 91 (1994), 5657-5661). FAP, in its glycosylated form, has both post-prolyl dipeptidyl peptidase and gelatinase activities (Sun et al., Protein Expr. Purif. 24 (2002), 274-281). Thus, FAP is a serine protease with both dipeptidyl peptidase, as well as endopeptidase activity cleaving gelatin and type I collagen.

Human FAP was originally identified in cultured fibroblasts using the monoclonal antibody (mAb) F19 (described in WO 93/05804, ATCC Number HB 8269). Homologues of the protein were found in several species, including mice (Niedermeyer et al., Int. J. Cancer 71, 383-389 (1997), Niedermeyer et al., Eur. J. Biochem. 254, 650-654 (1998); GenBank Accession Number AAH19190; NCBI Reference Sequence: NP_032012.1). Human and murine FAP share an 89% sequence identity and have similar functional homology. FAP has a unique tissue distribution: its expression was found to be highly upregulated on reactive stromal fibroblasts of more than 90% of all primary and metastatic epithelial tumors, including lung, colorectal, bladder, ovarian and breast carcinomas, while it is generally absent from normal adult tissues (Rettig et al., Proc. Natl. Acad. Sci. USA 85 (1988), 3110-3114; Garin-Chesa et al., Proc. Natl. Acad. Sci. USA 87 (1990), 7235-7239). Subsequent reports showed that FAP is not only expressed in stromal fibroblasts but also in some types of malignant cells of epithelial origin, and that FAP expression directly correlates with the malignant phenotype (Jin et al., Anticancer Res. 23 (2003), 3195-3198).

Due to its expression in many common cancers and its restricted expression in normal tissues, FAP has been considered a promising antigenic target for imaging, diagnosis and therapy of a variety of carcinomas. Thus, multiple monoclonal antibodies have been raised against FAP for research, diagnostic and therapeutic purposes, almost always aiming at targeting a detectable label or cytotoxic agent to carcinoma cells expressing FAP. For example, Sibrotuzumab/BIBHI, a humanized version of the FI9 antibody that specifically binds to human FAP (described in international application WO 99/57151) but is not inhibitory, and further humanized or human-like antibodies against the FAP antigen with F19 epitope specificity (described in Mersmann et al., Int. J. Cancer 92 (2001), 240-10 248; Schmidt et al., Eur. J. Biochem. 268 (2001), 1730-1738; and international application WO 01/68708) were developed, using phage display technology and human V-repertoires, where VL and VH regions of F19 were replaced by analogous human V-regions while retaining the original 15-amino acid long HCDR3 sequence in order to maintain F19 epitope specificity or the F19 antibody has been used as guide for selecting scFvs which recognize the same or a closely related epitope as the original mouse antibody. The OS4 antibody is another humanized (CDR-grafted) version of the F19 antibody (Wuest et al., J. Biotech. 92 (2001), 159-168), while scFv 33 and scFv 36 have a different binding specificity from F19 and are cross-reactive for the human and mouse FAP protein (Bracks et al., Mol. Med. 7 (2001), 461-469). Other murine anti-FAP antibodies, as well as chimeric and humanized versions thereof, were developed (international application WO 2007/077173, Ostermann et al., Clin. Cancer Res. 14 (2008), 4584-4592. In addition, human-like anti-FAP antibodies, *i.e*. Fab fragments using phage display technology were described (international application WO2012/020006), wherein selections were carried out against the ectodomain of human or murine FAP.

Proteases in the tumor stroma, through proteolytic degradation of extracellular matrix (ECM) components, facilitate processes such as angiogenesis and/or tumor cell migration. Moreover, the tumor stroma plays an important role in nutrient and oxygen supply of tumors, as well as in tumor invasion and metastasis. These essential functions make it not only a diagnostic but also a potential therapeutic target. Evidence for the feasibility of the concept of tumor stroma targeting *in vivo* using anti-FAP antibodies was obtained in a phase 1 clinical study with 131-iodine-lableled F19 antibody, which demonstrated specific enrichment of the antibody in the tumors and detection of metastases (Welt et al., J. Clin. Oncol. 12 (1994), 1193-1203). Similarly, a phase 1 study with Sibrotuzumab demonstrated specific tumor accumulation of the 131I-labeled antibody (Scott et al., Clin. Cancer Res. 9, 1639-1647 (2003)). An early phase II trial of unconjugated Sibrotuzumab in patients with metastatic colorectal cancer, however, was discontinued due to the lack of efficacy of the antibody in inhibiting tumor progression (Hofheinz et al., Onkologie 26 (2003), 44-48). In addition, 8 of 26 Sibrotuzumab-treated patients developed human-anti-human antibodies (HAHA) with a change in pharmacokinetics and reduced tumor uptake in 4 of 26 patients (Welt *et al.*, 1994, supra). Also a more recently developed anti-FAP antibody failed to show anti-tumor effects *in vivo* in unconjugated form (WO 2007/077173).

More recently, again using phage display techniques single-chain variable fragments (scFvs) after three rounds of panning against FAP yielded an inhibitory scFv antibody, named E3, which could attenuate 35% of FAP cleavage of the fluorescent substrate Ala-Pro-7-amido-4-trifluoromethylcoumarin compared with nonfunctional scFv control which displayed a 1.5 magnitude higher affinity (Zhang et al., FASEB J. 27 (2013), 581-589). However, the putative EC50 value was quite low, *i.e*. having a KD of about 2x 10-7 M and only approximately 35% inhibition of FAP enzymatic activity was seen at 17.85 μM (100 μg) of E3, and even after yeast affinity maturation the best mutant showed only a higher affinity (4-fold) and enhanced inhibitory effect on FAP enzyme activity of 4- and 3-fold, respectively, than E3. Therefore, in view of both the rather low affinity and inhibitory effect therapeutic utility of the scFv per se may not be expected. Rather, the authors concluded that the scFv itself or its derived IgG may nevertheless be a useful clinical reagent for investigating *in vivo* targeting of FAP positive tumor stroma. In view of the reports on FAP targeting so far it appears as if the development an anti-FAP antibody which has high affinity and a pronounced inhibitory effect on protease activity of FAP is not feasible.

Another FAP-targeting drug is Talabostat developed by Point Therapeutics. Talabostat (also known as PT-100 or Val-boroPro), a prolyl boronic acid, was originally developed as a DPPIV inhibitor and has been shown to also inhibit FAP, DPP8, DPP9, and POP/PREP. Experimental treatment considerations of metastatic cancer raised the possibility that Talabostat, could also be useful for inhibiting FAP and, as a consequence, cancer growth (Cunningham, Expert Opinion on Investigational Drugs. 16 (2007), 1459-1465; Narra et al., Cancer Biology & Therapy 6 (2007), 1691-1699). Talabostat also rapidly loses inhibitory activity due to cyclization in aqueous media, pH 7.8 (Kelly et al., Journal of the American Chemical Society 115 (1993), 12637-12638). Despite this limitation, when Val-boroPro treatment was used over several days to treat cancer patients, Met-a2AP/Asn-a2AP ratios increased significantly in humans, suggesting that the medication does inhibit FAP activity to some degree (Lee et al., Journal of Thrombosis and Haemostasis 9 (2011), 987-996).

In metastatic colorectal cancer patients, Talabostat was given PO at 200 mg BID. Despite reports that 1'200 μg is the maximum tolerated dose of Talabostat in healthy patients (Uprichard and Jones, ASH Annual Meeting Abstracts 104 (2004), 4215), the trial was initiated with patients receiving Val-boroPro orally at 400 μg taken twice per day (800 μg total daily). However, the protocol of 200 μg BID (400 μg total daily) was amended after enrolling three patients when the third patient died of renal failure and the first two patients experienced moderate toxicities (edema, fever) thought probably related to Val-boroPro. One intrapatient dose escalation to 300 μg twice per day (600 μg total daily) was allowed after four weeks of treatment if no non-hematologic toxicity greater than grade I was experienced. Therefore, between 400-600 μg daily doses were evaluated, due to dose limiting toxicities.

On this dosing regimen of 400-600μg total daily, Talabostat inhibited approx. 95% plasma dipeptidyl peptidase activity (mostly a combination of FAP and DPPIV), but only approx. 18% of post-proline-specific endopeptidase (mostly FAP specific) activity, suggesting that FAP was not effectively inhibited. Higher concentration of talabostat added *ex-vivo* (10 μM) resulted in 75% further inhibition of the FAP-specific activity, revealing that only a fraction of plasma FAP activity was inhibited in these patients. These data also suggest that FAP activity in the tumors was marginally inhibited.

Therefore, it is evident that clinical results of the Talabostat studies are not due to FAP inhibition *per se*, but rather that clinical effects were largely due to off target binding and inhibition of DPPIV, DPP8, and DPP9 (Narra *et al.*, (2007), *supra*).

Val-boroPro also inhibited dipeptidyl peptidases such as DPPIV, DPP8, DPP9, and prolylopigopeptidase (POP) and upregulated cytokine and chemokine activities (Narra *et al.*, (2007), *supra*). Dose limiting toxicities reported in these patients were predominantly consistent with cytokine effects, thought due to inhibition of cytoplasmic DPP8 and DPP9 resulting in severe side effects in animal trials. In rats, the DPP8/9 inhibition produced alopecia, thrombocytopenia, reticulocytopenia, enlarged spleen, multiorgan histopathological changes, and mortality. In dogs, DPP8/9 inhibitor produced gastrointestinal toxicity (Lankas et al., Diabetes 54 (2005), 2988-2994). DPPIV inhibition however has been shown to be safe in animals and humans (Nauck et al., Diabetes Care. 2014, published online before print April 17, 2014, doi: 10.2337/dc13-2761).

Although the clinical endpoints for the Val-boroPro treatment were not met in treating patients with epithelial cancer, this single agent study allowed investigation of the pharmacodynamic effects of FAP inhibition, thus providing valuable information about the effects of Val-boroPro on FAP enzymatic activity *in vivo* (Narra *et al.*, (2007), *supra*). FAP enzymatic activity was analyzed in patient plasma samples before and during Val-boroPro treatment using an endopeptidase substrate that cannot be cleaved by exopeptidases like DPP-IV. FAP selective enzymatic activity was reduced during treatment compared to pre-treatment levels, indicating that Val-boroPro is able to inhibit the enzymatic activity of FAP *in vivo*. However the inhibition of FAP was only partial at the doses utilized, as only approximately 20% of presumed FAP enzymatic activity was blocked. Higher concentrations of Val-boroPro *ex-vivo* (10$\mu$M) resulted in 60% more inhibition of the presumed FAP activity. However these concentrations are difficult to achieve in patients given the clinical toxicities seen with this agent at higher doses. Thus although robust and potent DPP exopeptidase inhibition with Val-boroPro was seen, only partial inhibition of FAP endopeptidase enzymatic activity was achieved. This partial inhibition of FAP may be another contributing factor explaining the minimal clinical activity seen with Val-boroPro treatment. New compounds that uncouple the cytokine toxicities mediated by inhibition of other dipeptidyl peptidase enzymes (e.g., cytosolic DPP8 and DPP9), from FAP inhibition may be advantageous to maximally inhibit FAP in the tumor stroma. Such therapeutics may yield improved clinical efficacy with less toxicity, as the U.S. Food and Drug Administration (FDA) placed the clinical program of talabostat on clinical hold as a result of an interim analysis of two Phase 3 studies of talabostat in combination with chemotherapy in patients with metastatic non-small cell lung cancer (NSCLC) (Narra et al., Cancer Biology & Therapy 6 (2007), 1691-1699).

Proof of concept has been shown in mouse models that inhibiting FAP with a small molecule inhibitor PT-630 and genetic knockout can abrogate tumor growth in NSCLC and MCRC indirectly through effects on stromagenesis, vascularization, and ECM remodeling (Santos et al., Journal of Clinical Investigation 119 (2009), 3613-3625). However, PT-630 also inhibits DPPPIV, DPP8 and DPP9 while simultaneously experiencing poor in-vivo stability due to cyclization.

Proof of concept has also been shown that a small molecule FAP inhibitor "Inhibitor 6", decreases FAP's proteolytic conversion of Met-$\alpha$2AP to Asn-$\alpha$2AP in a dose-response manner, with the resultant increased Met-$\alpha$2AP/Asn-$\alpha$2AP ratios corresponding to shortened lysis times for fibrin made from human plasma. It was demonstrated that FAP inhibition (arrest of Met-$\alpha$2AP conversion) results in increased lysis. However "Inhibitor 6" was not selective against POP/PREP. Despite this limitation of the inhibitor, the publication concluded that persistent exposure to the FAP inhibitor in the presence of normal *in vivo* protein turnover should ultimately shift total $\alpha$2AP to Met-$\alpha$2AP, which if maintained at maximal levels, would become crosslinked into fibrin much more slowly than derivative Asn-$\alpha$2AP. Just as naturally occurs in persons who are heterozygous for functionally impaired $\alpha$2AP, it was concluded that it may be possible to mimic a similar long-term state of increased fibrinolysis without significant risk of bleeding and thus present potential therapeutic benefit to persons at high risk for chronic progressive intravascular fibrin deposition (Lee et al., Journal of Thrombosis and Haemostasis 9 (2011), 1268-1269).

So called Compound 60 is a novel small molecule FAP inhibitor that has demonstrated promising biochemical characteristics in pre-clinical trials in the context of FAP selectivity and pharmacokinetics (Jansen et al., J. Med. Chem. 57 (2014), 3053-3074). However of concern, a structurally similar molecule "compound 4" (Figure A2) killed rats with 6 hours when injected at 5mg/kg (iv), and therefore safety may be a concern in humans. Another concern regarding compound 60 are the $IC_{50}$ values against PREP (1.8$\mu$M) and DPP9 (12.5 $\mu$M). These relatively low $IC_{50}$ values indicate that $C_{max}$ for compound 6 would need to in the sub $\mu$M range in order to prevent inhibition of these homologues (of which DPP9 inhibition is considered toxic), and it also remains to be seen if enough drug can be delivered to block FAP systemically with a $C_{max}$ for the medication remaining in the sub $\mu$M range (Jansen et al., Journal of Medicinal Chemistry 57 (2014), 3053-3074).

Next to antibodies, a further immunotherapeutic approach for the treatment of diseases associated with FAP, in particular for the treatment of cancer, includes the adoptive transfer of modified T cells transduced with chimeric antigen receptors (CARs) directed against the FAP antigen.

CARs are typically engineered fusion proteins that contain an extracellular antigen-binding domain composed of a single chain variable fragment derived from an antibody specific for the intended target region, a transmembrane domain and a T cell-specific intracellular signaling domain, such as the CD3$\zeta$ domain and might contain in addition either the 4-1BB or the CD28 costimulatory domain. Details on the structure, mechanisms of action, applications, etc. have been summarized in various literature, *e.g.,* in Maus and June, Clin. Cancer Res. 22 (2016), 1875-1884; Zhang et al., Cancer Transl. Med. 1 (2015), 43-49; Dai et al., JNCI J. Natl. Cancer Inst. 108 (2016); Rodgers et al., Proc. Natl. Acad. Sci. USA 113 (2016), E459-68; Brower, The Scientist Magazine®, April 2015 Issue.

Proof of concept with respect to anti-FAP CAR T cells has been described for example by Tran et al. (J. Exp. Med. 210 (2013), 1125-1135), Schuberth al. (J. Transl. Med. 11 (2013), 187), Kakarla et al. (Mol. Ther. 21 (2013), 1611-1620), Wang et al. (Cancer Immunol. Res. 2 (2014), 154-166 as well as in WO 2014055442 A2. The anti-FAP CAR mouse T cells comprising the binding domain of the FAP-5 monoclonal antibody that targets human and mouse FAP used by Tran *et al.* showed only limited anti-tumor efficacy. In Schuberth *et al.*, a CAR comprising the binding domain of monoclonal antibody F19 has been analyzed in mouse models. However, anti-tumor efficacy has not been evaluated since the F19 antibody does not cross-react with the mouse FAP. Furthermore, Kakarla *et al.* could show anti-tumor efficacy using anti-FAP CAR T cells comprising the binding domain of an anti-human / anti-mouse FAP monoclonal antibody in an immunodeficient mouse model. Similarly, Wang *et al.* developed CAR constructs specific for mouse FAP that target tumor-promoting stromal cells using the corresponding CAR T cells. In WO 2014/055442 A2, similar to Schuberth *et al.*, CARs comprising the binding domain of either murine anti-FAP antibodies or the mentioned F19 antibody have been constructed and analyzed.

Considering the above, so far FAP-targeting medications evaluated to date in human clinical trials suffer from several drawbacks, e.g., being non-selective and having a short biological half-life such as Talabostat or though being capable of specifically binding FAP lack a therapeutic effect and are immunogenic in human such as a Sibrotuzumab and other F19 based anti-FAP antibodies. In addition, previous evaluation of both PT-100 and Sibrotuzumab in clinical studies suggests that certain performance criteria are needed for an FAP-targeting medication to have the desired therapeutic effect. In case of the anti-FAP CARs, they have mainly be developed on basis of antibodies targeting mouse FAP or on basis of the mouse monoclonal F19 antibody which suffer from the drawbacks mentioned above.

Therefore, there is a need for FAP-binding molecules, FAP-selective inhibitors as well as CARs directed against FAP which are tolerable in human, effective for the treatment of diseases associated with FAP, as well for as for reliable diagnostic assays for diseases caused by and associated with FAP, and which indicate whether or not a patient suffering from such disease is amenable to FAP-targeted therapy.

The above technical problems are solved by the embodiments characterized in the claims and described further below and illustrated in the Examples and Figures.

## SUMMARY OF THE INVENTION

The present invention provides Fibroblast Activation Protein (FAP) antibodies, corresponding chimeric antigen receptors (CARs) and equivalent FAP-binding agents useful as a human and/or veterinary medicine, in particular for the treatment and/or prevention of FAP-related disorders such as but not limited to proliferative disorders. More specifically, therapeutically useful human-derived recombinant antibodies as well as fragments and derivatives thereof that recognize human FAP and/or fragments thereof are provided.

Thus, the present invention relates to the embodiments recited in any one of the following items *[1] et al.*, which are further disclosed in the detailed description of the present invention and/or may be supplemented with applications and embodiments described for FAP specific drugs such as anti-FAP antibodies and CARs directed against human FAP described in the documents referred to herein including those cited in the background section or known to the person skilled in the art otherwise:

[1] A monoclonal human memory B cell-derived anti-Fibroblast Activation Protein (FAP) antibody.

As illustrated in the appended Examples, experiments performed in accordance with the present invention were successful in the isolation of monoclonal FAP-specific antibodies from human memory B cells. This is surprising since hitherto the presence of autoantibodies against FAP has not been reported. In addition, controversial reports on the level and significance of FAP in the serum of patients suffering from carcinoma may be the reason that the possibility of existing anti-FAP autoantibodies and memory B cells, respectively has not been investigated at first place.

Because of human origin, *i.e.* derived from memory B cell and thus selected for self-tolerance and affinity matured in the human body contrary "human-like" antibodies such as generated by phage display or Xeno-Mouse it is prudent to expect that the human monoclonal anti-FAP antibodies of the present invention and derivatives thereof are non-immunogenic in human and useful in therapy and/or diagnostic uses *in vivo.* The present invention is thus directed to human-derived recombinant antibodies and biotechnological and synthetic derivatives thereof, and equivalent FAP-binding molecules which are capable of specifically recognizing FAP. If not indicated otherwise, by "antibody specifically recognizing FAP", "antibody specific to/for FAP" and "anti-FAP antibody" antibodies are meant which specifically, generally, and collectively bind to FAP but not substantially to FAP homologues, for example DPPIV, DPP8, DPP9, and POP/PREP; see Example 6 and Figure 7.

[2] The antibody of [1], wherein at least one of the complementarity determining regions (CDRs) and/or variable heavy ($V_H$) and/or variable light ($V_L$) chain of the antibody are derived encoded by a cDNA derived from an mRNA obtained from a human memory B cell which produced an anti-FAP antibody.

As known in the art, in order to retain the binding specificity and affinity of a given antibody it is not necessary that a cognate antibody contains all six CDR regions of the original human antibody, but only one original CDR region, in particular CDRH3 as described in international application WO01/68708 and Mersmann, *supra*. Furthermore, by retaining one or more of the CDRs of the original human monoclonal antibody the anti-FAP antibody of the present invention and equivalent FAP-binding molecules containing the CDR(s) advantageously have a lesser xeno-antigenic potential than any anti-FAP antibody engineered from mouse monoclonal antibodies. The same holds true for "human-like" antibodies such as generated by phage display or Xeno-Mouse since, as mentioned the scFvs, Fab-fragments and antibodies, respectively, are still artificial and foreign to the human body for which reason they are still immunogenic and known to induce Anti-Drug Antibody (ADA) responses. A process for preparing antibodies equivalent to anti-FAP antibody F19, *supra*, by CDRH3 retaining guided selection method is described in international application WO01/68708 and may be adapted to the human-derived monoclonal anti-FAP antibody of the present invention illustrated in the Examples.

[3] The antibody of [1] or [2] or a biotechnological or synthetic derivative thereof, which is capable of binding to captured or directly coated human FAP and/or fragments thereof (378-HYIKDTVENAIQITS-392 (SEQ ID NO: 27), 622-GWSYGGYVSSLALAS-636 (SEQ ID NO: 28) and 721-QVDFQAMWYSDQNHGL-736 (SEQ ID NO: 29)) with an EC50 of $\leq$ 0.1 $\mu$M.

As illustrated in Example 1 and shown in Figure 2 the antibodies of the present invention display a binding affinity to human FAP, *i.e.* EC50 values as determined by a non-linear regression in the nano- and sub-nanomolar range. Therefore, preferably the anti-FAP antibody of the present invention binds human FAP with an affinity to the captured FAP (sFAP) with an EC50 of $\leq$ 10 nM, more preferably $\leq$ 1 nM, and most preferably $\leq$ 0.1 nM. In addition, or alternatively the anti-FAP antibody of the present invention binds human FAP with an affinity to the directly coated FAP (FAP) with an EC50 of $\leq$ 10 nM, more preferably $\leq$ 1 nM, and most preferably $\leq$ 0.1 nM. In a still further embodiment, the anti-FAP antibody of the present invention in addition or alternatively binds to directly coated mixture of FAP fragments indicated in the legend to Figure 2(K) (cFAP) with an EC50 of $\leq$ 10 nM, more preferably $\leq$ 5 nM. The binding specificity and EC50 value of a candidate anti-FAP antibody may be determined by methods such as direct ELISA well known in the art, preferably as illustrated in the Examples. In addition, or alternatively any one of the subject antibodies illustrated in the Examples and Figures may be used as a reference antibody in a FAP binding competition assay; see, for example, international applications WO 01/68708, WO 2011/040972 and WO 2012/020006 as well as the description further below.

Besides the high affinity and specificity, the subject anti-FAP antibodies are preferably characterized by its capability of targeting human carcinoma tissue, human breast cancer tissue, colorectal cancer tissue, (murine) myeloma tissue and tumor stroma, and coronary thrombi and/or atherosclerotic plaque; see Examples 8 to 12 and 18 as well as corresponding Figures 9 to 14 and 24.

Hence, due to their high affinity to FAP and specificity of binding FAP positive carcinoma cells and tissue, coronary thrombi and atherosclerotic plaques the anti-FAP antibodies and equivalent FAP-binding agents of the present invention are particularly suited for therapeutic and diagnostic settings hitherto described for anti-FAP antibodies such as F19 and Sibrotuzumab, for example as potent radioimmunoconjugates, *in vivo* imaging agents or antibody-drug conjugates for diagnostic and therapeutic use in patients with FAP-expressing tumors; see, e.g., biodistribution and therapeutic effects of antibody phage library derived human-like Fab fragments (ESC11 and ESC 14) that bind to human and murine FAP and were engineered into fully human IgG1 antibodies labeled with the $\beta$-emitting radionuclide (177) Lu in a melanoma xenograft nude mouse model described in Fischer et al., Clin. Cancer Res. 15 (2012), 6208-6018.

[4] The antibody of any one of [1] to [3] or a biotechnological or synthetic derivative thereof, which is capable of binding a FAP epitope in a peptide of 15 amino acids in length, which epitope comprises or consists of the amino acid sequence NI-206-18H2 (501-IQLPKEEIKKL-511) (SEQ ID NO: 60) NI-206.82C2 (521-KMILPPQFDRSKKYP-535 (SEQ ID NO: 30); 525-PPQFDRSKKYPLLIQ-539 (SEQ ID NO: 31); 525-PPQFDRSKKYP-535 (SEQ ID NO: 32)); and/or 113-YLESDYSKLWR-123 (SEQ ID NO: 61)); NI-206.59B4 (53-SYKTFFP-59 (SEQ ID NO: 33)); NI-206.22F7 (381-KDTVENAIQIT-391 (SEQ ID NO: 34)); NI-206.27E8 (169-NIYLKQR-175 (SEQ ID NO: 35)); NI-206.12G4 (481-TDQEIKILEENKELE-495 (SEQ ID NO: 36)); or NI-206.17A6 (77-VLYNIETGQSY-87 (SEQ ID NO: 37)).

As described in Example 3, binding analyses have been performed to determine the binding epitope of the antibodies of the present invention. Exemplary, for NI-206.18H2 it has been shown that it recognizes peptides corresponding to the amino acid sequence 501-IQLPKEEIKKL-511 (SEQ ID NO: 60); see Fig. 4C. Furthermore, the minimum epitope region of NI-206.82C2 was identified by stepwise truncated peptides from the N- and C-terminus of a peptide fragment consisting of amino acids 521 to 539 of FAP covering the epitope of NI-206.82C2 (with spot 21 corresponding to the full length peptide and spots 22 to 33 to stepwise one amino acid truncations form the C-terminus and spots 34 to 45 corresponding to stepwise one amino acid truncations form the N-terminus) synthesized and spotted onto nitrocellulose membranes which revealed that antibody NI-206.82C2 recognizes spots 21-28 and 34-41 which correspond to the sequence 528-FDRSK-532 (SEQ ID NO: 39) on FAP; see Figure 26. Furthermore, due to the sequential mutation of every single amino acid in the mentioned FAP fragment 521-KMILPPQFDRSKKYPLLIQ-539 (SEQ ID NO: 38) into an alanine amino acids D-529 and K-532 of FAP were identified to be essential for NI-206.82C2 binding; see Figure 27. Therefore, whether or not an anti-FAP antibody is derived from and equivalent to antibody NI-206.82C2, respectively, may be identified by determining whether a given candidate antibody displays substantially the same binding characteristics as described for antibody NI-206.82C2 in Example 3, *i.e.* a core epitope of amino acids 528-FDRSK-532 of FAP and/or one or both key amino acids D-529 and K-532 of FAP for binding. The assessment of these features can be preferably performed in accordance with the Examples of the present application. Similarly, anti-FAP antibodies derived from and equivalent to any of the other antibodies disclosed herein, e.g., antibody NI-206.18H2 may be identified by determining whether a given candidate antibody displays substantially the same binding characteristics and competes with the exemplary antibody for binding the cognate epitope having, *e.g.*, the amino acid sequence of SEQ ID NO: 60.

[5] The antibody of any one of [1] to [4], or a biotechnological or synthetic derivative thereof which is capable of inhibiting protease activity of FAP, preferably wherein the antibody is capable of inhibiting recombinant human FAP (rhuFAP)-mediated cleavage of Prolyl Endopeptidase (PEP) substrate N-carbobenzoxy-Gly-Pro-7-amido-4-methylcoumarin (Z-Gly-Pro-AMC) or direct quenched gelatin (DQ-gelatin) with an IC50 of $\leq 0.1$ $\mu$M.

As mentioned above, FAP-targeting agents which display both, *i.e.* (i) high affinity and selectivity for FAP like in principle feasible with respect to anti-FAP antibodies and (ii) potent inhibitory effect on the enzymatic activity of FAP like shown for low molecular weight pseudo-peptide substrates and inhibitors such as Talabostat have not been provided so far. As described in Examples 4 to 7 and 18 as well as illustrated in Figures 5 to 8 and 24 the present invention for the first time provides such an agent, *i.e.* anti-FAP antibody NI-206.82C2. Moreover, the anti-FAP antibody of the present invention can be expected to have a considerable longer half-life than for example Talabostat and similar compounds, in particular when provided as IgG type antibody since human IgG is typically associated with a half-life of ~25 days. On the other hand, in case a long serum half-life is not desired for applications such as radioimmunotherapy or imaging as it may lead to irradiation of healthy tissues and high background respectively, antibody fragments such as Fab fragments and biotechnological and synthetic derivatives of the subject antibody may be used as an attractive alternative as they can be monovalent and rapidly eliminated by renal clearance. In particular, as described in Example 19 and shown in Figure 25 non-radioactive but fluorescently labeled antibody NI-206.82C2 accumulated selectively in the tumor stroma of a tumor mouse model and that the antibody concentration peaks in the animals from 6 h to 48 h post antibody injection and declines to almost zero after 6 d post antibody injection demonstrating efficient removal from the animals' body. Hence, once an anti-FAP antibody with the binding characteristics and biological properties as demonstrated for exemplary antibody NI-206.82C2 has been provided, various techniques are at the disposal of the person skilled in art to prepare biotechnological and synthetic derivatives thereof, for example with either enhanced or reduced bioavailability and half-life depending on the intended use; see for review, *e.g.,* Chames et al., British Journal of Pharmacology 157 (2009), 220-233 and Vugmeyster et al, World J. Biol. Chem. 3 (2012), 73-92. Since anti-FAP antibodies NI-206-18H2 and NI.206-6D3 also specifically bind to captured or directly coated FAP and do not bind to other human antigens, the characteristics of antibody NI-206.82C2 may be attributed to those antibodies too.

[6] The antibody of any one of [1] to [5] or a biotechnological or synthetic derivative thereof, which is capable of prolonging the clot formation time or decreasing clot rigidity of human blood plasma.

As described in Example 13 and illustrated in Figure 15 the present invention for the first time provides an anti-FAP antibody capable of prolonging human blood plasma clotting time, decreasing clotting rate, clot elasticity, and clot rigidity. Thus, in this embodiment the anti-FAP antibody and equivalent FAP-binding agent is useful as anti-coagulant and thus suitable for the treatment of corresponding disorders and *in vitro* uses. Hence, the present invention in general relates to a monoclonal antibody which is capable of inhibiting blood clotting/prolonging blood clotting time, decreasing clotting rate, clot elasticity, and/or clot rigidity by specific binding to FAP. Furthermore, as described in Example 14 and illustrated in Figure 16 immunoprecipitation of FAP from human plasma results in significant reduction of the rate of FAP substrate alpha 2 anti-plasmin ($\alpha$2AP-AMC) cleavage in the resulting plasma, compared to plasma before NI-206.82C2 immunoprecipitation. These data establish that inhibition of FAP might represent a therapeutic approach for enhancing thrombolytic activity.

[7] The antibody of any one of [1] to [6] or a biotechnological or synthetic derivative thereof comprising in its variable region or binding domain

(a) at least one CDR of the $V_H$ and/or $V_L$ chain amino acid sequence depicted in any one of Figs. 1G-1K and 1A-1F;

(b) an amino acid sequence of the $V_H$ and/or $V_L$ chain amino acid sequence as depicted in Figs. 1G-1K and 1A-1F;
(c) at least one CDR consisting of an amino acid sequence resulted from a partial alteration of any one of the amino acid sequences of (a); or
(d) a $V_H$ and/or $V_L$ chain comprising an amino acid sequence resulted from a partial alteration of the amino acid sequence of (b);

preferably wherein the number of alteration in the amino acid sequence is below 50%.

The pH of solid tumors is acidic due to increased fermentative metabolism. This, combined with poor perfusion results in an acidic extracellular pH in malignant tumors (pH 6.5-6.9) compared with normal tissue under physiologic conditions (7.2-7.4); see, *e.g.* Gillies et al., Am. J. Physiol. 267 (1994), (1 Pt 1), C195-203; Stubbs et al., Mol. Med. Today 6 (2000), 15-19. It has been hypothesized that acid pH promotes local invasive growth and metastasis. The hypothesis that acid mediates invasion proposes that H(+) diffuses from the proximal tumor microenvironment into adjacent normal tissues where it causes tissue remodeling that permits local invasion; see, *e.g.* Schornack et al., Neoplasia 5 (2003), 135-145. Such remodeling even may alter epitopes and thus affect antibody avidity which may be one reason for the failed anti-tumor effects of an anti-FAP antibody in unconjugated form; see WO 2007/077173, *supra*. In contrast, as demonstrated in Example 18 and illustrated in Figure 24, the anti-FAP antibody of the present invention, in particular antibodies NI-206.82C2, NI.206-59B4, NI206.27E8, NI206.18H2 and NI206.6D3 surprisingly revealed increased avidity to transmembrane FAP in the acidic pH found in tumors, compared to lower avidity at the neutral pH of healthy tissue. The pH dependent avidity is important because FAP is expressed also in healthy tissues, and antibody binding to healthy tissues may cause side effects. Therefore, due to the preferential binding of antibodies of the present invention to FAP in the tumor microenvironment a higher therapeutic effect can be achieved without the side effects associated with binding to transmembrane FAP in other tissues. These data further support that anti-FAP antibodies of the present invention capable of targeting (transmembrane) FAP within the acidic tumor microenvironment should represent an effective therapy for malignant disease.

[8] The antibody of any one of [1] to [7] or a biotechnological or synthetic derivative thereof, which is capable of binding to transmembrane FAP.

[9] The antibody of any one of [1] to [8] or a biotechnological or synthetic derivative thereof, which shows a higher avidity of, *i.e.* preferential binding to FAP under acidic pH as compared to neutral or physiological pH, preferably wherein the acidic pH is 6.4 or 6.8 and the physiological pH is 7.4; see also Example 18 and Figure 24 according to which the preferential binding of antibodies of the present invention to transmembrane FAP in an acidic environment can be tested.
In contrast, as demonstrated in Example 18 and illustrated in Figure 24D, the present invention also provides human and recombinant human-derived anti-FAP antibodies, in particular antibody NI-206.12G4, which show a decreased avidity to FAP in the acidic pH as compared to neutral or physiological pH. This characteristic may be advantageous when rather than the treatment of a tumor targeting of FAP associated with an inflammatory or cardiovascular disease is intended, where FAP and FAP expressing cells may be present in an environment with neutral pH.

[10] The antibody of any one of [1] to [8] or a biotechnological or synthetic derivative thereof, which shows a lower avidity of binding to FAP under acidic pH as compared to neutral or physiological pH, preferably wherein the acidic pH is 6.4 or 6.8 and the physiological pH is 7.4; see also Example 18 and Figure 24D according to which the preferential binding of antibodies of the present invention to transmembrane FAP in a neutral environment can be tested.
As mentioned before, preferably the anti-FAP antibody of the present invention is a recombinant antibody, wherein at least one, preferably two or more preferably all three complementarity determining regions (CDRs) of the variable heavy and/or light chain, and/or substantially the entire variable region are encoded by a cDNA derived from an mRNA obtained from a human memory B cell which produced an anti-FAP antibody. In a preferred embodiment, the anti-FAP antibody of the present invention displays, in any combination one or more of the binding characteristics and biological properties as demonstrated for the subject antibodies illustrated in the appended Examples and Figures, preferably one more of the binding characteristics and biological properties as demonstrated for exemplary antibody NI-206.82C2 or NI-206.18H2. Instead of the amino acid sequences of the above-mentioned CDRs and $V_H$ and/or $V_L$ chain, the amino acid sequences resulted from a partial alteration of these amino acid sequences can be used. However, alteration of the amino acid sequences can be carried out only in the range in which the antibody of the present invention substantially retains any one of the binding characteristics and biological activities mentioned before and illustrated in the Examples. As long as the antibody has any one of the mentioned activities, the respective activity may be increased or reduced by the alteration of the amino acid sequence. The number of amino acids to be altered is preferably 50 % or less, more preferably 40 % or less, still more preferably 30 % or less, even more preferably 20 % or less, and most preferably 10 % or less, respectively, with respect to the entire amino acids of the amino acid sequence of the above-mentioned CDRs or of the $V_H$ and/or $V_L$ chain. Means and methods for preparing biotechnological or synthetic derivatives and variants of a parent antibody are well known in the art; see the literature cited herein, e.g., international application WO 2012/020006 for substitution, insertion, and deletion variants; glycosylation variants; Fc region variants; cysteine engineered antibody variants; and antibody derivatives which may be equally applied to the subject antibodies illustrated in the Examples. In a particularly preferred embodiment of the present invention, the anti-FAP antibody or FAP-binding fragment thereof demonstrates the immunological binding characteristics of an antibody characterized by the variable regions VH and/or VL as set forth in Fig. 1G-1K and 1A-1F.

[11] The antibody of any one of [1] to [9] or a biotechnological or synthetic derivative thereof comprising in its variable region or binding domain

(a) at least one CDR of the $V_H$ and/or $V_L$ chain amino acid sequence depicted in any one of Fig. 1A, 1B, 1D, 1G, 1I;
(b) an amino acid sequence of the $V_H$ and/or $V_L$ chain amino acid sequence as depicted in Fig. 1A, 1B, 1D, 1G, 1I;
(c) at least one CDR consisting of an amino acid sequence resulted from a partial alteration of any one of the amino acid sequences of (a); or
(d) a $V_H$ and/or $V_L$ chain comprising an amino acid sequence resulted from a partial alteration of the amino acid sequence of (b);

preferably wherein the antibody is capable of binding a FAP epitope in a peptide of 15 amino acids in length, which epitope comprises or consists of the amino acid sequence of any one of SEQ ID NOs: 30 to 33, 35, 60 and 61.

[12] The antibody of any one of claims 1 to 8 and 10 or a biotechnological or synthetic derivative thereof comprising in its variable region or binding domain

(a) at least one CDR of the $V_H$ and/or $V_L$ chain amino acid sequence depicted in any one of Fig. 1E;
(b) an amino acid sequence of the $V_H$ and/or $V_L$ chain amino acid sequence as depicted in Fig. 1E;
(c) at least one CDR consisting of an amino acid sequence resulted from a partial alteration of any one of the amino acid sequences of (a); or
(d) a $V_H$ and/or $V_L$ chain comprising an amino acid sequence resulted from a partial alteration of the amino acid sequence of (b);

preferably wherein the antibody is capable of binding a FAP epitope in a peptide of 15 amino acids in length, which epitope comprises or consists of the amino acid sequence of SEQ ID NO: 36.

As demonstrated in the appended Examples and illustrated in the Figures, one anti-FAP antibody, NI-206.82C2 is provided characterized by unique binding and biological properties. Thus, in this embodiment the antibody of the present invention is preferably characterized by being capable of

(i) inhibiting protease activity of FAP;
(ii) prolonging the clot formation time or decreasing clot rigidity of human blood plasma; and/or
(iii) binding a FAP epitope in a peptide of 15 amino acids in length, which epitope comprises or consists of the amino acid sequence 525-PPQFDRSKKYP-535 (SEQ ID NO: 32).

Put in other words, the present invention generally relates to a FAP inhibitory antibody and equivalent FAP-binding agent which are characterized by any one of the functional features (i) to (iii). In addition, or alternatively to any one of the functional features (i) to (iii) the antibody of the present invention is preferably characterized by preferential binding to transmembrane FAP in an acidic environment as described *supra*. For example, the antibody NI.206-18H2 specifically binds to a FAP epitope in a peptide of 15 amino acids in length, which epitope comprises or consists of the amino acid sequence 501-IQLPKEEIKKL-511 (SEQ ID NO: 60) and is further characterized by preferential binding to transmembrane FAP in an acidic environment.

[13] An agent which is capable of inhibiting protease activity of FAP and/or prolonging the clot formation time or delaying clot rigidity of human blood plasma, characterized in that the agent is capable of competing with the antibody of [10] to bind an epitope of FAP comprising or consisting of the amino acid sequence of SEQ ID NOs: 32 or 60, preferably wherein the agent is an anti-FAP antibody.
As apparent form the Examples, the epitope (SEQ ID NO: 32) of antibody NI-206.82C2 is unique and entirely unexpected since when bound by the antibody FAP activity is inhibited though the epitope lies outside the FAP catalytic triad which is composed of residues $Ser^{624}$, $Asp^{702}$, and $His^{734}$; see, *e.g.* Liu et al., Cancer Biology & Therapy 13 (2012), 123-129. As demonstrated in the Examples, this epitope is also useful for the diagnosis of several human diseases when it is quantified, for example using a sandwich ELISA. Thus, with respect to this novel FAP epitope the present invention generally relates to any agent being capable of (a) binding a FAP epitope in a peptide of 15 amino acids in length, which epitope comprises or consists of the amino acid sequence 525-PPQFDRSKKYP-535 (SEQ ID NO: 32) and (b) inhibiting protease activity of FAP and/or prolonging the clot formation time or decreasing clot rigidity of human blood plasma. As already explained *supra*, such agent may be obtained by antibody NI-206.82C2 guided selection of biotechnological or synthetic derivatives of the antibody or in screening/competition assays which employ human FAP or a correspond fragment or peptide thereof comprising the epitope as a target. Exemplary competition assay are described, for example, in international applications WO 01/68708, WO 2011/040972 and WO 2012/020006 as well as in the description further below. Thus, the nature of the agent is not confined to antibody but includes other types of compounds as well. For example, protein and peptide displays other than antibodies are investigated and provided with similar loop structures as the CDRs of antibodies but less structural requirements and/or the possibility of CDR grafting; see, *e.g.,* Nicaise et al., Protein Science 13 (2004), 1882-1891 and Hosse et al., Protein Science 15 (2006), 14-27. Furthermore, antibody-enabled small-molecule drug discovery is described, e.g., in Lawson, Nature Reviews Drug Discovery 11 (2012), 519-525. Since the epitope of antibody NI.206-18H2 - which has been subsequently isolated with antibody NI-206.82C2 as a reference - is quite close to that of antibody NI-206.82C2 and thus also lies outside the FAP catalytic triad it is prudent to expect that antibody NI.206-18H2 and its biotechnological or synthetic derivatives exhibit substantially the same properties as antibody NI-206.82C2. Indeed, as illustrated in Example 18 and illustrated in Figure 24, the anti-FAP antibody NI-206.18H2 - as well as antibodies NI-206.59B4, NI-206.27E8 and NI-206.6D3 which epitopes have not been characterized yet - display pH-dependent binding of human FAP in substantially the same manner as antibody NI-206.82C2. Therefore, in accordance with the present invention all embodiments described and disclosed for antibody NI-206.82C2 either alone or in combination equally apply to antibodies NI-206.18H2, NI-206.59B4, NI-206.27E8, and NI-206.6D3 and are subject of the present application.

[14] The antibody of any one of [1] to [13], wherein the antibody comprises a human constant region and/or comprises an Fc region or a region equivalent to the Fc region of an immunoglobulin, preferably wherein the Fc region is an IgG Fc region.

[15] The antibody of any one of [1] to [14], wherein the antibody is a full-length IgG class antibody.

[16] The antibody of any one of [1] to [15], wherein the antibody comprises a glyco-engineered Fc region and has an increased proportion of non-fucosylated oligosaccharides in the Fc region, as compared to a non-glyco-engineered antibody.
Means and methods for glyco-engineering anti-FAP antibodies are known to the person skilled in the art; see, e.g., international application WO 2012/020006, in particular Example 1 for preparation of (glyco-engineered) antibodies and Example 14 for antibody-dependent cell-mediated cytotoxicity (ADCC) mediated by glyco-engineered anti-FAP IgG1 antibodies.

[17] The antibody of any one of [1] to [16], which is chimeric human-rodent or rodentized antibody such as murine or murinized, rat or ratinized antibody, the rodent versions being particularly useful for diagnostic methods and studies in animals.

[18] The antibody of any one of [1] to [17], which is selected from the group consisting of a single chain Fv fragment (scFv), an F(ab') fragment, an F(ab) fragment, and an $F(ab')_2$ fragment.

[19] The antibody of any one of [1] to [18], wherein the antibody binds to FAP and at least one further epitope and/or target, preferably wherein the antibody is bispecific or multivalent; for example wherein the antibody is a bispecific antibody, a trifunctional antibody, a tetrabody, a bivalent single chain fragment (ScFv), a bispecific T-cell engager (BiTE), a bispecific killer cell engager (BiKE), a dual affinity retargeting molecule (DART) or a DuoBody, preferably wherein the antibody is a bispecific antibody that binds in addition to FAP to death receptor 5 (DR5) and/or CD3.

Bispecific antibody targeting of FAP in the stroma and DR5 on the tumor cell are reported to induce apoptosis despite the targets being situated on different cells (international application WO 2014/161845). Such bispecific antibodies combine a Death Receptor 5 (DR5) targeting antigen binding site with a second antigen binding site that targets FAP. By that the death receptors become cross linked and apoptosis of the targeted tumor cell is induced. The advantage of these bispecific death receptor agonistic antibodies over conventional death receptor targeting antibodies is the specificity of induction of apoptosis only at the site where FAP is expressed as well as the higher potency of these bispecific antibodies due to the induction of DR5 hyperclustering. Means and methods for preparing DR5-FAP death receptor agonistic bispecific antibody including variable heavy chain and a variable light chain amino acid sequences for the antigen binding site specific for DR5 and testing its ability to mediate apoptosis of one cell line via cross-linking by a second cell line are known to the person skilled in the art; see, e.g., international application WO 2014/161845, in particular Example 1 and subsequent Examples. In a further embodiment, the bispecific antibody of the present invention targets FAP and the CD3 antigen which is also known as CD3 receptor and activates cytotoxic T cells. The CD3 receptor comprises a CD3γ chain, a CD3δ chain, and two CD3ε chains, wherein these chains associate with the T cell receptor and the ζ-chain generating an activation signal in T lymphocytes. Means and methods for preparing such kind of bispecific antibodies are known in the art and are described, for example, in Hornig et al., J. Immunother. 35 (2012), 418-429.

The trifunctional antibody of the present invention binds preferentially to the FAP antigen and to CD3 and/or DR5 and its intact Fc-part in addition binds to an Fc receptor on accessory cells. Thus, the trifunctional antibody is preferably capable of linking T cells via CD3 (as described above) to Fc receptor expressing cells like monocytes/macrophages, natural killer cells or dendritic cells to the tumor cell, leading to its lysis. The principle underlying the construction and mechanism of trifunctional antibodies is exemplarily shown in Eissler et al., Cancer Res. 72 (2012), 3958-66. Diabodies, triabodies and tetrabodies for cancer targeting are also described in Hudson et al., Nat. Med. 9 (2003), 129-134 and Todorovska et al., J. Immunol. Methods. 248 (2001), 47-66.

In another embodiment, the scFv of the anti-FAP antibody of the present invention can be fused to a scFV that binds to the CD3ε subunit of the TCR/CD3 complex. This fusion protein serves as a soluble, injectable product that has recently been termed bispecific T-cell engager (BiTE). Bispecific T cell activating binding molecules specific for FAP have been described in international application WO 2013/026837, the embodiments described which may be adapted to the anti-FAP antibody of the present invention. The most advanced CD3 bispecific protein is Blinatumomab, a CD3/CD19 BiTE® that received FDA approval for the treatment of refractory B-precursor acute lymphoblastic leukemia (ALL); see, e.g., European patent application EP 1 077 1102 A1.

Furthermore, the antibody of the present invention can be a DART (dual affinity retargeting) molecule, which is based on a bispecific antibody but features a C-terminal bridge for additional stabilization. The principle design and mechanism of action is described, *e.g.,* in Moore et al., Blood 117 (2001), 4542-5451.

[20] A polynucleotide or polynucleotides, preferably cDNA encoding at least an antibody $V_H$ and/or $V_L$ chain that forms part of the antibody according to any one of [1] to [19].
The present invention also relates to polynucleotides encoding at least a variable region of an immunoglobulin chain of the antibody of the invention. Preferably, said variable region comprises at least one complementarity determining region (CDR) of the VH and/or VL of the variable region as set forth in any one of Figs. 1G-1K and 1A-1F. In a preferred embodiment of the present invention, the polynucleotide is a cDNA, preferably derived from mRNA obtained from human memory B cells which produce antibodies reactive with FAP.

[21] A vector or vectors comprising the polynucleotide(s) of [20], optionally operably linked to an expression control sequence.

[22] A host cell comprising the polynucleotide(s) of [20] or vector(s) of [21], wherein the polynucleotide is heterologous to the host cell.

[23] A method for preparing an anti-FAP antibody or a biotechnological or synthetic derivative thereof, said method comprising

(a) culturing the cell of [22]; and
(b) isolating the antibody from the culture.

[24] An antibody encoded by a polynucleotide(s) of [20] or obtainable by the method of [23].

[25] The antibody of any one of [1] to [19] or [24], which

(i) comprises a detectable label, preferably wherein the detectable label is selected from the group consisting of an enzyme, a radioisotope, a fluorophore and a heavy metal;
(ii) is attached to a drug, preferably a cytotoxic agent;
(iii) comprises a active biological agent, preferably wherein the active biological agent is selected from the group consisting of cytokines, chemokines, and signaling pathway mediators; and/or

(iv) comprises a radioisotope for diagnostic or therapeutic use.

Appropriate labels and drugs, in particular cytotoxic agents are known to the person skilled in the art and are described, e.g., in the patent and non-patent literature concerning FAP targeted immunotherapy and- diagnostic cited herein; see also the description which follows.

[26] A peptide, preferably 10 or 11 to 20, preferably 15 or 16 amino acids in length having an epitope of FAP specifically recognized by an antibody of any one of [4] to [12], wherein the peptide comprises or consist of an amino acid sequence as defined in [4], preferably the amino acid sequence of SEQ ID NO: 60 or a modified sequence thereof in which one or more amino acids are substituted, deleted and/or added.

Such peptide can be used as an antigen, *i.e.* being an immunogen and thus useful for eliciting an immune response in a subject and stimulating the production of an antibody of the present invention *in vivo.* Accordingly, the peptide of the present invention is particularly useful as a vaccine. For review of peptide-based cancer vaccines, see, *e.g.,* Kast et al Leukemia (2002) 16, 970-971 and Buonaguro et al., Clin. Vac. Immunol. 18 (2011), 23-34. On the other hand, such antigen may be used for the immunization of a laboratory animal in order to raise corresponding antibodies, for example for research purposes.

[27] A composition comprising the antibody of any one of [1] to [19], [24] or [25], the agent of [13], the polynucleotide(s) of [20], the vector(s) of [21], the cell of [22] or the peptide of [26], preferably wherein the composition

(i) is a pharmaceutical composition and further comprises a pharmaceutically acceptable carrier, preferably wherein the composition is a vaccine and/or comprises an additional agent useful for preventing or treating diseases associated with FAP; or

(ii) a diagnostic composition, preferably further comprising reagents conventionally used in immuno or nucleic acid based diagnostic methods.

Furthermore, the present invention relates to immunotherapeutic and immunodiagnostic methods for the prevention, diagnosis or treatment of FAP-related diseases, wherein an effective amount of the anti-FAP antibody, agent, peptide or composition of the present invention is administered to a patient in need thereof.

[28] An anti-FAP antibody of any one of [1] to [19], [24] or [25], the agent of [13], the polynucleotide(s) of [20], the vector(s) of [21], the cell of [22], the peptide of [26] or the composition of [27] for use in the prophylactic or therapeutic treatment of a disease associated with FAP, preferably selected from the group consisting of cancer such as breast cancer, colorectal cancer, ovarian cancer, prostate cancer, pancreatic cancer, kidney cancer, lung cancer, epithelial cancer, melanoma, fibrosarcoma, bone and connective tissue sarcomas, renal cell carcinoma, giant cell carcinoma, squamous cell carcinoma, adenocarcinoma, multiple myeloma; diseases characterized by tissue remodeling and/or chronic inflammation such as fibrotic diseases, wound healing disorders, keloid formation disorders, osteoarthritis, rheumatoid arthritis, cartilage degradation disorders, atherosclerotic disease and Crohn's disease; cardiovascular disorders such as atherosclerosis, stroke or an acute coronary syndrome such as myocardial infarction, heart attack, cerebral venous thrombosis, deep venous thrombosis or pulmonary embolism, vulnerable atherosclerotic plaques or atherothrombosis; disorders involving endocrinological dysfunction, such as disorders of glucose metabolism; and blood clotting disorders.

As demonstrated in Examples 16 and 17 and illustrated in Figures 21, 22 and 23 the anti-FAP antibody of the present invention is capable of prolonging arterial occlusion times and thrombosis in a murine thrombosis model and abrogating orthotopic tumor growth in a syngeneic colorectal cancer mouse model. Thus, based on the experiments performed in accordance with the present invention and FAP's known role in (patho-)physiology, documented extensively in the literature, for example the documents cited in the background section on the one hand, it is reasonable and credible to envisage potential applications of anti-FAP antibodies, epitopes and agents of the present invention in FAP-related disorders and diseases characterized by: (a) proliferation (including but not limited to cancer); (b) tissue remodeling and/or chronic inflammation (including but not limited to fibrotic disease, chronic liver disease, wound healing, keloid formation, osteoarthritis, rheumatoid arthritis and related disorders involving cartilage degradation); (c) endocrinological disorders (including but not limited to disorders of glucose metabolism); (d) cardiovascular diseases (including but not limited to thrombosis, atherosclerosis, stroke, myocardial infarction, heart attack, vulnerable atherosclerotic plaques and atherothrombosis); and (e) diseases involving blood clotting disorders; for possible therapeutic and diagnostic applications see also the documents referred to herein.

[29] A method of preparing a pharmaceutical composition for use in the treatment of a FAP-related disorder as defined in [28], the method comprising:

(a) culturing the cell of [22];

(b) purifying the antibody, biotechnological or synthetic derivative or immunoglobulin chain(s) thereof from the culture to pharmaceutical grade; and

(c) admixing the antibody or biotechnological or synthetic derivative thereof with a pharmaceutically acceptable carrier.

Further therapeutic and diagnostic methods as well as formulation of corresponding compositions which may be employed for the anti-FAP antibody, agent, peptide and composition of the present invention will be apparent to the person skilled in the art from the literature relating to the treatment and diagnosis of FAP-related diseases cited herein, e.g., international application WO 2012/020006. For example, FAP for use a novel therapeutic and diagnostic target in cardiovascular diseases is disclosed international application WO 2012/025633.

In addition, with respect to the inhibitory anti-FAP antibodies and equivalent FAP-binding agents therapeutic and diagnostic utility can be envisaged described for cleaving enzyme (APCE). APCE is reported to be a soluble isoform or derivative of FAP, the latter being a type II integral membrane protein, which is predicted to have its first six N-terminal residues within fibroblast cytoplasm, followed by a 20-residue transmembrane domain, and then a 734-residue extracellular C-terminal catalytic domain. Like FAP, APCE is also a prolyl-specific enzyme that exhibits both endopeptidase and dipeptidyl peptidase activities. It has also been reported that FAP and APCE are essentially identical in amino acid sequence, except that APCE lacks the first 23 amino terminal residues of FAP, but otherwise the two molecules have essentially identical physico-chemical properties; see, *e.g.,* Lee et al., Blood 107 (2006), 1397-1404, international application WO 2004/072240 and US patent application US 2011/0144037 A1, in particular paragraph [0009] and paragraphs [0095] ff. for utility of inhibitors of FAP/APCE.

As demonstrated in Example 19 and shown in Figure 25 antibody NI-206.82C2 is a reliable *in vivo* imaging agent of cancer which accumulates selectively in the tumor stroma over time versus a biologically inactive isotype-matched control antibody. As mentioned, since anti-FAP antibody NI-206.18H2 as well as antibodies NI-206.59B4, NI-206.27E8 and NI-206.6D3 display pH-dependent binding of human FAP in substantially the same manner as antibody NI-206.82C2, it is prudent to expect that they exhibit the same biological activity towards a tumor *in vivo.*

[30] A FAP-binding molecule comprising at least one CDR of an antibody of any one of [1] to [12], [14] to [19], [24] or [25] for use in *in vivo* detection or imaging of or targeting a therapeutic and/or diagnostic agent to a FAP expressing cell or tissue thereof in the human or animal body, preferably wherein said *in vivo* imaging comprises scintigraphy, positron emission tomography (PET), single photon emission tomography (SPECT), near infrared (NIR), optical imaging or magnetic resonance imaging (MRI).

[31] An *in vitro* method of diagnosing whether a subject suffers from a disease associated with FAP as defined in [28] or whether a subject is amenable to the treatment with a FAP-specific therapeutic agent, the method comprising determining in a sample derived from a body fluid of the subject, preferably blood the presence of FAP, wherein an elevated level of FAP compared to the level in a control sample from a healthy subject is indicative for the disease and possibility for the treatment with the agent, wherein the method is characterized in that the level of FAP is determined by way of detecting an epitope of FAP comprising or consisting of the amino acid sequence of any one of SEQ ID NOS: 30 to 32 or 60.

As demonstrated in Example 15 and illustrated in Figures 17-20 a novel assay for assaying FAP in a body fluid, in particular blood has been developed based on the novel epitope of subject antibody NI-206.82C2 of the present invention. In previous international application WO 2012/025633, the blood test is different in that unknown FAP epitopes were measured, whereas the new assay specifically measures the FAP epitope "525-PPQFDRSKKYP-535" (SEQ ID NO: 32). In WO 2012/025633 a rabbit polyclonal antibody against FAP (Ab28246; Abcam, Cambridge, MA) has been used as the capture antibody and F19 as the detection antibody. The binding epitopes for both of these antibodies are not known. For the novel assay of the present invention F19 antibody may be used as the capture antibody and NI-206.82C2 or an equivalent anti-FAP antibody as the detection antibody to specifically quantify levels of the FAP epitope SEQ ID NO: 32. Quantifying the FAP epitope SEQ ID NO: 32 for diagnostics delivers unexpected and clinically valuable information. This information is unexpected, because other FAP blood tests published to date which measure various other FAP epitopes actually report that circulating FAP levels are lower in cancer patients versus healthy control patients; see, *e.g.,* Javidroozi et al., Disease Markers 32 (2012), 309-320; Tillmanns et al., International Journal of Cardiology 168 (2013), 3926-3931 and Keane et al., FEBS Open Bio 4 (2014), 43-54. However - quite unexpectedly - the novel assay of the present invention shows that levels of the FAP-specific epitope SEQ ID NO: 32 is increased in patients with cancer and cardiovascular disease (Figures 18-20). Without intending to be bound by theory this unexpected result is explained by the possibility that various forms of FAP exist in human blood (e.g. complexed, truncated, monomers, dimers, etc.) with different biological roles and different value as diagnostic and therapeutic targets. While FAP assays described in the prior art do not seem to specify a certain epitope or quantify an appropriate epitope for which reason a reliable method for the prediction of FAP-related diseases such as cancer had not been established, the information provided is clinically valuable because patients with high levels of the SEQ ID NO: 32 epitope are expected to be at an increased risk of a clinical event (*e.g.* cancer, heart attack, stroke, or clotting disorder), and these same patients with elevated epitope levels are also expected to benefit most from receiving FAP-targeted medication, preferably antibody NI-206.82C2 a biotechnological or synthetic variant thereof or equivalent FAP-binding agent which binds to the SEQ ID NO: 32 epitope. As described in Example 14 and illustrated in Figure 17, the FAP detection assay of the present invention is specific to rhFAP since SB9 oligopeptidase homologues rhDDP4, rhDPP8, rhDPP9, and rhPOP/PREP gave no signal, *i.e.* increase of OD. Furthermore, as illustrated in Figures 18 to 20, FAP detection assay of the present invention is particular suitable for the detection of metastatic colorectal cancer (MCRC), coronary artery disease (CAD) and ST Segment Elevation Myocardial Infarction (STEMI), carotid plaques in a patient. In view of similar binding properties of antibody NI-206.82C2 and NI-206.18H2 and close location of their epitopes this embodiment as well as the following embodiments may be equally performed with the FAP epitope of antibody NI-206.18H2 "501-IQLPKEEIKKL-511" (SEQ ID NO: 60).

[32] A therapeutic agent for use in the treatment of a patient suffering from or being at risk of developing a disease associated with FAP as defined in [28], characterized in that a sample of the patient's blood, compared to a control shows an elevated level of FAP as determined by detecting an epitope of FAP consisting of or comprising the amino acid sequence of any one of SEQ ID NOS: 30 to 32 or 60, preferably wherein the patient has been diagnosed in accordance with the method of [31].

[33] The method of [31] or the agent for use according to [32], wherein the level of FAP is determined by subjecting the sample to an anti-FAP antibody and detecting the presence of the complex formed between FAP and the antibody, preferably by Sandwich ELISA.
As described in Example 14, the sandwich-type immunoassay format (=sandwich immunoassay or ELISA) is particular preferred. Sandwich immunoassay formats are well known to the person skilled in the art and have also been described for the detection of FAP; see, e.g., international applications WO 2009/074275, WO 2010/127782 and WO 2012/025633. In this, context, detecting an epitope of FAP comprising or consisting of the amino acid sequence of any one of SEQ ID NOS: 30 to 32 is preferably performed with an anti-FAP antibody of the present invention, *e.g*. antibody NI-206.82C2 or a biotechnological or synthetic derivative thereof as the detection antibody and anti-FAP antibody F19 or a derivative thereof as the capture antibody. However, the assay may be performed vice versa. Instead of antibody F19 or derivatives thereof further anti-FAP antibodies may be used, for example rat monoclonal anti-FAP/Seprase antibodies (clones D8, D28 and D43); see Pineiro-Sanchez et al., J. Biol. Chem. 12 (1997), 7595-7601 and international application WO 2009/074275.

[34] An anti-FAP antibody for use in the treatment of blood clotting disorders or use of an anti-FAP antibody for slowing coagulation of blood *in vitro.*
As demonstrated in Examples 13 and 14 and illustrated in Figures 15 and 16, the anti-FAP antibody NI-206.82C2 interferes with the clotting cascade and prolong blood clotting time, thus meeting the definition of an anticoagulant and pro-thrombotic agent, respectively. Accordingly, possible therapeutic uses of the anti-FAP antibody NI-206.82C2 and NI-206.18H2 and their biotechnological and synthetic derivatives as well as equivalent FAP-binding agents include but are not limited to the treatment, amelioration and prevention of thrombotic disorders in general, atrial fibrillation (fast irregular heartbeat), disorders associated with a mechanical heart valve, endocarditis (infection of the inside of the heart), mitral stenosis (one of the valves in the heart does not fully open), certain blood disorders that affect how blood clots (inherited thrombophilia, antiphospholipid syndrome), disorders associated with surgery to replace a hip or knee. Furthermore, anti-FAP antibody of the present invention and equivalent agents may be used in medical equipment, such as test tubes, blood transfusion bags, and renal dialysis equipment. The anticoagulant activity of an anti-FAP antibody or equivalent FAP-binding agent can be determined by subjecting a candidate anti-FAP antibody to a clot formation assay with a sample derived from blood, preferably human blood, wherein a prolonged blood plasma clotting time, decreased clotting rate, decreased clot elasticity, and/or decreased clot rigidity compared to a sample subjected to an isotyped-matched control antibody is indicative for the anticoagulant activity of the anti-FAP antibody and agent, respectively, preferably wherein clot formation is determined by thromboelastography such as rotational thromboelastometry (ROTEM™); see also Example 13. In addition, or alternatively the anti-FAP antibody or agent may be tested for ability to reduce the rate of FAP substrate alpha 2 anti-plasmin ($\alpha$2AP-AMC) cleavage in human plasma as described in Example 14.

[35] The method or the agent for use according to [33], the anti-FAP antibody for use according to [34], or the use of [34], wherein the antibody is an antibody of any one [1] to [19], [24] or [25].

[36] A kit useful in a method of any one of [31], [33] or [35] or in the use of [34] or [35], the kit comprising at least one antibody of any one of [1] to [19], [24] or [25], the agent of [13], the polynucleotide(s) of [20], the vector(s) of [21], the cell of [22], the peptide of [26] or the composition of [27], optionally with reagents and/or instructions for use.

[37] A pharmaceutical package or article of manufacture comprising (i) means for performing the method of any one of [31], [32] or [35], preferably any one of the components of the kit of [36] and (ii) a FAP-targeting drug, preferably a therapeutic agent for use according to [31], [33] or [35], optionally with instructions for use.
In practice, it can be expected that the medication with FAP-targeting drugs, in particular anti-FAP antibody NI-206.82C2 and its biotechnological and synthetic derivatives as well as equivalent FAP-binding agents will most often be combined with the method and assay [29], *supra* and described in the Examples that quantifies the epitope "525-PPQFDRSKKYP-535" or the FAP epitope of antibody NI-206.18H2 "501-IQLPKEEIKKL-511" (SEQ ID NO: 60); see also *supra*. Preferably, the assay is performed as a sandwich ELISA-based blood test using an NI-206.82C2 derived antibody or agent as the detection antibody, and specifically measuring the amount of unbound epitope (or "drug target") which NI-206.82C2 will bind in the patient once the medication is injected. Therefore the assay of the present invention, preferably in the form of a blood test will identify which patients to treat (*i.e.* patients with high levels of the drug target) and how to dose the medication (*i.e.* specifically according to each patient's personal levels of the "525-PPQFDRSKKYP-535" or "501-IQLPKEEIKKL-511" epitope). Therefore, advantageously the FAP-targeting drug, in particular anti-FAP antibody NI-206.82C2 and NI-206.18H2, respectively, and its biotechnological and synthetic derivatives as well as equivalent FAP-binding agents are designed to be used together with the novel FAP detection assay of the present invention, for example as a clinical package, combining components necessary and sufficient to perform the assay and/or instructions for doing so. In addition, it is prudent to expect that using the FAP detection assay of the present invention in the assessment of FAP serum level in statistically significant population of representative subjects and patients, respectively, the present invention reference levels will be established which generally provide for the medical setting, *e.g.* dosing the FAP-binding agent.

[38] The invention as described herein, especially refers to the appended Examples and antibodies which show substantially the same binding and biological activities as any antibody selected from NI-206.18H2, NI-206.20A8, NI-206.6D3, NI-206.14C5, NI-206.34C11, NI-206.82C2, NI-206.59B4, NI-206.22F7, NI-206.27E8, NI-206.12G4, and NI-206.17A6. The anti-FAP antibody can also be altered to facilitate the handling of the method of diagnosing including the labeling of the antibody as described in detail below.

[39] A chimeric antigen receptor (CAR), wherein the antigen is fibroblast activation protein (FAP) and a first receptor comprises a variable region or binding domain derived from the anti-FAP antibody of any one of [1] to [19], preferably wherein the CAR or a cell expressing the CAR exhibits a higher avidity of binding to FAP or cell expressing FAP on its cell surface under acidic pH as compared to neutral or physiological pH, preferably wherein the acidic pH is 6.4 or 6.8 and the physiological pH is 7.4.
In a further aspect, the present invention relates to chimeric antigen receptors (CARs) specific for FAP, which are based on the anti-FAP antibodies of the present invention and particularly useful for targeting stromal cells in the treatment of cancer. A CAR targeting FAP is referred to herein as a FAP CAR. The FAP CARs of the present invention have certain advantages over prior art cancer treatments in that antibody conjugates can have limited tumor penetration and often induce an immune response in the host, and vaccination may lead to long lasting endogenous immunity to FAP. The present CARs, especially in combination with T cells (CAR T cells) are designed to circumvent these limitations. In general, CARs are molecules that combine antibody-based specificity for a desired antigen with a T cell receptor-activating intracellular domain to generate a chimeric protein that exhibits a specific anti-antigen cellular immune activity. The CARs of the present invention comprise an anti-FAP antibody, or an FAP binding fragment thereof. In particular, the CAR comprises a first receptor comprising a variable region or binding domain derived from any one of the anti-FAP antibodies mentioned *supra*. In a preferred embodiment of the present invention, the antigen binding domain specific for FAP is derived from any one of the antibodies of the present invention, which exhibits pH-dependent binding of FAP, *i.e.* increased avidity in an acidic and thus tumor environment. Most preferably, the FAP CAR of the present invention makes use of antibody NI-206.82C2 or NI-206.18H2; see Example 20. Hence, the present invention generally relates to FAP CARs which are substantially non-immunogenic in human inter alia due to the human origin of the anti-FAP antibody and/or wherein preferably the CAR or a cell expressing the CAR exhibits a higher avidity of binding to FAP or cell expressing FAP on its cell surface under acidic pH as compared to neutral or physiological pH, preferably wherein the acidic pH is 6.4 or 6.8 and the physiological pH is 7.4; see also the Examples.

[40] The CAR of [39], wherein said first receptor or a second receptor comprises at least one further antigen binding domain, preferably wherein said further antigen binding domain is specific for a CD3 receptor or a death receptor, preferably wherein the death receptor is the death receptor 5 (DR5).

[41] The CAR of [39] or [40], comprising an extracellular domain (receptor), a transmembrane domain and an intracellular signaling domain (endo- or cytoplasmic domain), preferably wherein the intracellular signaling domain comprises the 4-1BB costimulatory domain and/or CD28 costimulatory domains and/or the CD3ζ (zeta) endodomain.

In one embodiment, the CAR of the present invention comprises an extracellular domain having an antigen recognition domain, *i.e*. the first receptor specific for FAP, a transmembrane domain and a cytoplasmic domain, *i.e*. the second receptor specific for the CD3ζ (zeta) endodomain. In one embodiment, the transmembrane domain that naturally is associated with one of the domains in the CAR is used. In another embodiment, the transmembrane domain can be selected or modified by amino acid substitution to avoid binding of such domains to the transmembrane domains of the same or different surface membrane proteins to minimize interactions with other members of the receptor complex. Preferably, the transmembrane domain comprises the CD8 hinge domain.

With respect to the cytoplasmic domain, the CAR of the invention can be designed to comprise the CD28 and/or 4- IBB signaling domain by itself or be combined with any other desired cytoplasmic domain(s) useful in the context of the CAR of the invention. In one embodiment, the cytoplasmic domain of the CAR can be designed to further comprise the signaling domain of CD3-zeta. For example, the cytoplasmic domain of the CAR can include but is not limited to CD3-zeta, 4- IBB and CD28 signaling modules and combinations thereof.

In one embodiment, the costimulatory signaling region in the CAR comprises the intracellular domain of a costimulatory molecule selected from the group consisting of CD27, CD28, 4-1BB, OX40, CD30, CD40, PD- 1, ICOS, lymphocyte function-associated antigen- 1 (LFA- 1), CD2, CD 7, LIGHT, NKG2C, B7- H3, a ligand that specifically binds with CD83, and any combination thereof.

[42] A polynucleotide or polynucleotides, preferably a cDNA or cDNAs encoding the CAR of any one of [39] to [41] and optionally the antibody of any one of [1] to [19].

The present invention also relates to polynucleotides encoding the CAR of the present invention and additionally at least a variable region of an immunoglobulin chain of the antibody of the invention. Preferably, said variable region comprises at least one complementarity determining region (CDR) of the VH and/or VL of the variable region as set forth in any one of Figs. 1G-1K and 1A-1F. In a preferred embodiment of the present invention, the polynucleotide is a cDNA.

[43] A vector or vectors comprising the polynucleotide(s) of [42], optionally operably linked to an expression control sequence.

[44] A host cell genetically modified to express the CAR of any one of [39] to [41], wherein the host cell comprises the polynucleotide(s) of [42] or the vector(s) of [43], and optionally expresses and secretes the antibody of any one of claims [1] to [19].

[45] The cell of [44], which is a T cell, a cytotoxic T lymphocyte (CTL), a natural killer cell, a hematopoietic stem cell (HSC), an embryonic stem cell, or a pluripotent stem cell, preferably wherein the cell is a T cell (CAR T cell), preferably wherein the cell exhibits a higher avidity of binding to FAP or cell cell expressing FAP on its surface under acidic pH as compared to neutral or physiological pH, preferably wherein the acidic pH is 6.4 or 6.8 and the physiological pH is 7.4.

The present invention generally relates to a host cell modified to express the FAP CAR of the present invention and optionally expresses and secretes additionally the antibody of the present invention, preferably the NI-206.18H2 or the NI-206.82C2 antibody or a further FAP CAR of the present invention. The additional expression of an antibody or a FAP CAR is performed to *e.g.* enhance tumor targeting and/or tumor selectivity.

In one embodiment, the host cell of the present invention is a T cell, such as a primary T cell, an autologous T cell, a naive T cell, a central memory T cell, an effector memory T cell or a regulatory T cell, a cytotoxic T lymphocyte (CTL), a natural killer cell, a hematopoietic stem cell (HSC), an embryonic stem cell, or a pluripotent stem cell. Preferably, the T cell is an isolated primary human T cell isolated from a human donor. Furthermore, the T cell can be modified to functionally impair or to reduce expression of the endogenous T cell receptor (TCR). In particular, an isolated modified primary human T cell, which is derived form a primary human T cell isolated form a human is modified (i) to reduce expression of the endogenous T cell receptor (TCR) and is further modified (ii) to express at least one functional exogenous non-TCR that comprises a chimeric receptor comprising a ligand binding domain attached to a signaling domain, wherein said modified primary human T cell is suitable for use in human therapy, and further wherein said modified isolated primary human T cell elicits no or a reduced graft-versus-host disease (GVHD) response in a histoincompatible human recipient as compared to the GVHD response elicited by a primary human T cell isolated from the same human donor that is only modified as in (ii); see, *e.g.,* international application WO 2011/059836.

T cells expressing a CAR are referred to herein as CAR T cells or CAR modified T cells. In one embodiment, the invention relates to genetically modified T cells expressing the FAP CAR for use in the treatment of a patient with cancer. The present invention is based upon the finding that the anti-FAP antigen binding domain of the anti-FAP antibodies of the present invention are particular suited for use in a CAR allowing specific recognition of FAP expressing tumor cells and tumor reduction, wherein the T cell exhibits an anti-tumor immunity when the FAP binding domain binds to human FAP, *e.g.,* release of cytokines upon binding of CAR T cells to the target epitope leading to damage of cancer cells and its environment is one mechanism of anti-tumor effects of CAR T cells. Accordingly, the invention provides CAR T cells and methods of their use for adoptive therapy. In Example 22 it is illustrated how the specific binding of FAP CAR T cells to coated recombinant FAP and the release of effector cytokines can be determined.

[46] A method for generating a CAR expressing T cell (CAR T cell), said method comprising

(a) culturing a cell of [44] or [45]; and
(b) isolating said CAR T cell.

In one embodiment, the CAR T cells of the invention can be generated by introducing a lentiviral vector comprising the FAP CAR of the present invention into the cells. Thus, the CAR T cells of the invention are able to replicate *in vivo* resulting in long-term persistence that can lead to sustained tumor control. In another embodiment, the CAR T cells of the invention can be generated by transfecting an RNA encoding the FAP CAR of the present invention into the cells leading to a transient expression of the CAR in the genetically modified CAR T cells. A detailed exemplary production method for CAR T cells in given in Example 21.

[47] A CAR T cell obtainable by the method of [46]
Furthermore, the present invention relates to immunotherapeutic methods for the prevention or treatment of FAP-related diseases, especially tumor diseases, wherein an effective amount of the CAR T cell of the present invention is administered to a patient in need thereof. In particular, the CAR, the polynucleotide, the vector or the cell, preferably the CAR T cell can be used in the treatment of tumors expressing the FAP antigen, preferably wherein the treatment causes tumor regression by ablation of FAP-positive malignant cells and/or FAP-positive stromal cells with or without chemotherapy, and/or increases chemotherapy uptake and/or increases T-cell infiltration by ablation of FAP-positive stromal cells.
[48] The CAR of any one of [39] to [41], the polynucleotide(s) of [42], the vector(s) of [43], the cell of [44] or [45] or the CAR T cell of [47] for use in the prophylactic or therapeutic treatment of a disease associated with FAP, preferably selected from the group consisting of cancer such as breast cancer, colorectal cancer, ovarian cancer, prostate cancer, pancreatic cancer, kidney cancer, lung cancer, epithelial cancer, melanoma, fibrosarcoma, bone and connective tissue sarcomas, renal cell carcinoma, giant cell carcinoma, squamous cell carcinoma, adenocarcinoma, multiple myeloma; diseases characterized by tissue remodeling and/or chronic inflammation such as fibrotic diseases, wound healing disorders, keloid formation disorders, osteoarthritis, rheumatoid arthritis, cartilage degradation disorders, and Crohn's disease.
As mentioned above, the pH of solid tumors is acidic due to increased fermentative metabolism and since the antibodies of the present invention, in particular the antibodies NI-206.82C2, NI206.18H2, NI.206-59B4, NI206.27E8 and NI206.6D3 surprisingly revealed increased avidity to transmembrane FAP in the acidic pH environment found in tumors, compared to lower avidity at the neutral pH of healthy tissue it is reasonable to expect that the CARs comprising a variable region or binding domain derived from any of those antibodies also show this increased avidity to FAP in the acidic pH. Experiments allowing the determination of FAP CAR binding to human FAP-expressing cells and release of cytokines under normal and low pH conditions are exemplary described in Examples 23 to 25.
[49] The CAR of any one of [39] to [41], the polynucleotide(s) of [42], the vector(s) of [43], the cell of [44] or [45] or the CAR T cell of [47] for use

(i) in the treatment of tumors expressing the FAP antigen, preferably wherein the treatment

(a) causes tumor regression by ablation of FAP-positive malignant cells and/or FAP-positive stromal cells with or without chemotherapy; and/or
(b) increases chemotherapy uptake and/or increases T-cell infiltration by ablation of FAP-positive stromal cells.

Further embodiments of the present invention will be apparent from the description and Examples that follow.

BRIEF DESCRIPTION OF THE DRAWINGS

**Fig. 1:** Amino acid sequences of the variable regions of exemplary human NI-206.82C2, NI-206.59B4, NI-206.22F7, NI-206.27E8, NI-206.12G4, NI-206.17A6, NI-206.18H2, NI-206.20A8, NI-206.6D3, NI-206.14C5 and NI-206.34C11 antibodies. Framework (FR) and complementarity determining regions (CDRs) are indicated with the CDRs being underlined. The Kabat numbering scheme was used (cf. http://www.bioinf.org.uk/abs/).

**Fig. 2:** Specific binding to FAP of the recombinant human-derived antibodies assessed by ELISA and $EC_{50}$ determination.

**(A)** Plates were incubated with the indicated concentrations of recombinant human-derived antibodies. Exemplary antibody NI-206.82C2 binds with high affinity to rFAP (●) that was captured via its his-tag with a coated anti-His antibody. The antibody NI-206.82C2 does not bind to BSA (A). The data are expressed as OD values at 450 nm.
**(B)** Plates were incubated with the indicated concentrations of recombinant human-derived antibodies. Exemplary antibody NI-206.82C2 binds with good affinity to rFAP (●) that was directly coated onto the ELISA plates. The antibody NI-206.82C2 does not bind to BSA (▲). The data are expressed as OD values at 450 nm.
**(C)** Plates were incubated with the indicated concentrations of recombinant human-derived antibodies. Exemplary antibody NI-206.59B4 binds with high affinity to rFAP (●) that was captured via its his-tag with a coated anti-His antibody. The antibody NI-206.59B4 does not bind to BSA (A). The data are expressed as OD values at 450 nm.
**(D)** Plates were incubated with the indicated concentrations of recombinant human-derived antibodies. Exemplary antibody NI-206.59B4 binds with good affinity to rFAP (●) that was directly coated onto the ELISA plates. The antibody NI-206.59B4 does not bind to BSA (A). The data are expressed as OD values at 450 nm.
**(E)** Plates were incubated with the indicated concentrations of recombinant human-derived antibodies. Exemplary antibody NI-206.18H2 binds with good affinity to rFAP (●) that was captured via its his-tag with a coated anti-His antibody. The antibody NI-206.18H2 does not bind to anti-His antibody with BSA (A). The data are expressed as OD values at 450 nm.
**(F)** Plates were incubated with the indicated concentrations of recombinant human-derived antibodies. Exemplary antibody NI-206.18H2 binds with good affinity to rFAP (●) that was directly coated onto the ELISA plates. The antibody NI-206.18H2 does not bind to BSA (A).The data are expressed as OD values at 450 nm.
**(G)** Plates were incubated with the indicated concentrations of recombinant human-derived antibodies. Exemplary antibody NI-206.20A8 binds with good affinity to rFAP (●) that was captured via its his-tag with a coated anti-His antibody. The antibody NI-206.20A8 does not bind to anti-His antibody with BSA (A). The data are expressed as OD values at 450 nm.
**(H)** Plates were incubated with the indicated concentrations of recombinant human-derived antibodies. Exemplary antibody NI-206.20A8 binds with good affinity to rFAP (●) that was directly coated onto the ELISA plates. The antibody NI-206.20A8 does not bind to BSA (A).The data are expressed as OD values at 450 nm.
**(I)** Plates were incubated with the indicated concentrations of recombinant human-derived antibodies. Exemplary antibody NI-206.6D3 binds with good affinity to rFAP (●) that was captured via its his-tag with a coated anti-His antibody. The antibody NI-206.6D3 does not bind to anti-His antibody with BSA (A). The data are expressed as OD values at 450 nm.
**(J)** Plates were incubated with the indicated concentrations of recombinant human-derived antibodies. Exemplary antibody NI-206.6D3 binds with good affinity to rFAP (●) that was directly coated onto the ELISA plates. The antibody NI-206.6D3 does not bind to BSA (A).The data are expressed as OD values at 450 nm.
**(K)** The $EC_{50}$ values for the antibodies NI-206.82C2, NI-206.59B4, NI-206.22F7, NI-206.27E8, NI-206.12G4, NI-206.17A6, NI-206.18H2, NI-206.20A8 andNI-206.6D3 were estimated by a non-linear regression using GraphPad Prism software. The values for sFAP correspond to the measurements done with the ELISA plates where FAP was captured via its his-tag with a coated anti-His antibody. The values for FAP correspond to the measurements done with the ELISA plates where FAP was directly coated. The values for cFAP correspond to the measurements done with the ELISA plates where a mixture of FAP-specific peptides (378-HYIKDTVENAIQITS-392, 622-GWSYGGYVSSLALAS-636 and 721-QVDFQAMWYSDQNHGL-736) was directly coated. N/A stands for not applicable, the antibody did not show binding and the EC50 could therefore not be determined. The binding of the antibody NI-206.12G4 towards sFAP was not tested.

**Fig. 3:** Kinetic analysis of NI-206.82C2 on ProteOn™ analysis. Antibody was injected as a five-membered serial dilution starting at 16, 8, 4, 2 and 1 μg/ml, respectively, and analyzed in a single injection over three differing capacity reaction surfaces, one of which is shown.

**Fig. 4:** FAP binding epitopes of human-derived recombinant antibodies assessed by pepscan analysis.

**(A)** Pepscan image of recombinant NI-206.82C2 human-derived antibody (1 μg/ml). NI-206.82C2 binding occurred at peptides 131 and 132 (line G, 11th and 12th spot) covering amino acids 525-535 (peptide 131: 521-KMILPPQFDRSKKYP-535, peptide 132: 525-PPQFDRSKKYPLLIQ-539, consensus binding sequence: PPQFDRSKKYP); and at peptides 28 and 29 (line B, $8^{th}$ and $9^{th}$ spot) covering amino acids 113-123 (peptide 28: 109-RQFVYLESDYSKLWR-123, peptide 29: 113-YLESDYSKLWRYSYT-127, consensus binding sequence: YLESDYSKLWR)
**(B)** Pepscan image of secondary HRP-conjugated donkey anti-human IgG Fcγ only (1:20,000; secondary antibody only) was used as a specificity control;
**(C)** Pepscan image of recombinant NI-206.18H2 human-derived antibody (10 μg/ml). NI-206.18H2 binding occurred at peptides 125 and 126 (line G, 5th and 6th spot) covering amino acids 501-511 (peptide 125: 497-ALKNIQLPKEEIKKL-511, peptide 126: 501-IQLPKEEIKKLEVDE-515, consensus binding sequence: IQLPKEEIKKL);
**(D)** Pepscan image of secondary HRP-conjugated donkey anti-human IgG Fcγ only (1:20,000; secondary antibody only) was used as a specificity control;
**(E)** Identified binding epitopes of the different human-derived FAP-specific antibodies within the indicated amino acids of the FAP protein sequence.

**Fig. 5:** Inhibition FAP enzymatic activity with recombinant human monoclonal antibodies. Inhibition of recombinant human FAP gelatinase activity by NI-206.82C2 **(A),** NI-206.59B4 **(B),** NI-206.22F7 **(C),** NI-206.27E8 **(D),** NI-206.12G4 **(E),** NI-206.17A6 **(F).** Table summarizing human antibody inhibition characteristics **(G).**

**Fig. 6:** Mechanism of NI-206.82C2 inhibition of rhuFAP-mediated PEP (Z-Gly-Pro-AMC) cleavage. PEP-cleavage (measured as emission at 450nM) by active recombinant human FAP by 0, 10, 100, 1000nM NI-206.82C2 at different PEP fluorogenic substrate (Z-Gly-Pro-AMC) concentrations: 100μM **(A),** 80μM **(B),** 70μM **(C),** 60μM **(D),** 50μM **(E),** 40μM **(F),** 30μM (G), and 20μM **(H).** A velocity vs. [substrate] plot (I) and Lineweaver-Burk plot **(J)** are shown, which suggest that NI-206.82C2 has the characteristic properties of a non-competitive inhibitor of FAP-mediated PEP cleavage.

**Fig. 7:** Human-derived recombinant antibody selectively binds FAP.

**(A)** NI-206.82C2 used as a detection antibody at concentration 20, 4, and 0.8 nM results in a significantly greater colorimetric signal (OD450nM) against recombinant human FAP that was captured via its his-tag with a coated anti-His antibody (sFAP), versus CD26, and a panel of additional unrelated human antigens (A-N) using sandwich ELISA.
**(B)** NI-206.18H2 used as a detection antibody at concentration 20, 4, and 0.8 nM results in a significantly greater colorimetric signal (OD450nM) against recombinant human FAP that was captured via its his-tag with a coated anti-His antibody (sFAP) or recombinant human FAP that was directly coated onto the ELISA plates (FAP) versus a panel of additional unrelated human antigens (A-N).
**(C)** NI-206.20A8 used as a detection antibody at concentration 20, 4, and 0.8 nM results in a significantly greater colorimetric signal (OD450nM) against recombinant human FAP that was captured via its his-tag with a coated anti-His antibody (sFAP) or recombinant human FAP that was directly coated onto the ELISA plates (FAP) versus a panel of additional unrelated human antigens (A-N).
**(D)** NI-206.6D3 used as a detection antibody at concentration 20, 4, and 0.8 nM results in a significantly greater colorimetric signal (OD450nM) against recombinant human FAP that was captured via its his-tag with a coated anti-His antibody (sFAP) or recombinant human FAP that was directly coated onto the ELISA plates (FAP) versus a panel of additional unrelated human antigens (A-N).
**(E)** Affinity (EC50) assays using sandwich ELISA reveal that NI-206.82C2 selectively binds to recombinant human FAP (rhFAP) but not FAP SB9 oligopeptidase homologues (rhDPPIV, rhPOP/PREP, rhDPP8, and rhDPP9).
**(F)** Inhibition assays reveal that NI-206.82C2 selectively inhibits rhFAP, but not FAP homologues.

**Fig. 8:** NI-206.82C2 inhibits the enzymatic activity active recombinant human FAP and active recombinant mouse FAP. NI-206.82C2 demonstrate a higher potency for inhibiting active recombinant human FAP **(A)** and active recombinant mouse FAP **(B)** compared to previously tested FAP-targeting agents Val-boro-Pro, and F19 (*i.e.* the murine F19 monoclonal antibody of which Sibrotuzumab is the humanized version having subsattially the same binding affinity; see the description in the background section, supra).

**Fig. 9:** NI-206.82C2 binding to human carcinoma tissue sections. NI-206.82C2 specifically binds to human invasive ductal carcinoma **(A)** and invasive lobular carcinoma **(B)** sections by confocal immunofluorescence. 3A1 is a human isotype control antibody and DAPI counterstains cell nuclei.

**Fig. 10:** NI-206.82C2 staining of human invasive ductal carcinoma tissue. Positive staining (DAB, brown) is observed in the tissue section staining with NI-206.82C2 **(D, E, F)** but not in tissue stained with the 43A11 human isotype control antibody **(A, B, C).** Nuclei are counterstained blue with hematoxylin blue. NI-206.82C2 shows extracellular staining in the perimeter (closed arrows F) of a malignant tumor (+) versus no extracellular staining in the surrounding connective tissue (shown by *, Figure F). Positive staining is also observed in the cytoplasm of cells in the surrounding tissue (shown with open arrows, **F**).

**Fig. 11:** NI-206.82C2 staining of human ductal carcinoma in-situ tissue. Positive staining (DAB, brown) is observed in the tissue section staining with NI-206.82C2 **(B, D, F, H)** but not in an adjacent tissue section stain with isotype control antibody 43A11 **(A, C, E, G).** Nuclei and counterstained blue with hematoxylin. NI-206.82C2 shows elevated binding to a malignant "remodeling" DCIS tumor (+) vs. weaker staining in a large non-malignant "encapsulated" DCIS tumor (shown by *). Encapsulation of the large tumor (+) can be seen in A and B. No staining is observed from the 43A11 isotype control antibody **(E).** NI-206.82C2 staining is the highest on the outer perimeter of the smaller malignant tumor (shown with closed arrows, **F**). Connective tissue surrounding the tumor tissue (shown by $\tau$ in **G** and **H**) is negative with the exception of cell cytoplasm (shown with open arrows, **H**).

**Fig. 12:** NI-206.82C2 binding to murine CT-26 colorectal cancer tissue sections. NI-206.82C2 staining (red) is found around the perimeter of green fluorescent protein (GFP) transfected CT-26 syngeneic liver metastasis (green). Cell nuclei are shown in cyan (DAPI).

**Fig. 13:** NI-206.82C2 binds to multiple myeloma cells and tumor stroma in the syngeneic MOPC315.BM mouse model. **(A)** H&E staining of femur of MOPC315.BM challenged mice upon development of paraplegia. Massive plasma cell expansion is observed throughout the bone marrow. Immunofluorescence staining with NI-206.82C2 and anti-CD138 (plasma cell marker) on MOPC315.BM BALB/c **(B)** and control BALB/c mice (C) on 15 days after tumor cell injection. Arrows show colocalization of NI-206.82C2 with CD138-positive multiple myeloma plasma cells.

**Fig. 14:** NI-206.82C2 binds to myocardial infarction causing obstructive human coronary thrombi and aortic atherosclerotic plaque. Obstructive coronary thrombus retrieved from a patient suffering from a myocardial infarction **(A),** and to a human aortic atherosclerotic plaque **(B)** are staining with Cyanine 3 labeled NI-206.82C2 and visualized by confocal immunofluorescence. 3A1 is a human isotype matched antibody with no known binding epitope as a specificity control.

**Fig. 15:** NI-206.82C2 prolongs human blood plasma clotting time, decreases clotting rate, decreases clot elasticity, and decreases clot rigidity. ROTEM™ analysis shows a dose dependent prolongation of clot formation time by treatment with NI-206.82C2, but not with an inactive isotyped-matched control antibody 43A11 **(A).** NI-206.82C2 also reduces the maximum clotting angle **(B),** decreases maximum clot elasticity **(C),** and reduces clot rigidity, **(D)** proportional to increasing concentrations of NI-206.82C2 compared to saline vehicle (0.0nM) or an isotype matched control antibody (43A11).

**Fig. 16:** Immunoprecipitation of FAP from human plasma. Immunoprecipitation performed on human blood plasma using NI-206.82C2, significantly reduces the rate of FAP substrate alpha 2 anti-plasmin ($\alpha$2AP-AMC) cleavage in the resulting plasma, compared to plasma before NI-206.82C2 immunoprecipitation. By comparison, immunoprecipitation with human isotype control antibodies 43A11 and 3A1 did not result in a reduction in the rate $\alpha$2AP-AMC cleavage.

**Fig. 17:** Characterization of a sandwich ELISA for measuring NI-206.82C2 antigen in human samples. **(A)** A standard curve was performed using recombinant human FAP as a control. **(B)** Linearity of the samples at increasing serum dilutions was determined. **(C)** The ELISA was shown to be specific to rhFAP, but not for other SB9 oligopeptidase homologues rhDDP4, rhDPP8, rhDPP9, and rhPOP/PREP.

**Fig. 18:** NI-206.82C2 antigen was significantly increased in the serum of patients with metastatic colorectal cancer (MCRC) compared with healthy control patients as measured by Sandwich ELISA.

**Fig. 19:** NI-206.82C2 antigen was significantly increased in the serum of patients with coronary artery disease (CAD) and ST Segment Elevation Myocardial Infarction (STEMI) compared to healthy control patients as measured by Sandwich ELISA.

**Fig. 20:** NI-206.82C2 antigen was significantly increased in the plasma of patients with carotid plaques compared to healthy control patients as measured by Sandwich ELISA.

**Fig. 21:** **(A)** A micrograph of the photochemical carotid injury model showing the Doppler flow meter (i), carotid artery (ii), and laser-induced carotid artery injury site (iii, bar = 1mm).
**(B)** The occlusion time of NI-206.82C2 treated mice (20mg/kg i.v.) was significantly prolonged compared to animals treated with the vehicle control. Statistics, unpaired Student's T-Test (*; $p < 0.05$, n=4).

**Fig. 22:** Treatment with NI-206.82C2 reduced the cumulative tumor diameter **(A)** and number of metastases **(B)** versus treatment with a phosphate buffered saline vehicle control as assessed by magnetic resonance imaging in mice bearing orthotopic syngeneic MC38 colorectal tumors (* = $p < 0.05$).

**Fig. 23:** Anti-FAP antibody NI-206.82C2 inhibits thrombosis in mice. Antibody NI-206.82C2 prolongs photochemical injury induced arterial occlusion times versus 43A11 (biologically inactive isotype-matched control antibody) in living mice (A, Log-rank hazard ratio = 0.04, 95% confidence interval = 0.01-0.16, p<0.0001). NI-206.82C2 exhibits a dose-dependent increase in the median time to occlusion in mice (B, n=10-11 mice / group).

**Fig. 24:** Cy5 labelled Anti-FAP RTM™ derived antibodies share a pH dependent binding behavior to FAP+ HEK293 Cells in pH adjusted PBS. RTM™ derived human monoclonal anti-FAP antibodies bind to $FAP^+$ cells in a pH dependent manner (A) NI-206.82C2, (B) NI-206.59B4, (C) NI-206.27E8 (D) NI-206.12G4, (E) NI-206.18H2, (F) NI-206.6D3. Δ MFI = MFI-anti-FAP RTM™ derived antibody minus MFI-43A11; an isotype matched biologically inactive control.

**Fig. 25:** NI-206.82C2 Target Engagement. NI-206.282C2 accumulates selectively in the tumor stroma **(A)** over time vs. biologically inactive isotype-matched control antibody 43A11 **(B).**

**Fig. 26:** Minimum epitope region of antibody NI-206.82C2. FAP peptides covering the epitope of antibody NI-206.82C2 were sequentially truncated by one amino acid from the N- and C-terminus to determine the minimum epitope region of NI-206.82C2 covering amino acids 528-FDRSK-532 (SEQ ID NO: 38) of FAP; see also *supra*.

**Fig. 27:** Amino acids essential for NI-206.82C2 binding. Every single amino acid from a FAP peptide fragment 521-KMILPPQFDRSKKYPLLIQ-539 (SEQ ID NO: 39) was mutated sequentially into an alanine to determine the essential amino acids, *i.e.* those which cause a loss of NI-206.82C2 binding when mutated. This strategy revealed that amino acids D-529 and K-532 of FAP are essential for NI-206.82C2 binding; see also *supra*.

## DETAILED DESCRIPTION OF THE INVENTION

The present invention generally relates to human auto-antibodies against fibroblast activation protein (FAP) and recombinant derivatives thereof. More specifically, the present invention relates to monoclonal anti-FAP antibodies which are characterized in that at least one of their CDRs are derived from an FAP specific antibody produced by a human memory B cell. The anti-FAP antibodies of the present invention are particular useful in immunotherapy and *in vivo* detection and labeling of FAP-related diseases, *i.e.* diseases which affected cells and tissue are characterized by the (elevated) expression of FAP. Due to their human derivation, the resulting recombinant antibodies of the present invention can be reasonably expected to be efficacious and safe as therapeutic agent, and highly specific as a diagnostic reagent for the detection of FAP both *in vitro* as well as *in vivo* on cells and in tissue without giving false positives.

Furthermore, the present invention relates to FAP-binding molecules, in particular anti-FAP antibodies, which exhibit an increased avidity to transmembrane FAP at acidic pH found in tumors, compared to lower avidity at the neutral pH of healthy tissue. The pH dependent avidity is important because FAP is expressed also in healthy tissue, and antibody binding to healthy tissue may cause side effects. Therefore, due to the preferential binding of antibodies of the present invention to FAP in the tumor microenvironment a higher therapeutic effect can be achieved without the side effects associated with binding to transmembrane FAP in other tissue.

In a further aspect, the present invention relates to an anti-FAP antibody and equivalent agent which selectively binds to and inhibits the enzymatic activity of FAP, which in addition is characterized by anti-coagulant activity and thus useful as an anti-thrombolytic agent. Furthermore, based on a unique and novel epitope of FAP recognized by a human-derived anti-FAP antibody of the present invention a novel *in vitro* assay for determining FAP in a body fluid, in particular blood and blood plasma, respectively, is provided, wherein an increased level of FAP reliably correlates with the presence of the FAP-related disease such as cancer and atherosclerosis.

In addition, the present invention relates to diagnostic and pharmaceutical compositions comprising the subject anti-FAP antibody or equivalent FAP-binding agent, in particular for use in diagnosis and treatment of tumor and cardiovascular diseases such as thrombosis.

In a particular aspect, the present invention relates to a chimeric antigen receptor (CAR) directed against human FAP, wherein one receptor comprises a variable region or binding domain derived from the anti-FAP antibody of the present invention and preferably a further receptor is preferentially specific for the CD3ζ antigen allowing the CAR to graft an arbitrary specificity onto an immune effector cell. Thus, the CAR of the present invention is useful for therapeutic purposes, preferably for T cell mediated treatment of FAP induced or associated tumors.

The embodiments of the present invention derived from the results of the appending Examples as illustrated in the Figures are summarized in the claims and in items [1] to [48], *supra*, and are supplemented with the following description. Furthermore, for the avoidance of any doubt the technical content of the prior art referred to in the background section form part of the disclosure of the present invention and may be relied upon for any embodiment claimed herein. However, this is not an admission that these documents represent relevant prior art as to the present invention.

## I. Definitions

Unless otherwise stated, a term as used herein is given the definition as provided in the Oxford Dictionary of Biochemistry and Molecular Biology, Oxford University Press, 1997, revised 2000 and reprinted 2003, ISBN 0 19 850673 2.

It is to be noted that the term "a" or "an" entity refers to one or more of that entity; for example, "an antibody", is understood to represent one or more antibodies. As such, the terms "a" (or "an"), "one or more", and "at least one" can be used interchangeably herein.

"Activation", as used herein, refers to the state of a T cell that has been sufficiently stimulated to induce detectable cellular proliferation. Activation can also be associated with induced cytokine production, and detectable effector functions. The term "activated T cells" refers to, among other things, T cells that are undergoing cell division.

As used herein, reference to an antibody or equivalent agent that "specifically binds", "selectively binds", or "preferentially binds" FAP refers to an antibody that does not bind other unrelated proteins. In one example, an anti-FAP antibody or equivalent FAP-binding agent disclosed herein can bind human recombinant FAP or an epitope thereof and shows no binding above about 2 times background for other proteins. Information and databank accession numbers for the nucleotide and amino acid sequence of human FAP is given the background section, *supra*. In a preferred embodiment, the antibody of the present invention does not substantially recognize FAP homologues such as rhDPPIV, rhPOP/PREP, rhDPP8, and rhDPP9 or other unrelated human antigens; see Example 6 and Fig. 7A - E, in particular when assessed in accordance the Example. In addition, in one preferred embodiment the anti-FAP antibody or equivalent FAP-binding agent is capable of binding murine FAP as well; see Examples 7, 10 and 11 and Figures 8, 12 and 13. Information and databank accession numbers for the nucleotide and amino acid sequence of mouse FAP is given the background section, *supra*.

Furthermore, as used herein, reference to a "FAP inhibitory" antibody or equivalent agent that "specifically inhibits", "selectively inhibits", or "preferentially inhibits" FAP refers to an antibody or agent that selectively binds to and inhibits the enzymatic activity of FAP but does not substantially inhibit the enzymatic activity FAP homologues such as rhDPPIV, rhPOP/PREP, rhDPP8, and rhDPP9; see Example 6 and Fig. 7F as well as Example 7 and Figure 8, in particular when assessed in accordance the Examples. In a preferred embodiment, the anti-FAP antibody and FAP-binding agent of the present invention demonstrates a higher potency for inhibiting active recombinant human FAP compared to previously tested FAP-targeting agent Val-boro-Pro and/or F19; see Example 7 and Figure 8, in particular when assessed in accordance the Example. In addition, in one preferred embodiment the anti-FAP antibody or equivalent FAP-binding agent is capable of inhibiting active recombinant mouse FAP as well; see Example 7 and Figures 8.

The term "pH" refers to the Latin term "pondus hydrogenii" and symbolizes the logarithm of the reciprocal of hydrogen ion concentration in gram atoms per liter, used to express the acidity or alkalinity of a solution on a scale of 0 to 14, where less than 7 represents acidity, 7 neutrality, and more than 7 alkalinity. Pure water has a pH of about 7. The typical physiological pH of a normal human organ, tissue or microenvironment of a cell is 7.2-7.4 (average 7.4) while tumor tissues have been shown to have a more acidic extracellular pH (pHe) (pH = 6.5 - 6.9); see, *e.g.,* Estrella et al.

Cancer Res. 73 (2013), 1524-1535 and references cited *supra*. As demonstrated in Example 18 and illustrated in Figure 24, in one preferred embodiment of the present invention the anti-FAP antibody or equivalent FAP-binding agent disclosed herein binds to transmembrane FAP preferentially at an acidic pH, preferably at pH 6.4 or pH 6.8 compared to its binding to FAP at a neutral pH or more particularly physiological pH of 7.4. The preferential binding of an anti-FAP antibody to transmembrane FAP at an acidic pH can be tested in accordance with the experimental setup described in Example 18. In contrast, as demonstrated in Example 18 and illustrated in Figure 24D, the present invention also provides human and recombinant human-derived anti-FAP antibodies, in particular antibody NI-206.12G4, which show a decreased avidity to FAP in the acidic pH as compared to neutral or physiological pH. This characteristic may be advantageous when rather than the treatment of a tumor targeting of FAP associated with an inflammatory or cardiovascular disease is intended, where FAP and FAP expressing cells may be present in an environment with neutral pH.

In addition, as used herein, reference to an anti-coagulant refers an anti-FAP antibody or equivalent FAP-binding agent which is capable in a dose dependent manner to prolong clot formation time of human blood plasma, preferably accompanied by reduction of the maximum clotting angle, decrease of maximum clot elasticity, and reduction of clot rigidity; see Example 13 and Figure 15, in particular when assessed in accordance the Example.

In this context, as used herein, reference to a thrombolytic agent or thrombolytic therapy refers an anti-FAP antibody or equivalent FAP-binding agent and use thereof, respectively, which is capable of inhibiting FAP mediated activation of $\alpha$2-Antiplasmin, a coagulation factor which inhibits plasmin-mediated thrombolysis; see Example 14 and Figure 16, in particular when assessed in accordance the Example.

Since the sequences of the FAP antibodies of the present invention have been obtained from human subjects, the FAP antibodies of the present invention may also be called "human auto-antibodies" or "human-derived antibodies" in order to emphasize that those antibodies were indeed expressed initially by the subjects and are not synthetic constructs generated, for example, by means of human immunoglobulin expressing phage libraries, which hitherto represented one common method for trying to provide human-like antibodies. On the other hand, the human-derived antibody of the present invention may be denoted synthetic, recombinant, and/or biotechnological in order distinguish it from human serum antibodies per se, which may be purified via protein A or affinity column.

*Peptides:*

The term "peptide" is understood to include the terms "polypeptide" and "protein" (which, at times, may be used interchangeably herein) within its meaning. Similarly, fragments of proteins and polypeptides are also contemplated and may be referred to herein as "peptides". Nevertheless, the term "peptide" preferably denotes an amino acid polymer including at least 5 contiguous amino acids, preferably at least 10 contiguous amino acids, more preferably at least 15 contiguous amino acids, still more preferably at least 20 contiguous amino acids, and particularly preferred at least 25 contiguous amino acids. In addition, the peptide in accordance with present invention typically has no more than 100 contiguous amino acids, preferably less than 80 contiguous amino acids, more preferably less than 50 contiguous amino acids and still more preferred no more than 15 contiguous amino acids of the FAP polypeptide.

*Polypeptides:*

As used herein, the term "polypeptide" is intended to encompass a singular "polypeptide" as well as plural "polypeptides", and refers to a molecule composed of monomers (amino acids) linearly linked by amide bonds (also known as peptide bonds). The term "polypeptide" refers to any chain or chains of two or more amino acids, and does not refer to a specific length of the product. Thus, "peptides", "dipeptides", "tripeptides", "oligopeptides", "protein", "amino acid chain", or any other term used to refer to a chain or chains of two or more amino acids, are included within the definition of "polypeptide", and the term "polypeptide" may be used instead of, or interchangeably with any of these terms.

The term "polypeptide" is also intended to refer to the products of post-expression modifications of the polypeptide, including without limitation glycosylation, acetylation, phosphorylation, amidation and derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis.

A polypeptide of the invention may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded. As used herein, the term glycoprotein refers to a protein coupled to at least one carbohydrate moiety that is attached to the protein via an oxygen-containing or a nitrogen-containing side chain of an amino acid residue, *e.g.,* a serine residue or an asparagine residue.

By an "isolated" polypeptide or a fragment, variant, or derivative thereof is intended a polypeptide that is not in its natural milieu. No particular level of purification is required. For example, an isolated polypeptide can be removed from its native or natural environment. Recombinantly produced polypeptides and proteins expressed in host cells are considered isolated for purposed of the invention, as are native or recombinant polypeptides which have been separated, fractionated, or partially or substantially purified by any suitable technique.

"Recombinant peptides, polypeptides or proteins" refer to peptides, polypeptides or proteins produced by recombinant DNA techniques, *i.e.* produced from cells, microbial or mammalian, transformed by an exogenous recombinant DNA expression construct encoding the fusion protein including the desired peptide. Proteins or peptides expressed in most bacterial cultures will typically be free of glycan. Proteins or polypeptides expressed in yeast may have a glycosylation pattern different from that expressed in mammalian cells.

Included as polypeptides of the present invention are fragments, derivatives, analogs or variants of the foregoing polypeptides and any combinations thereof as well. The terms "fragment", "variant", "derivative", and "analog" include peptides and polypeptides having an amino acid sequence sufficiently similar to the amino acid sequence of the natural peptide. The term "sufficiently similar" means a first amino acid sequence that contains a sufficient or minimum number of identical or equivalent amino acid residues relative to a second amino acid sequence such that the first and second amino acid sequences have a common structural domain and/or common functional activity. For example, amino acid sequences that comprise a common structural domain that is at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 100%, identical are defined herein as sufficiently similar. Preferably, variants will be sufficiently similar to the amino acid sequence of the preferred peptides of the present invention, in particular to FAP, variants, derivatives or analogs of either of them. Such variants generally retain the functional activity of the peptides of the present invention. Variants include peptides that differ in amino acid sequence from the native and wildtype peptide, respectively, by way of one or more amino acid deletion(s), addition(s), and/or substitution(s). These may be naturally occurring variants as well as artificially designed ones.

Furthermore, the terms "fragment", "variant", "derivative", and "analog" when referring to antibodies or antibody polypeptides of the present invention include any polypeptides which retain at least some of the antigen-binding properties of the corresponding native binding molecule, antibody, or polypeptide. Fragments of polypeptides of the present invention include proteolytic fragments, as well as deletion fragments, in addition to specific antibody fragments discussed elsewhere herein. Variants of antibodies and antibody polypeptides of the present invention include fragments as described above, and also polypeptides with altered amino acid sequences due to amino acid substitutions, deletions, or insertions. Variants may occur naturally or be non-naturally occurring. Non-naturally occurring variants may be produced using art-known mutagenesis techniques. Variant polypeptides may comprise conservative or non-conservative amino acid substitutions, deletions or additions. Derivatives of FAP-specific binding molecules, e.g., antibodies, antibody polypeptides or chimeric antigen receptors (CARs) of the present invention, are polypeptides which have been altered so as to exhibit additional features not found on the native polypeptide. Examples include fusion proteins. Variant polypeptides may also be referred to herein as "polypeptide analogs". As used herein a "derivative" of a binding molecule or fragment thereof, an antibody, an antibody polypeptide, a CAR refers to a subject polypeptide having one or more residues chemically derivatized by reaction of a functional side group. Also included as "derivatives" are those peptides which contain one or more naturally occurring amino acid derivatives of the twenty standard amino acids. For example, 4-hydroxyproline may be substituted for proline; 5-hydroxylysine may be substituted for lysine; 3-methylhistidine may be substituted for histidine; homoserine may be substituted for serine; and ornithine may be substituted for lysine.

*Determination of similarity and/or identity of molecules:*

"Similarity" between two peptides is determined by comparing the amino acid sequence of one peptide to the sequence of a second peptide. An amino acid of one peptide is similar to the corresponding amino acid of a second peptide if it is identical or a conservative amino acid substitution. Conservative substitutions include those described in Dayhoff, M.O., ed., The Atlas of Protein Sequence and Structure 5, National Biomedical Research Foundation, Washington, D.C. (1978), and in Argos, EMBO J. 8 (1989), 779-785. For example, amino acids belonging to one of the following groups represent conservative changes or substitutions: -Ala, Pro, Gly, Gln, Asn, Ser, Thr; -Cys, Ser, Tyr, Thr; -Val, Ile, Leu, Met, Ala, Phe; -Lys, Arg, His; -Phe, Tyr, Trp, His; and - Asp, Glu.

"Similarity" between two polynucleotides is determined by comparing the nucleic acid sequence of one polynucleotide to the sequence of a polynucleotide. A nucleic acid of one polynucleotide is similar to the corresponding nucleic acid of a second polynucleotide if it is identical or, if the nucleic acid is part of a coding sequence, the respective triplet comprising the nucleic acid encodes for the same amino acid or for a conservative amino acid substitution.

The determination of percent identity or similarity between two sequences is preferably accomplished using the mathematical algorithm of Karlin and Altschul (1993) Proc. Natl. Acad. Sci USA 90: 5873-5877. Such an algorithm is incorporated into the BLASTn and BLASTp programs of Altschul et al. (1990) J. Mol. Biol. 215: 403-410 available at NCBI (http://www.ncbi.nlm.nih.gov/blast/Blast.cge).

The determination of percent identity or similarity is performed with the standard parameters of the BLASTn programs for BLAST polynucleotide searches and BLASTp programs for BLAST protein search, as recommended on the NCBI webpage and in the "BLAST Program Selection Guide" in respect of sequences of a specific length and composition. BLAST polynucleotide searches are performed with the BLASTn program.

For the general parameters, the "Max Target Sequences" box may be set to 100, the "Short queries" box may be ticked, the "Expect threshold" box may be set to 1000 and the "Word Size" box may be set to 7 as recommended for short sequences (less than 20 bases) on the NCBI webpage. For longer sequences the "Expect threshold" box may be set to 10 and the "Word Size" box may be set to 11. For the scoring parameters the "Match/mismatch Scores" may be set to 1,-2 and the "Gap Costs" box may be set to linear. For the Filters and Masking parameters, the "Low complexity regions" box may not be ticked, the "Species-specific repeats" box may not be ticked, the "Mask for lookup table only" box may be ticked, the "DUST Filter Settings" may be ticked and the "Mask lower case letters" box may not be ticked. In general the "Search for short nearly exact matches" may be used in this respect, which provides most of the above indicated settings. Further information in this respect may be found in the "BLAST Program Selection Guide" published on the NCBI webpage.

BLAST protein searches are performed with the BLASTp program. For the general parameters, the "Max Target Sequences" box may be set to 100, the "Short queries" box may be ticked, the "Expect threshold" box may be set to 10 and the "Word Size" box may be set to "3". For the scoring parameters the "Matrix" box may be set to "BLOSUM62", the "Gap Costs" Box may be set to "Existence: 11 Extension: 1", the "Compositional adjustments" box may be set to "Conditional compositional score matrix adjustment". For the Filters and Masking parameters the "Low complexity regions" box may not be ticked, the "Mask for lookup table only" box may not be ticked and the "Mask lower case letters" box may not be ticked.

Modifications of both programs, *e.g.*, in respect of the length of the searched sequences, are performed according to the recommendations in the "BLAST Program Selection Guide" published in a HTML and a PDF version on the NCBI webpage.

*Polynucleotides:*

The term "polynucleotide" is intended to encompass a singular nucleic acid as well as plural nucleic acids, and refers to an isolated nucleic acid molecule or construct, *e.g.,* messenger RNA (mRNA) or plasmid DNA (pDNA). A polynucleotide may comprise a conventional phosphodiester bond or a non-conventional bond *(e.g.,* an amide bond, such as found in peptide nucleic acids (PNA)). The term "nucleic acid" refers to any one or more nucleic acid segments, *e.g.,* DNA or RNA fragments, present in a polynucleotide. By "isolated" nucleic acid or polynucleotide is intended a nucleic acid molecule, DNA or RNA, which has been removed from its native environment. For example, a recombinant polynucleotide encoding an antibody contained in a vector is considered isolated for the purposes of the present invention. Further examples of an isolated polynucleotide include recombinant polynucleotides maintained in heterologous host cells or purified (partially or substantially) polynucleotides in solution. Isolated RNA molecules include *in vivo* or *in vitro* RNA transcripts of polynucleotides of the present invention. Isolated polynucleotides or nucleic acids according to the present invention further include such molecules produced synthetically. In addition, polynucleotide or a nucleic acid may be or may include a regulatory element such as a promoter, ribosome binding site, or a transcription terminator.

As used herein, a "coding region" is a portion of nucleic acid which consists of codons translated into amino acids. Although a "stop codon" (TAG, TGA, or TAA) is not translated into an amino acid, it may be considered to be part of a coding region, but any flanking sequences, for example promoters, ribosome binding sites, transcriptional terminators, introns, and the like, are not part of a coding region. Two or more coding regions of the present invention can be present in a single polynucleotide construct, *e.g.,* on a single vector, or in separate polynucleotide constructs, *e.g.,* on separate (different) vectors. Furthermore, any vector may contain a single coding region, or may comprise two or more coding regions, *e.g.,* a single vector may separately encode an immunoglobulin heavy chain variable region and an immunoglobulin light chain variable region. In addition, a vector, polynucleotide, or nucleic acid of the invention may encode heterologous coding regions, either fused or unfused to a nucleic acid encoding a binding molecule, an antibody, or fragment, variant, a CAR, or derivative thereof. Heterologous coding regions include without limitation specialized elements or motifs, such as a secretory signal peptide or a heterologous functional domain.

In certain embodiments, the polynucleotide or nucleic acid is DNA. In the case of DNA, a polynucleotide comprising a nucleic acid which encodes a polypeptide normally may include a promoter and/or other transcription or translation control elements operable associated with one or more coding regions. An operable association is when a coding region for a gene product, *e.g.,* a polypeptide, is associated with one or more regulatory sequences in such a way as to place expression of the gene product under the influence or control of the regulatory sequence(s). Two DNA fragments (such as a polypeptide coding region and a promoter associated therewith) are "operable associated" or "operable linked" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operable associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operable associated with the polynucleotide to direct cell-specific transcription. Suitable promoters and other transcription control regions are disclosed herein.

A variety of transcription control regions are known to those skilled in the art. These include, without limitation, transcription control regions which function in vertebrate cells, such as, but not limited to, promoter and enhancer segments from cytomegaloviruses (the immediate early promoter, in conjunction with intron-A), simian virus 40 (the early promoter), and retroviruses (such as Rous sarcoma virus). Other transcription control regions include those derived from vertebrate genes such as actin, heat shock protein, bovine growth hormone and rabbit ß-globin, as well as other sequences capable of controlling gene expression in eukaryotic cells. Additional suitable transcription control regions include tissue-specific promoters and enhancers as well as lymphokine-inducible promoters (e.g., promoters inducible by interferons or interleukins).

Similarly, a variety of translation control elements are known to those of ordinary skill in the art. These include, but are not limited to ribosome binding sites, translation initiation and termination codons, and elements derived from picornaviruses (particularly an internal ribosome entry site, or IRES, also referred to as a CITE sequence).

In other embodiments, a polynucleotide of the present invention is RNA, for example, in the form of messenger RNA (mRNA).

Polynucleotide and nucleic acid coding regions of the present invention may be associated with additional coding regions which encode secretory or signal peptides, which direct the secretion of a polypeptide encoded by a polynucleotide of the present invention. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the complete or "full-length" polypeptide to produce a secreted or "mature" form of the polypeptide. In certain embodiments, the native signal peptide, *e.g.,* an immunoglobulin heavy chain or light chain signal peptide is used, or a functional derivative of that sequence that retains the ability to direct the secretion of the polypeptide that is operable associated with it. Alternatively, a heterologous mammalian signal peptide, or a functional derivative thereof, may be used. For example, the wild-type leader sequence may be substituted with the leader sequence of human tissue plasminogen activator (TPA) or mouse ß-glucuronidase.

A "binding molecule" or "FAP-binding agent" as used in the context of the present invention relates primarily to antibodies, CARs, and fragments thereof, but may also refer to other non-antibody molecules that bind to FAP including but not limited to hormones, receptors, ligands, major histocompatibility complex (MHC) molecules, chaperones such as heat shock proteins (HSPs) as well as cell-cell adhesion molecules such as members of the cadherin, intergrin, C-type lectin and immunoglobulin (Ig) superfamilies. Thus, for the sake of clarity only and without restricting the scope of the present invention most of the following embodiments are discussed with respect to antibodies and antibody-like molecules, in particular CARs which represent the preferred binding molecules for the development of therapeutic and diagnostic agents.

*Antibodies:*

The terms "antibody" and "immunoglobulin" are used interchangeably herein. An antibody or immunoglobulin is a binding molecule which comprises at least the variable domain of a heavy chain, and normally comprises at least the variable domains of a heavy chain and a light chain. Basic immunoglobulin structures in vertebrate systems are relatively well understood; see, *e.g.,* Harlow et al., Antibodies: A Laboratory Manual, (Cold Spring Harbor Laboratory Press, 2nd ed. 1988).

As will be discussed in more detail below, the term "immunoglobulin" comprises various broad classes of polypeptides that can be distinguished biochemically. Those skilled in the art will appreciate that heavy chains are classified as gamma, mu, alpha, delta, or epsilon, ($\gamma$, $\mu$, $\alpha$, $\delta$, $\varepsilon$) with some subclasses among them (*e.g.*, $\gamma$1-$\gamma$4). It is the nature of this chain that determines the "class" of the antibody as IgG, IgM, IgA IgG, or IgE, respectively. The immunoglobulin subclasses (isotypes) *e.g.,* IgG1, IgG2, IgG3, IgG4, IgA1, etc. are well characterized and are known to confer functional specialization. Modified versions of each of these classes and isotypes are readily discernible to the skilled artisan in view of the instant disclosure and, accordingly, are within the scope of the instant invention. All immunoglobulin classes are clearly within the scope of the present invention, the following discussion will generally be directed to the IgG class of immunoglobulin molecules. With regard to IgG, a standard immunoglobulin molecule comprises two identical light chain polypeptides of molecular weight approximately 23,000 Daltons, and two identical heavy chain polypeptides of molecular weight 53,000-70,000. The four chains are typically joined by disulfide bonds in a "Y" configuration wherein the light chains bracket the heavy chains starting at the mouth of the "Y" and continuing through the variable region.

Light chains are classified as either kappa or lambda (κ, λ). Each heavy chain class may be bound with either a kappa or lambda light chain. In general, the light and heavy chains are covalently bonded to each other, and the "tail" portions of the two heavy chains are bonded to each other by covalent disulfide linkages or non-covalent linkages when the immunoglobulins are generated either by hybridomas, B cells or genetically engineered host cells. In the heavy chain, the amino acid sequences run from an N-terminus at the forked ends of the Y configuration to the C-terminus at the bottom of each chain.

Both the light and heavy chains are divided into regions of structural and functional homology. The terms "constant" and "variable" are used functionally. In this regard, it will be appreciated that the variable domains of both the light ($V_L$) and heavy ($V_H$) chain portions determine antigen recognition and specificity. Conversely, the constant domains of the light chain (CL) and the heavy chain (CH1, CH2 or CH3) confer important biological properties such as secretion, transplacental mobility, Fc receptor binding, complement binding, and the like. By convention the numbering of the constant region domains increases as they become more distal from the antigen-binding site or amino-terminus of the antibody. The N-terminal portion is a variable region and at the C-terminal portion is a constant region; the CH3 and CL domains actually comprise the carboxy-terminus of the heavy and light chain, respectively.

As indicated above, the variable region allows the antibody to selectively recognize and specifically bind epitopes on antigens. That is, the $V_L$ domain and $V_H$ domain, or subset of the complementarity determining regions (CDRs), of an antibody combine to form the variable region that defines a three dimensional antigen-binding site. This quaternary antibody structure forms the antigen-binding site present at the end of each arm of the Y. More specifically, the antigen-binding site is defined by three CDRs on each of the $V_H$ and $V_L$ chains. Any antibody or immunoglobulin fragment which contains sufficient structure to specifically bind to FAP is denoted herein interchangeably as a "binding fragment" or an "immunospecific fragment".

In naturally occurring antibodies, an antibody comprises six hypervariable regions, sometimes called "complementarity determining regions" or "CDRs" present in each antigen-binding domain, which are short, non-contiguous sequences of amino acids that are specifically positioned to form the antigen-binding domain as the antibody assumes its three dimensional configuration in an aqueous environment. The "CDRs" are flanked by four relatively conserved "framework" regions or "FRs" which show less inter-molecular variability. The framework regions largely adopt a β-sheet conformation and the CDRs form loops which connect, and in some cases form part of, the β-sheet structure. Thus, framework regions act to form a scaffold that provides for positioning the CDRs in correct orientation by inter-chain, non-covalent interactions. The antigen-binding domain formed by the positioned CDRs defines a surface complementary to the epitope on the immunoreactive antigen. This complementary surface promotes the non-covalent binding of the antibody to its cognate epitope. The amino acids comprising the CDRs and the framework regions, respectively, can be readily identified for any given heavy or light chain variable region by one of ordinary skill in the art, since they have been precisely defined; see, "Sequences of Proteins of Immunological Interest," Kabat, E., et al., U.S. Department of Health and Human Services, (1983); and Chothia and Lesk, J. Mol. Biol., 196 (1987), 901-917.

In the case where there are two or more definitions of a term which is used and/or accepted within the art, the definition of the term as used herein is intended to include all such meanings unless explicitly stated to the contrary. A specific example is the use of the term "complementarity determining region" ("CDR") to describe the non-contiguous antigen combining sites found within the variable region of both heavy and light chain polypeptides. This particular region has been described by Kabat et al., U.S. Dept. of Health and Human Services, "Sequences of Proteins of Immunological Interest" (1983) and by Chothia and Lesk, J. Mol. Biol., 196 (1987), 901-917, which are incorporated herein by reference, where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table I as a comparison. The exact residue numbers which encompass a particular CDR will vary depending on the sequence and size of the CDR. Those skilled in the art can routinely determine which residues comprise a particular hypervariable region or CDR of the human IgG subtype of antibody given the variable region amino acid sequence of the antibody.

| **Table I:** CDR Definitions[1] | **Kabat** | **Chothia** |
|---|---|---|
| VH CDR1 | 31-35 | 26-32 |
| VH CDR2 | 50-65 | 52-58 |

(continued)

| **Table I:** CDR Definitions[1] | **Kabat** | **Chothia** |
|---|---|---|
| VH CDR3 | 95-102 | 95-102 |
| VL CDR1 | 24-34 | 26-32 |
| VL CDR2 | 50-56 | 50-52 |
| VL CDR3 | 89-97 | 91-96 |
| [1]Numbering of all CDR definitions in Table I is according to the numbering conventions set forth by Kabat *et al.* (see below). | | |

Kabat *et al.* also defined a numbering system for variable domain sequences that is applicable to any antibody. One of ordinary skill in the art can unambiguously assign this system of "Kabat numbering" to any variable domain sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody or antigen-binding fragment, variant, or derivative thereof of the present invention are according to the Kabat numbering system, which however is theoretical and may not equally apply to every antibody of the present invention. For example, depending on the position of the first CDR the following CDRs might be shifted in either direction.

Antibodies or antigen-binding fragments, immunospecific fragments, variants, or derivatives thereof of the invention include, but are not limited to, polyclonal, monoclonal, multispecific, human, humanized, primatized, murinized or chimeric antibodies, single chain antibodies, epitope-binding fragments, *e.g.,* Fab, Fab' and $F(ab')_2$, Fd, Fvs, single-chain Fvs (scFv), single-chain antibodies, disulfide-linked Fvs (sdFv), fragments comprising either a $V_L$ or $V_H$ domain, fragments produced by a Fab expression library, and anti-idiotypic (anti-Id) antibodies (including, *e.g.,* anti-Id antibodies to antibodies disclosed herein). ScFv molecules are known in the art and are described, *e.g.,* in US patent 5,892,019. Immunoglobulin or antibody molecules of the invention can be of any type (*e.g.*, IgG, IgE, IgM, IgD, IgA, and IgY), class (*e.g.*, IgG1, IgG2, IgG3, IgG4, IgA1 and IgA2) or subclass of immunoglobulin molecule.

In one embodiment, antibodies or antigen-binding fragments, immunospecific fragments, variants, or derivatives thereof of the invention further include, but are not limited to bispecific antibodies, trifunctional antibodies, tetrabodies, bispecific T-cell engagers (BiTEs), bispecific killer cell engagers (BiKEs), dual affinity retargeting molecules (DARTs) and DuoBodies, which are all well known in the art and are described; see the documents cited *supra.* Thus, in this embodiment the antibodies or antigen-binding fragments, immunospecific fragments, variants, or derivatives thereof of the present invention do not only bind to FAP, but at least one further epitope/target. In a preferred embodiment, in addition to FAP the antibody of the present invention binds to the death receptor 5 (DR5) or to CD3.

In one embodiment, the antibody of the present invention is not IgM or a derivative thereof with a pentavalent structure. Particular, in specific applications of the present invention, especially therapeutic use, IgMs are less useful than IgG and other bivalent antibodies or corresponding binding molecules since IgMs due to their pentavalent structure and lack of affinity maturation often show unspecific cross-reactivities and very low affinity.

In a particularly preferred embodiment, the antibody of the present invention is not a polyclonal antibody, *i.e.* it substantially consists of one particular antibody species rather than being a mixture obtained from a plasma immunoglobulin sample.

Antibody fragments, including single-chain antibodies, may comprise the variable region(s) alone or in combination with the entirety or a portion of the following: hinge region, CH1, CH2, and CH3 domains. Also included in the invention are FAP binding fragments which comprise any combination of variable region(s) with a hinge region, CH1, CH2, and CH3 domains. Antibodies or immunospecific fragments thereof of the present invention may be from any animal origin including birds and mammals. Preferably, the antibodies are human, murine, donkey, rabbit, goat, guinea pig, camel, llama, horse, or chicken antibodies. In another embodiment, the variable region may be condricthoid in origin (e.g., from sharks).

In one aspect, the antibody of the present invention is a human monoclonal antibody isolated from a human. Optionally, the framework region of the human antibody is aligned and adopted in accordance with the pertinent human germ line variable region sequences in the database; see, *e.g.,* Vbase (http://vbase.mrc-cpe.cam.ac.uk/) hosted by the MRC Centre for Protein Engineering (Cambridge, UK). For example, amino acids considered to potentially deviate from the true germ line sequence could be due to the PCR primer sequences incorporated during the cloning process. Compared to artificially generated human-like antibodies such as single chain antibody fragments (scFvs) from a phage displayed antibody library or xenogeneic mice the human monoclonal antibody of the present invention is characterized by (i) being obtained using the human immune response rather than that of animal surrogates, *i.e.* the antibody has been generated in response to natural FAP in its relevant conformation in the human body, (ii) having protected the individual or is at least significant for the presence of FAP, and (iii) since the antibody is of human origin the risks of cross-reactivity against self-antigens is minimized. Thus, in accordance with the present invention the terms "human monoclonal antibody", "human monoclonal autoantibody", "human antibody" and the like are used to denote a FAP binding molecule which is of human origin, *i.e.* which has been isolated from a human cell such as a B cell or hybridoma thereof or the cDNA of which has been directly cloned from mRNA of a human cell, for example a human memory B cell. A human antibody is still "human", *i.e.* human-derived even if amino acid substitutions are made in the antibody, *e.g.,* to improve binding characteristics.

In one embodiment the human-derived antibodies of the present invention comprises heterologous regions compared to the natural occurring antibodies, e.g. amino acid substitutions in the framework region, constant region exogenously fused to the variable region, different amino acids at the C- or N- terminal ends and the like.

Antibodies derived from human immunoglobulin libraries or from animals transgenic for one or more human immunoglobulins and that do not express endogenous immunoglobulins, as described *infra* and, for example in, US patent no 5,939,598 by Kucherlapati et al., are denoted human-like antibodies in order distinguish them from truly human antibodies of the present invention.

For example, the paring of heavy and light chains of human-like antibodies such as synthetic and semi-synthetic antibodies typically isolated from phage display do not necessarily reflect the original paring as it occurred in the original human B cell. Accordingly Fab and scFv fragments obtained from recombinant expression libraries as commonly used in the prior art can be considered as being artificial with all possible associated effects on immunogenicity and stability.

In contrast, the present invention provides isolated affinity-matured antibodies from selected human subjects, which are characterized by their therapeutic utility and their tolerance in man.

As used herein, the term "rodentized antibody" or "rodentized immunoglobulin" refers to an antibody comprising one or more CDRs from a human antibody of the present invention; and a human framework region that contains amino acid substitutions and/or deletions and/or insertions that are based on a rodent antibody sequence. When referred to rodents, preferably sequences originating in mice and rats are used, wherein the antibodies comprising such sequences are referred to as "murinized" or "ratinized" respectively. The human immunoglobulin providing the CDRs is called the "parent" or "acceptor" and the rodent antibody providing the framework changes is called the "donor". Constant regions need not be present, but if they are, they are usually substantially identical to the rodent antibody constant regions, *i.e.* at least about 85 % to 90 %, preferably about 95 % or more identical. Hence, in some embodiments, a full-length murinized human heavy or light chain immunoglobulin contains a mouse constant region, human CDRs, and a substantially human framework that has a number of "murinizing" amino acid substitutions. Typically, a "murinized antibody" is an antibody comprising a murinized variable light chain and/or a murinized variable heavy chain. For example, a murinized antibody would not encompass a typical chimeric antibody, *e.g.,* because the entire variable region of a chimeric antibody is non-mouse. A modified antibody that has been "murinized" by the process of "murinization" binds to the same antigen as the parent antibody that provides the CDRs and is usually less immunogenic in mice, as compared to the parent antibody. The above explanations in respect of "murinized" antibodies apply analogously for other "rodentized" antibodies, such as "ratinized antibodies", wherein rat sequences are used instead of the murine.

As used herein, the term "heavy chain portion" includes amino acid sequences derived from an immunoglobulin heavy chain. A polypeptide comprising a heavy chain portion comprises at least one of: a CH1 domain, a hinge *(e.g.,* upper, middle, and/or lower hinge region) domain, a CH2 domain, a CH3 domain, or a variant or fragment thereof. For example, a binding polypeptide for use in the invention may comprise a polypeptide chain comprising a CH1 domain; a polypeptide chain comprising a CH1 domain, at least a portion of a hinge domain, and a CH2 domain; a polypeptide chain comprising a CH1 domain and a CH3 domain; a polypeptide chain comprising a CH1 domain, at least a portion of a hinge domain, and a CH3 domain, or a polypeptide chain comprising a CH1 domain, at least a portion of a hinge domain, a CH2 domain, and a CH3 domain. In another embodiment, a polypeptide of the invention comprises a polypeptide chain comprising a CH3 domain. Further, a binding polypeptide for use in the invention may lack at least a portion of a CH2 domain (e.g., all or part of a CH2 domain). As set forth above, it will be understood by one of ordinary skill in the art that these domains (e.g., the heavy chain portions) may be modified such that they vary in amino acid sequence from the naturally occurring immunoglobulin molecule.

In certain antibodies, or antigen-binding fragments, variants, or derivatives thereof disclosed herein, the heavy chain portions of one polypeptide chain of a multimer are identical to those on a second polypeptide chain of the multimer. Alternatively, heavy chain portion-containing monomers of the invention are not identical. For example, each monomer may comprise a different target binding site, forming, for example, a bispecific antibody or diabody.

In another embodiment, the antibodies, or antigen-binding fragments, variants, or derivatives thereof disclosed herein are composed of a single polypeptide chain such as scFvs and are to be expressed intracellularly (intrabodies) for potential *in vivo* therapeutic and diagnostic applications.

The heavy chain portions of a binding polypeptide for use in the diagnostic and treatment methods disclosed herein may be derived from different immunoglobulin molecules. For example, a heavy chain portion of a polypeptide may comprise a CH1 domain derived from an IgG1 molecule and a hinge region derived from an IgG3 molecule. In another example, a heavy chain portion can comprise a hinge region derived, in part, from an IgG1 molecule and, in part, from an IgG3 molecule. In another example, a heavy chain portion can comprise a chimeric hinge derived, in part, from an IgG1 molecule and, in part, from an IgG4 molecule.

As used herein, the term "light chain portion" includes amino acid sequences derived from an immunoglobulin light chain. Preferably, the light chain portion comprises at least one of a $V_L$ or CL domain.

The minimum size of a peptide or polypeptide epitope for an antibody is thought to be about four to five amino acids. Peptide or polypeptide epitopes preferably contain at least seven, more preferably at least nine and most preferably between at least about 15 to about 30 amino acids. Since a CDR can recognize an antigenic peptide or polypeptide in its tertiary form, the amino acids comprising an epitope need not be contiguous, and in some cases, may not even be on the same peptide chain. In the present invention, a peptide or polypeptide epitope recognized by antibodies of the present invention contains a sequence of at least 4, at least 5, at least 6, at least 7, more preferably at least 8, at least 9, at least 10, at least 15, at least 20, at least 25, or between about 15 to about 30 contiguous or non-contiguous amino acids of FAP.

As previously indicated, the subunit structures and three dimensional configuration of the constant regions of the various immunoglobulin classes are well known. As used herein, the term "$V_H$ domain" includes the amino terminal variable domain of an immunoglobulin heavy chain and the term "CH1 domain" includes the first (most amino terminal) constant region domain of an immunoglobulin heavy chain. The CH1 domain is adjacent to the $V_H$ domain and is amino terminal to the hinge region of an immunoglobulin heavy chain molecule.

As used herein the term "CH2 domain" includes the portion of a heavy chain molecule that extends, *e.g.,* from about residue 244 to residue 360 of an antibody using conventional numbering schemes (residues 244 to 360, Kabat numbering system; and residues 231-340, EU numbering system; see Kabat EA *et al. op. cit*). The CH2 domain is unique in that it is not closely paired with another domain. Rather, two N-linked branched carbohydrate chains are interposed between the two CH2 domains of an intact native IgG molecule. It is also well documented that the CH3 domain extends from the CH2 domain to the C-terminal of the IgG molecule and comprises approximately 108 residues.

As used herein, the term "hinge region" includes the portion of a heavy chain molecule that joins the CH1 domain to the CH2 domain. This hinge region comprises approximately 25 residues and is flexible, thus allowing the two N-terminal antigen-binding regions to move independently. Hinge regions can be subdivided into three distinct domains: upper, middle, and lower hinge domains; see Roux et al., J. Immunol. 161 (1998), 4083-4090.

As used herein the term "disulfide bond" includes the covalent bond formed between two sulfur atoms. The amino acid cysteine comprises a thiol group that can form a disulfide bond or bridge with a second thiol group. In most naturally occurring IgG molecules, the CH1 and CL regions are linked by a disulfide bond and the two heavy chains are linked by two disulfide bonds at positions corresponding to 239 and 242 using the Kabat numbering system (position 226 or 229, EU numbering system).

As used herein, the terms "linked", "fused" or "fusion" are used interchangeably. These terms refer to the joining together of two more elements or components, by whatever means including chemical conjugation or recombinant means. An "in-frame fusion" refers to the joining of two or more polynucleotide open reading frames (ORFs) to form a continuous longer ORF, in a manner that maintains the correct translational reading frame of the original ORFs. Thus, a recombinant fusion protein is a single protein containing two or more segments that correspond to polypeptides encoded by the original ORFs (which segments are not normally so joined in nature). Although the reading frame is thus made continuous throughout the fused segments, the segments may be physically or spatially separated by, for example, in-frame linker sequence. For example, polynucleotides encoding the CDRs of an immunoglobulin variable region may be fused, in-frame, but be separated by a polynucleotide encoding at least one immunoglobulin framework region or additional CDR regions, as long as the "fused" CDRs are co-translated as part of a continuous polypeptide.

The term "expression" as used herein refers to a process by which a gene produces a biochemical, for example, an RNA or polypeptide. The process includes any manifestation of the functional presence of the gene within the cell including, without limitation, gene knockdown as well as both transient expression and stable expression. It includes without limitation transcription of the gene into messenger RNA (mRNA), transfer RNA (tRNA), small hairpin RNA (shRNA), small interfering RNA (siRNA) or any other RNA product, and the translation of mRNA into polypeptide(s). If the final desired product is a biochemical, expression includes the creation of that biochemical and any precursors. Expression of a gene produces a "gene product". As used herein, a gene product can be either a nucleic acid, *e.g.,* a messenger RNA produced by transcription of a gene, or a polypeptide which is translated from a transcript. Gene products described herein further include nucleic acids with post transcriptional modifications, e.g., polyadenylation, or polypeptides with post translational modifications, *e.g.,* methylation, glycosylation, the addition of lipids, association with other protein subunits, proteolytic cleavage, and the like.

As used herein, the term "sample" refers to any biological material obtained from a subject or patient. In one aspect, a sample can comprise blood, peritoneal fluid, CSF, saliva or urine. In other aspects, a sample can comprise whole blood, blood plasma, blood serum, B cells enriched from blood samples, and cultured cells *(e.g.,* B cells from a subject). A sample can also include a biopsy or tissue sample including neural tissue. In still other aspects, a sample can comprise whole cells and/or a lysate of the cells. Blood samples can be collected by methods known in the art. In one aspect, the pellet can be resuspended by vortexing at 4°C in 200 μl buffer (20 mM Tris, pH. 7.5, 0.5 % Nonidet, 1 mM EDTA, 1 mM PMSF, 0.1 M NaCl, IX Sigma Protease Inhibitor, and IX Sigma Phosphatase Inhibitors 1 and 2). The suspension can be kept on ice for 20 min. with intermittent vortexing. After spinning at 15,000 x g for 5 min at about 4°C, aliquots of supernatant can be stored at about -70°C.

*Chimeric antigen receptors (CARs):*

As used herein, the term "Chimeric Antigen Receptor" and "CAR", respectively, refers to a recombinant polypeptide construct, e.g. fusion protein comprising at least an extracellular antigen binding domain, a transmembrane domain and a cytoplasmic signaling domain comprising a functional signaling domain derived from a stimulatory molecule as defined below. Thus, CARs are generally generated by fusing the antigen-binding domain/region, for example single chain fragment of the variable region (scFv) of a monoclonal antibody (mAb) to membrane-spanning and intracellular-signaling domains; see, *e.g.* Figure 1 of Dai *et al.* (Dai et al., JNCI J. Natl. Cancer. Inst. 108 (2016), 1-15: djv439) for schematic representation of the CAR structure. In one embodiment, the stimulatory molecule is the zeta chain associated with the T cell receptor complex. In one embodiment, the cytoplasmic signaling domain further comprises one or more functional signaling domains derived from at least one costimulatory molecule as defined below. In one aspect, the costimulatory molecule is chosen from the group consisting of CD27, CD28, 4-1BB (*i.e.*, CD137), OX40, CD30, CD40, PD- 1, ICOS, lymphocyte function-associated antigen- 1 (LFA- 1), CD2, CD 7, LIGHT, NKG2C, B7- H3, a ligand that specifically binds with CD83, and any combination thereof. Unless indicated otherwise, the definition of the basic structure and components of CARs as defined in the international application WO 2014/ 055442 apply.
In one embodiment the CAR comprises an optional leader sequence at the amino-terminus (N-ter) of the CAR fusion protein. In one aspect, the CAR further comprises a leader sequence at the N-terminus of the extracellular antigen recognition domain, wherein the leader sequence is optionally cleaved from the antigen binding domain during cellular processing and localization of the CAR to the cellular membrane.

As used herein, a "signaling domain" is the functional portion of a protein which acts by transmitting information within the cell to regulate cellular activity via defined signaling pathways by generating second messengers or functioning as effectors by responding to such messengers.

By the term "stimulation," used in the context of CAR T cell, is meant a primary response induced by binding of a stimulatory molecule (e.g., a TCR/CD3 complex) with its cognate ligand thereby mediating a signal transduction event, such as, but not limited to, signal transduction via the TCR/CD3 complex. Stimulation can mediate altered expression of certain molecules, such as downregulation of TGF-ß, and/or reorganization of cytoskeletal structures, and the like.

A "stimulatory molecule," used in the context of CAR T, means a molecule expressed by a T cell that provide the primary cytoplasmic signaling sequence(s) that regulate primary activation of the TCR complex in a stimulatory way of the T cell signaling pathway. In one embodiment, the primary signal is initiated by, for instance, binding of a TCR/CD3 complex with an MHC molecule loaded with peptide, and which leads to mediation of a T cell response, including, but not limited to, proliferation, activation, differentiation, and the like. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences that are of particular use in the invention include those derived from TCR zeta, FcR gamma, FcR beta, CD3 gamma, CD3 delta, CD3 epsilon, CDS, CD22, CD79a, CD79b, CD278 (also known as "ICOS") and CD66d. In a specific CAR of the invention, the cytoplasmic signaling molecule in any one or more CARS of the invention, including CARs comprises a cytoplasmic signaling sequence derived from CD3-zeta.

As used herein "zeta" or alternatively "zeta chain", "CD3-zeta" or "TCR-zeta" is defined as the protein provided as GenBank Accession No. BAG36664.1, or the equivalent residues from a non-human species, e.g., mouse, rodent, monkey, ape and the like, and a "zeta stimulatory domain" or alternatively a "CD3-zeta stimulatory domain" or a "TCR-zeta stimulatory domain" is defined as the amino acid residues from the cytoplasmic domain of the zeta chain that are sufficient to functionally transmit an initial signal necessary for T cell activation. In one aspect the cytoplasmic domain of zeta comprises residues 52 through 164 of GenBank Accession No. BAG36664.1 or the equivalent residues from a non-human species, e.g., mouse, rodent, monkey, ape and the like, that are functional orthologs thereof.

A "costimulatory molecule" refers to the cognate binding partner on a T cell that specifically binds with a costimulatory ligand, thereby mediating a costimulatory response by the T cell, such as, but not limited to, proliferation. Costimulatory molecules are cell surface molecules other than antigen receptors or their ligands that are required for an efficient immune response. Costimulatory molecules include, but are not limited to an MHC class I molecule, BTLA and a Toll ligand receptor, as well as OX40, CD27, CD28, CDS, ICAM-1, LFA-1 (CD1 la/CD18) and 4-1BB (CD 137).

As used herein "4-lBB" is defined as member of the TNFR superfamily with an amino acid sequence provided as GenBank Accession No. AAA62478.2, or the equivalent residues from a non-human species, e.g., mouse, rodent, monkey, ape and the like; and a "4-lBB costimulatory domain" are defined amino acid residues 214-255 of GenBank Accession No. AAA62478.2, or the equivalent residues from a non-human species, *e.g.,* mouse, rodent, monkey, ape and the like.

As used herein, the terms "linked", "fused" or "fusion" are used interchangeably. These terms refer to the joining together of two more elements or components, by whatever means including chemical conjugation or recombinant means. An "in-frame fusion" refers to the joining of two or more polynucleotide open reading frames (ORFs) to form a continuous longer ORF, in a manner that maintains the correct translational reading frame of the original ORFs. Thus, a recombinant fusion protein is a single protein containing two or more segments that correspond to polypeptides encoded by the original ORFs (which segments are not normally so joined in nature). Although the reading frame is thus made continuous throughout the fused segments, the segments may be physically or spatially separated by, for example, in-frame linker sequence. For example, polynucleotides encoding the CDRs of an immunoglobulin variable region may be fused, in-frame, but be separated by a polynucleotide encoding at least one immunoglobulin framework region or additional CDR regions, as long as the "fused" CDRs are co-translated as part of a continuous polypeptide.

The term "expression" as used herein refers to a process by which a gene produces a biochemical, for example, an RNA or polypeptide. The process includes any manifestation of the functional presence of the gene within the cell including, without limitation, gene knockdown as well as both transient expression and stable expression. It includes without limitation transcription of the gene into messenger RNA (mRNA), transfer RNA (tRNA), small hairpin RNA (shRNA), small interfering RNA (siRNA) or any other RNA product, and the translation of mRNA into polypeptide(s). If the final desired product is a biochemical, expression includes the creation of that biochemical and any precursors. Expression of a gene produces a "gene product". As used herein, a gene product can be either a nucleic acid, *e.g.,* a messenger RNA produced by transcription of a gene, or a polypeptide which is translated from a transcript. Gene products described herein further include nucleic acids with post transcriptional modifications, *e.g.,* polyadenylation, or polypeptides with post translational modifications, *e.g.,* methylation, glycosylation, the addition of lipids, association with other protein subunits, proteolytic cleavage, and the like.

*Binding characteristics of antibodies and chimeric antigen receptors (CARs):*

By "specifically binding", or "specifically recognizing", used interchangeably herein, it is generally meant that a binding molecule, *e.g.,* an antibody or CAR binds to an epitope via its antigen-binding domain, and that the binding entails some complementarity between the antigen-binding domain and the epitope. According to this definition, the binding molecule is said to "specifically bind" to an epitope when it binds to that epitope, via its antigen-binding domain more readily than it would bind to a random, unrelated epitope. The term "specificity" is used herein to qualify the relative affinity by which a certain antibody or CAR binds to a certain epitope. For example, antibody or CAR "A" may be deemed to have a higher specificity for a given epitope than antibody or CAR "B", or antibody or CAR "A" may be said to bind to epitope "C" with a higher specificity than it has for related epitope "D".

Where present, the term "immunological binding characteristics", or other binding characteristics of an antibody or a CAR with an antigen, in all of its grammatical forms, refers to the specificity, affinity, cross-reactivity, and other binding characteristics of an antibody or CAR.

By "preferentially binding", it is meant that the binding molecule, *e.g.,* antibody or CAR specifically binds to an epitope more readily than it would bind to a related, similar, homologous, or analogous epitope. Thus, a binding molecule which "preferentially binds" to a given epitope would more likely bind to that epitope than to a related epitope, even though such an antibody or CAR may cross-react with the related epitope.

By way of non-limiting example, a binding molecule, *e.g.,* an antibody or CAR may be considered to bind a first epitope preferentially if it binds said first epitope with a dissociation constant ($K_D$) that is less than the binding molecule's $K_D$ for the second epitope. In another non-limiting example, a binding molecule may be considered to bind a first antigen preferentially if it binds the first epitope with an affinity that is at least one order of magnitude less than the binding molecule's $K_D$ for the second epitope. In another non-limiting example, an antibody or CAR may be considered to bind a first epitope preferentially if it binds the first epitope with an affinity that is at least two orders of magnitude less than the binding molecule's $K_D$ for the second epitope.

In another non-limiting example, a binding molecule, *e.g.,* an antibody or CAR may be considered to bind a first epitope preferentially if it binds the first epitope with an off rate (k(off)) that is less than the binding molecule's k(off) for the second epitope. In another non-limiting example, an binding molecule may be considered to bind a first epitope preferentially if it binds the first epitope with an affinity that is at least one order of magnitude less than the binding molecule's k(off) for the second epitope. In another non-limiting example, an antibody or CAR may be considered to bind a first epitope preferentially if it binds the first epitope with an affinity that is at least two orders of magnitude less than the binding molecule's k(off) for the second epitope.

A binding molecule, *e.g.,* an antibody or antigen-binding fragment, variant, CAR, or derivative disclosed herein may be said to bind FAP or a fragment, variant or specific conformation thereof with an off rate (k(off)) of less than or equal to 5 x $10^{-2}$ $sec^{-1}$, $10^{-2}$ $sec^{-1}$, 5 x $10^{-3}$ $sec^{-1}$ or $10^{-3}$ $sec^{-1}$. More preferably, an antibody or CAR of the invention may be said to bind FAP or a fragment, variant or specific conformation thereof with an off rate (k(off)) less than or equal to 5 x $10^{-1}$ $sec^{-1}$, $10^{-4}$ $sec^{-1}$, 5 x $10^{-5}$ $sec^{-1}$, or $10^{-5}$ $sec^{-1}$ 5 x $10^{-6}$ $sec^{-1}$, $10^{-6}$ $sec^{-1}$, 5 x $10^{-7}$ $sec^{-1}$ or $10^{-7}$ $sec^{-1}$.

A binding molecule, *e.g.,* an antibody or antigen-binding fragment, variant, CAR, or derivative disclosed herein may be said to bind FAP or a fragment, variant or specific conformation thereof with an on rate (k(on)) of greater than or equal to $10^3$ $M^{-1}$ $sec^{-1}$, 5 x $10^3$ $M^{-1}$ $sec^{-1}$, $10^4$ $M^{-1}$ $sec^{-1}$ or 5 x $10^4$ $M^{-1}$ $sec^{-1}$. More preferably, an antibody or CAR of the invention may be said to bind FAP or a fragment, variant or specific conformation thereof with an on rate (k(on)) greater than or equal to $10^5$ $M^{-1}$ $sec^{-1}$, 5 x $10^5$ $M^{-1}$ $sec^{-1}$, $10^6$ $M^{-1}$ $sec^{-1}$, or 5 x $10^6$ $M^{-1}$ $sec^{-1}$ or $10^7$ $M^{-1}$ $sec^{-1}$.

A binding molecule, *e.g.,* an antibody or CAR is said to competitively inhibit binding of a reference antibody or CAR to a given epitope if it preferentially binds to that epitope to the extent that it blocks, to some degree, binding of the reference antibody or CAR to the epitope. Competitive inhibition may be determined by any method known in the art, for example, competition ELISA assays. A binding molecule may be said to competitively inhibit binding of the reference antibody or CAR to a given epitope by at least 90%, at least 80%, at least 70%, at least 60%, or at least 50%.

As used herein, the term "affinity" refers to a measure of the strength of the binding of an individual epitope with the CDR of a binding molecule, *e.g.,* an immunoglobulin molecule or CAR; *see*, *e.g.,* Harlow et al., Antibodies: A Laboratory Manual, Cold Spring Harbor Laboratory Press, 2nd ed. (1988) at pages 27-28. As used herein, the term "avidity" refers to the overall stability of the complex between a population of binding molecules and an antigen, that is, the functional combining strength of an binding molecule mixture with the antigen; see, *e.g.,* Harlow at pages 29-34. Avidity is related to both the affinity of individual binding molecules in the population with specific epitopes, and also the valences of the binding molecules and the antigen. For example, the interaction between a bivalent monoclonal antibody and an antigen with a highly repeating epitope structure, such as a polymer, would be one of high avidity. The affinity or avidity of an antibody for an antigen can be determined experimentally using any suitable method; see, for example, Berzofsky et al., "Antibody-Antigen Interactions" In Fundamental Immunology, Paul, W. E., Ed., Raven Press New York, N Y (1984), Kuby, Janis Immunology, W. H. Freeman and Company New York, N Y (1992), and methods described herein. General techniques for measuring the affinity of an antibody or CAR for an antigen include ELISA, RIA, and surface plasmon resonance. The measured affinity of a particular antibody/CAR-antigen interaction can vary if measured under different conditions, *e.g.,* salt concentration, pH. Thus, measurements of affinity and other antigen-binding parameters, *e.g.,* $K_D$, $IC_{50}$, are preferably made with standardized solutions of antibody/CAR and antigen, and a standardized buffer.

Binding molecules, *e.g.,* antibodies or antigen-binding fragments, variants, CARs or derivatives thereof of the invention may also be described or specified in terms of their cross-reactivity. As used herein, the term "cross-reactivity" refers to the ability of a binding molecule, specific for one antigen, to react with a second antigen; a measure of relatedness between two different antigenic substances. Thus, a binding molecule is cross reactive if it binds to an epitope other than the one that induced its formation. The cross-reactive epitope generally contains many of the same complementary structural features as the inducing epitope, and in some cases, may actually fit better than the original.

For example, certain antibodies or CARs have some degree of cross-reactivity, in that they bind related, but non-identical epitopes, *e.g.,* epitopes with at least 95%, at least 90%, at least 85%, at least 80%, at least 75%, at least 70%, at least 65%, at least 60%, at least 55%, and at least 50% identity (as calculated using methods known in the art and described herein) to a reference epitope. An antibody or CAR may be said to have little or no cross-reactivity if it does not bind epitopes with less than 95%, less than 90%, less than 85%, less than 80%, less than 75%, less than 70%, less than 65%, less than 60%, less than 55%, and less than 50% identity (as calculated using methods known in the art and described herein) to a reference epitope. An antibody or CAR may be deemed "highly specific" for a certain epitope, if it does not bind any other analog, ortholog, or homolog of that epitope.

Binding molecules, *e.g.,* antibodies or antigen-binding fragments, variants, CARs or derivatives thereof of the invention may also be described or specified in terms of their binding affinity to FAP and/or fragments thereof. Preferred binding affinities include those with a dissociation constant or Kd less than 5 x $10^{-2}$ M, $10^{-2}$ M, 5 x $10^{-3}$ M, $10^{-3}$ M, 5 x $10^{-4}$ M, $10^{-4}$ M, 5 x $10^{-5}$ M, $10^{-5}$ M, 5 x $10^{-6}$ M, $10^{-6}$ M, 5 x $10^{-7}$ M, $10^{-7}$ M, 5 x $10^{-8}$ M, $10^{-8}$ M, 5 x $10^{-9}$ M, $10^{-9}$ M, 5 x $10^{-10}$ M, $10^{-10}$ M, 5 x $10^{-11}$ M, $10^{-11}$ M, 5 x $10^{-12}$ M, $10^{-12}$ M, 5 x $10^{-13}$ M, $10^{-13}$ M, 5 x $10^{-14}$ M, $10^{-14}$ M, 5 x $10^{-15}$ M, or $10^{-15}$ M.

*Diseases:*

Unless stated otherwise, the terms "disorder" and "disease" are used interchangeably herein and comprise any undesired physiological change in a subject, an animal, an isolated organ, tissue or cell/cell culture. FAP-related diseases and disorders comprise but are not limited to:

- Proliferative diseases including metastatic breast cancer, colorectal cancer, kidney cancer, chronic lymphocytary leukemia, pancreatic adenocarcinoma, carcinoma, invasive lobular carcinoma, non-small cell lung cancer, myeloma and tumor stroma.

- Diseases involving tissue remodeling and/or chronic inflammation (including but not limited to fibrotic disease, wound healing, keloid formation, osteoarthritis, rheumatoid arthritis and related disorders involving cartilage degradation, atherosclerotic disease and Crohn's disease).
- Diseases involving endocrinological disorder (including but not limited to disorders of glucose metabolism) and diseases involving blood clotting disorders.
- Cardiovascular diseases including heart disorders, as well as disorders of the blood vessels of the circulation system caused by, *e.g.,* abnormally high concentrations of lipids in the blood vessels, atherosclerosis, atherosclerotic plaques, atherothrombosis, myocardial infarction heart attack, chronic liver disease, cerebral venous thrombosis, deep venous thrombosis and pulmonary embolism.

The term "cancer" and "tumor" may be used interchangeably herein and defines as disease characterized by the rapid and uncontrolled growth of aberrant cells. Cancer cells can spread locally or through the bloodstream and lymphatic system to other parts of the body. Examples of various cancers include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like. Preferably, cancer and tumor cells are characterized by aberrant expression of FAP on their cell surface.

*Treatment:*

As used herein, the terms "treat" or "treatment" refer to both therapeutic treatment and prophylactic or preventative measures, wherein the object is to prevent or slow down (lessen) an undesired physiological change or disorder, such as the development of cardiac deficiency or tumors, preferably as characterized above. Beneficial or desired clinical results include, but are not limited to, alleviation of symptoms, diminishment of extent of disease, stabilized (*i. e.,* not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the manifestation of the condition or disorder is to be prevented.

If not stated otherwise the term "drug", "medicine", or "medicament" are used interchangeably herein and shall include but are not limited to all (A) articles, medicines and preparations for internal or external use, and any substance or mixture of substances intended to be used for diagnosis, cure, mitigation, treatment, or prevention of disease of either man or other animals; and (B) articles, medicines and preparations (other than food) intended to affect the structure or any function of the body of man or other animals; and (C) articles intended for use as a component of any article specified in clause (A) and (B). The term "drug", "medicine", or "medicament" shall include the complete formula of the preparation intended for use in either man or other animals containing one or more "agents", "compounds", "substances" or "(chemical) compositions" as and in some other context also other pharmaceutically inactive excipients as fillers, disintegrants, lubricants, glidants, binders or ensuring easy transport, disintegration, disaggregation, dissolution and biological availability of the "drug", "medicine", or "medicament" at an intended target location within the body of man or other animals, *e.g.,* at the skin, in the stomach or the intestine. The terms "agent", "compound", or "substance" are used interchangeably herein and shall include, in a more particular context, but are not limited to all pharmacologically active agents, *i.e.* agents that induce a desired biological or pharmacological effect or are investigated or tested for the capability of inducing such a possible pharmacological effect by the methods of the present invention.

The term "anti-tumor effect" as used herein, refers to a biological effect which can be manifested by various means, including but not limited to, a decrease in tumor volume, a decrease in the number of tumor cells, a decrease in the number of metastases, an increase in life expectancy, a decrease in tumor cell proliferation, a decrease in tumor cell survival, or amelioration of various physiological symptoms associated with the cancerous condition. An "anti-tumor effect" can also be manifested by the ability of the peptides, polynucleotides, cells, antibodies and CARs of the invention in prevention of the occurrence of tumor in the first place.

By "subject" or "individual" or "animal" or "patient" or "mammal", is meant any subject, particularly a mammalian subject, *e.g.,* a human patient, for whom diagnosis, prognosis, prevention, or therapy is desired.

*Pharmaceutical carriers:*

Pharmaceutically acceptable carriers and administration routes can be taken from corresponding literature known to the person skilled in the art. The pharmaceutical compositions of the present invention can be formulated according to methods well known in the art; see for example Remington: The Science and Practice of Pharmacy (2000) by the University of Sciences in Philadelphia, ISBN 0-683-306472, Vaccine Protocols 2nd Edition by Robinson et al.,

Humana Press, Totowa, New Jersey, USA, 2003; Banga, Therapeutic Peptides and Proteins: Formulation, Processing, and Delivery Systems. 2nd Edition by Taylor and Francis. (2006), ISBN: 0-8493-1630-8. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well-known conventional methods. These pharmaceutical compositions can be administered to the subject at a suitable dose. Administration of the suitable compositions may be effected by different ways. Examples include administering a composition containing a pharmaceutically acceptable carrier via oral, intranasal, rectal, topical, intraperitoneal, intravenous, intramuscular, subcutaneous, subdermal, transdermal, intrathecal, and intracranial methods. Aerosol formulations such as nasal spray formulations include purified aqueous or other solutions of the active agent with preservative agents and isotonic agents. Such formulations are preferably adjusted to a pH and isotonic state compatible with the nasal mucous membranes. Pharmaceutical compositions for oral administration, such as single domain antibody molecules (e.g., "nanobodies™") etc. are also envisaged in the present invention. Such oral formulations may be in tablet, capsule, powder, liquid or semi-solid form. A tablet may comprise a solid carrier, such as gelatin or an adjuvant. Formulations for rectal or vaginal administration may be presented as a suppository with a suitable carrier; see also O'Hagan et al., Nature Reviews, Drug Discovery 2(9) (2003), 727- 735. Further guidance regarding formulations that are suitable for various types of administration can be found in Remington's Pharmaceutical Sciences, Mace Publishing Company, Philadelphia, PA, 17th ed. (1985) and corresponding updates. For a brief review of methods for drug delivery see Langer, Science 249 (1990), 1527-1533.

## II. Antibodies of the present invention

The present invention generally relates to human-derived anti-FAP antibodies and antigen-binding fragments thereof, which preferably demonstrate the immunological binding characteristics and/or biological properties as outlined for the antibodies illustrated in the Examples. In accordance with the present invention human monoclonal antibodies specific for FAP were cloned from a pool of healthy human subjects. However, in another embodiment of the present invention, the human monoclonal anti-FAP antibodies might also be cloned from patients showing symptoms of a FAP-related disease and/or disorder associated with FAP.

In the course of the experiments performed in accordance with the present invention, antibodies present in the conditioned media of cultured human memory B cell were evaluated for their capacity to bind to FAP and to more than 10 other proteins including bovine serum albumin (BSA). Only the B-cell supernatants able to bind to the FAP protein but not to any of the other proteins in the screen were selected for further analysis, including determination of the antibody class and light chain subclass. The selected B-cells were then processed for antibody cloning.

In brief, this consisted in the extraction of messenger RNAs from the selected B-cells, retro-transcription by RT-PCR, amplification of the antibody-coding regions by PCR, cloning into plasmid vectors and sequencing. Selected human antibodies were then produced by recombinant expression in HEK293 or CHO cells and purification, and subsequently characterized for their capacity to bind human FAP protein. The combination of various tests, e.g. recombinant expression of the antibodies in HEK293 or CHO cells and the subsequent characterization of their binding specificities towards human FAP protein, and their distinctive binding to FAP and not to FAP homologues confirmed that for the first time human antibodies have been cloned that are highly specific for FAP and distinctively recognize and selectively bind FAP protein. In some cases, mouse chimeric antibodies are generated on the basis of the variable domains of the human antibodies of the present invention. As described in Example 9 and shown in Figure 10 and 11 the mouse chimeric antibodies have equal binding affinity, specificity and selectivity to human FAP as the human antibodies in that FAP positive human breast cancer tissue sections were specifically stained with recombinantly engineered chimeric form of NI-206.82C2 with a murine constant domain and the human variable domain of the original antibody.

Thus, the present invention generally relates to recombinant human-derived monoclonal anti-FAP antibody and biotechnological and synthetic derivatives thereof. In one embodiment of the present invention, the antibody is capable of binding human and murine FAP; see Example 7 and Figure 8.

In one embodiment, the present invention is directed to an anti-FAP antibody, or antigen-binding fragment, variant or derivatives thereof, where the antibody specifically binds to the same epitope of FAP as a reference antibody selected from the group consisting of NI-206.18H2, NI-206.82C2, NI-206.59B4, NI-206.22F7, NI-206.27E8, NI-206.12G4, and NI-206.17A6; see Example 3 and Figure 4 for the respective epitopes. As explained in Example 3, the entire sequences of FAP were synthesized as a total of 188 linear 15-mer peptides with an 11 amino acid overlap between individual peptides. Thus, the subject antibodies of the present invention illustrated in the Examples are different from antibodies which recognize any of the mentioned epitopes in context with additional N- and/or C-terminal amino acids only. Therefore, in a preferred embodiment of the present invention, specific binding of an anti-FAP antibody to a FAP epitope which comprises the amino acid sequence of any one of the epitopes identified for anti-FAP antibodies NI-206.18H2, NI-206.82C2, NI-206.59B4, NI-206.22F7, NI-206.27E8, NI-206.12G4, and NI-206.17A6 is determined with sequential peptides 15 amino acid long and 11 amino acid overlap in accordance with Example 3 and Figure 4. Accordingly, the present invention generally relates to any anti-FAP antibody and antibody-like molecule which binds to the same epitope as an antibody illustrated in the Examples having the CDRs and/or variable heavy and light region as depicted in any one of Figures 1A-1K.

In one embodiment, the antibody of the present invention exhibits the binding properties of the exemplary NI-206.82C2, NI-206.59B4, NI-206.22F7, NI-206.27E8, NI-206.12G4, NI-206.17A6, NI-206.18H2, NI-206.20A8, NI-206.6D3, NI-206.14C5 and NI-206.34C11 antibodies as described in the Examples.

The term "does not substantially recognize" when used in the present application to describe the binding affinity of a molecule of a group comprising an antibody, a fragment thereof or a binding molecule for a specific target molecule, antigen and/or conformation of the target molecule and/or antigen means that the molecule of the aforementioned group binds said molecule, antigen and/or conformation with a binding affinity which is at least 2-fold, 3-fold, 4-fold, 5-fold, 6-fold, 7-fold, 8-fold or 9-fold less than the binding affinity of the molecule of the aforementioned group for binding another molecule, antigen and/or conformation. Very often the dissociation constant (KD) is used as a measure of the binding affinity. Sometimes, it is the EC50 on a specific assay as for example an ELISA assay that is used as a measure of the binding affinity. Preferably the term "does not substantially recognize" when used in the present application means that the molecule of the aforementioned group binds said molecule, antigen and/or conformation with a binding affinity which is at least or 10-fold, 20-fold, 50-fold, 100-fold, 1000-fold or 10000-fold less than the binding affinity of said molecule of the aforementioned group for binding to another molecule, antigen and/or conformation. In this context, the binding affinities may be in the range as shown for the NI-206.82C2, NI-206.59B4, NI-206.22F7, NI-206.27E8, NI-206.12G4, NI-206.17A6, NI-206.18H2, NI-206.20A8 and NI-206.6D3 antibodies in Fig. 2, *i.e.* having half maximal effective concentrations (EC50) of about 1 pM to 500 nM, preferably an EC50 of about 50 pM to 100 nM, most preferably an EC50 of about 1 nM to 20 nM or even below 1 nM for human FAP, *i.e.* captured FAP (sFAP), directly coated FAP (FAP) and/or directly coated FAP peptides mixture (cFAP) as shown in Figure 2.

Some antibodies are able to bind to a wide array of biomolecules, *e.g.,* proteins. As the skilled artisan will appreciate, the term specific is used herein to indicate that other biomolecules than FAP protein or fragments thereof do not significantly bind to the antigen-binding molecule, *e.g.,* one of the antibodies of the present invention. Preferably, the level of binding to a biomolecule other than FAP results in a binding affinity which is at most only 20% or less, 10% or less, only 5% or less, only 2% or less or only 1% or less (*i.e.* at least 5, 10, 20, 50 or 100 fold lower, or anything beyond that) of the affinity to FAP, respectively; see *e.g.,* Fig. 7. The present invention is also drawn to an antibody, or antigen-binding fragment, variant or biotechnological or synthetic derivatives thereof, where the antibody comprises an antigen-binding domain identical to that of an antibody selected from the group consisting of NI-206.82C2, NI-206.59B4, NI-206.22F7, NI-206.27E8, NI-206.12G4, NI-206.17A6, NI-206.18H2, NI-206.20A8, NI-206.6D3, NI-206.14C5 and NI-206.34C11.

The present invention further exemplifies several binding molecules, *e.g.,* antibodies and binding fragments thereof, which may be characterized by comprising in their variable region, *e.g.,* binding domain at least one complementarity determining region (CDR) of the $V_H$ and/or $V_L$ variable region comprising any one of the amino acid sequences depicted in Figs. 1G-K and 1A-1F. The corresponding nucleotide sequences encoding the above-identified variable regions are set forth in Table II below. Exemplary sets of CDRs of the above amino acid sequences of the $V_H$ and/or $V_L$ region are depicted in Figs. 1G-K and 1A-1F. However, as discussed in the following the person skilled in the art is well aware of the fact that in addition or alternatively CDRs may be used, which differ in their amino acid sequence from those set forth in Figs. 1G-K and 1A-1F by one, two, three or even more amino acids in case of CDR2 and CDR3. Therefore, in one embodiment the antibody of the present invention or a FAP-binding fragment thereof is provided comprising in its variable region at least one complementarity determining region (CDR) as depicted in Figs. 1G-K and 1A-1F and/or one or more CDRs thereof comprising one or more amino acid substitutions.

In one embodiment, the antibody of the present invention is any one of the antibodies comprising an amino acid sequence of the $V_H$ and/or $V_L$ region as depicted in Figs. 1G-K and 1A-1F or a $V_H$ and/or $V_L$ region thereof comprising one or more amino acid substitutions. Preferably, the antibody of the present invention is characterized by the preservation of the cognate pairing of the heavy and light chain as was present in the human B-cell.

In a further embodiment of the present invention the anti-FAP antibody, FAP-binding fragment, synthetic or biotechnological variant thereof can be optimized to have appropriate binding affinity to the target and pharmacokinetic properties. Therefore, at least one amino acid in the CDR or variable region, which is prone to modifications selected from the group consisting of glycosylation, oxidation, deamination, peptide bond cleavage, iso-aspartate formation and/or unpaired cysteine is substituted by a mutated amino acid that lack such alteration or wherein at least one carbohydrate moiety is deleted or added chemically or enzymatically to the antibody. Examples for amino acid optimization can be found in e.g. international applications WO 2010/121140 and WO 2012/049570. Additional modification optimizing the antibody properties are described in Gavel et al., Protein Engineering 3 (1990), 433-442 and Helenius et al., Annu. Rev. Biochem. 73 (2004), 1019-1049.

Alternatively, the antibody of the present invention is an antibody or antigen-binding fragment, derivative or variant thereof, which competes for binding to FAP with at least one of the antibodies having the $V_H$ and/or $V_L$ region as depicted in any one of Fig. 1A-1F.

The antibody of the present invention may be human, in particular for therapeutic applications. Alternatively, the antibody of the present invention is a rodent, rodentized or chimeric rodent-human antibody, preferably a murine, murinized or chimeric murine-human antibody or a rat, ratinized or chimeric rat-human antibody which are particularly useful for diagnostic methods and studies in animals. In one embodiment the antibody of the present invention is a chimeric rodent-human or a rodentized antibody.

As mentioned above, due to its generation upon a human immune response the human monoclonal antibody of the present invention will recognize epitopes which are of particular pathological relevance and which might not be accessible or less immunogenic in case of immunization processes for the generation of, for example, mouse monoclonal antibodies and *in vitro* screening of phage display libraries, respectively. Accordingly, it is prudent to stipulate that the epitope of the human anti-FAP antibody of the present invention is unique and no other antibody which is capable of binding to the epitope recognized by the human monoclonal antibody of the present invention exists. A further indication for the uniqueness of the antibodies of the present invention is the fact that, as indicated in the Examples, for the first time a selective and potent FAP inhibitory anti-FAP antibody NI-206.82C2 has been provided, which may not be obtainable by the usual processes for antibody generation, such as immunization or *in vitro* library screenings.

Therefore, in one embodiment the present invention also extends generally to anti-FAP antibodies and FAP-binding molecules which compete with the human monoclonal antibody of the present invention for specific binding to FAP. The present invention is more specifically directed to an antibody, or antigen-binding fragment, variant or derivatives thereof, where the antibody specifically binds to the same epitope of FAP as a reference antibody selected from the group consisting of NI-206.18H2, NI-206.82C2, NI-206.59B4, NI-206.22F7, NI-206.27E8, NI-206.12G4 and NI-206.17A6.

Competition between antibodies is determined by an assay in which the immunoglobulin under test inhibits specific binding of a reference antibody to a common antigen, such as FAP. Numerous types of competitive binding assays are known, for example: solid phase direct or indirect radioimmunoassay (RIA), solid phase direct or indirect enzyme immunoassay (EIA), sandwich competition assay; see Stahli et al., Methods in Enzymology 9 (1983), 242-253; solid phase direct biotin-avidin EIA; see Kirkland et al., J. Immunol. 137 (1986), 3614-3619 and Cheung et al., Virology 176 (1990), 546-552; solid phase direct labeled assay, solid phase direct labeled sandwich assay; see Harlow and Lane, Antibodies, A Laboratory Manual, Cold Spring Harbor Press (1988); solid phase direct label RIA using $I^{125}$ label; see Morel et al., Molec. Immunol. 25 (1988), 7-15 and Moldenhauer et al., Scand. J. Immunol. 32 (1990), 77-82. Typically, such an assay involves the use of purified FAP or epitope containing antigen thereof bound to a solid surface or cells bearing either of these, an unlabeled test immunoglobulin and a labeled reference immunoglobulin, *i.e.* the human monoclonal antibody of the present invention. Competitive inhibition is measured by determining the amount of label bound to the solid surface or cells in the presence of the test immunoglobulin. Usually the test immunoglobulin is present in excess. Preferably, the competitive binding assay is performed under conditions as described for the ELISA assay in the appended Examples. Antibodies identified by competition assay (competing antibodies) include antibodies binding to the same epitope as the reference antibody and antibodies binding to an adjacent epitope sufficiently proximal to the epitope bound by the reference antibody for steric hindrance to occur. Usually, when a competing antibody is present in excess, it will inhibit specific binding of a reference antibody to a common antigen by at least 50% or 75%. Hence, the present invention is further drawn to an antibody, or antigen-binding fragment, variant or derivatives thereof, where the antibody competitively inhibits a reference antibody selected from the group consisting of NI-206.82C2, NI-206.59B4, NI-206.22F7, NI-206.27E8, NI-206.12G4, NI-206.17A6, NI-206.18H2, NI-206.20A8, NI-206.6D3, NI-206.14C5 and NI-206.34C11 from binding to FAP or fragments thereof

In another embodiment, the present invention provides an isolated polypeptide comprising, consisting essentially of, or consisting of an immunoglobulin heavy chain variable region ($V_H$), where at least one of $V_H$-CDRs of the heavy chain variable region or at least two of the $V_H$-CDRs of the heavy chain variable region are at least 80%, 85%, 90% or 95% identical to reference heavy chain $V_H$-CDR1, $V_H$-CDR2 or $V_H$-CDR3 amino acid sequences from the antibodies disclosed herein. Alternatively, the $V_H$-CDR1, $V_H$-CDR2 and $V_H$-CDR3 regions of the $V_H$ are at least 80%, 85%, 90% or 95% identical to reference heavy chain $V_H$ -CDR1, $V_H$-CDR2 and $V_H$-CDR3 amino acid sequences from the antibodies disclosed herein. Thus, according to this embodiment a heavy chain variable region of the invention has $V_H$-CDR1, $V_H$-CDR2 and $V_H$-CDR3 polypeptide sequences related to the groups shown in Figs. 1G-K and 1A-1F respectively. While Figs. 1G-K and 1A-1F show $V_H$-CDRs defined by the Kabat system, other CDR definitions, *e.g.,* $V_H$-CDRs defined by the Chothia system, are also included in the present invention, and can be easily identified by a person of ordinary skill in the art using the data presented in Figs. 1G-F and 1A-1F.

In another embodiment, the present invention provides an isolated polypeptide comprising, consisting essentially of, or consisting of an immunoglobulin heavy chain variable region ($V_H$) in which the $V_H$-CDR1, $V_H$-CDR2 and $V_H$-CDR3 regions have polypeptide sequences which are identical to the $V_H$-CDR1, $V_H$-CDR2 and $V_H$-CDR3 groups shown in Figs. 1G-K and 1A-1F respectively.

In another embodiment, the present invention provides an isolated polypeptide comprising, consisting essentially of, or consisting of an immunoglobulin heavy chain variable region ($V_H$) in which the $V_H$-CDR1, $V_H$-CDR2 and

$V_H$-CDR3 regions have polypeptide sequences which are identical to the $V_H$-CDR1, $V_H$-CDR2 and $V_H$-CDR3 groups shown in Figs. 1G-K and 1A-1F respectively, except for one, two, three, four, five, or six amino acid substitutions in any one $V_H$-CDR. In certain embodiments the amino acid substitutions are conservative.

In another embodiment, the present invention provides an isolated polypeptide comprising, consisting essentially of, or consisting of an immunoglobulin light chain variable region ($V_L$), where at least one of the $V_L$-CDRs of the light chain variable region or at least two of the $V_L$-CDRs of the light chain variable region are at least 80%, 85%, 90% or 95% identical to reference light chain $V_L$-CDR1, $V_L$-CDR2 or $V_L$-CDR3 amino acid sequences from antibodies disclosed herein.

Alternatively, the $V_L$-CDR1, $V_L$-CDR2 and $V_L$-CDR3 regions of the $V_L$ are at least 80%, 85%, 90% or 95% identical to reference light chain $V_L$-CDR1, $V_L$-CDR2 and $V_L$-CDR3 amino acid sequences from antibodies disclosed herein. Thus, according to this embodiment a light chain variable region of the invention has $V_L$-CDR1, $V_L$-CDR2 and $V_L$-CDR3 polypeptide sequences related to the polypeptides shown in Fig. 1G-K and 1A-1F respectively. While Figs. 1G-K and 1A-1F shows $V_L$-CDRs defined by the Kabat system, other CDR definitions, e.g., $V_L$-CDRs defined by the Chothia system, are also included in the present invention.In another embodiment, the present invention provides an isolated polypeptide comprising, consisting essentially of, or consisting of an immunoglobulin light chain variable region ($V_L$) in which the $V_L$-CDR1, $V_L$-CDR2 and $V_L$-CDR3 regions have polypeptide sequences which are identical to the $V_L$-CDR1, $V_L$-CDR2 and $V_L$-CDR3 groups shown in Figs. 1G-K and 1A-1F respectively.

In another embodiment, the present invention provides an isolated polypeptide comprising, consisting essentially of, or consisting of an immunoglobulin heavy chain variable region ($V_L$) in which the $V_L$-CDR1, $V_L$-CDR2 and $V_L$-CDR3 regions have polypeptide sequences which are identical to the $V_L$-CDR1, $V_L$-CDR2 and $V_L$-CDR3 groups shown in Figs. 1G-K and 1A-1F respectively, except for one, two, three, four, five, or six amino acid substitutions in any one $V_L$-CDR. In certain embodiments the amino acid substitutions are conservative.

An immunoglobulin or its encoding cDNA may be further modified. Thus, in a further embodiment the method of the present invention comprises any one of the step(s) of producing a chimeric antibody, murinized antibody, single-chain antibody, Fab-fragment, bi-specific antibody, fusion antibody, labeled antibody or an analog of any one of those. Corresponding methods are known to the person skilled in the art and are described, *e.g.,* in Harlow and Lane "Antibodies, A Laboratory Manual", CSH Press, Cold Spring Harbor (1988). When derivatives of said antibodies are obtained by the phage display technique, surface plasmon resonance as employed in the BIAcore system can be used to increase the efficiency of phage antibodies which bind to the same epitope as that of any one of the antibodies described herein (Schier, Human Antibodies Hybridomas 7 (1996), 97-105; Malmborg, J. Immunol. Methods 183 (1995), 7-13). The production of chimeric antibodies is described, for example, in international application WO 89/09622. Methods for the production of humanized antibodies are described in, *e.g.,* European application EP-A1 0 239 400 and international application WO 90/07861. Further sources of antibodies to be utilized in accordance with the present invention are so-called xenogeneic antibodies. The general principle for the production of xenogeneic antibodies such as human-like antibodies in mice is described in, *e.g.,* international applications WO 91/10741, WO 94/02602, WO 96/34096 and WO 96/33735. As discussed above, the antibody of the invention may exist in a variety of forms besides complete antibodies; including, for example, Fv, Fab and $F(ab)_2$, as well as in single chains; see *e.g.* international application WO 88/09344. In one embodiment therefore, the antibody of the present invention is provided, which is selected from the group consisting of a single chain Fv fragment (scFv), a F(ab') fragment, a F(ab) fragment, and a $F(ab')_2$ fragment.

The antibodies of the present invention or their corresponding immunoglobulin chain(s) can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of an immunoglobulin chain are well known to the person skilled in the art; see, *e.g.,* Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994). Modifications of the antibody of the invention include chemical and/or enzymatic derivatizations at one or more constituent amino acids, including side chain modifications, backbone modifications, and N- and C-terminal modifications including acetylation, hydroxylation, methylation, amidation, and the attachment of carbohydrate or lipid moieties, cofactors, and the like. Likewise, the present invention encompasses the production of chimeric proteins which comprise the described antibody or some fragment thereof at the amino terminus fused to heterologous molecule such as an immunostimulatory ligand at the carboxyl terminus; see, *e.g.,* international application WO 00/30680 for corresponding technical details.

Additionally, the present invention encompasses peptides including those containing a binding molecule as described above, for example containing the CDR3 region of the variable region of any one of the mentioned antibodies, in particular CDR3 of the heavy chain since it has frequently been observed that heavy chain CDR3 (HCDR3) is the region having a greater degree of variability and a predominant participation in antigen-antibody interaction. Such peptides may easily be synthesized or produced by recombinant means to produce a binding agent useful according to the invention. Such methods are well known to those of ordinary skill in the art. Peptides can be synthesized for example, using automated peptide synthesizers which are commercially available. The peptides can also be produced by recombinant techniques by incorporating the DNA expressing the peptide into an expression vector and transforming cells with the expression vector to produce the peptide.

Hence, the present invention relates to any FAP-binding molecule, *e.g.,* an antibody or binding fragment thereof which is oriented towards the anti-FAP antibodies and/or antibodies capable of binding FAP and/or fragments thereof and displays the mentioned properties for exemplary recombinant human NI-206.82C2, NI-206.59B4, NI-206.22F7, NI-206.27E8, NI-206.12G4, NI-206.17A6, NI-206.18H2, NI-206.20A8, NI-206.6D3, NI-206.14C5 and NI-206.34C11. Such antibodies and binding molecules can be tested for their binding specificity and affinity by ELISA and immunohistochemistry as described herein, see, *e.g.,* the Examples. These characteristics of the antibodies and binding molecules can be tested by Western Blot as well.

As an alternative to obtaining immunoglobulins directly from the culture of B cells or memory B cells, the cells can be used as a source of rearranged heavy chain and light chain loci for subsequent expression and/or genetic manipulation. Rearranged antibody genes can be reverse transcribed from appropriate mRNAs to produce cDNA. If desired, the heavy chain constant region can be exchanged for that of a different isotype or eliminated altogether. The variable regions can be linked to encode single chain Fv regions. Multiple Fv regions can be linked to confer binding ability to more than one target or chimeric heavy and light chain combinations can be employed. Once the genetic material is available, design of analogs as described above which retain both their ability to bind the desired target is straightforward. Methods for the cloning of antibody variable regions and generation of recombinant antibodies are known to the person skilled in the art and are described, for example, Gilliland et al., Tissue Antigens 47 (1996), 1-20; Doenecke et al., Leukemia 11 (1997), 1787-1792.

Once the appropriate genetic material is obtained and, if desired, modified to encode an analog, the coding sequences, including those that encode, at a minimum, the variable regions of the heavy and light chain, can be inserted into expression systems contained on vectors which can be transfected into standard recombinant host cells. A variety of such host cells may be used; for efficient processing, however, mammalian cells are preferred. Typical mammalian cell lines useful for this purpose include, but are not limited to, CHO cells, HEK 293 cells, or NSO cells.

The production of the antibody or analog is then undertaken by culturing the modified recombinant host under culture conditions appropriate for the growth of the host cells and the expression of the coding sequences. The antibodies are then recovered by isolating them from the culture. The expression systems are preferably designed to include signal peptides so that the resulting antibodies are secreted into the medium; however, intracellular production is also possible.

In accordance with the above, the present invention also relates to a polynucleotide encoding the antibody or equivalent binding molecule of the present invention, in case of the antibody preferably at least a variable region of an immunoglobulin chain of the antibody described above. Typically, said variable region encoded by the polynucleotide comprises at least one complementarity determining region (CDR) of the $V_H$ and/or $V_L$ of the variable region of the said antibody. In one embodiment of the present invention, the polynucleotide is a cDNA.

The person skilled in the art will readily appreciate that the variable domain of the antibody having the above-described variable domain can be used for the construction of other polypeptides or antibodies of desired specificity and biological function. Thus, the present invention also encompasses polypeptides and antibodies comprising at least one CDR of the above-described variable domain and which advantageously have substantially the same or similar binding properties as the antibody described in the appended examples. The person skilled in the art knows that binding affinity may be enhanced by making amino acid substitutions within the CDRs or within the hypervariable loops (Chothia and Lesk, J. Mol. Biol. 196 (1987), 901-917) which partially overlap with the CDRs as defined by Kabat; see, *e.g.,* Riechmann, et al, Nature 332 (1988), 323-327. Thus, the present invention also relates to antibodies wherein one or more of the mentioned CDRs comprise one or more, preferably not more than two amino acid substitutions. Preferably, the antibody of the invention comprises in one or both of its immunoglobulin chains two or all three CDRs of the variable regions as set forth in Figs. 1G-K and 1A-1F.

Binding molecules, *e.g.,* antibodies, or antigen-binding fragments, variants, or derivatives thereof of the invention, as known by those of ordinary skill in the art, can comprise a constant region which mediates one or more effector functions. For example, binding of the C1 component of complement to an antibody constant region may activate the complement system. Activation of complement is important in the opsonization and lysis of cell pathogens. The activation of complement also stimulates the inflammatory response and may also be involved in autoimmune hypersensitivity. Further, antibodies bind to receptors on various cells via the Fc region, with a Fc receptor binding site on the antibody Fc region binding to a Fc receptor (FcR) on a cell. There are a number of Fc receptors which are specific for different classes of antibody, including IgG (gamma receptors), IgE (epsilon receptors), IgA (alpha receptors) and IgM (mu receptors). Binding of antibody to Fc receptors on cell surfaces triggers a number of important and diverse biological responses including engulfment and destruction of antibody-coated particles, clearance of immune complexes, lysis of antibody-coated target cells by killer cells (called antibody-dependent cell-mediated cytotoxicity, or ADCC), release of inflammatory mediators, placental transfer and control of immunoglobulin production.

Accordingly, certain embodiments of the present invention include an antibody, or antigen-binding fragment, variant, or derivative thereof, in which at least a fraction of one or more of the constant region domains has been deleted or otherwise altered so as to provide desired biochemical characteristics such as reduced effector functions, the ability to non-covalently dimerize, increased ability to localize at the site of FAP expression on the cell surface, *e.g.,* on tumor cells, reduced serum half-life, or increased serum half-life when compared with a whole, unaltered antibody of approximately the same immunogenicity. For example, certain antibodies for use in the diagnostic and treatment methods described herein are domain deleted antibodies which comprise a polypeptide chain similar to an immunoglobulin heavy chain, but which lack at least a portion of one or more heavy chain domains. For instance, in certain antibodies, one entire domain of the constant region of the modified antibody will be deleted, for example, all or part of the CH2 domain will be deleted. In other embodiments, certain antibodies for use in the diagnostic and treatment methods described herein have a constant region, *e.g.,* an IgG heavy chain constant region, which is altered to eliminate glycosylation, referred to elsewhere herein as aglycosylated or "agly" antibodies. Such "agly" antibodies may be prepared enzymatically as well as by engineering the consensus glycosylation site(s) in the constant region. While not being bound by theory, it is believed that "agly" antibodies may have an improved safety and stability profile *in vivo.* Methods of producing aglycosylated antibodies, having desired effector function are found for example in international application WO 2005/018572, which is incorporated by reference in its entirety.

In certain antibodies, or antigen-binding fragments, variants, or derivatives thereof described herein, the Fc portion may be mutated to decrease effector function using techniques known in the art. For example, the deletion or inactivation (through point mutations or other means) of a constant region domain may reduce Fc receptor binding of the circulating modified antibody thereby increasing FAP localization. In other cases it may be that constant region modifications consistent with the instant invention moderate complement binding and thus reduce the serum half-life and nonspecific association of a conjugated cytotoxin. Yet other modifications of the constant region may be used to modify disulfide linkages or oligosaccharide moieties that allow for enhanced localization due to increased antigen specificity or antibody flexibility. The resulting physiological profile, bioavailability and other biochemical effects of the modifications, such as FAP localization, biodistribution and serum half-life, may easily be measured and quantified using well know immunological techniques without undue experimentation.

In certain antibodies, or antigen-binding fragments, variants, or derivatives thereof described herein, the Fc portion may be mutated or exchanged for alternative protein sequences to increase the cellular uptake of antibodies by way of example by enhancing receptor-mediated endocytosis of antibodies via Fcγ receptors, LRP, or Thy1 receptors or by 'SuperAntibody Technology', which is said to enable antibodies to be shuttled into living cells without harming them (Expert Opin. Biol. Ther. (2005), 237-241). For example, the generation of fusion proteins of the antibody binding region and the cognate protein ligands of cell surface receptors or bi- or multi-specific antibodies with a specific sequences binding to FAP as well as a cell surface receptor may be engineered using techniques known in the art.

In certain antibodies, or antigen-binding fragments, variants, or derivatives thereof described herein, the Fc portion may be mutated or exchanged for alternative protein sequences or the antibody may be chemically modified to increase its blood brain barrier penetration.

Modified forms of antibodies, or antigen-binding fragments, variants, or derivatives thereof of the invention can be made from whole precursor or parent antibodies using techniques known in the art. Exemplary techniques are discussed in more detail herein. Antibodies, or antigen-binding fragments, variants, or derivatives thereof of the invention can be made or manufactured using techniques that are known in the art. In certain embodiments, antibody molecules or fragments thereof are "recombinantly produced", *i.e.*, are produced using recombinant DNA technology. Exemplary techniques for making antibody molecules or fragments thereof are discussed in more detail elsewhere herein.

Antibodies, or antigen-binding fragments, variants, or derivatives thereof of the invention also include derivatives that are modified, *e.g.,* by the covalent attachment of any type of molecule to the antibody such that covalent attachment does not prevent the antibody from specifically binding to its cognate epitope. For example, but not by way of limitation, the antibody derivatives include antibodies that have been modified, *e.g.,* by glycosylation, acetylation, pegylation, phosphorylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the derivative may contain one or more non-classical amino acids.

In particular preferred embodiments, antibodies, or antigen-binding fragments, variants, or derivatives thereof of the invention will not elicit a deleterious immune response in the animal to be treated, *e.g.,* in a human. In certain embodiments, binding molecules, *e.g.,* antibodies, or antigen-binding fragments thereof of the invention are derived from a patient, *e.g.,* a human patient, and are subsequently used in the same species from which they are derived, *e.g.,* human, alleviating or minimizing the occurrence of deleterious immune responses.

De-immunization can also be used to decrease the immunogenicity of an antibody. As used herein, the term "de-immunization" includes alteration of an antibody to modify T cell epitopes; *see, e.g.,* international applications WO 98/52976 and WO 00/34317. For example, $V_H$ and $V_L$ sequences from the starting antibody are analyzed and a human T cell epitope "map" from each V region showing the location of epitopes in relation to complementarity determining regions (CDRs) and other key residues within the sequence. Individual T cell epitopes from the T cell epitope map are analyzed in order to identify alternative amino acid substitutions with a low risk of altering activity of the final antibody. A range of alternative $V_H$ and $V_L$ sequences are designed comprising combinations of amino acid substitutions and these sequences are subsequently incorporated into a range of binding polypeptides, *e.g.,* FAP-specific antibodies or immunospecific fragments thereof for use in the diagnostic and treatment methods disclosed herein, which are then tested for function. Typically, between 12 and 24 variant antibodies are generated and tested. Complete heavy and light chain genes comprising modified V and human C regions are then cloned into expression vectors and the subsequent plasmids introduced into cell lines for the production of whole antibody. The antibodies are then compared in appropriate biochemical and biological assays, and the optimal variant is identified.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, Cold Spring Harbor Laboratory Press, 2nd ed. (1988); Hammerling et al., in: Monoclonal Antibodies and T-Cell Hybridomas Elsevier, N.Y., 563-681 (1981), said references incorporated by reference in their entireties. The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced. Thus, the term "monoclonal antibody" is not limited to antibodies produced through hybridoma technology. In certain embodiments, antibodies of the present invention are derived from human B cells which have been immortalized via transformation with Epstein-Barr virus, as described herein.

In the well-known hybridoma process (Kohler et al., Nature 256 (1975), 495) the relatively short-lived, or mortal, lymphocytes from a mammal, *e.g.,* B cells derived from a human subject as described herein, are fused with an immortal tumor cell line (*e.g.,*. a myeloma cell line), thus, producing hybrid cells or "hybridomas" which are both immortal and capable of producing the genetically coded antibody of the B cell. The resulting hybrids are segregated into single genetic strains by selection, dilution, and re-growth with each individual strain comprising specific genes for the formation of a single antibody. They produce antibodies, which are homogeneous against a desired antigen and, in reference to their pure genetic parentage, are termed "monoclonal".

Hybridoma cells thus prepared are seeded and grown in a suitable culture medium that preferably contains one or more substances that inhibit the growth or survival of the unfused, parental myeloma cells. Those skilled in the art will appreciate that reagents, cell lines and media for the formation, selection and growth of hybridomas are commercially available from a number of sources and standardized protocols are well established. Generally, culture medium in which the hybridoma cells are growing is assayed for production of monoclonal antibodies against the desired antigen. The binding specificity of the monoclonal antibodies produced by hybridoma cells is determined by *in vitro* assays such as immunoprecipitation, radioimmunoassay (RIA) or enzyme-linked immunoabsorbent assay (ELISA) as described herein. After hybridoma cells are identified that produce antibodies of the desired specificity, affinity and/or activity, the clones may be subcloned by limiting dilution procedures and grown by standard methods; *see, e.g.,* Goding, Monoclonal Antibodies: Principles and Practice, Academic Press (1986), 59-103. It will further be appreciated that the monoclonal antibodies secreted by the subclones may be separated from culture medium, ascites fluid or serum by conventional purification procedures such as, for example, protein-A, hydroxylapatite chromatography, gel electrophoresis, dialysis or affinity chromatography.

In another embodiment, lymphocytes can be selected by micromanipulation and the variable genes isolated. For example, peripheral blood mononuclear cells can be isolated from an immunized or naturally immune mammal, *e.g.,* a human, and cultured for about 7 days *in vitro.* The cultures can be screened for specific IgGs that meet the screening criteria. Cells from positive wells can be isolated. Individual Ig-producing B cells can be isolated by FACS or by identifying them in a complement-mediated hemolytic plaque assay. Ig-producing B cells can be micromanipulated into a tube and the $V_H$ and $V_L$ genes can be amplified using, *e.g.,* RT-PCR. The $V_H$ and $V_L$ genes can be cloned into an antibody expression vector and transfected into cells (e.g., eukaryotic or prokaryotic cells) for expression.

Alternatively, antibody-producing cell lines may be selected and cultured using techniques well known to the skilled artisan. Such techniques are described in a variety of laboratory manuals and primary publications. In this respect, techniques suitable for use in the invention as described below are described in Current Protocols in Immunology, Coligan et al., Eds., Green Publishing Associates and Wiley-Interscience, John Wiley and Sons, New York (1991) which is herein incorporated by reference in its entirety, including supplements.

Antibody fragments that recognize specific epitopes may be generated by known techniques. For example, Fab and $F(ab')_2$ fragments may be produced recombinantly or by proteolytic cleavage of immunoglobulin molecules, using enzymes such as papain (to produce Fab fragments) or pepsin (to produce $F(ab')_2$ fragments). $F(ab')_2$ fragments contain the variable region, the light chain constant region and the CH1 domain of the heavy chain. Such fragments are sufficient for use, for example, in immunodiagnostic procedures involving coupling the immunospecific portions of immunoglobulins to detecting reagents such as radioisotopes.

In one embodiment, an antibody of the invention comprises at least one CDR of an antibody molecule. In another embodiment, an antibody of the invention comprises at least two CDRs from one or more antibody molecules. In another embodiment, an antibody of the invention comprises at least three CDRs from one or more antibody molecules. In another embodiment, an antibody of the invention comprises at least four CDRs from one or more antibody molecules. In another embodiment, an antibody of the invention comprises at least five CDRs from one or more antibody molecules. In another embodiment, an antibody of the invention comprises at least six CDRs from one or more antibody molecules. Exemplary antibody molecules comprising at least one CDR that can be included in the subject antibodies are described herein.

Antibodies of the present invention can be produced by any method known in the art for the synthesis of antibodies, in particular, by chemical synthesis or preferably by recombinant expression techniques as described herein.

In one embodiment, an antibody, or antigen-binding fragment, variant, or derivative thereof of the invention comprises a synthetic constant region wherein one or more domains are partially or entirely deleted ("domain-deleted antibodies"). In certain embodiments compatible modified antibodies will comprise domain deleted constructs or variants wherein the entire CH2 domain has been removed (ΔCH2 constructs). For other embodiments a short connecting peptide may be substituted for the deleted domain to provide flexibility and freedom of movement for the variable region. Those skilled in the art will appreciate that such constructs are particularly preferred due to the regulatory properties of the CH2 domain on the catabolic rate of the antibody. Domain deleted constructs can be derived using a vector encoding an $IgG_1$ human constant domain, *see, e.g.,* international applications WO 02/060955 and WO 02/096948A2. This vector is engineered to delete the CH2 domain and provide a synthetic vector expressing a domain deleted $IgG_1$ constant region.

In certain embodiments, antibodies, or antigen-binding fragments, variants, or derivatives thereof of the present invention are minibodies. Minibodies can be made using methods described in the art, *see, e.g.,* US patent 5,837,821 or international application WO 94/09817.

In one embodiment, an antibody, or antigen-binding fragment, variant, or derivative thereof of the invention comprises an immunoglobulin heavy chain having deletion or substitution of a few or even a single amino acid as long as it permits association between the monomeric subunits. For example, the mutation of a single amino acid in selected areas of the CH2 domain may be enough to substantially reduce Fc binding and thereby increase FAP localization. Similarly, it may be desirable to simply delete that part of one or more constant region domains that control the effector function (e.g. complement binding) to be modulated. Such partial deletions of the constant regions may improve selected characteristics of the antibody (serum half-life) while leaving other desirable functions associated with the subject constant region domain intact. Moreover, as alluded to above, the constant regions of the disclosed antibodies may be synthetic through the mutation or substitution of one or more amino acids that enhances the profile of the resulting construct. In this respect it may be possible to disrupt the activity provided by a conserved binding site *(e.g.* Fc binding) while substantially maintaining the configuration and immunogenic profile of the modified antibody. Yet other embodiments comprise the addition of one or more amino acids to the constant region to enhance desirable characteristics such as an effector function or provide for more cytotoxin or carbohydrate attachment. In such embodiments it may be desirable to insert or replicate specific sequences derived from selected constant region domains.

The present invention also provides antibodies that comprise, consist essentially of, or consist of, variants (including derivatives) of antibody molecules (e.g., the $V_H$ regions and/or $V_L$ regions) described herein, which antibodies or fragments thereof immunospecifically bind to FAP. Standard techniques known to those of skill in the art can be used to introduce mutations in the nucleotide sequence encoding an antibody, including, but not limited to, site-directed mutagenesis and PCR-mediated mutagenesis which result in amino acid substitutions. Preferably, the variants (including derivatives) encode less than 50 amino acid substitutions, less than 40 amino acid substitutions, less than 30 amino acid substitutions, less than 25 amino acid substitutions, less than 20 amino acid substitutions, less than 15 amino acid substitutions, less than 10 amino acid substitutions, less than 5 amino acid substitutions, less than 4 amino acid substitutions, less than 3 amino acid substitutions, or less than 2 amino acid substitutions relative to the reference $V_H$ region, $V_H$-CDR1, $V_H$-CDR2, $V_H$-CDR3, $V_L$ region, $V_L$-CDR1, $V_L$-CDR2, or $V_L$-CDR3. A "conservative amino acid substitution" is one in which the amino acid residue is replaced with an amino acid residue having a side chain with a similar charge. Families of amino acid residues having side chains with similar charges have been defined in the art. These families include amino acids with basic side chains *(e.g.,* lysine, arginine, histidine), acidic side chains *(e.g.,* aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain activity (e.g., the ability to bind and inhibit FAP).

For example, it is possible to introduce mutations only in framework regions or only in CDR regions of an antibody molecule. Introduced mutations may be silent or neutral missense mutations, *e.g.,* have no, or little, effect on an antibody's ability to bind antigen, indeed some such mutations do not alter the amino acid sequence whatsoever.

These types of mutations may be useful to optimize codon usage, or improve a hybridoma's antibody production. Codon-optimized coding regions encoding antibodies of the present invention are disclosed elsewhere herein. Alternatively, non-neutral missense mutations may alter an antibody's ability to bind antigen. The location of most silent and neutral missense mutations is likely to be in the framework regions, while the location of most non-neutral missense mutations is likely to be in CDR, though this is not an absolute requirement. One of skill in the art would be able to design and test mutant molecules with desired properties such as no alteration in antigen-binding activity or alteration in binding activity (e.g., improvements in antigen-binding activity or change in antibody specificity). Following mutagenesis, the encoded protein may routinely be expressed and the functional and/or biological activity of the encoded protein, (e.g., ability to immunospecifically bind at least one epitope of FAP) can be determined using techniques described herein or by routinely modifying techniques known in the art.

**III. Chimeric antigen receptors (CARs) of the present invention**

As mentioned, next to human-derived anti-FAP antibodies, in a further aspect the present invention generally relates to chimeric antigen receptors (CARs), comprising a variable domain or binding domain, e.g. scFv fragment from said human-derived anti-FAP antibodies of the present invention. Thus, it is prudent to expect that the CARs of the present invention exhibit the same or similar characteristics, especially binding characteristics like the anti-FAP antibodies of the present invention. The CARs of the present invention are preferably used for treating cancer, in particular cancer that may be selected from hematological malignancy, a solid tumor, a primary or a metastasizing tumor. Since FAP is mainly associated with stromal cells, said CARs preferentially target the stromal cell population in tumor microenvironment. Thus, in one embodiment the present invention provides CARs targeting stromal cells, rather than tumor cells directly, as it was seen that stromal cells existing in the tumor microenvironment have tumorigenic activity. For example, stromal cells in tumor microenvironments promote tumor growth and metastasis. Therefore, targeting of FAP expressing stromal cells affects a tumor cell so that the tumor cell fails to grow, is prompted to die, or otherwise is affected so that the tumor burden in a patient is diminished or eliminated.

As mentioned hereinbefore, the basic structure of CARs is well known in the art; see also the documents cited in the background section and, for example Gross and Eshhar, Therapeutic Potential of T Cell Chimeric Antigen Receptors (CARs) in Cancer Treatment: Counteracting Off-Tumor Toxicities for Safe CAR T Cell Therapy in Annual Review of Pharmacology and Toxicology 56 (2016), 59-83 and Zhang et al., New Strategies for the Treatment of Solid Tumors with CAR-T Cells in Int. J. Biol. Sci. 12 (2016), 718-729. In one embodiment of the present invention, the FAP binding domain (extracellular domain) of said CAR is preferably fused with an intracellular domain (cytoplasmic domain) comprising a costimulatory signaling region and a zeta chain portion. Preferably, the antigen binding domain is fused with one or more intracellular domains selected from the group of a CD 137 (4-lBB) signaling domain, a CD28 signaling domain, a CD3zeta signal domain, and any combination thereof. The costimulatory signaling region refers to a portion of the CAR comprising the intracellular domain of a costimulatory molecule. Costimulatory molecules are cell surface molecules other than antigens receptors or their ligands that are required for an efficient response of lymphocytes to antigen.

Between the extracellular domain and the transmembrane domain of the CAR, or between the cytoplasmic domain and the transmembrane domain of the CAR, there may be incorporated a spacer domain. As used herein, the term "spacer domain" generally means any oligo- or polypeptide that functions to link the transmembrane domain to, either the extracellular domain or, the cytoplasmic domain in the polypeptide chain. A spacer domain may comprise up to 300 amino acids, preferably 10 to 100 amino acids and most preferably 25 to 50 amino acids. Preferably, the CAR comprises an extracellular domain, a transmembrane domain and a cytoplasmic domain. Detailed description of the preparation of such CARs and amino acid sequences of the spacer domain, hinge domain, signaling domains and transmembrane domains that can be used for the construction of CARs in accordance with the present invention can be found, *e.g.,* in international application WO 2014/055442.

In general, the extracellular domain, *i.e.* the FAP antigen binding domain of the CAR of the present invention may comprise or consist of the anti-FAP antibody heavy chain variable region which may in turn be covalently associated with the anti-FAP antibody light chain variable region by virtue of the presence of CH1 constant domain and hinge region or by virtue of the presence hinge, CH2 and CH3 domain. Thus, the CAR of the present invention can be engineered to include any FAP-binding moiety of any one of the antibodies of the present invention. In one embodiment, the FAP binding domain and its encoding nucleic acid sequence, respectively, comprises a nucleic acid sequence comprising SEQ ID NOs: 1, 3, 5, 7, 9, 11, 13, 15, 17, 19, 21, 23, 25, 40, 42, 44, 46, 48, 50, 52, 54, 56 or 58 or a part of said nucleic acid sequences, at least a part encoding one CDR of the light or heavy chain of the antibodies encoded by said nucleic acids. In one embodiment, the FAP binding domain comprises an amino acid sequence comprising SEQ ID NOs: 2, 4, 6, 8, 10, 12, 14, 16, 18, 20, 22, 24, 26, 41, 43, 45, 47, 51, 53, 55, 57 or 59 or a part of said amino acid sequences, at least a part constituting at least one CDR of the light or heavy chain of said antibodies.

With respect to the transmembrane domain, in general the CAR can be designed to comprise a transmembrane domain that is fused to the extracellular domain of the CAR. In one embodiment, the transmembrane domain that naturally is associated with one of the domains in the CAR is used. In some instances, the transmembrane domain can be selected or modified by amino acid substitution to avoid binding of such domains to the transmembrane domains of the same or different surface membrane proteins to minimize interactions with other members of the receptor complex.

The transmembrane domain may be derived either from a natural or from a synthetic source. Where the source is natural, the domain may be derived from any membrane-bound or transmembrane protein. Transmembrane regions of particular use in this invention may be derived from (*i.e.* comprise) at least the transmembrane region(s) of) the alpha, beta or zeta chain of the T-cell receptor, CD28, CD3 epsilon, CD45, CD4, CDS, CD8, CD9, CD16, CD22, CD33, CD37, CD64, CD80, CD86, CD134, CD137, CD154. Alternatively the transmembrane domain may be synthetic, in which case it will comprise predominantly hydrophobic residues such as leucine and valine. Preferably a triplet of phenylalanine, tryptophan and valine will be found at each end of a synthetic transmembrane domain. Optionally, a short oligo- or polypeptide linker, preferably between 2 and 10 amino acids in length may form the linkage between the transmembrane domain and the cytoplasmic signalling domain of the CAR. A glycine-serine doublet provides a particularly suitable linker.

The cytoplasmic domain or otherwise the intracellular signalling domain of the CAR of the present invention is responsible for activation of at least one of the normal effector functions of the immune cell in which the CAR has been placed in. The term "effector function" refers to a specialized function of a cell. Effector function of a T cell, for example, may be cytolytic activity or helper activity including the secretion of cytokines. Thus the term "intracellular signalling domain" refers to the portion of a protein which transduces the effector function signal and directs the cell to perform a specialized function. While usually the entire intracellular signalling domain can be employed, in many cases it is not necessary to use the entire chain. To the extent that a truncated portion of the intracellular signalling domain is used, such truncated portion may be used in place of the intact chain as long as it transduces the effector function signal. The term intracellular signalling domain is thus meant to include any truncated portion of the intracellular signalling domain sufficient to transduce the effector function signal.

Preferred examples of intracellular signalling domains for use in the CAR of the invention include the cytoplasmic sequences of the T cell receptor (TCR) and co-receptors that act in concert to initiate signal transduction following antigen receptor engagement, as well as any derivative or variant of these sequences and any synthetic sequence that has the same functional capability. It is known that signals generated through the TCR alone are insufficient for full activation of the T cell and that a secondary or co-stimulatory signal is also required. Thus, T cell activation can be said to be mediated by two distinct classes of cytoplasmic signalling sequence: those that initiate antigen-dependent primary activation through the TCR (primary cytoplasmic signalling sequences) and those that act in an antigen-independent manner to provide a secondary or co-stimulatory signal (secondary cytoplasmic signalling sequences).

Primary cytoplasmic signalling sequences regulate primary activation of the TCR complex either in a stimulatory way, or in an inhibitory way. Primary cytoplasmic signalling sequences that act in a stimulatory manner may contain signalling motifs which are known as immunoreceptor tyrosine-based activation motifs or ITAMs.

Examples of ITAM containing primary cytoplasmic signalling sequences that are of particular use in the invention include those derived from TCR zeta, FcR gamma, FcR beta, CD3 gamma, CD3 delta, CD3 epsilon, CDS, CD22, CD79a, CD79b, and CD66d. It is particularly preferred that cytoplasmic signalling molecule in the CAR of the invention comprises a cytoplasmic signalling sequence derived from CD3 zeta.

In a preferred embodiment, the cytoplasmic domain of the CAR can be designed to comprise the CD3-zeta signalling domain by itself or combined with any other desired cytoplasmic domain(s) useful in the context of the CAR of the invention. For example, the cytoplasmic domain of the CAR can comprise a CD3 zeta chain portion and a costimulatory signalling region. The costimulatory signalling region refers to a portion of the CAR comprising the intracellular domain of a costimulatory molecule. A costimulatory molecule is a cell surface molecule other than an antigen receptor or their ligands that is required for an efficient response of lymphocytes to an antigen. Examples of such molecules include CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, and a ligand that specifically binds with CD83, and the like. Thus, while the invention in exemplified primarily with 4-1BB and CD28 as the co-stimulatory signaling element, other costimulatory elements are within the scope of the invention.

The cytoplasmic signaling sequences within the cytoplasmic signaling portion of the CAR of the invention may be linked to each other in a random or specified order. Optionally, a short oligo- or polypeptide linker, preferably between 2 and 10 amino acids in length may form the linkage. A glycine-serine doublet provides a particularly suitable linker.

In one embodiment, the cytoplasmic domain is designed to comprise the signaling domain of CD3-zeta and the signaling domain of CD28. In yet another embodiment, the cytoplasmic domain is designed to comprise the signaling domain of CD3-zeta and the signaling domain of CD28 and 4-lBB.

Furthermore, the person skilled in the art will appreciate that in principle the design of FAP CARs described in the prior art, for example in Tran et al. (J. Exp. Med. 210 (2013), 1125-1135), Schuberth et al. (J. Transl. Med. 11 (2013), 187), Kakarla et al. (Mol. Ther. 21 (2013),1611-1620), Wang et al. (Cancer Immunol. Res. 2 (2014), 154-166 as well as in WO 2014055442 A2 using FAP binding domains derived from mouse monoclonal anti-FAP antibodies such as antibody F19 can be used to construct the FAP CARs of the present invention via replacing the described FAP binding domain with the FAP binding domain of the human monoclonal anti-FAP antibodies described in the present invention.

**IV. Polynucleotides Encoding Antibodies and CARs**

The present invention also relates to DNA constructs comprising a nucleic acid sequence encoding a CAR and/or an antibody, or antigen-binding fragment, variant, or derivative thereof; see *supra.* In case of the CAR, the DNA construct comprises the nucleic acid sequence encoding any one of the FAP binding domains of the present invention operably liked to the nucleic acid sequence encoding an intracellular domain. An exemplary intracellular domain that can be used in the CAR of the invention includes but is not limited to the intracellular domain of CD3-zeta, CD28, 4-lBB, and the like. In some instances, the CAR can comprise any combination of CD3-zeta, CD28, 4-lBB, and the like.

A polynucleotide encoding a CAR or an antibody, or antigen-binding fragment, variant, or derivative thereof can be composed of any polyribonucleotide or polydeoxribonucleotide, which may be unmodified RNA or DNA or modified RNA or DNA. For example, a polynucleotide encoding a CAR or an antibody, or antigen-binding fragment, variant, or derivative thereof can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single-stranded and double-stranded regions. In addition, a polynucleotide encoding a CAR or an antibody, or antigen-binding fragment, variant, or derivative thereof can be composed of triple-stranded regions comprising RNA or DNA or both RNA and DNA. A polynucleotide encoding a CAR or an antibody, or antigen-binding fragment, variant, or derivative thereof may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

An isolated polynucleotide encoding a non-natural variant of a polypeptide derived from an immunoglobulin (e.g., an immunoglobulin heavy chain portion or light chain portion) or a CAR can be created by introducing one or more nucleotide substitutions, additions or deletions into the nucleotide sequence such that one or more amino acid substitutions, additions or deletions are introduced into the encoded protein. Mutations may be introduced by standard techniques, such as site-directed mutagenesis and PCR-mediated mutagenesis. Preferably, conservative amino acid substitutions are made at one or more non-essential amino acid residues.

As is well known, RNA may be isolated from the original B cells, hybridoma cells or from other transformed cells by standard techniques, such as a guanidinium isothiocyanate extraction and precipitation followed by centrifugation or chromatography. Where desirable, mRNA may be isolated from total RNA by standard techniques such as chromatography on oligo dT cellulose.

Suitable techniques are familiar in the art. In one embodiment, cDNAs that encode the light and the heavy chains of the antibody may be made, either simultaneously or separately, using reverse transcriptase and DNA polymerase in accordance with well-known methods. PCR may be initiated by consensus constant region primers or by more specific primers based on the published heavy and light chain DNA and amino acid sequences. As discussed above, PCR also may be used to isolate DNA clones encoding the antibody light and heavy chains. In this case the libraries may be screened by consensus primers or larger homologous probes, such as human constant region probes.

DNA, typically plasmid DNA, may be isolated from the cells using techniques known in the art, restriction mapped and sequenced in accordance with standard, well known techniques set forth in detail, *e.g.,* in the foregoing references relating to recombinant DNA techniques. Of course, the DNA may be synthetic according to the present invention at any point during the isolation process or subsequent analysis.

In this context, the present invention also relates to a polynucleotide encoding at least the binding domain or variable region of an immunoglobulin chain of the antibody of the present invention, preferably which can constitute the binding domain (extracellular domain) of the CAR of the present invention. In one embodiment, the present invention provides an isolated polynucleotide comprising, consisting essentially of, or consisting of a nucleic acid encoding an immunoglobulin heavy chain variable region ($V_H$), where at least one of the CDRs of the heavy chain variable region or at least two of the $V_H$-CDRs of the heavy chain variable region are at least 80%, 85%, 90%, or 95% identical to reference heavy chain $V_H$-CDR1, $V_H$-CDR2, or $V_H$-CDR3 amino acid sequences from the antibodies disclosed herein. Alternatively, the $V_H$-CDR1, $V_H$-CDR2, or $V_H$-CDR3 regions of the $V_H$ are at least 80%, 85%, 90%, or 95% identical to reference heavy chain $V_H$-CDR1, $V_H$-CDR2, and $V_H$-CDR3 amino acid sequences from the antibodies disclosed herein. Thus, according to this embodiment a heavy chain variable region of the invention has $V_H$-CDR1, $V_H$-CDR2, or $V_H$-CDR3 polypeptide sequences related to the polypeptide sequences shown in Figs. 1G-K and 1A-1F.

In another embodiment, the present invention provides an isolated polynucleotide comprising, consisting essentially of, or consisting of a nucleic acid encoding an immunoglobulin light chain variable region ($V_L$), preferably which can constitute the binding domain (extracellular domain) of the CAR of the present invention, where at least one of the

$V_L$-CDRs of the light chain variable region or at least two of the $V_L$-CDRs of the light chain variable region are at least 80%, 85%, 90%, or 95% identical to reference light chain $V_L$-CDR1, $V_L$-CDR2, or $V_L$-CDR3 amino acid sequences from the antibodies disclosed herein. Alternatively, the $V_L$-CDR1, $V_L$-CDR2, or $V_L$-CDR3 regions of the $V_L$ are at least 80%, 85%, 90%, or 95% identical to reference light chain $V_L$-CDR1, $V_L$-CDR2, and $V_L$-CDR3 amino acid sequences from the antibodies disclosed herein. Thus, according to this embodiment a light chain variable region of the invention has $V_L$-CDR1, $V_L$-CDR2, or $V_L$-CDR3 polypeptide sequences related to the polypeptide sequences shown in Figs. 1G-K and 1A-1F.

In another embodiment, the present invention provides an isolated polynucleotide comprising, consisting essentially of, or consisting of a nucleic acid encoding an immunoglobulin heavy chain variable region ($V_H$), preferably which can constitute the binding domain (extracellular domain) of the CAR of the present invention, in which the $V_H$-CDR1, $V_H$-CDR2, and $V_H$-CDR3 regions have polypeptide sequences which are identical to the $V_H$-CDR1, $V_H$-CDR2, and $V_H$-CDR3 groups shown in Figs. 1G-K and 1A-1F.

As known in the art, "sequence identity" between two polypeptides or two polynucleotides is determined by comparing the amino acid or nucleic acid sequence of one polypeptide or polynucleotide to the sequence of a second polypeptide or polynucleotide. When discussed herein, whether any particular polypeptide is at least about 40%, 45%, 50%, 55%, 60%, 65%, 70%, 75%, 80%, 85%, 90%, or 95% identical to another polypeptide can be determined using methods and computer programs/software known in the art such as, but not limited to, the BESTFIT program (Wisconsin Sequence Analysis Package, Version 8 for Unix, Genetics Computer Group, University Research Park, 575 Science Drive, Madison, WI 53711). BESTFIT uses the local homology algorithm of Smith and Waterman, Advances in Applied Mathematics 2 (1981), 482-489, to find the best segment of homology between two sequences. When using BESTFIT or any other sequence alignment program to determine whether a particular sequence is, for example, 95% identical to a reference sequence according to the present invention, the parameters are set, of course, such that the percentage of identity is calculated over the full length of the reference polypeptide sequence and that gaps in homology of up to 5% of the total number of amino acids in the reference sequence are allowed.

In a preferred embodiment of the present invention, the polynucleotide comprises, consists essentially of, or consists of a nucleic acid having a polynucleotide sequence of the $V_H$ or $V_L$ region of an anti-FAP antibody and/or antibody recognizing FAP species and/or fragments thereof, preferably which can constitute the binding domain (extracellular domain) of the CAR of the present invention, as depicted in and Table II. In this respect, the person skilled in the art will readily appreciate that the polynucleotides encoding at least the variable domain of the light and/or heavy chain may encode the variable domain of both immunoglobulin chains or only one. In one embodiment therefore, the polynucleotide comprises, consists essentially of, or consists of a nucleic acid having a polynucleotide sequence of the $V_H$ and the $V_L$ region of an anti-FAP antibody as depicted in Table II.

**Table II:** Nucleotide sequences of the $V_H$ and $V_L$ region of antibodies recognizing human FAP or peptides thereof.

| Antibody | Nucleotide sequences of variable heavy (VH) and variable light (VL) chains or variable kappa-light chains (VK) |
|---|---|
| NI-206.82C2-VH (not PIMC) | CAGGTGCAGCTGCAGGAGTCGGGTCCAGGACTGGTGAAGCCCTCGCAGACCCTCTCAC TCACCTGTGCCATCTCCGGGGACAGTGTCTCTAGCAACAGTGTTACTTGGAACTGGAT CAGGCAGTCCCCATCGAGAGGCCTTGAGTGGCTGGGAAGGACATACTACAGGTCCAAG TGGTATAATGATTATGCAGTATCTGTGAAAGGTCGAATAACCATCAATCCAGACACTT CCAAGAACCAGTTCTACCTGCAGTTGAAATCTGTGACTCCCGAGGATGCGGCTGTCTA TTATTGTGCAAGAGATAGTAGCATCTTATATGGGGACTACTGGGGCCAGGGAACCCTG GTCACCGTCTCCTCG<br>*SEQ ID NO.: 1* |
| NI-206.82C2-VH (PIMC) | CAGGTACAGCTGCAGCAGTCAGGTCCAGGACTGGTGAAGCCCTCGCAGACCCTCTCAC TCACCTGTGCCATCTCCGGGGACAGTGTCTCTAGCAACAGTGTTACTTGGAACTGGAT CAGGCAGTCCCCATCGAGAGGCCTTGAGTGGCTGGGAAGGACATACTACAGGTCCAAG TGGTATAATGATTATGCAGTATCTGTGAAAGGTCGAATAACCATCAATCCAGACACTT CCAAGAACCAGTTCTACCTGCAGTTGAAATCTGTGACTCCCGAGGATGCGGCTGTCTA TTATTGTGCAAGAGATAGTAGCATCTTATATGGGGACTACTGGGGCCAGGGAACCCTG GTCACCGTCTCCTCG<br>*SEQ ID NO.: 3* |

(continued)

| Antibody | Nucleotide sequences of variable heavy (VH) and variable light (VL) chains or variable kappa-light chains (VK) |
|---|---|
| NI-206.82C2-VL (PIMC by default) | CAGGCTGTGCTGACTCAGCCGTCTTCCCTCTCTGCATCTCCTGGAGCATCAGCCAGTC<br>TCACCTGCACCTTGCCCAGTGGCATCAATGTTGGTACCTACAGGATATTCTGGTTCCA<br>GCAGAAGCCAGGGAGTCCTCCCCAGTATCTCCTGAGTTACAAATCAGACTCAGATAAT<br>CACCAGGGCTCTGGAGTCCCCAGCCGCTTCTCTGGATCCAAAGATGCTTCGGCCAATG<br>CAGGGATTTTACTCATCTCTGGGCTCCAGTCTGAGGATGAGGCTGACTATTACTGTAT<br>GATTTGGCACAGCAGCGCTTGGGTGTTCGGCGGAGGGACCAAGCTGACCGTCCTA<br>*SEQ ID NO.: 5* |
| NI-206.59B4-VH (PIMC) | CAGGTACAGCTGGTGCAATCTGGGGCTGAGGTGAAGAAGCCTGGGGCCTCAGTGAAGG<br>TCTCCTGCAAGACTTCTGGATACACCTTCACCGACTACTATATACACTGGGTGCGACA<br>GGCCCCTGGACAAGGGCTTGAATGGATGGGATGGATCAACCCTAACAGAGGTGGCACA<br>AACTATGCACAAAAATTTCAGGGCAGGGTCACCATGACCAGGGACACCTCCATCGCTA<br>CAGCCTACATGGAGTTGAGTAGACTGAGATCTGACGACACGGCCGTGTATTACTGTGC<br>GACTGCGTCGCTAAAAATAGCAGCAGTTGGTACATTTGACTGCTGGGGCCAGGGCACC<br>CTGGTCACCGTCTCCTCG<br>*SEQ ID NO.: 7* |
| NI-206.59B4-VL (PIMC) | TCCTATGAGCTGACTCAGCCACCCTCGGTGTCAGTGTCCCCAGGACAGACGGCCAGGA<br>TCACCTGCTCTGGAGATGCATTGTCAAAGCAATATGCTTTTTGGTTCCAGCAGAAGCC<br>AGGCCAGGCCCCTATATTGGTGATATATCAAGACACTAAGAGGCCCTCAGGGATCCCT<br>GGGCGATTCTCTGGCTCCAGCTCAGGGACAACAGTCACGTTGACCATCAGTGGAGCCC<br>AGGCAGACGACGAGGCTGACTATTATTGTCAATCAGCAGACAGCAGTGGTACTTATGT<br>CTTCGGAACTGGGACCAAGGTCACCGTCCTA<br>*SEQ ID NO.: 9* |
| NI-206.22F7-VH (not PIMC) | GAGGTGCAGCTGGTGGAGACTGGGGGAGGCGTGGTCCAGCCTGGGAGGTCCCTGAGAC<br>TCTCCTGTGCAGCCTCTGGATTCAGCTTCAGTACCCATGGCATGTACTGGGTCCGCCA<br>GCCTCCAGGCAAGGGGCTGGAGTGGGTGGCAGTTATATCATATGATGGAAGTGATAAA<br>AAGTATGCAGACTCCGTGAAGGGCCGGTTCACCATCTCCAGAGACAATTCCAAGAACA<br>CGGTGTATTTGGAAATGAGCAGCGTGAGAGCTGAGGACACGGCTCTATATTACTGTTT<br>CTGCCGCCGGGATGCTTTTGATCTCTGGGGCCAAGGGACAATGGTCACCGTCTCTTCG<br>*SEQ ID NO.: 11* |
| NI-206.22F7-VL (not PIMC) | TCCTATGTGCTGACTCAGCCACCCTCGGTGTCAGTGTCCCCAGGACAAACGGCCAGGA<br>TCACCTGCTCTGGAGATGCATTGCCAAAAAAGTATGCTTATTGGTACCAGCAGAAGTC<br>AGGCCAGGCCCCTGTGCTGGTCATCTATGAGGACACCAAACGACCCTCCGGGATCCCT<br>GAGAGATTCTCTGGCTCCAGCTCAGGGACAATGGCCACCTTGACTATCAGTGGGGCCC<br>AGGTGGAGGATGAAGCTGACTATTACTGTTACTCAACAGACAGCAGCGGTAATTATTG<br>GGTATTCGGCGGAGGGACCGAGGTGACCGTCCTA<br>*SEQ ID NO.: 13* |
| NI-206.27E8-VH (PIMC by default) | GAGGTGCAGCTGGTGGAGTCTGGGGGAGGCTTGGTTGAGCCTGGGGGGTCCCTAAGAC<br>TCTCCTGTGCAGCCTCTGGTTTCACTTTCAGTGATGCCTGGATGAACTGGGTCCGCCA<br>GGCTCCAGGGAAGGGGCTGGAGTGGGTCGGGCGTATTAAAACGAAAAGCGATGGTGGG<br>ACAACAGACTACGCTGCACCCGTGAGAGGCAGATTTTCCATCTCAAGAGATGATTCAA<br>AAAACACACTGTTTCTGGAAATGAACAGCCTGAAGACCGAGGACACAGCCATATATTA<br>TTGTTTTATTACTGTCATAGTAGTATCCTCCGAATCTCCACTTGACCACTGGGGCCAG<br>GGAACCCTGGTCACCGTCTCCTCG<br>*SEQ ID NO.: 15* |

(continued)

| Antibody | Nucleotide sequences of variable heavy (VH) and variable light (VL) chains or variable kappa-light chains (VK) |
|---|---|
| NI-206.27E8-VL (PIMC by default) | TCCTATGAGCTGACTCAGCCACCCTCGGTGTCAGTGTCCCCAGGACAGACGGCCAGGA TCACCTGCTCTGGAGACGAACTGCCAAAACAATATGCTTATTGGTACCAGCAGAAGCC AGGCCAGGCCCCTGTGTTGGTGATATATAAGGACAGACAGAGGCCCTCAGGGATCCCT GAGCGATTCTCTGGCTCCAGCTCAGGGACAACAGTCACGTTGACCATCAGTGGAGTCC AGGCAGAAGACGAGGCTGACTATTACTGTCAATCAGCATACAGCATTAATACTTATGT GATTTTCGGCGGAGGGACCAAGCTGACCGTCCTA<br>*SEQ ID NO.: 17* |
| NI-206.12G4-VH (not PIMC) | GAGGTGCAGCTGGTGGAGTCTGGGGGAGGCTTGGTCAAGCCTGGAGGGTCCCTGAGAC TCTCCTGTGCAGCCTCTGGATTCACCTTCAGTGACTACTACATGAGCTGGATCCGCCA GGCTCCAGGGAAGGGGCTGGAATGGATTTCTTATATTAGTAGTGGTAGTAGTTACACA AACTATGCAGACTCTGTGAAGGGCCGATTCACCATCTCCAGAGACAACGCCAAGAAGT CAGTGTATCTGGAAGTCAACGGCCTGACAGTCGAGGACACGGCTGTGTATTACTGTGC GAGAGTTCGATATGGGGACCGGGAGATGGCAACAATCGGAGGATTTGATTTCTGGGGC CAGGGAACCCTGGTCACCGTCTCCTCG<br>*SEQ ID NO.: 19* |
| NI-206.12G4-VL (PIMC by default) | TCCTATGAGCTGACTCAGCCACCCTCGGTGTCAGTGTCCCCAGGACAGACGGCCAGGA TCACCTGCTCTGGAGATGCACTGCCAAAGCAATATGCTTATTGGTATCAACAGAGCCC AGGCCAGGCCCCTGTGTTGGTGATATATAAAGACAGTGAGAGGCCCTCAGGGATCCCT GAGCGATTCTCTGGCTCCAGCTCAGGGACAACAGTCACGTTGACCATCAGTGGAGTCC AGGCAGAAGACGAGGCTGACTATTACTGTCAATCAGCAGACAGCGGTGGTACTTCTAG GATATTCGGCGGAGGGACCAAGTTGACCGTCCTG<br>*SEQ ID NO.: 21* |
| NI-206.17A6-VH (PIMC by default) | CAGGTGCAGCTGCAGGAGTCGGGCCCAGGACTGGTGAGGTCTACGGAGACCCTGTCCC TCACCTGCCTTGTCTCTGGTGACTCCATCAACAGTCACTACTGGAGTTGGCTCCGGCA GTCCCCAGGGAGGGGCCTGGAATGGATTGGGTACATTTACTACACTGGGCCCACCAAC TACAATCCCTCCCTCAAGAGTCGAGTCTCCATATCACTGGGCACGTCCAAGGACCAGT TCTCCCTGAAGCTGAGTTCTGTGACCGCTGCGGACACGGCCAGATATTACTGTGCGAG AAATAAGGTCTTTTGGCGTGGTTCTGACTTCTACTACTACATGGACGTCTGGGGCAAA GGGACCACGGTCACCGTCTCCTCG<br>*SEQ ID NO.: 23* |
| NI-206.17A6-VK (not PIMC) | GAAATTGTGTTGACACAGTCTCCAGGCACCCTGTCTTTGTCTCTAGGGGAAAGAGCCA CCCTCTCCTGCAGGGCCAGTCAGAGTCTTGCCAACAACTACTTAGCCTGGTACCAGCA GAAACCTGGCCAGGCTCCCAGGCTCCTCATGTATGACGCATCCACCAGGGCCACTGGC ATCCCTGACAGGTTCAGTGGCAGTGGGTCTGGGACAGACTTCACTCTCACCATCAGCA GACTGGAGCCTGAAGATTTTGCAGTGTATTACTGCCAGCAATTTGTTACCTCACACCA CATGTACATTTTTGGCCAGGGGACCAAGGTGGAAATCAAA<br>*SEQ ID NO.: 25* |
| NI-206.18H2-VH (not PIMC) | CAGGTGCAGCTGCAGGAGTCGGGCCCACGACTGGTGAAGCCTTCGGAGACCCTGTCTC TCACCTGCACTGTCTCTGGTTACTCCATTAGTAATGGTTACTACTGGGGCTGGATCCG ACAGCCCCCAGGGCAGGGGCTGGAGTGGATTGCGAGTGTCTGGCATAGTGGGAACACC TACCACAACCCGTCCCTCAAAAGTCGAGTCACCATTTTAGTGGACACGTTGAAGAACC AATTTTCCCTGAACCTGAAGTCTGTGACCGCCGCAGACACGGCCTTATATTACTGTGC GAGATCATATACACGAAACGACGTGGGCGCTTTTGAGACGTGGGGCCAAGGGACAATG GTCACCGTCTCTTCG<br>*SEQ ID NO.: 40* |

(continued)

| Antibody | Nucleotide sequences of variable heavy (VH) and variable light (VL) chains or variable kappa-light chains (VK) |
|---|---|
| NI-206.18H2-VL (PIMC by default) | TCCTATGAGCTGACTCAGCCACCCTCGGTGTCTGTGTCCCCAGGACAAACGGCCAGGA TCGCCTGCTCTGGAGATGAATTGCCAAAAAGATATGCTTATTGGTACCAGCAGAAGTC AGGCCAGGCCCCTGTACTGGTCATGTATGAGGACACCAAGCGACCCTCCGGGATTCCT GAGAGATTGTCTGGCTCCTCCTCAGGGACAGTGACCACTCTGACTATTAGTGGGGCCC AGGTGGAGGATGAGGCTGACTACTACTGTTACTCAACAGCCCCCAGTGGCAATCACAC TTTTGTCTTCGGAACTGGGACCAGGGTCACCGTCCTT<br>*SEQ ID NO.: 42* |
| NI-206.20A8-VH (PIMC by default) | GAGGTGCAGCTGGTGGAGTCCGGGGGAGGCTTGGTCCAGCCGGGGGGGTCCCTCAAAC TCTCCTGTGCAGGCTCTGGCTTAGACCTCAGTGCCTCTGCTGTGCACTGGGTCCGCCA GGCCTCCGGGAAAGGGCTGGAGTGGATTGGCCGCATCAGAAGCAAGCCTAATCACTAT GCGACAACATATCTGGCGTCGGTGAGAGGCAGATTCATCCTCTCCAGAGATGATTCAG AGAACACGGCCTATCTCCAAATGAACAGCCTGAGAACCGAGGACACAGCCGTATATTA CTGCAGAATTGGAATTCTGAATTCTGACCACTGGGGCCGGGGAACCCTGGTCACCGTC TCCTCG<br>*SEQ ID NO.: 44* |
| NI-206.20A8-VL (PIMC by default) | CAGTCTGTGCTGACTCAGCCACCCTCAGCGTCTGGGACCCCCGGGCAGACGGTCATCA TCTCTTGCTCTGGAAGCAGCTCCAATCTCGGAAGGAAAACTCTAAACTGGTACCAGCA ACTCCCAGGAGCGGCCCCCAAACTCCTCATTTTTAAAAATGATCAGCGGGCCTCAGGG GTCCCTGACCGATTCTCTGCCTCCAAGTCTGGCACCTCAGCCTCCCTGACCATCAGTG GACTCCAGTCTGACGATGAGGCTGATTATTACTGTGGAACATGGGATGACAGCCTGGA TAATTGGGTGTTCGGCGGAGGGACCAAGGTGACCGTCCTA<br>*SEQ ID NO.: 46* |
| NI-206.6D3-VH (PIMC by default) | GAGGTGCAGCTGGTGGAGTCTGGGGGAGGCTTGGTAAAGCCTGGGGAGTCCCTTAGAC TCTCCTGTGCAGCCTCTGGATTCACTTTCAGTAAGGCCTGGATGAACTGGGTCCGCCA GGGTCCAGGGAAGGGGCTGGAGTGGGTTGGCCGTGTTAAAAGCAAAACTGATGGTGGG ACAACAGACTACGCTGCACCCGTGAAAGGCAGATTCACCATCTCAAGAGACGATTCAA AAGACACAGTGTTTCTGCAAATGAACAGCCTGAAAACCGAAGATACAGCCGTATATTA CTGTTCCATCCAGTTTATTGTAGTAGGTGATAGGGGCCGAGACGACCAGTACATGGAC GTCTGGGGCAAAGGGACCACGGTCACCGTCTCCTCG<br>*SEQ ID NO.: 48* |
| NI-206.6D3-VL (not PIMC) | TCCTATGTGCTGACTCAGCCACCCTCGGTGTCAGTGTCCCCAGGACAGACGGCCAGGA TCACCTGCTCTGGAGATGCATTGCCAAAGCAATATGCTTTTTGGTACCAGCAGAAGCC AGGCCAGGCCCCTGTAATTATGATATATAAAGACAATCAGAGGCCCTCAGGGATCCCT GAGCGATTCTCTGGCTCCAGCTCAGGGACAACAGTCACGTTGACCATCAGTGGAGTCC AGACAGAAGACGAGGCTGACTATTACTGTCAATCAGCAGACAGCACTAATACTCATGT GGTATTCGGCGGAGGGACCAAGCTGACCGTCCTA<br>*SEQ ID NO.: 50* |
| NI-206.14C5-VH (PIMC by default) | CAGGTGCAGCTGCAGGAGTCGGGCCCAGGACTGGTGAAGCCTTCGGAGACCCTGTCCC TCACTTGCACTGTCTCTAATGGCTCCATCAATAATAACTACTGGAGCTGGCTCCGGCA GCCCCCCGGGAAGGGGCTGCAATGGATTGGTTATGTCTATTACAGTGGGAGCGCAAAG TATAACCCCTCCCTCCAGAGTCGAGTCTCTCTTTCAGTAGACAGATCCAAGAACCAAT TCTCCCTGGAGCTGAGCTCTGTCACCGCTGCGGACACGGCCGTCTATTACTGTGCGAG GACCTATTGTAGTGGTCGTGACACTTGTTTCTATTTCTTTGACAACTGGGGCCAGGGA ACCCTGGTCACCGTCTCCTCG<br>*SEQ ID NO.: 52* |

(continued)

| Antibody | Nucleotide sequences of variable heavy (VH) and variable light (VL) chains or variable kappa-light chains (VK) |
|---|---|
| NI-206.14C5-VL (PIMC by default) | CAGTCTGCCCTGACTCAGCCTGCCTCCGTGTCTGGGTCTCCTAGACAGTCGCTCACCA TCTCCTGCACTGGAACCATGAGTGATGTTGGGAGGTATGACCTTGTCTCATGGTACCA ACAACACCCTGGCAAAGCCCCCAACGTCATCATTTATGCCGTCACTAAGCGGCCCTCA GGGGTTTCTGATCGCTTCTCTGGCTCCAAGTCTGGCACCACGGCCTCCCTGACAATCT CTGGGCTCCAGGCTGAGGACGAGGCTTATTATTACTGCTGTTCATATGCAACTGTTAA CAGTTGGCTATTCGGCGGAGGGACCAAGGTGACCGTCCTA<br>*SEQ ID NO.: 54* |
| NI-206.34C11-VH (not PIMC) | GAGGTGCAGCTGGTGGAGACTGGGGGAGGCTTGGTACAGCCTGGGGGGTCCCTGAGAC TCGCCTGTGCAGCCTCTGGATTCACCTTTAGCAGCTATGCCATGAGCTGGGTCCGCCA GGCTCCAGGGAAGGGGCTGGAGTGGGTCTCAGGTATTAGTGATAGTGGTGGTAGCACA TACTACGCTGACGCCGTGAAGGGCCGGTTCACCATTTCCAAAGACAATTCCAAGAACA CCCTGTATCTGCAAATGAACAGCCTGAGAGCCGAGGACACGGCCGTATATTACTGTGC GAGAGTTGACTCTAATAGTATGGACGTCTGGGGCCAAGGGACCACGGTCACCGTCTCC TCG<br>*SEQ ID NO.: 56* |
| NI-206.34C11-VL (PIMC by default) | CAGTCTGTGTTGACGCAGCCGCCCTCACTGTCTGCGGCCCCAGGACAGAAGGTCACCA TCTCCTGCTCTGGAAGCAGCTCCAACATTGGGAATAATTATGTATCCTGGTACCAGCA ACTCCCAGGAACAGCCCCCAAACTCCTCATTTATAACAATGATAAGCGGCCCTCAGGG ATTTCTGACCGATTCTCTGGCTCCAAGTCTGGCACGTCAGCCACCCTGGGCATCACCG GACTCCAGACTGGGGACGAGGCCGATTATTACTGCGGAACATGGGATAGGAGCCTGAG TGGTAGGGTGTTCGGCGGAGGGACCAAGCTGACCGTCCTA<br>*SEQ ID NO.: 58* |

Due to the cloning strategy the amino acid sequence at the N- and C-terminus of the heavy chain and light chains may potentially contain primer-induced alterations in FR1 and FR4, which however do not substantially affect the biological activity of the antibody. In order to provide a consensus human antibody, the nucleotide and amino acid sequences of the original clone can be aligned with and tuned in accordance with the pertinent human germ line variable region sequences in the database; see, *e.g.,* Vbase2, as described above. The amino acid sequence of human antibodies are indicated in bold when N- and C-terminus amino acids are considered to potentially deviate from the consensus germ line sequence due to the PCR primer and thus have been replaced by primer-induced mutation correction (PIMC).

The present invention also includes fragments of the polynucleotides of the invention, as described elsewhere. Additionally polynucleotides which encode fusion polynucleotides, Fab fragments, and other derivatives, as described herein, are also contemplated by the invention. The polynucleotides may be produced or manufactured by any method known in the art. For example, if the nucleotide sequence of the antibody is known, a polynucleotide encoding the antibody may be assembled from chemically synthesized oligonucleotides, *e.g.,* as described in Kutmeier et al., BioTechniques 17 (1994), 242, which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding a CAR or an antibody, or antigen-binding fragment, variant, or derivative thereof may be generated from nucleic acid from a suitable source. If a clone containing a nucleic acid encoding a particular antibody or a CAR is not available, but the sequence of the antibody or CAR molecule is known, a nucleic acid encoding the antibody or the CAR may be chemically synthesized or obtained from a suitable source (*e.g.,* a cDNA library, or a cDNA library generated from, or nucleic acid, preferably polyA$^+$ RNA, isolated from, any tissue or cells expressing the FAP-specific antibody or the corresponding CAR, by PCR amplification using synthetic primers hybridizable to the 3' and 5' ends of the sequence or by cloning using an oligonucleotide probe specific for the particular gene sequence to identify, *e.g.,* a cDNA clone from a cDNA library that encodes the antibody. Amplified nucleic acids generated by PCR may then be cloned into replicable cloning vectors using any method well known in the art. Accordingly, in one embodiment of the present invention the cDNA encoding an antibody, immunoglobulin chain, or fragment thereof is used for the production of an anti-FAP antibody or the corresponding CAR.

Once the nucleotide sequence and corresponding amino acid sequence of the CAR or the antibody, or antigen-binding fragment, variant, or derivative thereof is determined, its nucleotide sequence may be manipulated using methods well known in the art for the manipulation of nucleotide sequences, *e.g.,* recombinant DNA techniques, site directed mutagenesis, PCR, etc. (see, for example, the techniques described in Sambrook et al., Molecular Cloning, A Laboratory Manual, 2d Ed., Cold Spring Harbor Laboratory, Cold Spring Harbor, N.Y. (1990) and Ausubel et al., eds., Current Protocols in Molecular Biology, John Wiley & Sons, NY (1998), which are both incorporated by reference herein in their entireties), to generate antibodies and/or CARs having a different amino acid sequence, for example to create amino acid substitutions, deletions, and/or insertions.

**V. Expression of Antibody Polypeptides**

Following, manipulation of the isolated genetic material to provide antibodies, or antigen-binding fragments, variants, or derivatives thereof of the invention, are provided. The polynucleotides encoding the antibodies are typically inserted in an expression vector for introduction into host cells that may be used to produce the desired quantity of antibody. Recombinant expression of an antibody, or fragment, derivative, or analog thereof, *e.g.,* a heavy or light chain of an antibody which binds to a target molecule is described herein. Once a polynucleotide encoding an antibody molecule or a heavy or light chain of an antibody, or portion thereof (preferably containing the heavy or light chain variable domain), of the invention has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. The invention, thus, provides replicable vectors comprising a nucleotide sequence encoding an antibody molecule of the invention, or a heavy or light chain thereof, or a heavy or light chain variable domain, operable linked to a promoter. Such vectors may include the nucleotide sequence encoding the constant region of the antibody molecule (*see, e.g.,* international applications WO 86/05807 and WO 89/01036; and US patent no. 5,122,464) and the variable domain of the antibody may be cloned into such a vector for expression of the entire heavy or light chain.

The term "vector" or "expression vector" is used herein to mean vectors used in accordance with the present invention as a vehicle for introducing into and expressing a desired gene in a host cell.

As known to those skilled in the art, such vectors may easily be selected from the group consisting of plasmids, phages, viruses, and retroviruses. In general, vectors compatible with the instant invention will comprise a selection marker, appropriate restriction sites to facilitate cloning of the desired gene and the ability to enter and/or replicate in eukaryotic or prokaryotic cells. For the purposes of this invention, numerous expression vector systems may be employed. For example, one class of vector utilizes DNA elements which are derived from animal viruses such as bovine papilloma virus, polyoma virus, adenovirus, vaccinia virus, baculovirus, retroviruses (RSV, MMTV or MOMLV), or SV40 virus. Others involve the use of polycistronic systems with internal ribosome binding sites. Additionally, cells which have integrated the DNA into their chromosomes may be selected by introducing one or more markers or reporters which allow selection of transfected host cells. The marker may provide for prototrophy to an auxotrophic host, biocide resistance (e.g., antibiotics), or resistance to heavy metals such as copper. The selectable marker gene can either be directly linked to the DNA sequences to be expressed, or introduced into the same cell by co-transformation. Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, *e.g.,* enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene; see, *e.g.,* Ui-Tei et al., FEBS Letters 479 (2000), 79-82. Additional elements may also be needed for optimal synthesis of mRNA. These elements may include signal sequences, splice signals, as well as transcriptional promoters, enhancers, and termination signals.

In particularly preferred embodiments the cloned variable region genes are inserted into an expression vector along with the heavy and light chain constant region genes (preferably human) as discussed above. This vector contains the cytomegalovirus promoter/enhancer, the mouse beta globin major promoter, the SV40 origin of replication, the bovine growth hormone polyadenylation sequence, neomycin phosphotransferase exon 1 and exon 2, the dihydrofolate reductase gene, and leader sequence. This vector has been found to result in very high level expression of antibodies upon incorporation of variable and constant region genes, transfection in CHO cells, followed by selection in G418 containing medium and methotrexate amplification. Of course, any expression vector which is capable of eliciting expression in eukaryotic cells may be used in the present invention. Examples of suitable vectors include, but are not limited to plasmids pcDNA3, pHCMV/Zeo, pCR3.1, pEF1/His, pIND/GS, pRc/HCMV2, pSV40/Zeo2, pTRACER-HCMV, pUB6/V5-His, pVAX1, and pZeoSV2 (available from Invitrogen, San Diego, CA), and plasmid pCI (available from Promega, Madison, WI). In general, screening large numbers of transformed cells for those which express suitably high levels if immunoglobulin heavy and light chains is routine experimentation which can be carried out, for example, by robotic systems. Vector systems are also taught in US patent nos. 5,736,137 and 5,658,570, each of which is incorporated by reference in its entirety herein. This system provides for high expression levels, *e.g.,* > 30 pg/cell/day. Other exemplary vector systems are disclosed *e.g.,* in US patent no. 6,413,777.

In other preferred embodiments the antibodies, or antigen-binding fragments, variants, or derivatives thereof of the invention may be expressed using polycistronic constructs such as those disclosed in US patent application publication no. 2003-0157641 A1 and incorporated herein in its entirety. In these expression systems, multiple gene products of interest such as heavy and light chains of antibodies may be produced from a single polycistronic construct. These systems advantageously use an internal ribosome entry site (IRES) to provide relatively high levels of antibodies. Compatible IRES sequences are disclosed in US patent no. 6,193,980 which is also incorporated herein. Those skilled in the art will appreciate that such expression systems may be used to effectively produce the full range of antibodies disclosed in the instant application. Therefore, in one embodiment the present invention provides a vector comprising the polynucleotide encoding at least the binding domain or variable region of an immunoglobulin chain of the antibody, optionally in combination with a polynucleotide that encodes the variable region of the other immunoglobulin chain of said binding molecule.

More generally, once the vector or DNA sequence encoding a monomeric subunit of the antibody has been prepared, the expression vector may be introduced into an appropriate host cell. Introduction of the plasmid into the host cell can be accomplished by various techniques well known to those of skill in the art. These include, but are not limited to, transfection including lipotransfection using, *e.g.,* Fugene® or lipofectamine, protoplast fusion, calcium phosphate precipitation, cell fusion with enveloped DNA, microinjection, and infection with intact virus. Typically, plasmid introduction into the host is via standard calcium phosphate co-precipitation method. The host cells harboring the expression construct are grown under conditions appropriate to the production of the light chains and heavy chains, and assayed for heavy and/or light chain protein synthesis. Exemplary assay techniques include enzyme-linked immunosorbent assay (ELISA), radioimmunoassay (RIA), or fluorescence-activated cell sorter analysis (FACS), immunohistochemistry and the like.

The expression vector is transferred to a host cell by conventional techniques and the transfected cells are then cultured by conventional techniques to produce an antibody for use in the methods described herein. Thus, the invention includes host cells comprising a polynucleotide encoding an antibody of the invention, or a heavy or light chain thereof, or at least the binding domain or variable region of an immunoglobulin thereof, which preferably are operable linked to a heterologous promoter. In addition or alternatively the invention also includes host cells comprising a vector, as defined hereinabove, comprising a polynucleotide encoding at least the binding domain or variable region of an immunoglobulin chain of the antibody, optionally in combination with a polynucleotide that encodes the variable region of the other immunoglobulin chain of said binding molecule. In preferred embodiments for the expression of double-chained antibodies, a single vector or vectors encoding both the heavy and light chains may be co-expressed in the host cell for expression of the entire immunoglobulin molecule, as detailed below.

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain is advantageously placed before the heavy chain to avoid an excess of toxic free heavy chain; *see* Proudfoot, Nature 322 (1986), 52; Kohler, Proc. Natl. Acad. Sci. USA 77 (1980), 2197. The coding sequences for the heavy and light chains may comprise cDNA or genomic DNA.

As used herein, "host cells" refers to cells which harbor vectors constructed using recombinant DNA techniques and encoding at least one heterologous gene. In descriptions of processes for isolation of antibodies from recombinant hosts, the terms "cell" and "cell culture" are used interchangeably to denote the source of antibody unless it is clearly specified otherwise. In other words, recovery of polypeptide from the "cells" may mean either from spun down whole cells, or from the cell culture containing both the medium and the suspended cells.

A variety of host-expression vector systems may be utilized to express antibody molecules for use in the methods described herein. Such host-expression systems represent vehicles by which the coding sequences of interest may be produced and subsequently purified, but also represent cells which may, when transformed or transfected with the appropriate nucleotide coding sequences, express an antibody molecule of the invention *in situ.* These include but are not limited to microorganisms such as bacteria *(e.g., Escherichia coli, Bacillus subtilis*) transformed with recombinant bacteriophage DNA, plasmid DNA or cosmid DNA expression vectors containing antibody coding sequences; yeast (*e.g., Saccharomyces, Pichia*) transformed with recombinant yeast expression vectors containing antibody coding sequences; insect cell systems infected with recombinant virus expression vectors (e.g., baculovirus) containing antibody coding sequences; plant cell systems infected with recombinant virus expression vectors (e.g., cauliflower mosaic virus, CaMV; tobacco mosaic virus, TMV) or transformed with recombinant plasmid expression vectors (*e.g.,* Ti plasmid) containing antibody coding sequences; or mammalian cell systems *(e.g.,* COS, CHO, NSO, BLK, 293, 3T3 cells) harboring recombinant expression constructs containing promoters derived from the genome of mammalian cells (e.g., metallothionein promoter) or from mammalian viruses (e.g., the adenovirus late promoter; the vaccinia virus 7.5K promoter). Preferably, bacterial cells such as *E. coli,* and more preferably, eukaryotic cells, especially for the expression of whole recombinant antibody molecule, are used for the expression of a recombinant antibody molecule. For example, mammalian cells such as Chinese Hamster Ovary (CHO) cells, in conjunction with a vector such as the major intermediate early gene promoter element from human cytomegalovirus is an effective expression system for antibodies; *see, e.g.,* Foecking et al., Gene 45 (1986), 101; Cockett et al., Bio/Technology 8 (1990), 2.

The host cell line used for protein expression is often of mammalian origin; those skilled in the art are credited with ability to preferentially determine particular host cell lines which are best suited for the desired gene product to be expressed therein. Exemplary host cell lines include, but are not limited to, CHO (Chinese Hamster Ovary), DG44 and DUXB 11 (Chinese Hamster Ovary lines, DHFR minus), HELA (human cervical carcinoma), CVI (monkey kidney line), COS (a derivative of CVI with SV40 T antigen), VERY, BHK (baby hamster kidney), MDCK, WI38, R1610 (Chinese hamster fibroblast) BALBC/3T3 (mouse fibroblast), HAK (hamster kidney line), SP2/O (mouse myeloma), P3x63-Ag3.653 (mouse myeloma), BFA-1c1BPT (bovine endothelial cells), RAJI (human lymphocyte) and 293 (human kidney). CHO and 293 cells are particularly preferred. Host cell lines are typically available from commercial services, the American Tissue Culture Collection or from published literature.

In addition, a host cell strain may be chosen which modulates the expression of the inserted sequences, or modifies and processes the gene product in the specific fashion desired. Such modifications *(e.g.,* glycosylation) and processing *(e.g.,* cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which stably express the antibody molecule.

A number of selection systems may be used, including but not limited to the herpes simplex virus thymidine kinase (Wigler et al., Cell 11 (1977), 223), hypoxanthine-guanine phosphoribosyltransferase (Szybalska and Szybalski, Proc. Natl. Acad. Sci. USA 48 (1992), 202), and adenine phosphoribosyltransferase (Lowy et al., Cell 22 (1980), 817) genes can be employed in tk-, hgprt- or aprt-cells, respectively. Also, anti-metabolite resistance can be used as the basis of selection for the following genes: dhfr, which confers resistance to methotrexate (Wigler et al., Natl. Acad. Sci. USA 77 (1980), 357; O'Hare et al., Proc. Natl. Acad. Sci. USA 78 (1981), 1527); gpt, which confers resistance to mycophenolic acid (Mulligan and Berg, Proc. Natl. Acad. Sci. USA 78 (1981), 2072); neo, which confers resistance to the aminoglycoside G-418 Goldspiel et al., Clinical Pharmacy 12 (1993), 488-505; Wu and Wu, Biotherapy 3 (1991), 87-95; Tolstoshev, Ann. Rev. Pharmacol. Toxicol. 32 (1993), 573-596; Mulligan, Science 260 (1993), 926-932; and Morgan and Anderson, Ann. Rev. Biochem. 62 (1993), 191-217; TIB TECH 11 (1993), 155-215; and hygro, which confers resistance to hygromycin (Santerre et al., Gene 30 (1984), 147. Methods commonly known in the art of recombinant DNA technology which can be used are described in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., J. Mol. Biol. 150:1 (1981), which are incorporated by reference herein in their entireties.

The expression levels of an antibody molecule can be increased by vector amplification, for a review; *see* Bebbington and Hentschel, The use of vectors based on gene amplification for the expression of cloned genes in mammalian cells in DNA cloning, Academic Press, New York, Vol. 3. (1987). When a marker in the vector system expressing antibody is amplifiable, increase in the level of inhibitor present in culture of host cell will increase the number of copies of the marker gene. Since the amplified region is associated with the antibody gene, production of the antibody will also increase; *see* Crouse et al., Mol. Cell. Biol. 3 (1983), 257.

*In vitro* production allows scale-up to give large amounts of the desired polypeptides. Techniques for mammalian cell cultivation under tissue culture conditions are known in the art and include homogeneous suspension culture, *e.g.* in an airlift reactor or in a continuous stirrer reactor, or immobilized or entrapped cell culture, *e.g.* in hollow fibers, microcapsules, on agarose microbeads or ceramic cartridges. If necessary and/or desired, the solutions of polypeptides can be purified by the customary chromatography methods, for example gel filtration, ion-exchange chromatography, chromatography over DEAE-cellulose or (immuno-) affinity chromatography, *e.g.,* after preferential biosynthesis of a synthetic hinge region polypeptide or prior to or subsequent to the HIC chromatography step described herein.

Genes encoding antibodies, or antigen-binding fragments, variants or derivatives thereof of the invention can also be expressed in non-mammalian cells such as bacteria or insect or yeast or plant cells. Bacteria which readily take up nucleic acids include members of the enterobacteriaceae, such as strains of *E. coli* or *Salmonella*; Bacillaceae, such as *B. subtilis*; *Pneumococcus*; *Streptococcus*, and *Haemophilus influenzae.* It will further be appreciated that, when expressed in bacteria, the heterologous polypeptides typically become part of inclusion bodies. The heterologous polypeptides must be isolated, purified and then assembled into functional molecules. Where tetravalent forms of antibodies are desired, the subunits will then self-assemble into tetravalent antibodies; *see, e.g.,* international application WO 02/096948.

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited, to the *E. coli* expression vector pUR278 (Ruther et al., EMBO J. 2 (1983), 1791), in which the antibody coding sequence may be ligated individually into the vector in frame with the lacZ coding region so that a fusion protein is produced; pIN vectors (Inouye and Inouye, Nucleic Acids Res. 13 (1985), 3101-3109; Van Heeke and Schuster, J. Biol. Chem. 24 (1989), 5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix of glutathione-agarose beads followed by elution in the presence of free glutathione. The pGEX vectors are designed to include thrombin or factor Xa protease cleavage sites so that the cloned target gene product can be released from the GST moiety.

In addition to prokaryotes, eukaryotic microbes may also be used. *Saccharomyces cerevisiae,* or common baker's yeast, is the most commonly used among eukaryotic microorganisms although a number of other strains are commonly available, *e.g., Pichia pastoris.* For expression in *Saccharomyces,* the plasmid YRp7, for example, (Stinchcomb et al., Nature 282 (1979), 39; Kingsman et al., Gene 7 (1979), 141; Tschemper et al., Gene 10 (1980), 157) is commonly used. This plasmid already contains the TRP1 gene which provides a selection marker for a mutant strain of yeast lacking the ability to grow in tryptophan, for example ATCC No. 44076 or PEP4-1 (Jones, Genetics 85 (1977), 12). The presence of the trpl lesion as a characteristic of the yeast host cell genome then provides an effective environment for detecting transformation by growth in the absence of tryptophan.

In an insect system, *Autographa californica* nuclear polyhedrosis virus (AcNPV) is typically used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter).

Once an antibody molecule of the invention has been recombinantly expressed, the whole antibodies, their dimers, individual light and heavy chains, or other immunoglobulin forms of the present invention, can be purified according to standard procedures of the art, including for example, by chromatography (*e.g.*, ion exchange, affinity, particularly by affinity for the specific antigen after Protein A, and sizing column chromatography), centrifugation, differential solubility, e.g. ammonium sulfate precipitation, or by any other standard technique for the purification of proteins; see, *e.g.,* Scopes, "Protein Purification", Springer Verlag, N.Y. (1982). Alternatively, a preferred method for increasing the affinity of antibodies of the invention is disclosed in US patent publication 2002-0123057 A1. In one embodiment therefore, the present invention also provides a method for preparing an anti-FAP antibody or a biotechnological or synthetic derivative thereof or immunoglobulin chain(s) thereof, said method comprising:

(a) culturing the host cell as defined hereinabove, which cell comprises a polynucleotide or a vector as defined hereinbefore; and
(b) isolating said antibody, biotechnological or synthetic derivative or immunoglobulin chain(s) thereof from the culture.

Furthermore, in one embodiment the present invention also relates to an antibody or immunoglobulin chain(s) thereof encoded by a polynucleotide as defined hereinabove or obtainable by the method for preparing an anti-FAP antibody.

### VI. Expression of chimeric antigen receptor (CAR) polynucleotides

Regarding the manipulation of the isolated genetic material to provide CARs and CAR T cells of the present invention means and methods for expressing polynucleotides in general are already described with respect to antibodies hereinbefore, e.g. the choice of suitable expression vectors and their design as well as means and methods for transfection of mammalian cells (see section V) and can be applied to the expression of CARs as well. Furthermore, the general design and expression of CARs is described in detail in prior art, *e.g.* in Tran et al. (J. Exp. Med. 210 (2013), 1125-1135), Schuberth et al. (J. Transl. Med. 11 (2013), 187), Kakarla et al. (Mol. Ther. 21 (2013), 1611-1620), Wang et al. (Cancer Immunol. Res. 2 (2014), 154-166 as well as in WO 2014055442 A2.

The expression of natural or synthetic nucleic acids encoding the FAP CAR of the present invention is typically achieved by operably linking a nucleic acid encoding the CAR polypeptide or portions thereof to a promoter, and incorporating the construct into an expression vector. The vectors can be suitable for replication and integration eukaryotes. Typical cloning vectors contain transcription and translation terminators, initiation sequences, and promoters useful for regulation of the expression of the desired nucleic acid sequence. The term "expression vector" has been defined hereinbefore; see section V. The expression constructs of the present invention may also be used for nucleic acid immunization and gene therapy, using standard gene delivery protocols. Methods for gene delivery are known in the art; see, *e.g.,* US Patent. 5,399,346; 5,580,859; and 5,589,466, incorporated by reference herein in their entireties. In another embodiment, the invention provides a gene therapy vector.

In one embodiment of the present invention, the sequence of the CAR of the present invention is delivered into eukaryotic cells, tissue or whole organisms using a retroviral or lentiviral vector. In a preferred embodiment, the sequence of the CAR of the present invention is delivered into a T cell. Thus, the present invention is directed to a retroviral or lentiviral vector encoding a CAR that is stably integrated into a T cell and stably expressed therein.

Vectors derived from retroviruses such as the lentivirus are particularly suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity.

A "lentivirus" as used herein refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses. Vectors derived from lentiviruses offer the means to achieve significant levels of gene transfer *in vivo.* A "lentiviral vector" is a vector derived from at least a portion of a lentivirus genome, including especially a self-inactivating lentiviral vector as provided in Milone et al., Mol. Ther. 17 (2009), 1453-1464. Other Examples or lentivirus vectors that may be used in the clinic as an alternative to the pELPS vector, include but not limited to, *e.g.,* the LENTIVECTOR® gene delivery technology from Oxford BioMedica, the LENTIMAX™ vector system from Lentigen and the like. Nonclinical types of lentiviral vectors are also available and would be known to one skilled in the art.

The expression of natural or synthetic nucleic acids encoding the FAP CAR of the present invention can also be achieved by generating an RNA construct encoding the CAR of the present invention which can be directly transferred to the desired cell type.

In one embodiment of the present invention, the sequence of the CAR of the present invention is delivered into eukaryotic cells, tissue or whole organisms using *in vitro* transcribed mRNA. In a preferred embodiment, the sequence of the CAR of the present invention is delivered into a T cell.

Thus, the present invention also discloses an RNA construct encoding the CAR of the present invention that can be directly transfected into a T cell and transiently expressed therein. Transient, non-integrating expression of the CAR in a cell mitigates concerns associated with permanent and integrated expression of CAR in a cell. A method for generating mRNA for use in transfection involves *in vitro* transcription (IVT) of a the CAR with specially designed primers, followed by poly A addition, to produce a construct containing 3' and 5' untranslated sequence ("UTR"), a 5' cap and/or Internal Ribosome Entry Site (IRES), the gene to be expressed, and a polyA tail, typically 50-2000 bases in length. RNA produced by this means can efficiently transfect different kinds of cells; for detailed methods see, e.g., international application WO 2014/055442 and Schutsky et al., Oncotarget. 6 (2015), 28911-28928.

One advantage of the RNA transfection method is that RNA transfection is essentially transient and vector-free. An RNA transgene can be delivered to a lymphocyte and expressed therein following a brief *in vitro* cell activation, as a minimal expressing cassette without the need for any additional viral sequences. Under these conditions, integration of the transgene into the host cell genome is unlikely. Cloning of cells is not necessary because of the efficiency of transfection of the RNA and its ability to uniformly modify the entire lymphocyte population.

Genetic modification of T cells with *in* vitro-transcribed RNA (IVTRNA) makes use of two different strategies both of which have been successively tested in various animal models. Cells are transfected with *in* vitro-transcribed RNA by means of lipofection or electroporation. Preferably, it is desirable to stabilize IVTRNA using various modifications in order to achieve prolonged expression of transferred IVT-RNA.

Some IVT vectors are known in the literature which are utilized in a standardized manner as template for *in vitro* transcription and which have been genetically modified in such a way that stabilized RNA transcripts are produced. Currently protocols used in the art are based on a plasmid vector with the following structure: a 5' RNA polymerase promoter enabling RNA transcription, followed by a gene of interest which is flanked either 3' and/or 5' by untranslated regions (UTR), and a 3' polyadenyl cassette containing 50-70 A nucleotides. Prior to *in vitro* transcription, the circular plasmid is linearized downstream of the polyadenyl cassette by type II restriction enzymes (recognition sequence corresponds to cleavage site). The polyadenyl cassette thus corresponds to the later poly(A) sequence in the transcript. As a result of this procedure, some nucleotides remain as part of the enzyme cleavage site after linearization and extend or mask the poly(A) sequence at the 3' end. It is not clear, whether this non-physiological overhang affects the amount of protein produced intracellularly from such a construct. RNA has several advantages over more traditional plasmid or viral approaches. Gene expression from an RNA source does not require transcription and the protein product is produced rapidly after the transfection. Further, since the RNA has to only gain access to the cytoplasm, rather than the nucleus, and therefore typical transfection methods result in an extremely high rate of transfection. In addition, plasmid based approaches require that the promoter driving the expression of the gene of interest be active in the cells under study.

In another aspect, the RNA construct can be delivered into the cells by electroporation; see, *e.g.,* the formulations and methodology of electroporation of nucleic acid constructs into mammalian cells as taught in US applications US 2004/0014645A1, US 2005/0052630A1, US 2005/0070841A1, US 2004/0059285A1, US 2004/0092907A1. The various parameters including electric field strength required for electroporation of any known cell type are generally known in the relevant research literature as well as numerous patents and applications in the field; see *e.g.,* US patent 6,678,556, US patent 7,171,264, and US patent 7,173,116. Apparatus for therapeutic application of electroporation are available commercially, *e.g.,* the MedPulser™ DNA Electroporation Therapy System (Inovio/Genetronics, San Diego, Calif.), and are described in patents such as US patent 6,567,694, US patent 6,516,223, US patent 5,993,434, US patent 6,181,964, US patent 6,241,701, and US patent 6,233,482. Electroporation may also be used for transfection of cells *in vitro* as described *e.g.* in US20070128708A1. Electroporation may also be utilized to deliver nucleic acids into cells *in vitro.* Accordingly, electroporation-mediated administration into cells of nucleic acids including expression constructs utilizing any of the many available devices and electroporation systems known to those of skill in the art presents an exciting new means for delivering an RNA of interest to a target cell.

In one embodiment, the present invention provides a cell *(e.g.,* T cell) engineered to express any one of the above-described CAR comprising an FAP binding domain of the anti-FAP antibodies of the present invention (also referred to as FAP CAR T cells), wherein the CAR T cell exhibits an antitumor property and wherein the CAR of the invention when expressed in a T cell is able to redirect antigen recognition based on the FAP antigen binding specificity. In one embodiment, the FAP CAR T cells of the invention can undergo robust *in vivo* T cell expansion and can establish FAP-specific memory cells that persist at high levels for an extended amount of time in blood and bone marrow. In some instances, the FAP CAR T cells of the invention infused into a patient can eliminate tumor cells *in vivo* in patients with cancer.

Prior to expansion and genetic modification of the T cells of the invention, a source of T cells has to be obtained from a subject. T cells can be obtained from a number of sources, including peripheral blood mononuclear cells, bone marrow, lymph node tissue, cord blood, thymus tissue, tissue from a site of infection, ascites, pleural effusion, spleen tissue, and tumors. In certain embodiments of the present invention, any number of T cell lines available in the art, may be used. Methods to obtain and enrich those T cells are known to a person skilled in the art and are described for example in the international application WO 2014/055442.

Whether prior to or after genetic modification of the T cells to express the desirable CAR, the T cells can be activated and expanded generally using methods as described, for example, in US Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and US patent application US 2006/0121005 A1.

Generally, the T cells of the invention are expanded by contact with a surface having attached thereto an agent that stimulates a CD3/TCR complex associated signal and a ligand that stimulates a co-stimulatory molecule on the surface of the T cells. In particular, T cell populations may be stimulated as described herein, such as by contact with an anti-CD3 antibody, or antigen-binding fragment thereof, or an anti-CD2 antibody immobilized on a surface, or by contact with a protein kinase C activator (*e.g.*, bryostatin) in conjunction with a calcium ionophore. For costimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. To stimulate proliferation of either CD4 + T cells or CD8+ T cells, an anti-CD3 antibody and an anti-CD28 antibody. Examples of an anti-CD28 antibody include 9.3, B-T3, XR-CD28 (Diaclone, Besancon, France) can be used as can other methods commonly known in the art (Berg et al., Transplant Proc. 30 (1998), 3975-3977; Haanen et al., J. Exp. Med. 190 (1999), 13191328; Garland et al., J. Immunol. Meth. 227 (1999), 53-63). Further details about the activation and expansion of the CAR T cells of the present invention are given in international application WO 2014/055442.

## VII. Fusion Proteins and Conjugates

In certain embodiments, the antibody polypeptide comprises an amino acid sequence or one or more moieties not normally associated with an antibody. Exemplary modifications are described in more detail below. For example, a single-chain Fv antibody fragment of the invention may comprise a flexible linker sequence, or may be modified to add a functional moiety (e.g., PEG, a drug, a toxin, or a label such as a fluorescent, radioactive, enzyme, nuclear magnetic, heavy metal or an active biological agent such as cytokines, chemokines and chemical pathway mediators and the like). Also the CARs of the present invention can be designated as fusion protein.

An antibody polypeptide of the invention may comprise, consist essentially of, or consist of a fusion protein. Fusion proteins are chimeric molecules which comprise, for example, an immunoglobulin FAP-binding domain with at least one target binding site, and at least one heterologous portion, *i.e.*, a portion with which it is not naturally linked in nature. The amino acid sequences may normally exist in separate proteins that are brought together in the fusion polypeptide or they may normally exist in the same protein but are placed in a new arrangement in the fusion polypeptide. Fusion proteins may be created, for example, by chemical synthesis, or by creating and translating a polynucleotide in which the peptide regions are encoded in the desired relationship.

The term "heterologous" as applied to a polynucleotide or a polypeptide, means that the polynucleotide or polypeptide is derived from a distinct entity from that of the rest of the entity to which it is being compared. For instance, as used herein, a "heterologous polypeptide" to be fused to an antibody, or an antigen-binding fragment, variant, or analog thereof is derived from a non-immunoglobulin polypeptide of the same species, or an immunoglobulin or non-immunoglobulin polypeptide of a different species. As discussed in more detail elsewhere herein, antibodies, or antigen-binding fragments, variants, or derivatives thereof of the invention may further be recombinantly fused to a heterologous polypeptide at the N- or C-terminus or chemically conjugated (including covalent and non-covalent conjugations) to polypeptides or other compositions. For example, antibodies may be recombinantly fused or conjugated to molecules useful as labels in detection assays and effector molecules such as heterologous polypeptides, drugs, radionuclides, or toxins; *see, e.g.,* international applications WO 92/08495; WO 91/14438; WO 89/12624; US patent no. 5,314,995; and European patent application EP 0 396 387. They can also be fused to signaling and transmembrane domains as described above to form the chimeric antigen receptor (CARs) of the present invention.

Antibodies, or antigen-binding fragments, variants, or derivatives thereof of the invention can be composed of amino acids joined to each other by peptide bonds or modified peptide bonds, *i.e.*, peptide isosteres, and may contain amino acids other than the 20 gene-encoded amino acids. Antibodies may be modified by natural processes, such as posttranslational processing, or by chemical modification techniques which are well known in the art. Such modifications are well described in basic texts and in more detailed monographs, as well as in a voluminous research literature. Modifications can occur anywhere in the antibodies, including the peptide backbone, the amino acid side-chains and the amino or carboxyl termini, or on moieties such as carbohydrates. It will be appreciated that the same type of modification may be present in the same or varying degrees at several sites in a given antibody. Also, a given antibody may contain many types of modifications. Antibodies may be branched, for example, as a result of ubiquitination, and they may be cyclic, with or without branching. Cyclic, branched, and branched cyclic antibodies may result from posttranslational natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphatidylinositol, cross-linking, cyclization, disulfide bond formation, demethylation, formation of covalent crosslinks, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination; *see*, *e.g.,* Proteins - Structure And Molecular Properties, T. E. Creighton, W. H. Freeman and Company, New York 2nd Ed., (1993); Posttranslational Covalent Modification Of Proteins, B. C. Johnson, Ed., Academic Press, New York, (1983) 1-12; Seifter et al., Meth. Enzymol. 182 (1990), 626-646; Rattan et al., Ann. NY Acad. Sci. 663 (1992), 48-62).

The present invention also provides for fusion proteins comprising an antibody, or antigen-binding fragment, variant, or derivative thereof, and a heterologous polypeptide. In one embodiment, a fusion protein of the invention comprises, consists essentially of, or consists of, a polypeptide having the amino acid sequence of any one or more of the $V_H$ regions of an antibody of the invention or the amino acid sequence of any one or more of the $V_L$ regions of an antibody of the invention or fragments or variants thereof, and a heterologous polypeptide sequence. In another embodiment, a fusion protein for use in the diagnostic and treatment methods disclosed herein comprises, consists essentially of, or consists of a polypeptide having the amino acid sequence of any one, two, three of the $V_H$-CDRs of an antibody, or fragments, variants, or derivatives thereof, or the amino acid sequence of any one, two, three of the $V_L$-CDRs of an antibody, or fragments, variants, or derivatives thereof, and a heterologous polypeptide sequence. In one embodiment, the fusion protein comprises a polypeptide having the amino acid sequence of a $V_H$-CDR3 of an antibody of the present invention, or fragment, derivative, or variant thereof, and a heterologous polypeptide sequence, which fusion protein specifically binds to FAP. In another embodiment, a fusion protein comprises a polypeptide having the amino acid sequence of at least one $V_H$ region of an antibody of the invention and the amino acid sequence of at least one $V_L$ region of an antibody of the invention or fragments, derivatives or variants thereof, and a heterologous polypeptide sequence. Preferably, the $V_H$ and $V_L$ regions of the fusion protein correspond to a single source antibody (or scFv or Fab fragment) which specifically binds FAP. In yet another embodiment, a fusion protein for use in the diagnostic and treatment methods disclosed herein comprises a polypeptide having the amino acid sequence of any one, two, three, or more of the $V_H$ CDRs of an antibody and the amino acid sequence of any one, two, three, or more of the $V_L$ CDRs of an antibody, or fragments or variants thereof, and a heterologous polypeptide sequence. Preferably, two, three, four, five, six, or more of the $V_H$-CDR(s) or $V_L$-CDR(s) correspond to single source antibody (or scFv or Fab fragment) of the invention. Nucleic acid molecules encoding these fusion proteins are also encompassed by the invention.

Exemplary fusion proteins reported in the literature include fusions of the T cell receptor (Gascoigne et al., Proc. Natl. Acad. Sci. USA 84 (1987), 2936-2940; CD4 (Capon et al., Nature 337 (1989), 525-531; Traunecker et al., Nature 339 (1989), 68-70; Zettmeissl et al., DNA Cell Biol. USA 9 (1990), 347-353; and Byrn et al., Nature 344 (1990), 667-670); L-selectin (homing receptor) (Watson et al., J. Cell. Biol. 110 (1990), 2221-2229; and Watson et al., Nature 349 (1991), 164-167); CD44 (Aruffo et al., Cell 61 (1990), 1303-1313); CD28 and B7 (Linsley et al., J. Exp. Med. 173 (1991),721-730); CTLA-4 (Lisley et al., J. Exp. Med. 174 (1991), 561-569); CD22 (Stamenkovic et al., Cell 66 (1991), 1133-1144); TNF receptor (Ashkenazi et al., Proc. Natl. Acad. Sci. USA 88 (1991), 10535-10539; Lesslauer et al., Eur. J. Immunol. 27 (1991), 2883-2886; and Peppel et al., J. Exp. Med. 174 (1991), 1483-1489 (1991); and IgE receptor a (Ridgway and Gorman, J. Cell. Biol. 115 (1991), Abstract No. 1448).

As discussed elsewhere herein, antibodies, or antigen-binding fragments, variants, or derivatives thereof of the invention may be fused to heterologous polypeptides to increase the *in vivo* half-life of the polypeptides or for use in immunoassays using methods known in the art. For example, in one embodiment, PEG can be conjugated to the antibodies of the invention to increase their half-life *in vivo*; see, *e.g.,* Leong et al., Cytokine 16 (2001), 106-119; Adv. in Drug Deliv. Rev. 54 (2002), 531; or Weir et al., Biochem. Soc. Transactions 30 (2002), 512.

Moreover, antibodies, or antigen-binding fragments, variants, or derivatives thereof of the invention can be fused to marker sequences, such as a peptide to facilitate their purification or detection. In preferred embodiments, the marker amino acid sequence is a hexa-histidine peptide (HIS), such as the tag provided in a pQE vector (QIAGEN, Inc., 9259 Eton Avenue, Chatsworth, Calif., 91311), among others, many of which are commercially available. As described in Gentz et al., Proc. Natl. Acad. Sci. USA 86 (1989), 821-824, for instance, hexa-histidine provides for convenient purification of the fusion protein. Other peptide tags useful for purification include, but are not limited to, the "HA" tag, which corresponds to an epitope derived from the influenza hemagglutinin protein (Wilson et al., Cell 37 (1984), 767), GST, c-mycand the "flag" tag; *see, e.g.,* Bill Brizzard, BioTechniques 44 (2008) 693-695 for a review of epitope tagging techniques, and Table 1 on page 694 therein listing the most common epitope tags usable in the present invention, the subject matter of which is hereby expressly incorporated by reference.

Fusion proteins can be prepared using methods that are well known in the art; *see* for example US patent nos. 5,116,964 and 5,225,538. The precise site at which the fusion is made may be selected empirically to optimize the secretion or binding characteristics of the fusion protein. DNA encoding the fusion protein is then transfected into a host cell for expression, which is performed as described hereinbefore.

Antibodies of the present invention may be used in non-conjugated form or may be conjugated to at least one of a variety of molecules, *e.g.,* to improve the therapeutic properties of the molecule, to facilitate target detection, or for imaging or therapy of the patient. Antibodies, or antigen-binding fragments, variants, or derivatives thereof of the invention can be labeled or conjugated either before or after purification, when purification is performed. In particular, antibodies, or antigen-binding fragments, variants, or derivatives thereof of the invention may be conjugated to therapeutic agents, prodrugs, peptides, proteins, enzymes, viruses, lipids, biological response modifiers, pharmaceutical agents, or PEG.

Conjugates that are immunotoxins including conventional antibodies have been widely described in the art. The toxins may be coupled to the antibodies by conventional coupling techniques or immunotoxins containing protein toxin portions can be produced as fusion proteins. The antibodies of the present invention can be used in a corresponding way to obtain such immunotoxins. Illustrative of such immunotoxins are those described by Byers, Seminars Cell. Biol. 2 (1991), 59-70 and by Fanger, Immunol. Today 12 (1991), 51-54.

Those skilled in the art will appreciate that conjugates may also be assembled using a variety of techniques depending on the selected agent to be conjugated. For example, conjugates with biotin are prepared, *e.g.,* by reacting a FAP-binding polypeptide with an activated ester of biotin such as the biotin N-hydroxysuccinimide ester. Similarly, conjugates with a fluorescent marker may be prepared in the presence of a coupling agent, *e.g.* those listed herein, or by reaction with an isothiocyanate, preferably fluorescein-isothiocyanate. Conjugates of the antibodies, or antigen-binding fragments, variants or derivatives thereof of the invention are prepared in an analogous manner.

The present invention further encompasses antibodies, biotechnological and synthetic derivatives thereof as well as equivalent FAP-binding agents of the invention conjugated to a diagnostic or therapeutic agent. Especially for therapeutic use, the antibody of the present invention and fusion proteins thereof as well as compositions according to the present invention can comprise a biologically active agent such as cytokines, chemokines or signaling pathway mediators or radioisotopes for therapeutic use. For example, cytokines, which are known to have anti-cancer activity, cannot find their way cannot find their way to clinical exploitation due to their devastating toxicity shown during dose escalation to therapeutically active concentrations. To circumvent these problems, an elegant and efficient way to accumulate therapeutic agents at the tumor site, thus reducing systemic side effects, is their conjugation to tumor-specific antibodies, e.g. to FAP. A review about immunocytokines conjugated to a single-chain human antibody that selectively targets tumor-associated stroma or blood vessels is given, *e.g.,* in Ronca et al., Immunobiology 214 (2009), 800-810.

The antibodies can be used diagnostically to, for example, demonstrate presence of a FAP-related disease to indicate the risk of getting a disease or disorder associated with FAP, to monitor the development or progression of such a disease, *i.e.* a disease showing the occurrence of, or related to elevated levels of FAP, or as part of a clinical testing procedure to, *e.g.,* determine the efficacy of a given treatment and/or prevention regimen. In one embodiment thus, the present invention relates to an antibody, which is detectably labeled. Furthermore, in one embodiment, the present invention relates to an antibody, which is attached to a drug. Detection can be facilitated by coupling the antibody, or antigen-binding fragment, variant, or derivative thereof to a detectable substance. The detectable substances or label may be in general an enzyme; a heavy metal, preferably gold; a dye, preferably a fluorescent or luminescent dye; or a radioactive label. Examples of detectable substances include various enzymes, prosthetic groups, fluorescent materials, luminescent materials, bioluminescent materials, radioactive materials, positron emitting metals using various positron emission tomographies, and nonradioactive paramagnetic metal ions; *see, e.g.,* US patent no. 4,741,900 for metal ions which can be conjugated to antibodies for use as diagnostics according to the present invention. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include $^{125}I$, $^{131}I$, $^{111}In$ or $^{99}Tc$. Therefore, in one embodiment the present invention provides a detectably labeled antibody, wherein the detectable label is selected from the group consisting of an enzyme, a radioisotope, a fluorophore and a heavy metal. Further suitable radioactive labels and cytotoxins for FAP-targeting are known to the person skilled in the art; see, e.g., international application WO 2011/040972.

An antibody, or antigen-binding fragment, variant, or derivative thereof also can be detectably labeled by coupling it to a chemiluminescent compound. The presence of the chemiluminescenttagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester. One of the ways in which an antibody, or antigen-binding fragment, variant, or derivative thereof can be detectably labeled is by linking the same to an enzyme and using the linked product in an enzyme immunoassay (EIA) (Voller, A., "The Enzyme Linked Immunosorbent Assay (ELISA)" Microbiological Associates Quarterly Publication, Walkersville, Md., Diagnostic Horizons 2 (1978), 1-7); Voller et al., J. Clin. Pathol. 31 (1978), 507-520; Butler, Meth. Enzymol. 73 (1981), 482-523; Maggio, (ed.), Enzyme Immunoassay, CRC Press, Boca Raton, Fla., (1980); Ishikawa, et al., (eds.), Enzyme Immunoassay, Kgaku Shoin, Tokyo (1981). The enzyme, which is bound to the antibody, will react with an appropriate substrate, preferably a chromogenic substrate, in such a manner as to produce a chemical moiety which can be detected, for example, by spectrophotometric, fluorimetric or by visual means. Enzymes which can be used to detectably label the antibody include, but are not limited to, malate dehydrogenase, staphylococcal nuclease, delta-5-steroid isomerase, yeast alcohol dehydrogenase, alphaglycerophosphate, dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase. Additionally, the detection can be accomplished by colorimetric methods which employ a chromogenic substrate for the enzyme. Detection may also be accomplished by visual comparison of the extent of enzymatic reaction of a substrate in comparison with similarly prepared standards.

Detection may also be accomplished using any of a variety of other immunoassays. For example, by radioactively labeling the antibody, or antigen-binding fragment, variant, or derivative thereof, it is possible to detect the antibody through the use of a radioimmunoassay (RIA) (*see,* for example, Weintraub, B., Principles of Radioimmunoassays, Seventh Training Course on Radioligand Assay Techniques, The Endocrine Society, (March, 1986)), which is incorporated by reference herein). The radioactive isotope can be detected by means including, but not limited to, a gamma counter, a scintillation counter, or autoradiography. An antibody, or antigen-binding fragment, variant, or derivative thereof can also be detectably labeled using fluorescence emitting metals such as $^{152}Eu$, or others of the lanthanide series. These metals can be attached to the antibody using such metal chelating groups as diethylenetriaminepentacetic acid (DTPA) or ethylenediaminetetraacetic acid (EDTA).

Techniques for conjugating various moieties to an antibody, or antigen-binding fragment, variant, or derivative thereof are well known, *see, e.g.,* Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. (1985); Hellstrom et al., "Antibodies For Drug Delivery", in Controlled Drug Delivery (2nd Ed.), Robinson et al. (eds.), Marcel Dekker, Inc., (1987) 623-53; Thorpe, "Antibody Carriers Of Cytotoxic Agents In Cancer Therapy: A Review", in Monoclonal Antibodies

’84: Biological And Clinical Applications, Pinchera et al. (eds.), (1985) 475-506; "Analysis, Results, And Future Prospective Of The Therapeutic Use Of Radiolabeled Antibody In Cancer Therapy", in Monoclonal Antibodies For Cancer Detection And Therapy, Baldwin et al. (eds.), Academic Press (1985) 303-16, and Thorpe et al., "The Preparation And Cytotoxic Properties Of Antibody-Toxin Conjugates", Immunol. Rev. 62 (1982), 119-158. As mentioned, in certain embodiments, a moiety that enhances the stability or efficacy of a binding molecule, *e.g.,* a binding polypeptide, *e.g.,* an antibody or immunospecific fragment thereof can be conjugated. For example, in one embodiment, PEG can be conjugated to the binding molecules of the invention to increase their half-life *in vivo.* Leong et al., Cytokine 16 (2001), 106; Adv. in Drug Deliv. Rev. 54 (2002), 531; or Weir et al., Biochem. Soc. Transactions 30 (2002), 512.

**VIII. Compositions and Methods of Use**

As demonstrated in the appended Examples and illustrated in the Figures the anti-FAP antibody of the present invention is capable of selectively binding FAP *in vitro* and its epitope provide for a reliable FAP non-invasive and tissue-free detection assay. Furthermore, the anti-FAP antibody of the present invention is capable of selectively binding FAP *in vivo* in human blood plasma and on diseased tissue characterized by the presence of FAP such as breast cancer tissue, carcinoma, multiple myeloma tissue as well as atherosclerotic plaque and obstructive coronary thrombi. Moreover, in some embodiments the anti-FAP antibody of the present has an inhibitory effect on FAP serine protease activity and is biologically active *in vivo,* exerting therapeutic effects such as prolonging blood coagulation and arterial occlusion times as well as anti-tumor effect on, *e.g.,* colorectal cancer. All these properties make the anti-FAP antibody of the present invention and equivalents thereof described in the preceding sections useful in variety of diagnostic and therapeutic applications.

Thus, the present invention relates to compositions comprising the aforementioned FAP-binding molecule, *e.g.,* antibody or biotechnological or synthetic derivative thereof of the present invention, or the polynucleotide, vector, cell or peptide of the invention as defined hereinbefore and uses thereof. In one embodiment, the composition of the present invention is a pharmaceutical composition and further comprises a pharmaceutically acceptable carrier. Furthermore, the pharmaceutical composition of the present invention may comprise further agents such as anti-tumor agents, interleukins or interferons depending on the intended use of the pharmaceutical composition. For use in the treatment of a disease or disorder showing the occurrence of, or related to increased level of FAP, the additional agent may be selected from the group consisting of small organic molecules, anti-FAP antibodies, and combinations thereof. Hence, in a particular preferred embodiment the present invention relates to the use of the FAP-binding molecule, *e.g.,* antibody or antigen-binding fragment thereof of the present invention or of a binding molecule having substantially the same binding specificities of any one thereof, the polynucleotide, the vector or the cell of the present invention for the preparation of a pharmaceutical or diagnostic composition for prophylactic and therapeutic treatment of a disease or disorder associated with FAP, monitoring the progression of a disease or disorder associated with FAP or a response to a FAP-targeted treatment in a subject or for determining a subject’s risk for developing a disease or disorder associated with FAP.

Hence, in one embodiment the present invention relates to a method of treating a disease or disorder characterized by abnormal expression of FAP in affected tissue and organs such as cancer, vascular system, see also *supra*, which method comprises administering to a subject in need thereof a therapeutically effective amount of any one of the afore-described FAP-binding agents, antibodies, polynucleotides, vectors, cells or peptides of the instant invention.

A particular advantage of the therapeutic approach of the present invention lies in the fact that the recombinant antibodies of the present invention are derived from human memory B cells which have already successfully gone through somatic maturation, *i.e.* the optimization with respect to selectivity and effectiveness in the high affinity binding to the target FAP molecule by means of somatic variation of the variable regions of the antibody.

The knowledge that such cells *in vivo, e.g.* in a human, have not been activated by means of related or other physiological proteins or cell structures in the sense of an autoimmunological or allergic reaction is also of great medical importance since this signifies a considerably increased chance of successfully living through the clinical test phases. So to speak, efficiency, acceptability and tolerability have already been demonstrated before the preclinical and clinical development of the prophylactic or therapeutic antibody in at least one human subject. It can thus be expected that the human anti-FAP antibodies of the present invention, both its target-specific efficiency as therapeutic agent and its decreased probability of side effects significantly increase its clinical probability of success.

The present invention also provides a pharmaceutical and diagnostic, respectively, pack or kit comprising one or more containers filled with one or more of the above described ingredients, e.g. anti-FAP antibody, binding fragment, derivative or variant thereof, polynucleotide, vector, cell and/or peptide of the present invention. Associated with such container(s) can be a notice in the form prescribed by a governmental agency regulating the manufacture, use or sale of pharmaceuticals or biological products, which notice reflects approval by the agency of manufacture, use or sale for human administration. In addition or alternatively the kit comprises reagents and/or instructions for use in appropriate diagnostic assays. The composition, e.g. kit of the present invention is of course particularly suitable for the risk assessment, diagnosis, prevention and treatment of a disease or disorder which is accompanied with the presence of FAP, and in particular applicable for the treatment of disorders generally characterized by FAP expression comprising diseases and/or disorders such as cancer, atherosclerosis and clotting disorders; see supra.

The pharmaceutical compositions of the present invention can be formulated according to methods well known in the art; see for example Remington: The Science and Practice of Pharmacy (2000) by the University of Sciences in Philadelphia, ISBN 0-683-306472. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well-known conventional methods. These pharmaceutical compositions can be administered to the subject at a suitable dose. Administration of the suitable compositions may be effected by different ways, *e.g.,* by intravenous, intraperitoneal, subcutaneous, intramuscular, intranasal, topical or intradermal administration or spinal or brain delivery. Aerosol formulations such as nasal spray formulations include purified aqueous or other solutions of the active agent with preservative agents and isotonic agents. Such formulations are preferably adjusted to a pH and isotonic state compatible with the nasal mucous membranes. Formulations for rectal or vaginal administration may be presented as a suppository with a suitable carrier.

The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0.001 to 1000 $\mu$g (or of nucleic acid for expression or for inhibition of expression in this range); however, doses below or above this exemplary range are envisioned, especially considering the aforementioned factors. Generally, the dosage can range, *e.g.,* from about 0.0001 to 100 mg/kg, and more usually 0.01 to 5 mg/kg (*e.g.,* 0.02 mg/kg, 0.25 mg/kg, 0.5 mg/kg, 0.75 mg/kg, 1 mg/kg, 2 mg/kg, etc.), of the host body weight. For example dosages can be 1 mg/kg body weight or 10 mg/kg body weight or within the range of 1-10 mg/kg, preferably at least 1 mg/kg. Doses intermediate in the above ranges are also intended to be within the scope of the invention. Subjects can be administered such doses daily, on alternative days, weekly or according to any other schedule determined by empirical analysis. An exemplary treatment entails administration in multiple dosages over a prolonged period, for example, of at least six months. Additional exemplary treatment regimens entail administration once per every two weeks or once a month or once every 3 to 6 months. Exemplary dosage schedules include 1-10 mg/kg or 15 mg/kg on consecutive days, 30 mg/kg on alternate days or 60 mg/kg weekly. In some methods, two or more monoclonal antibodies with different binding specificities are administered simultaneously, in which case the dosage of each antibody administered falls within the ranges indicated. Progress can be monitored by periodic assessment. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline, and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, antioxidants, chelating agents, and inert gases, and the like. Furthermore, the pharmaceutical composition of the invention may comprise further agents such as dopamine or psychopharmacologic drugs, depending on the intended use of the pharmaceutical composition.

In one embodiment, it may be beneficial to use recombinant Fab (rFab) and single chain fragments (scFvs) of the antibody of the present invention, which might more readily penetrate a cell membrane. The perceived advantages of using small Fab and scFv engineered antibody formats which lack the effector function include more efficient passage across the blood-brain barrier and minimizing the risk of triggering inflammatory side reactions. Furthermore, besides scFv and single-domain antibodies retain the binding specificity of full-length antibodies, they can be expressed as single genes and intracellularly in mammalian cells as intrabodies, with the potential for alteration of the folding, interactions, modifications, or subcellular localization of their targets; *see* for review, *e.g.,* Miller and Messer, Molecular Therapy 12 (2005), 394-401.

In a different approach Muller et al., Expert Opin. Biol. Ther. (2005), 237-241, describe a technology platform, so-called 'SuperAntibody Technology', which is said to enable antibodies to be shuttled into living cells without harming them. Such cell-penetrating antibodies open new diagnostic and therapeutic windows. The term 'TransMabs' has been coined for these antibodies.

In a further embodiment, co-administration or sequential administration of other FAP-targeting agents may be desirable. Examples of agents which can be used to treat a subject include, but are not limited to: Agents which stabilize the FAP-tetramer, such as Tafamidis Meglumin, diflusinal, doxycyclin with ursodeoxycholic acid; anti-inflammatory agents such as diflusinal, corticosteroids, 2-(2,6-dichloranilino) phenylacetic acid (diclofenac), iso-butyl-propanoic-phenolic acid (ibuprofen); diuretics, Epigallocatechin gallate, Melphalan hydrochloride, dexamethasone, Bortezomib, Bortezomib-Melphalan, Bortezomib-dexamethasone, Melphalan-dexamethasone, Bortezomib-Melphalan- dexamethasone; antidepressants, antipsychotic drugs, neuroleptics, antidementiva (*e.g.* the NMDA-rezeptor antagonist memantine), acetylcholinesterase inhibitors (*e.g.* Donepezil, HCl, Rivastigmine, Galantamine), glutamat-antagonists and other nootropics blood pressure medication (*e.g.* Dihydralazin, Methyldopa), cytostatics, glucocorticoides, angiotensin-converting-enzyme (ACE) inhibitors; anti-inflammatory agents or any combination thereof.

Examples of agents which may be used for treating or preventing organ rejection following clinical organ transplantation include but are not limited to the agents of the group which lead to a weakening of the immune system, *i.e.* immunosuppressive comprising such as calcineurin inhibitors such as cyclosporine and Tacrolimus, inhibitors of proliferation such as mTOR inhibitors comprising Everolimus and Sirolimus (rapamycin) as well as antimetabolites such as Azathioprin, Mycophenolat Mofetil/MMF and mycophenolic acid, and corticosteroids such as cortisone and cortisol as well as synthetical substances such as Prednison or Prednisolon can be used. Additionally antibodies can be used such as anti-IL2-receptor monoclonal antibodies (e.g. Basiliximab, Daclizumab) as well as anti-CD3 monoclonal antibodies (*e.g.* Muromonab-CD3), and polyclonal compositions such as anti-thymocyte globulin (ATG); and glucagon- like peptide-1 (GLP-1) receptor agonists (see, *e.g.,* Noguchi et al., Acta Med. Okayama, 60 (2006), and the international application WO 2012/088157). Furthermore, additional agents might comprise agents for the prophylaxis and or treatment of infections and other side effects after an organ transplantation comprising valganciclovir, cytomegalie-immunoglobulin, gancyclovir, amphotericin B, pyrimethamin, ranitidine, ramipril, furosemide, benzbromaron. Therefore, in one embodiment a composition is provided further comprising an additional agent useful for treating FAP amyloidosis and/or in treating or preventing organ rejection following, e.g. clinical liver transplantation.

In a particular preferred embodiment, the present invention relates to a therapeutic agent, preferably FAP-targeting agent for use in the treatment of a patient suffering from or being at risk of developing a disease associated with FAP as characterized hereinbefore, characterized in that a sample of the patient's blood, compared to a control sample from a healthy subject shows an elevated level of FAP as determined by detecting an epitope of FAP consisting of or comprising the amino acid sequence of any one of SEQ ID NOS: 30 to 32 and 60. Preferably, the patient has been diagnosed in accordance with the method of the present invention as described further below. In practice, it can be expected that the medication with FAP-targeting agents, in particular anti-FAP antibodies NI-206.82C2 and NI-206.18H2 and its biotechnological and synthetic derivatives as well as equivalent FAP-binding agents will most often be combined with the method and assay of the present invention, illustrated in the Examples that quantifies the epitopes "525-PPQFDRSKKYP-535" and "501-IQLPKEEIKKL-511", thereby specifically measuring the amount of the "drug target" FAP and thus also allowing to dose the therapeutically effective amount for medication.

A therapeutically effective dose or amount refers to that amount of the active ingredient sufficient to ameliorate the symptoms or condition. Therapeutic efficacy and toxicity of such compounds can be determined by standard pharmaceutical procedures in cell cultures or experimental animals, *e.g.,* $ED_{50}$ (the dose therapeutically effective in 50% of the population) and $LD_{50}$ (the dose lethal to 50% of the population). The dose ratio between therapeutic and toxic effects is the therapeutic index, and it can be expressed as the ratio, $LD_{50}/ED_{50}$.

From the foregoing, it is evident that the present invention encompasses any use of an FAP-binding molecule comprising at least one CDR of the above described antibodies, in particular for diagnosing and/or treatment of a FAP-related disease or disorder. Preferably, said binding molecule is an antibody of the present invention. In addition, the present invention relates to anti-idiotypic antibodies of any one of the mentioned antibodies described hereinbefore. These are antibodies or other binding molecules which bind to the unique antigenic peptide sequence located on an antibody's variable region near the antigen-binding site and are useful, *e.g.,* for the detection of anti-FAP antibodies in a sample obtained from a subject. In one embodiment thus, the present invention provides an antibody as defined hereinabove and below or a FAP-binding molecule having substantially the same binding specificities of any one thereof, the polynucleotide, the vector or the cell as defined herein or a pharmaceutical or diagnostic composition comprising any one thereof for use in prophylactic treatment, therapeutic treatment and/or monitoring the progression or a response to treatment of a disease or disorder related to FAP, see *supra.*

In another embodiment the present invention relates to a diagnostic composition comprising any one of the above described FAP-binding molecules, antibodies, antigen-binding fragments, polynucleotides, vectors, cells and/or peptides of the invention and optionally suitable means for detection such as reagents conventionally used in immuno- or nucleic acid-based diagnostic methods. The antibodies of the invention are, for example, suited for use in immunoassays in which they can be utilized in liquid phase or bound to a solid phase carrier. Examples of immunoassays which can utilize the antibody of the invention are competitive and non-competitive immunoassays in either a direct or indirect format. Examples of such immunoassays are the radioimmunoassay (RIA), the sandwich (immunometric assay), flow cytometry, and the Western blot assay. The antigens and antibodies of the invention can be bound to many different carriers and used to isolate cells specifically bound thereto. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. There are many different labels and methods of labeling known to those of ordinary skill in the art. Examples of the types of labels which can be used in the present invention include enzymes, radioisotopes, colloidal metals, fluorescent compounds, chemiluminescent compounds, and bioluminescent compounds; see also the embodiments discussed hereinabove.

By a further embodiment, the FAP-binding molecules, in particular antibodies of the present invention may also be used in a method for the diagnosis of a FAP-related disease or disorder in an individual by obtaining a body fluid sample from the tested individual which may be a blood sample, a plasma sample, a serum sample, a lymph sample or any other body fluid sample, such as a saliva or a urine sample and contacting the body fluid sample with an antibody of the instant invention under conditions enabling the formation of antibody-antigen complexes. The level of such complexes is then determined by methods known in the art, a level significantly higher than that formed in a control sample indicating the disease or disorder in the tested individual. In the same manner, the specific antigen bound by the antibodies of the invention may also be used. Thus, the present invention relates to an *in vitro* immunoassay comprising the binding molecule, *e.g.,* antibody or antigen-binding fragment thereof of the invention. Preferably, the FAP-binding molecule is anti-FAP antibody NI-206.82C2 or NI-206.18H2 or a recombinant, biotechnological or synthetic derivative thereof.

In a further embodiment of the present invention the FAP-binding molecules, in particular antibodies of the present invention may also be used in a method for the diagnosis of a disease or disorder in an individual by obtaining a biopsy from the tested individual which may be skin, salivary gland, hair roots, heart, colon, nerve, subcutaneous fat biopsies, or a biopsy from any affected organs.

In this context, the present invention also relates to means specifically designed for this purpose. For example, an antibody-based array may be used, which is for example loaded with antibodies or equivalent antigen-binding molecules of the present invention which specifically recognize FAP. Design of microarray immunoassays is summarized in Kusnezow et al., Mol. Cell Proteomics 5 (2006), 1681-1696. Accordingly, the present invention also relates to microarrays loaded with FAP-binding molecules identified in accordance with the present invention.

In one embodiment, the present invention relates to a method of diagnosing a disease or disorder related to FAP in a subject, the method comprising determining the presence of FAP in a sample from the subject to be diagnosed with at least one antibody of the present invention, a FAP-binding fragment thereof or an FAP-binding molecule having substantially the same binding specificities of any one thereof, wherein the presence of FAP is indicative for a FAP-related disease and an increase of the level of FAP in comparison to the level in a healthy control is indicative for progression of FAP amyloidosis in said subject.

The subject to be diagnosed may be asymptomatic or preclinical for the disease. Preferably, the control subject has a disease associated with FAP, wherein a similarity between the level of FAP and the reference standard indicates that the subject to be diagnosed has a FAP-related disease or is at risk to develop a FAP-related disease. Alternatively, or in addition as a second control the control subject does not have a FAP-related disease, wherein a difference between the level of physiological FAP and the reference standard indicates that the subject to be diagnosed has a FAP-related disease or is at risk to develop a FAP-related disease. Preferably, the subject to be diagnosed and the control subject(s) are age-matched. The sample to be analyzed may be any body fluid suspected to contain FAP, for example a blood, blood plasma, blood serum, urine, peritoneal fluid, saliva or cerebral spinal fluid (CSF).

The level of FAP may be assessed by any suitable method known in the art comprising, *e.g.,* analyzing FAP by one or more techniques chosen from Western blot, immunoprecipitation, enzyme-linked immunosorbent assay (ELISA), radioimmunoassay (RIA), fluorescent activated cell sorting (FACS), two-dimensional gel electrophoresis, mass spectroscopy (MS), matrix-assisted laser desorption/ionization-time of flight-MS (MALDI-TOF), surface-enhanced laser desorption ionization-time of flight (SELDI-TOF), high performance liquid chromatography (HPLC), fast protein liquid chromatography (FPLC), multidimensional liquid chromatography (LC) followed by tandem mass spectrometry (MS/MS), and laser densitometry. Preferably, said *in vivo* imaging of FAP comprises scintigraphy, positron emission tomography (PET), single photon emission tomography (SPECT), near infrared (NIR) optical imaging or magnetic resonance imaging (MRI).

In a particular preferred embodiment, the present invention relates to an *in vitro* method of diagnosing whether a subject suffers from a disease associated with FAP or whether a subject is amenable to the treatment with a FAP-specific therapeutic agent, the method comprising determining in a sample derived from a body fluid of the subject, preferably blood the presence of FAP, wherein an elevated level of FAP compared to the level in a control sample from a healthy subject is indicative for the disease and possibility for the treatment with the agent, wherein the method is characterized in that the level of FAP is determined by way of detecting an epitope of FAP comprising or consisting of the amino acid sequence of any one of SEQ ID NOS: 30 to 32 or 60. As demonstrated in Example 15 and illustrated in Figures 17-20 a novel assay for assaying FAP in a body fluid, in particular blood has been developed based on the novel epitope of subject antibody NI-206.82C2 of the present invention. As described in Example 14, the sandwich-type immunoassay format (=sandwich immunoassay or ELISA) is particular preferred. Most preferably, antibody NI-206.82C2 or a biotechnological or synthetic derivative thereof is used as the detection antibody and anti-FAP antibody F19 or a derivative thereof as the capture antibody. Alternatively, another anti-FAP antibody such as rat monoclonal anti-FAP antibody clones D8, D28 and D43 may be used as the capture antibody. In view of similar binding properties of antibody NI-206.82C2 and NI-206.18H2 and close location of their epitopes this embodiment as well as the following embodiments may be equally performed with the FAP epitope of antibody NI-206.18H2 "501-IQLPKEEIKKL-511" (SEQ ID NO: 60).

As indicated above, the antibodies of the present invention, fragments thereof and molecules of the same binding specificity as the antibodies and fragments thereof may be used not only *in vitro* but *in vivo* as well, wherein besides diagnostic, therapeutic applications as well may be pursued. In one embodiment thus, the present invention also relates to a FAP-binding molecule comprising at least one CDR of an antibody of the present invention for the preparation of a composition for *in vivo* detection/imaging of or targeting a therapeutic and/or diagnostic agent to FAP in the human or animal body. Potential therapeutic and/or diagnostic agents may be chosen from the non-exhaustive enumerations of the therapeutic agents useful in treatment FAP-related diseases and potential labels as indicated hereinbefore. In respect of the *in vivo* imaging, in one preferred embodiment the present invention provides said FAP-binding molecule comprising at least one CDR of an antibody of the present invention, wherein said *in vivo* imaging comprises scintigraphy, positron emission tomography (PET), single photon emission tomography (SPECT), near infrared (NIR) optical imaging or magnetic resonance imaging (MRI). In a further embodiment the present invention also provides said FAP-binding molecule comprising at least one CDR of an antibody of the present invention, or said molecule for the preparation of a composition for the above specified *in vivo* imaging methods, for the use in the method of diagnosing or monitoring the progression of a disease or disorder related to FAP in a subject, as defined hereinabove.

Since the antigen binding domain of the CARs of the present invention are derived from the human anti-FAP antibodies of the present invention, it is prudent to expect that said CARs substantially retain the binding characteristics of these antibodies, in particular the selectively and pH-dependent binding to FAP *in vivo* in human blood plasma and on diseased tissue characterized by the presence of FAP such as breast cancer tissue, carcinoma, multiple myeloma tissue, atherosclerotic plaque and obstructive coronary thrombi as well as the same or similar inhibitory effect on FAP serine protease activity and the anti-tumor effect. Furthermore, since the recombinant antibodies of the present invention are derived from human memory B cells it is possible to produce CARs entirely made up of human-derived components thus arriving at a FAP CAR and CAR T cell which less immunogenic, if at all compared to those described in the prior art using for example FAP binding domain from mouse monoclonal antibody..

Thus, the present invention *inter alia* relates a cell (*e.g.,* T cell) modified to express the FAP CAR of the present invention which is preferably entirely human in the sense of substantially lacking antigens foreign to the human body and, in some instances, can elicit a CAR-mediated T-cell response. The invention provides the use of the FAP CAR to redirect the specificity of a primary T cell to the FAP antigen. Thus, the present invention also provides a method for use in stimulating a T cell-mediated immune response to a population of stromal cells within a tumor microenvironment in a mammal comprising the step of administering to the mammal a T cell that expresses the FAP CAR of the present invention.

In one embodiment, the CAR of the present invention is for use in a type of cellular therapy where T cells are genetically modified to express a CAR and the CAR T cell is infused to a recipient in need thereof. The infused cell is able to reduce tumor burden in the recipient. Unlike antibody therapies, CAR T cells are able to replicate *in vivo* resulting in long term persistence that can lead to sustained tumor control. In one embodiment, the CAR T cells of the invention can undergo robust *in vivo* T cell expansion and can persist for an extended amount of time. In another embodiment, the CAR T cells of the invention evolve into specific memory T cells that can be reactivated to inhibit any additional tumor formation or growth. For example, CAR T cells of the invention can undergo robust *in vivo* T cell expansion and persist at high levels for an extended amount of time in blood and bone marrow and form specific memory T cells. Without wishing to be bound by any particular theory, CAR T cells may differentiate *in vivo* into a central memory-like state upon encounter and subsequent elimination of target cells expressing the surrogate antigen.

Without wishing to be bound by any particular theory, the response elicited by the CAR-modified T cells may be an active or a passive immune response. In addition, the CAR mediated immune response may be part of an adoptive immunotherapy approach in which CAR-modified T cells induce an immune response specific to the antigen binding domain in the CAR. For example, a FAP CAR T cell elicits an immune response specific against cells expressing FAP. While the data disclosed herein specifically disclose a CAR comprising an anti-FAP binding domain, along with 4-IBB and CD3zeta signaling domains, the invention should be construed to include any number of variations for each of the components of the construct as described elsewhere herein.

As described elsewhere herein, the present invention provides FAP CAR T cells for use in the targeting of stromal cells which exist in the tumor microenvironment to treat cancers. As such, the present invention includes the CARs of the present invention for use in the treatment of any cancer where stromal cells exist. Cancers that may be treated include tumors that are not vascularized, or not yet substantially vascularized, as well as vascularized tumors. The cancers may comprise non-solid tumors (such as hematological tumors, for example, leukemias and lymphomas) or may comprise solid tumors. Types of cancers to be treated with the CARs of the invention include, but are not limited to, carcinoma, blastoma, and sarcoma, and certain leukemia or lymphoid malignancies, benign and malignant tumors, and malignancies *e.g.,* sarcomas, carcinomas, and melanomas. Adult tumors/cancers and pediatric tumors/cancers are also included.

Hematologic cancers are cancers of the blood or bone marrow. Examples of hematological (or hematogenous) cancers include leukemias, including acute leukemias (such as acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors, such as sarcomas and carcinomas, include fibrosarcoma, myxosarcoma, liposarcoma, chondrosarcoma, osteosarcoma, and other sarcomas, synovioma, mesothelioma, Ewing's tumor, leiomyosarcoma, rhabdomyosarcoma, colon carcinoma, lymphoid malignancy, pancreatic cancer, breast cancer, lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, melanoma, and CNS tumors (such as a glioma (such as brainstem glioma and mixed gliomas), glioblastoma (also known as glioblastoma multiforme) astrocytoma, CNS lymphoma, germinoma, medulloblastoma, Schwannoma craniopharyogioma, ependymoma, pinealoma, hemangioblastoma, acoustic neuroma, oligodendroglioma, menangioma, neuroblastoma, retinoblastoma and brain metastases).

The CAR-modified T cells of the invention may also be used as a type of vaccine for *ex vivo* immunization and/or *in vivo* therapy in a mammal. Preferably, the mammal is a human.

With respect to *ex vivo* immunization, at least one of the following occurs *in vitro* prior to administering the cell into a mammal: i) expansion of the cells, ii) introducing a nucleic acid encoding a CAR to the cells, and/or iii) cryopreservation of the cells. *Ex vivo* procedures are well known in the art and are discussed more fully below. Briefly, cells are isolated from a mammal (preferably a human) and genetically modified (*i. e.,* transduced or transfected *in vitro*) with a vector expressing a CAR disclosed herein. The CAR-modified cell can be administered to a mammalian recipient to provide a therapeutic benefit. Tue mammalian recipient may be a human and the CAR-modified cell can be autologous with respect to the recipient. Alternatively, the cells can be allogeneic, syngeneic or xenogeneic with respect to the recipient.

The procedure for *ex vivo* expansion of hematopoietic stem and progenitor cells is described in U.S. Pat. No. 5,199,942, incorporated herein by reference, can be applied to the cells of the present invention. Other suitable methods are known in the art, therefore the present invention is not limited to any particular method of *ex vivo* expansion of the cells. Briefly, *ex vivo* culture and expansion of T cells comprises: (1) collecting CD34+ hematopoietic stem and progenitor cells from a mammal from peripheral blood harvest or bone marrow explants; and (2) expanding such cells *ex vivo.* In addition to the cellular growth factors described in U.S. Pat. No. 5,199,942, other factors such as flt3-L, IL-1, IL-3 and c-kit ligand, can be used for culturing and expansion of the cells. In addition to use a cell-based vaccine in terms of *ex vivo* immunization, the present invention also provides compositions and methods for use in *in vivo* immunization to elicit an immune response directed against an antigen in a patient.

Generally, the cells activated and expanded as described herein may be used in the treatment and prevention of diseases that arise in individuals who are immunocompromised. In particular, the CAR-modified T cells of the invention are used in the treatment of cancer. In certain embodiments, the cells of the invention are used in the treatment of patients at risk for developing cancer. Thus, the present invention provides methods for use in the treatment or prevention of cancer comprising administering to a subject in need thereof, a therapeutically effective amount of the CAR-modified T cells of the invention.

In one embodiment, the T cells modified to express the FAP CAR are used for administering as monotherapy. However, in another embodiment, the FAP CAR T cells are used for administering in a combination therapy, e.g. along with an antitumor vaccine as disclosed elsewhere herein.

The FAP CAR T cell of the present invention may be administered either alone, or as a pharmaceutical composition in combination with diluents and/or with other components such as IL-2 or other cytokines or cell populations. Briefly, pharmaceutical compositions of the present invention may comprise a target cell population as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an immunologically effective amount," "an anti-tumor effective amount," "an tumor-inhibiting effective amount," or "therapeutic amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the T cells described herein may be administered at a dosage of $10^4$ to $10^9$ cells/kg body weight, preferably $10^5$ to $10^6$ cells/kg body weight, including all integer values within those ranges. T cell compositions may also be administered multiple times at these dosages. Tue cells can be administered by using infusion techniques that are commonly known in immunotherapy (see, *e.g.,* Rosenberg et al., New Eng. J. Med 319 (1988), 1676). The optimal dosage and treatment regime for a particular patient can readily be determined by one skilled in the art of medicine by monitoring the patient for signs of disease and adjusting the treatment accordingly.

It may be desired to administer activated T cells to a subject and then subsequently redraw blood (or have an apheresis performed), activate T cells therefrom according to the present invention, and reinfuse the patient with these activated and expanded T cells. This process can be carried out multiple times every few weeks. In certain embodiments, T cells can be activated from blood draws of from 10cc to 400cc. In certain embodiments, T cells are activated from blood draws of 20cc, 30cc, 40cc, 50cc, 60cc, 70cc, 80cc, 90cc, or 100cc. Not to be bound by theory, using this multiple blood draw/multiple reinfusion protocol may serve to select out certain populations of T cells.

The administration of the subject compositions may be carried out in any convenient manner, including by aerosol inhalation, injection, ingestion, transfusion, implantation or transplantation. The compositions described herein may be administered to a patient subcutaneously, intradermally, intratumorally, intranodally, intramedullary, intramuscularly, by intravenous (i.v.) injection, or intraperitoneally. Thus, T cell compositions of the present invention can be administered to a patient by intradermal or subcutaneous injection or by i.v. injection. The compositions of T cells may be injected directly into a tumor, lymph node, or site of infection.

Cells activated and expanded using the methods described herein, or other methods known in the art where T cells are expanded to therapeutic levels, can be administered to a patient in conjunction with (e.g., before, simultaneously or following) any number of relevant treatment modalities, including but not limited to treatment with agents such as antiviral therapy, cidofovir and interleukin-2, Cytarabine (also known as ARA-C) or natalizumab treatment for MS patients or efalizumab treatment for psoriasis patients or other treatments for PML patients. Furthermore, the T cells of the invention may be used in combination with chemotherapy, radiation, immunosuppressive agents, such as cyclosporin, azathioprine, methotrexate, mycophenolate, and FK506, antibodies, or other immunoablative agents such as CAMPATH, anti-CD3 antibodies or other antibody therapies, cytoxin, fludaribine, cyclosporin, FK506, rapamycin, mycophenolic acid, steroids, FR901228, cytokines, and irradiation. These drugs inhibit either the calcium dependent phosphatase calcineurin (cyclosporine and FK506) or inhibit the p70S6 kinase that is important for growth factor induced signaling (rapamycin) (Liu et al., Cell 66 (1991), 807-815; Henderson et al., Immun. 73 (1991), 316-321; Bierer et al., Curr. Opin. Immun. 5 (1993), 763-773). Furthermore, the cell compositions of the present invention can be administered to a patient in conjunction with *(e.g.,* before, simultaneously or following) bone marrow transplantation, T cell ablative therapy using either chemotherapy agents such as, fludarabine, extemal-beam radiation therapy (XRT), cyclophosphamide, or antibodies such as OKT3 or CAMPATH. Moreover, the cell compositions of the present invention can be administered following B-cell ablative therapy such as agents that react with CD20, e.g., Rituxan. For example, subjects may undergo standard treatment with high dose chemotherapy followed by peripheral blood stem cell transplantation or, following the transplant, subjects receive an infusion of the expanded immune cells of the present invention. Expanded cells can be administered before or following surgery.

The dosage of the above treatments to be administered to a patient will vary with the precise nature of the condition being treated and the recipient of the treatment. Tue scaling of dosages for human administration can be performed according to art-accepted practices. Strategies for CAR T cell dosing and scheduling have been discussed; see, *e.g.,* Ertl et al., Cancer Res 71 (2011), 3175-3181; Junghans, Journal of Translational Medicine 8 (2010), 55.

IX. Peptides with specific FAP epitopes

In a further aspect the present invention relates to peptides having an epitope of FAP specifically recognized by any antibody of the present invention NI-206.18H2, NI-206.82C2, NI-206.59B4, NI-206.22F7, NI-206.27E8, NI-206.12G4 and NI-206.17A6. Preferably, such peptide comprises or consists of an amino acid sequence as indicated in SEQ ID NOs: 30 to 37 and 60 as the unique linear epitope recognized by the antibody or a modified sequence thereof in which one or more amino acids are substituted, deleted and/or added, wherein the peptide is recognized by any antibody of the present invention, preferably by antibody NI-206.18H2, NI-206.82C2, NI-206.59B4, NI-206.22F7, NI-206.27E8, NI-206.12G4 and NI-206.17A6.

In one embodiment of this invention such a peptide may be used for diagnosing or monitoring a disease or disorder related to FAP species and/or fragment thereof in a subject comprising a step of determining the presence of an antibody that binds to a peptide in a biological sample of said subject, and being used for diagnosis of such a disease in said subject by measuring the levels of antibodies which recognize the above described peptide of the present invention and comparing the measurements to the levels which are found in healthy subjects of comparable age and gender.

Furthermore, since the peptide of the present invention contains an epitope of a therapeutically useful antibody derived from a human such peptide can of course be used as an antigen, *i.e.* an immunogen for eliciting an immune response in a subject and stimulating the production of an antibody of the present invention *in vivo.* The peptide of the present invention may be formulated in an array, a kit and composition such as a vaccine, respectively, as described hereinbefore. In this context, the present invention also relates to a kit useful in the diagnosis or monitoring the progression of a FAP-related disease, said kit comprising at least one antibody of the present invention or a FAP-binding molecule having substantially the same binding specificities of any one thereof, the polynucleotide, the vector or the cell and/or the peptide as respectively defined hereinbefore, optionally with reagents and/or instructions for use.

X. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is used for treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection.

These and other embodiments are disclosed and encompassed by the description and Examples of the present invention. Further literature concerning any one of the materials, methods, uses, and compounds to be employed in accordance with the present invention may be retrieved from public libraries and databases, using for example electronic devices. For example the public database "Medline" may be utilized, which is hosted by the National Center for Biotechnology Information (NCBI) and/or the National Library of Medicine at the National Institutes of Health (NLM.NIH). Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The above disclosure generally describes the present invention. Unless otherwise stated, a term as used herein is given the definition as provided in the Oxford Dictionary of Biochemistry and Molecular Biology, Oxford University Press, 1997, revised 2000 and reprinted 2003, ISBN 0 19 850673 2. Several documents are cited throughout the text of this specification. Full bibliographic citations may be found at the end of the specification immediately preceding the claims. The contents of all cited references (including literature references, issued patents, published patent applications as cited throughout this application including the background section and manufacturer's specifications, instructions, etc.) are hereby expressly incorporated by reference; however, there is no admission that any document cited is indeed prior art as to the present invention. A more complete understanding can be obtained by reference to the following specific examples which are provided herein for purposes of illustration only and are not intended to limit the scope of the invention.

EXAMPLES

Human-derived antibodies targeting FAP were identified utilizing the method described in the international application WO 2008/081008 with modifications. In particular, human-derived antibodies targeting FAP were identified by high-throughput analyses of complements of the human memory B-cell repertoire derived from the clinically selected donors. For FAP antibody screening on directly coated target, 96-well microplates (Costar, Corning, USA) were coated overnight at 4°C with recombinant human FAP (rFAP), cFAP (a mixture of peptides: 378-HYIKDTVENAIQITS-392, 622-GWSYGGYVSSLALAS-636 and 721-QVDFQAMWYSDQNHGL-736) or BSA (Sigma-Aldrich, Buchs, Switzerland) diluted to a concentration of 5 $\mu$g/ml in carbonate buffer (15 mM $Na_2CO_3$, 35 mM $NaHCO_3$, pH 9.4). For the capture ELISA, the microplates were coated with mouse anti-His monoclonal antibody (Clontech) diluted to a concentration of 3 $\mu$g/ml in PBS, the plates were then blocked, rFAP or BSA were diluted to a concentration of 2 $\mu$g/ml in PBS and added to the plates to be captured. Plates were then washed in PBS-T pH 7.6 and non-specific binding sites were blocked for 1 hr at RT with PBS/0.1% Tween-20 containing 2% BSA. B cell conditioned medium was transferred from memory B cell culture plates to ELISA plates and incubated for one hour at RT. ELISA plates were washed in PBS-T and binding was determined using horseradish peroxidase (HRP)-conjugated anti-human immunoglobulins polyclonal antibodies (Jackson ImmunoResearch, Newmarket, UK) followed by measurement of HRP activity in a standard colorimetric assay. Only B cell cultures which have shown binding of the antibodies contained in the medium to FAP (either rFAP directly coated or captured, or cFAP) but not to BSA were subjected to antibody cloning.

The amino acid sequences of the variable regions of the above identified anti-FAP antibodies were determined on the basis of their mRNA sequences; *see* Fig. 1. In brief, living B cells of selected non-immortalized memory B cell cultures were harvested. Subsequently, the mRNAs from cells producing selected anti-FAP antibodies were extracted and converted in cDNA, and the sequences encoding the antibody's variable regions were amplified by PCR, cloned into plasmid vectors and sequenced. In brief, a combination of primers representing all sequence families of the human immunoglobulin germline repertoire was used for the amplifications of leader peptides, V-segments and J-segments. The first round of amplification was performed using leader peptide-specific primers in 5'-end and constant region-specific primers in 3'-end (Smith et al., Nat. Protoc. 4 (2009), 372-384). For heavy chains and kappa light chains, the second round of amplification was performed using V-segment-specific primers at the 5'-end and J-segment-specific primers at the 3'-end. For lambda light chains, the second round amplification was performed using V-segment-specific primers at the 5'-end and a C-region-specific primer at the 3'-end (Marks et al., Mol. Biol. 222 (1991), 581-597; de Haard et al., J. Biol. Chem. 26 (1999), 18218-18230).

Identification of the antibody clone with the desired specificity was performed by re-screening on ELISA upon recombinant expression of complete antibodies. Recombinant expression of complete human IgG1 antibodies was achieved upon insertion of the variable heavy and light chain sequences "in the correct reading frame" into expression vectors that complement the variable region sequence with a sequence encoding a leader peptide at the 5'-end and at the 3'-end with a sequence encoding the appropriate constant domain(s). To that end the primers contained restriction sites designed to facilitate cloning of the variable heavy and light chain sequences into antibody expression vectors. Heavy chain immunoglobulins were expressed by inserting the immunoglobulin heavy chain RT-PCR product in frame into a heavy chain expression vector bearing a signal peptide and the constant domains of human immunoglobulin gamma 1 or mouse immunoglobulin gamma 2a. Kappa light chain immunoglobulins were expressed by inserting the kappa light chain RT-PCR-product in frame into a light chain expression vector providing a signal peptide and the constant domain of human or mouse kappa light chain immunoglobulin. Lambda light chain immunoglobulins were expressed by inserting the lambda light chain RT-PCR-product in frame into a lambda light chain expression vector providing a signal peptide and the constant domain of human or mouse lambda light chain immunoglobulin.

Functional recombinant monoclonal antibodies were obtained upon co-transfection into HEK 293 or CHO cells (or any other appropriate recipient cell line of human or mouse origin) of an Ig-heavy-chain expression vector and a kappa or lambda Ig-light-chain expression vector. Recombinant human monoclonal antibody was subsequently purified from the conditioned medium using a standard Protein A column purification. Recombinant human monoclonal antibody can produced in unlimited quantities using either transiently or stably transfected cells. Cell lines producing recombinant human monoclonal antibody can be established either by using the Ig-expression vectors directly or by re-cloning of Ig-variable regions into different expression vectors. Derivatives such as F(ab), F(ab)2 and scFv can also be generated from these Ig-variable regions.

The framework and complementarity determining regions were determined by comparison with reference antibody sequences available in databases such as Abysis (http://www.bioinf.org.uk/abysis/), and annotated using the Kabat numbering scheme (http://www.bioinf.org.uk/abs/). The amino acid sequences of the variable regions of the subject antibodies NI-206-18H2, NI-206-20A8, NI-206-6D3, NI-206-14C5, NI-206-34C11, NI-206.82C2, NI-206.59B4, NI-206.22F7, NI-206.27E8, NI-206.12G4 and NI-206.17A6 including indication of the framework (FR) and complementarity determining regions (CDRs) are shown in Figure 1G-1K and 1A-1F.

Example 1: Binding specificity of FAP antibodies

ELISA assays were performed with varying antibody concentrations to validate the binding of the exemplary antibodies of the present invention to FAP and to be able to determine their half maximal effective concentration ($EC_{50}$). For the exemplary recombinant human NI-206-18H2, NI-206-20A8, NI-206-6D3, 'NI-206-14C5, NI-206-34C11, NI-206.82C2, NI-206.59B4, NI-206.22F7, NI-206.27E8, NI-206.12G4 and NI-206.17A6 antibodies, 96-well microplates (Costar, Corning, USA) were coated with FAP, with a mixture of the 3 peptides or with BSA (Sigma-Aldrich, Buchs, Switzerland) diluted to a concentration of 5 μg/ml in carbonate ELISA coating buffer (15 mM $Na_2CO_3$, 35 mM $NaHCO_3$, pH 9.4) for the direct ELISA. For the capture ELISA, the microplates were coated with mouse anti-His monoclonal antibody (Clontech) diluted to a concentration of 3 μg/ml in PBS, the plates were then blocked, FAP or BSA were diluted to a concentration of 2 μg/ml in PBS and added to the plates to be captured. The binding efficiency of the antibodies was then tested. The exemplary NI-206.82C2 antibody specifically and strongly binds to the captured FAP and less efficiently to the directly coated FAP. The exemplary NI-206.59B4 and NI-206.18H2 antibodies specifically, strongly and similarly bind to the captured FAP and to the directly coated FAP. In comparison to the aforementioned exemplary antibodies, the NI-206.20A8 antibody binds less efficiently to both the captured FAP and the directly coated FAP. No binding was observed to BSA. The exemplary antibody NI-206.6D3 strongly and similarly bind to the captured FAP and to the directly coated FAP, but especially in case of the captured FAP, at high antibody concentrations strong binding to BSA was observed as well; see Figure 2A-J.

The $EC_{50}$ values were estimated by a non-linear regression using GraphPad Prism (San Diego, USA) software. Recombinant human-derived antibodies NI-206.82C2, NI-206.59B4, NI-206.27E8, NI-206.17A6, NI-206.18H2 and NI-206.20A8 bound with a high affinity to the captured FAP (sFAP) with an $EC_{50}$ of 0.014 nM, 0.044 nM, 3.36 nM, 50.2 nM, 0.272 nM and 1.33 nM respectively, whereas the antibody NI-206.6D3 showed a lower binding affinity with an $EC_{50}$ of 118 nM. NI-206.22F7 did not show any binding to sFAP. The binding of NI-206.12G4 towards sFAP was not tested. Recombinant human-derived antibodies NI-206.82C2, NI-206.59B4, NI-206.27E8, NI-206.12G4, NI-206.17A6, NI-206.18H2 and NI-206.20A8 bound with a high affinity to the directly coated FAP (FAP) with an $EC_{50}$ of 0.61 nM, 0.096 nM, 0.33 nM, 1.4 nM, 10.5 nM, 3.54 nM and 2.90 nM respectively, whereas the antibody NI-206.6D3 showed a lower binding affinity with an $EC_{50}$ of 86.5 nM. NI-206.22F7 did not show any binding to directly coated FAP. Recombinant human-derived antibody NI-206.22F7 bound with a high affinity to the directly coated FAP peptides mixture (cFAP) with an $EC_{50}$ of 0.12 nM. NI-206.82C2, NI-206.59B4, NI-206.27E8, NI-206.12G4, NI-206.17A6, NI-206.18H2, NI-206.20A8 and NI-206.6D3 did not show any binding to cFAP; see Figure 2K.

**Example 2: Determination of NI-206.82C2 binding kinetics**

To address NI-206.82C2 kinetics, recombinant human FAP (rhuFAP, Sino Biologicals, 10464-H07H) was amine-coupled onto a GLC sensor chip (Biorad #176-5011), leaving one channel unmodified to provide an additional reference surface. This was achieved by varying the concentration of the activation reagents used in each channel. Three activation solutions were prepared using a threefold serial dilution of a stock mixture containing 0.4 M EDC + 0.1 M sulfo-NHS and injected for 360 s. Then rhuFAP at 2.5 μg/ml in coupling buffer (10 mM HEPES, 150 mM NaCl, 3,4 mM EDTA, 0.005% Tween 20, pH 5.2) was coupled for 1020 s at 25 μL/min. Excess reactive esters were blocked for 600 s with 1 M ethanolamine hydrochoride. This created uniform strips of rhuFAP spanning final immobilized levels of 700 RU. A five-fold serial dilution of rhuFAP starting at 16 μg/mL (106.7nM) was injected for 400 s at 60 μL/min. A single injection delivered a full concentration series, using buffer to complete a row of six samples and provide an in-line blank for double-referencing the response data. Association and dissociation phases were measured for 400 s and 3600 s, respectively. Immobilized rhuFAP was regenerated with 1.5 M glycine pH 3 for 30 s at 100 μL/min after each binding cycle, and NI-206.82C2 were analyzed in duplicate injections within the same experiment to confirm cycle-to-cycle reproducibility; see Figure 3.

**Example 3: Assessment of the binding epitope of the FAP specific antibodies**

To determine the binding epitope of the exemplary NI-206.82C2 and NI-206.18H2 antibodies, binding analyses were performed with overlapping peptides mapping the entire sequences of FAP. Binding capacity of the antibodies was tested on these peptides spotted onto a nitrocellulose membrane (JPT Peptide Technologies, Berlin, Germany) and using HRP-conjugated donkey anti-human IgG secondary antibody (Jackson immunoResearch, Newmarket, UK) followed by detection of HRP activity (Figure 4A and C). In brief, epitope mapping was performed using scans of overlapping peptides. The entire sequences of FAP were synthesized as a total of 188 linear 15-mer peptides with an 11 amino acid overlap between individual peptides. Those peptides were spotted onto nitrocellulose membranes (JPT Peptide Technologies, Berlin, Germany). The membrane was activated for 5 min in methanol and washed in TBS for 10 min at RT. Non-specific binding sites were blocked for 2h at RT with Roti®-Block (Carl Roth GmbH+Co. KG, Karlsruhe, Germany). Human antibodies (1 μg/ml) were incubated in Roti®-Block for 3h at RT. Binding of primary antibody was determined using HRP-conjugated donkey anti-human IgG secondary antibody. Blots were developed and evaluated using ECL and ImageQuant 350 detection (GE Healthcare, Otelfingen, Switzerland).

The antibody NI-206.82C2 recognizes the peptides 131 and 132 (line G, 11th and 12th spot) which correspond to the sequence 525-PPQFDRSKKYP-535 on FAP; see Figure 4A and the antibody NI-206.18H2 recognizes the peptides 125 and 126 (line G, 5th and 6th spot) which correspond to the sequence 501-IQLPKEEIKKL-511; see Figure 4C. The antibody NI-206.59B4 recognizes the sequence 53-SYKTFFP-59 on FAP. The antibody NI-206.22F7 recognizes the sequence 381-KDTVENAIQIT-391 on FAP. The antibody NI-206.27E8 recognizes the sequence 169-NIYLKQR-175 on FAP. The antibody NI-206.12G4 recognizes the sequence 481-TDQEIKILEENKELE-495 on FAP. The antibody NI-206.17A6 recognizes the sequence 77-VLYNIETGQSY-87 on FAP.

To determine the minimum epitope region of antibody NI-206.82C2 peptides (from the N- and C-terminal) covering the epitope of antibody NI-206.82C2 were sequentially truncated by one amino acid (with spot 21 corresponding to the full length peptide and spots 22 to 33 to stepwise one amino acid truncations form the C-terminus and spots 34 to 45 corresponding to stepwise one amino acid truncations form the N-terminus), synthesized and spotted onto nitrocellulose membranes (JPT Peptide Technologies, Berlin, Germany). NI-206.82C2 binding to these spotted peptides was visualized as described above. The antibody NI-206.82C2 recognizes spots 21-28 and 34-41 which correspond to the sequence 528-FDRSK-532 (SEQ ID NO: 39) on FAP; see Figure 26.

To determine the amino acids essential for NI-206.82C2 binding, every single amino acid from 521-KMILPPQFDRSKKYPLLIQ-539 (SEQ ID NO: 38) of FAP was mutated sequentially into an alanine to determine the essential amino acids (*i.e.* those which cause a loss of NI-206.82C2 binding when mutated). These linear peptide sequences with single alanine-mutated linear epitopes were synthesized and spotted onto nitrocellulose membranes (JPT Peptide Technologies, Berlin, Germany). NI-206.82C2 binding to these spotted peptides was visualized as described in the example above, with an absence of binding to two spots (spot 10 and spot 13) in which the corresponding FAP peptide contained alanine substitutions at position 529 and 532, respectively, thus revealing that amino acids D-529 and K-532 of FAP are essential for NI-206.82C2 binding; see Figure 27.

### Example 4: Determination of the ability of recombinant human monoclonal antibodies to inhibit FAP enzymatic activity

A black flat bottom standard 96-well ELISA plate was blocked for 1 hour at 37°C using sterile blocking buffer: 5% bovine serum albumin (BSA) in phosphate buffered saline (PBS). The blocking buffer was removed and replaced with: 40μL of fresh blocking buffer, 40μL of antibody in PBS, 10μL of 20nM active recombinant human FAP (Sino Biologicals, 10464-H07H) in PBS, and 10μL of DQ-Gelatin (Molecular Probes, D-12054) in PBS. Fluorescence intensity was measured at 485nM excitation and 530nM emission every 3min over a total time of 45min, while gently shaking the plate for twenty seconds before every measurement. To calculate the fractional activity, the steady state velocity (Δ 530nM emission / Δ time) is divided by the velocity observed at each concentration of antibody. The results are shown in Figure 5.

### Example 5: Determination of competitive, noncompetitive, or uncompetitive inhibition of rhuFAP-mediated PEP (Z-Gly-Pro-AMC) cleavage

A black flat bottom standard 96-well ELISA plate was blocked for 1 hr at 37°C using sterile blocking buffer: 5% bovine serum albumin (BSA) in phosphate buffered saline (PBS). 10μL of recombinant human FAP solution: 0,4 nM recombinant human FAP (Sino Biologicals, 10464-H07H), in 225mM Sodium phosphate buffer, pH 7,4) was added to each well. Then, 80μL of NI-206.82C2 at the indicated concentrations in PBS were added to the wells incubated for 1hr at 37°C. Then, 10μL of fluorogenic substrate solution (Z-Gly-Pro-AMC in 40% methanol and 60% 25nM PBS/10nM EDTA) was added to each well. The fluorescence intensity was measured at 360nM excitation and 460nM emission every 3min over a total reading time of 2hr. The results are shown in Figure 6.

### Example 6: Determination of the binding specificity of antibodies NI-206.82C2, NI-206.18H2, NI-206.20A8 and NI-206.6D3

A mouse anti-6X-histidine tag antibody (Clonetech) was coated at a concentration of 3 μg/ml in PBS to a 96 well ELISA plate, the plates were then blocked with 5% BSA in PBS, and enzymatically active recombinant human FAP with an N-terminus 6X histidine tag was added to the plates to be captured. The binding efficiency of NI-206.82C2, NI-206.18H2, NI-206.20A8 and NI-206.6D3 (at 20 nM, 4 nM, and 0.8 nM) against sFAP and of NI-206.18H2, NI-206.20A8 and NI-206.6D3 against FAP as well was then tested by 1 hour incubation, followed by washing with PBS and detection with an HRP-labelled goat anti-human antibody (Jackson Immunoresearch) using a colormetric assay. To assess NI-206.82C2, NI-206.18H2, NI-206.20A8 and NI-206.6D3 binding other targets, recombinant human CD26 (only in case of NI-206.82C2) and fourteen other unrelated recombinant human proteins (A-N) were individually added to a 96-well ELISA plate in triplicate, and the binding efficacy of NI-206.82C2 was evaluated. (Figure 7A to D)

To determine the binding efficiency and inhibitory ability of NI-206.82C2 against other members of the SB9 oligopeptidase family that have sequence similarity to FAP, indirect ELISA and inihibiton assays were performed on active enzyme targets. Active recombinant human enzymes evaluated in this assay include: Fibroblast Activation Protein (FAP; Sino Biologicals, 10464-H07H), Dipeptidyl Peptidase IV (DPPIV, BPS Bioscience 80040), Dipeptidyl Peptidase 8 (DPP8, BPS Bioscience 80080), Dipeptidyl Peptidase 9 (DPP9, BPS Bioscience 80080), and Prolyl Oligopeptidase / Prolyl Endopeptidase (POP/PREP, BPS Bioscience 80105). Each recombinant peptidase was expressed with a purification tag, and attached to the 96 well ELISA plate using either a mouse anti-Histidine antibody (CloneTech), or a mouse anti-Glutathione S-transferase (GST, Sino Biologicals, 111213-MM02) tag antibody. Each enzyme was validated to bind and remained active on the ELISA plate using enzyme specific fluorogenic substrates. H-Gly-Pro-AMC (ATT Bioquest, 13450) was used to validate the presence and activity of DPP4, DPP9, and DPP8. Z-Gly-Pro-AMC (BaChem, I1145) was used to evaluate the activity of FAP and POP/ PREP. To determine the binding efficiency of NI-206.82C2 to each enzyme target, the antibody was labelled with HRP and added at concentrations of 400, 126.5, 40.1, 12.7, 4, 1.3, 0.4, 0.13, 0.04, 0.013, 0.004, and OnM in PBS. Following a washing step, bound antibody was detected using 3,3',5,5'-Tetramethylbenzidine (TMB) substrate. The reaction was stopped after 10 minutes by adding 2N $H_2SO_4$ and the resulting change in optical density was quantified using an ELISA plate reader at 450nM absorbance (Fig. 7 E).

To determine the inhibitory ability of the antibody a black 96 well plate was blocked for 1 h with blocking buffer. NI-206.82C2 was pipetted at concentrations of 1000, 50, 10, 3.03, 1.01, 0.031, 0.001, and 0nM in PBS into the according wells. The enzymes were added at a concentration of 0.2nM. Finally, fluorgenic substrates were then added to the well at a concentration equal to the Michaelis constant ($K_m$) and the velocity of the substrate cleavage was quantified by fluorescence at 360nm excitation and 460nm emission. H-Gly-Pro-AMC (AAT Bioquest, 13450) was used for DPP4, DPP9, and DPP8. Z-Gly-Pro-AMC (BaChem, I1145) was used to evaluate the activity of POP/ PREP. FAP activity was assessed using DQ-gelatin (Life Technologies, D12054) and cleavage quantified at 495nM excitation and 515nM emission.

**Example 7: Determination of NI-206.82C2 inhibitory ability against active recombinant human FAP and active recombinant mouse FAP compared to previously tested FAP inhibitors**

To determine the inhibitory ability of NI-206.82C2 against active recombinant human FAP (Sino Biologicals, 10464-H07H) a black 96 well ELISA plate was blocked for 1h at 37°C with sterile 5% BSA in PBS. After removing the blocking solution, 40uL of 12.5% BSA in PBS was added to each well. Then FAP-targeting agents (NI-206.82C2, F19, and Val-Boro-Pro (PT-100; Point therapeutics)) were added to the appropriate wells for a final concentration of 500, 10, 3.03, 1.01, 0.031, 0.001, and 0nM. 10uL of active recombinant human FAP was then added to all the wells for a final assay concentration of 20nM. Finally, 10μL of DQ-gelatin solution was added to each well for a concentration of 60μg/mL. The fluorescent intensity was then measure at 485nM excitation and 530nM emission every 3min for 45min, gently shaking the plate for 20min before each measurement. Using GraphPad Prism 6 software steady state velocity was then used to calculate fractional activity at each concentration compared to the steady-state concentration where no inhibitor was given, and fit to a three parameter variable fit model to calculate the $IC_{50}$ (the concentration at which 50% of the maximum inhibition as achieved). (Fig. 8, A)

To determine the inhibitory ability of NI-206.82C2 against active recombinant murine FAP, recombinant murine FAP was expressed in a HEK293 cell line without the transmembrane and intracellular domains and an N-terminal 6X histidine tag. A 96-well ELISA plate was then coated with 50uL of murine anti-His tag antibody at 6μg/mL in PBS overnight at 4°C. The wells were then blocked with 60μL of 2% BSA in PBS for 1h at room temperature, and 50μL of recombinant mouse FAP containing cell culture supernatant diluted 1:4 was added to the well and incubated overnight at 4C while gently shaking. Following three washing steps with PBS, 20uL of sterile 12.5% BSA/PBS solution was added to all wells. FAP-targeting agents (NI-206.82C2, F19, and Val-Boro-Pro (PT-100; Point therapeutics) were then added to the appropriate wells for a final concentration of 500, 10, 3.03, 1.01, 0.031, 0.001, and 0nM. Finally 5μL of DQ Gelatin solution in PBS was then added for a final assay concentration of 24μg/mL. DQ gelatin cleavage was then measured by the fluorescence intensity a 485nM excitation and 530nM emission every 3 min over a total time of 60 min, while gently shaking the plate for 20 seconds before each measurement. Using GraphPad Prism 6 software, steady state velocity was used to calculate fractional activity at each concentration, and to preform three parameter variable model regression to calculate the IC50 (the concentration at which 50% of the maximum inhibition as achieved) (Fig. 8, B).

**Example 8: Determination of NI-206.82C2 binding to human carcinoma cryosections by confocal immunofluorescence**

NI-206.82C2 was labelled with Cyanine 3 for fluorescent imaging (Cy3 conjugation kit: Innova Biosciences, 340-0030) and antibody labelling was validated using a spectophotometer. Cryosections from human invasive ductal carcinoma tissues (Figure 9, A) and invasive lobular carcinoma (Figure 9, B) were fixed for 5 minutes in ice cold acetone and allowed to dry for 10 minutes before washing in PBS. The sections were then incubated for 1 hr at room temperature in 5% BSA in PBS. The tissue sections were then incubated with Cyanine 3 prelabelled antibodies overnight at 4°C, and then washed three times in PBS before incubation with DAPI at 0.5μg/mL. Sections were then washed three additional times in PBS and mounted in Lisbeth's mounting medium before imaging on a SP8 confocal microscope (Leica Microsystems).

**Example 9: Determination of NI-206.82C2 binding to human breast cancer tissue sections by immunohistochemistry**

Human breast cancer tissue sections were allowed to dry for 10 min at room temperature, and then fixed for 15 minutes in 4% paraformaldahyde, followed by washing three times 5 minutes in PBS. Slides were then incubated for 5 min in 0.3% $H_2O_2$ in PBS to block endogenous peroxidase activity, and subsquently washed 3 times 5 min in PBS. Endogenous biotin was then blocked using the Biotin-Blocking System (DAKO X0590), followed by times washing with PBS. Blocking of unspecific antibody binding and permiabilization of the tissue section was performed using blocking buffer (5% goat serum, 5% horse serum, 0.3M glycine, 5% BSA, and 0.5% Triton X100 in PBS). Tissue sections were then stained with a recombinantly engineered chimeric form of NI-206.82C2 with a murine constant domain and the human variable domain of the original antibody and a matched isotype control (43A11) at 10μg/mL in permiabilization buffer overnight at 4°C. Following three PBS washing steps, the sections were incubated with a biotinylated goat anti-mouse antibody for 1h at room temperature. For amplifying the target antigen the VECTASTAIN ABC kit (Vector Labs) was used, followed by development with 3,3'-diaminobenzidine (DAB) and counterstained with Mayer's haematoxylin blue. Samples were washed for 10 min in lukewarm running tapwater and mounted in an aqueous mounting medium before imaging with a histology slide scanner (Zeiss Mirax MIDI). The results are shown in Figure 10 and 11.

**Example 10: Determination of NI-206.82C2 binding to murine colorectal cancer tissues**

NI-206.82C2 was prelabelled with Cyanine 5 antibody labeling kit (Innova Biosciences, 342-0010), and sufficient antibody labelling was validated with a photospectrometer. Cryosections of mouse livers containing syngeneic CT-26 liver metastasis were allowed to dry for 30 min at room temperature the fixed for 10 min in 4% formalin in PBS. Slides were then washed 3 times for 5 minutes in PBS and blocked for 1hr at room temperature with 5% bovine serum albumin in PBS. The slides were then incubated overnight at 4°C with staining solution containing DAPI at 0.5μg/mL, and Alexa 547 phalloidin (Invitrogen) according to the manufacturer's instructions, and either Cy5 labelled NI-206.82C2 or a Cy5 labelled isotype-matched control antibody 3A1 at a concentration of 10μg/mL, in blocking solution with 0.5% Triton X100. Slides were then washed 3 times in PBS, mounted in Lisbeth's mounting medium, and imaged with an SP8 confocal microscope (Leica Microsystems). The results are shown in Figure 12.

**Example 11: Determination of NI-206.82C2 binding to murine multiple myeloma tissues**

BALB/c mice were injected with $2\times10^6$ of the murine multiple myeloma cell line MOPC315.4 intravenously. BALB/c mice were injected with a vehicle-only control. Bone tissue was harvested and fixed in 4% PFA, decalcified in 10% EDTA (pH 7.4), embedded in paraffin before being sectioned (4 μm) and rehydrated. Then, CD138 mAb (Clone 281-2, BD Pharmingen) and Cy5 labelled NI-206.82C2 were used to identify MOPC315.BM.Luc cell infiltration, and NI-206.82C2 binding to these cells and surrounding stromal cells in the tissue sections. Stained tissue sections were then imaged using a fluorescent microscope (Figure 13).

**Example 12: Determination of NI-206.82C2 binding human atherosclerotic plaque and obstructive coronary thrombi by confocal immunofluorescence**

NI-206.82C2 was labelled with Cyanine 3 for fluorescent imaging (Cy3 conjugation kit: Innova Biosciences, 340-0030) and antibody labelling was validated using a spectophotometer. Cryosections from myocardial infarction causing obstructive human coronary thrombi (Figure 14, A) and human aortic atherosclerotic plaque (Figure 14, B) were fixed for 5 minutes in ice cold acetone and allowed to dry for 10 minutes before washing in PBS. The sections were then incubated for 1 hr at room temperature in 5% BSA in PBS, and then incubated with Cyanine 3 labelled antibodies overnight at 4°C, before washing three times in PBS before incubation with DAPI at 0.5μg/mL. Sections were then washed three additional times in PBS and mounted in Lisbeth's mounting medium before imaging on a SP8 confocal microscope (Leica Microsystems).

**Example 13: Determination of the role of NI-206.82C2 in blood coagulation using rotational thromboelastometry (ROTEM™)**

Two batches of peripheral blood were taken from a single healthy subject in sodium citrate tubes and platelet-free blood plasma was prepared by centrifugation. Blood plasma from each tube was pooled and immediately aliquoted for storage at -80 °C. The plasma has an FAP level of 130 ng/ml by ELISA. Plasma samples were treated with NI-206.82C2 (n=3) against FAP or 43A11 control (n=3) diluted in sterile saline and added to fresh fast thawed plasma such that the final concentration of antibody in the sample was 0.000667 nM, 0.00667 nM, 0.0667 nM, 0.667 nM 6.667 nM of plasma. Following 1hr incubation with the antibody at 37°C, NATEM analysis was performed according to the manufacturer's instructions by using STAR-TEM reagent to observe the native blood clotting process after an incubation time with the antibodies of 1h at 37°C with the antibody before starting measurement. Statistical analysis was performed using a two-way ANOVA (*p < 0.05, ** p < 0.01, *** p < 0.005, **** p < 0.001). The results are shown in Figure 15.

**Example 14: Determination of FAP clearance from human plasma using NI-206.82C2 based immunoprecipitation**

100μL of PureProteome G Magnetic Beads (Millipore LSKMAGG02) were suspended in 500μL of 25mM TRIS, 0.15M NaCl, and 0.1% Tween 20. Human plasma was diluted 1:5 in PBS to an end volume of 125μL, separated into four tubes, and incubated for 30 min at RT with rotation. Beads were then collected with a magnetic stand, and the pre-cleared supernatant was transferred to a new tube containing magnetic beads coated with 82C2, 43A11, or 3A1. Antibody-conjugated beads were incubated with the plasma dilution overnight at 4°C while rotating. Beads were then collected with a magnetic stand and the supernatant was removed of analysis of α2AP-AMC cleavage activity. To determine α2AP-AMC activity in the supernatants, a half area black 96 well plate was blocked with sterile filtered 5% BSA at 37°C for lh. Then 40μL of PBS was added to all the wells, 5μL of the supernatant solutions to the appropriate wells, and 5μL of α2AP-AMC solution in methanol was added for a final assay concentration of 10μM. The fluorescence intensity (cleaved AMC) was measured at 360nM excitation and 460nM emission every 3 min over a total time of 30 min and the reaction velocity was calculated using Graphpad Prism 6 software. The results are shown in Figure 16.

**Example 15: Characterization of a sandwich ELISA to measure the levels of NI-206.82C2 antigen**

To quantify the levels of NI-206.82C2 antigen in human samples, a clear 96-well plate was coated with 30μL of F19 (CRL-2733) at 8μg/mL in carbonate coating buffer for 2-4 hours at room temperature. The coating solution was removed and the plate was blocked with 40μL 2% BSA in PBS blocking buffer for 1h at room temperature and then discarded. 30uL sample solution was then added. Sample solutions included recombinant human FAP standard (Figure 17A), human serum at varying dilutions (Figure 17B), FAP homologues (Figure 17C), serum samples from healthy patients (Figures 18 and 19), serum samples patients with metastatic colorectal cancer (Figure 18), serum samples from patients with cardiovascular disease (Figure 19), sodium citrate plasma samples from healthy patients (Figure 20), and sodium citrate samples from patients with symptomatic carotid atherosclerotic plaques (Figure 20). Sample solutions were added at a total volume of 30μL. The plate was washed three times with 40 μL PBS and blotted dry. 30μL of the HRP-labelled 82C2 to the all wells, incubated for 1h at RT. The plate was then washed again three times with 3x PBS and blotted dry. 30μL TMB (3,3',5,5'-Tetramethylbenzidine) solution was then added to each well and allowed to develop for 5-10 minutes at room temperature. The reaction was then stopped by the addition of 30μL 1M H2SO4, and absorbance at 450nM was read with a plate reader.

**Example 16: Anti-FAP antibody NI-206.82C2 is capable of prolonging arterial occlusion times in a murine thrombosis model**

The carotid artery photochemical injury-induced thrombosis model begins with anesthesia by intraperitoneal injection of sodium pentobarbital (87 mg/kg body weight). After slight tail warming (using warm water) rose bengal (10mg/mL in PBS) is injected into the tail vein in a volume of 0.12 mL at a concentration of 50mg/kg. Mice will then be secured in a supine position (with the head pointing towards the operator) and placed on a heating pad (rectal temperature will be monitored) under a dissecting microscope. Following a (2.5-3 cm) midline cervical incision and a small incision of the larynx to provide a tracheostoma, by blunt preparation the right common carotid artery is exposed and cleared of connective tissue. A surgical stitch is employed to fix the sternocleidomastoid muscle aside to the right and increase access area to the right carotid artery. Care must be taken to avoid excessive vessel manipulation during procedures. Curved-tip tweezers will be employed to slide under the vessel (from the left side) and gently lift it so as to place the probe under and around it. The probe will be placed as proximal as the access area allows it to be and its connection wire should then be placed on a micromanipulator to fine-adjust its position. (The probe should be perfectly aligned with the vessel so as not to cause any resistance to flow). Little surgical ultrasonic gel should be applied on top of the probe to increase signal quality. Within 6 minutes of Rose Bengal injection, a laser beam will be aimed at the carotid artery and kept at fixed distance of 6 cm for 60 minutes. Flow will be measured during these 60 minutes and for further 60 minutes (max time elapse 120 minutes) or until occlusion occurs. Occlusion is considered as a constant (≥1 min) flow below 0.1 ml/min. Mice are euthanized by an overdose of pentobarbital immediately (25mg) after the occlusion analysis is completed.

Mice will be placed on a heating pad, to avoid a drop of body temperature. Heart rate (probe measuring the blood flow will also measure the heart rate) will be monitored during the surgery. Anethesia depth will be checked before starting the surgery and during the experiment by pedal withdrawal reflex (animals hind limb will be extended and the interdigital webbing of the foot will firmly pinched by the use of an atraumatic forceps; if there is no withdrawal reaction to the toe pinch, animals will be judged deep enough).

The chosen dose of anesthesia sodium pentobarbital (87 mg/kg body weight) is sufficient to keep the animal in deep anesthesia for the whole duration of the experiment. No second dosing is necessary. 20mg/kg of NI-206.82C2 in Phosphate buffered saline (pH 7) vehicle is anticipated to saturate the mouse and negate any effects of pharmacokinetics (Tabrizi et al., Development of Antibody-Based Therapeutics, Chapter 8, 218-219). Phosphate buffered saline (pH 7) alone is administered as the vehicle only control. The results are shown in Figure 21. Indeed, experiments performed in accordance with the present invention demonstrate that anti-FAP antibody NI-206.82C2 reduces thrombosis in mice in a dose-dependent manner as evidenced by prolonging photochemical injury induced arterial occlusion times versus a control antibody 43A11 (biologically inactive isotype-matched control antibody) in living mice. Antibody NI-206.82C2 exhibits a dose-dependent increase in the median time to occlusion in mice with a significant increase starting at a dose of 2 mg/kg and further increase over 7 mg/kg and 20 mg/kg versus PBS and antibody 43A11 at a constant dose of 20 mg/kg as a control; see Figure 23.

**Example 17: Anti-FAP antibody NI-206.82C2 is capable of abrogating orthotopic tumor growth in a syngeneic colorectal cancer mouse model**

CJ57/BL6 mice were anesthetized by isofluorane inhalation, and the hepatic portal vein was accessed by median laparotomy (from xiphoid 4cm caudally). Mice were injected with 1 million murine MC38 colorectal cancer cells. The origin of these cell is described in Science 19 September 1986. MC-38 tumors were allowed to form for 7 days before treatment. Mice were treated with either PBS or NI-206.82C2 (20mg/kg by intraperitoneal injection) every 72 hours for 5 treatment cycles, before being anesthetized by isofluourane inhalation. Small animal MRI imaging was performed using a Bruker 4.7 Tesla MRI to aquire images of liver metastases. Tumor images were analyzed on Myrian Software (Intrasense) to quantify tumor metastases and cumulative tumor diameter overall tumor burden. The results are shown in Figure 22.

**Example 18: NI-206.82C2, NI-206.59B4, NI-206.27E8, NI-206.12G4, NI-206.18H2 and NI-206.6D3 binding to transmembrane FAP is pH dependent**

To evaluate the binding of NI-206.82C2, NI-206.59B4, NI-206.27E8, NI-206.12G4, NI-206.18H2 and NI-206.6D3 to transmembrane human FAP, an FAP expressing HEK293 cell line was received from the National Institutes of Health, Bethesda, MD. The generation of this FAP expressing HEK293 cells is described in: J. Exp. Med. 2013 Vol. 210 No. 6 1125-1135.

HEK293 cells were transduced with retrovirus encoding full length human FAP cDNA. Cloning was performed using Fast Cloning Pack and FastDigest restriction enzymes (both from Fermentas). Transient retroviral supernatants were generated by transfecting 293GP cells with the FAP plasmid using Lipofectamine 2000 (Invitrogen). Retroviral supernatants were collected at 48 h after transfection and centrifuged onto Retronectin-coated (10 μg/ml; Takara), non-tissue culture-treated 6-well plates at 2,000 G for 2 h at 32°C. These retroviral supernatants where then used to transduce HEK293 cells overnight. Transduced FAP-HEK293 cells were selected with 1 mg/ml G418 (CellGro).

To generate fluorescently labelled antibodies for fluorescence active flow cytometry, NI-206.82C2, NI-206.59B4, NI-206.27E8, NI-206.12G4, NI-206.18H2 and NI-206.6D3 as well as isotype matched biologically inactive control antibody 43A11 were labelled with a Cyanine Dye 5 dye (Cy5) using a Lightning-Link Cy5 Antibody Labeling Kit (Novus Biologicals) according to the manufacturer's instructions.

500'000 FAP-HEK293 cells were incubated for 1hr at 4°C either with Cy5 labelled NI-206.82C2, NI-206.59B4, NI-206.27E8, NI-206.12G4, NI-206.18H2 or NI-206.6D3 or with Cy5 labelled 43A11 at four different antibody concentrations (0.1, 1, 10, and 100 nM) in seven different pH-adjusted PBS buffers (pH 7.4, 6.8, 6.6, 6.5, 6.4, 6.3 and 6.2). PBS buffers were adjusted to using MES monohydrate (Sigma Aldrich). Following incubation, cells were washed 3 times with 200uL in matched pH-adjusted PBS, spun at 400G for 4min, and resuspended in 200μL of pH adjusted buffer.

To analyze antibody binding, fluorescent activated flow cytometry was performed using a BD Fortesa device for forward scatter, side scatter, and the mean fluorescent intensity (MFI) of the Cy5 channel was recorded using a 633nM laser excitation and 600/20 filter. Viable cells were gated by forward and side scatter, and singlets/doublet exclusion, and the MFI for Cy5 was quantified using FlowJo software Version 10.1. The MFI signal for Cy5 labelled 43A11 was subtracted from Cy5 labelled 82C2, 59B4, 27E8, 12G4, 18H2 and 6D3 to calculate ΔMFI at pH 7.4, 6.8, 6.6, 6.5, 6.4, 6.3 and 6.2 revealing increased paratope-specific 82C2, 59B4, 27E8, 18H2 and 6D3 avidity under acidic conditions vs. at pH 7.4 (Figure 24A, B, C, E and F) and decreases paratops-specific 12D3 avidity under acidic conditions vs. at pH 7.4 (Figure 24D).

**Example 19: NI-206.82C2 Tumor engagement in-vivo**

Anti-FAP antibody NI-206.82C2, and a biologically inactive isotype matched control antibody 43A11 were fluorescently labeled with Alexa 680 dye using Alexa Fluor 680 Antibody Labeling Kit (Thermo Fischer A20188) according to the manufacturer's instructions. These antibodies are designated as A680-82C2 (Alexa 680 labelled 82C2) and A680-43A11 (Alexa 680 labelled 43A11).

A murine cancer model was generated by the injection of 100'000 4T1 cultured breast tumor cells orthotopically into the 2nd left breast of Balb/c immunocompetent mice and allowed to grow for 7 days. Prior to in vivo imaging, the animals were shaved and de-epilated to remove fur for minimal absorbance and scattering of the incident optical light. In-vivo imaging was performed with the Maestro 500 imaging system (Cambridge Research Inc, Woburn, USA). For A680-82C2 detection, a band pass filter from 615 nm - 665 nm and a highpass filter over 700 nm were used for excitation and emission light respectively, and fluorescence was detected by a CCD camera (cooled to 11 °C). A series of images were acquired at different wavelengths and then subjected to spectral unmixing (deconvolution of collected optical spectra; this enabled the unmixing of the Alexa680 fluorescence pattern from tissue auto-fluorescence and other spectral contributions).

The 4T1-breast tumor bearing Balb/c mice (3-4 in each group) were then injected on day 7 after innoculation with 2mg/kg of A680-82C2 or A680-43A11. Whole mice images were acquired at the following timepoints: before antibody injection, immediately after antibody injection, 6h, 24h, 48h, and 6d after antibody injection. The intensity data were then normalized to auto-fluorescence and compared between the two groups and it was found that the antibody concentration peaks in the animals from 6h to 48h post antibody injection and that A680-82C2 antibody has significantly higher tumor uptake than the control antibody A680-43A11. The results are shown in Figure 25.

**Example 20: 82C2-41BB-CDζ (anti-FAP) chimeric antigen receptor (CAR) construction**

A chimeric antigen receptor (CAR) with the FAP-binding 82C2-derived ScFv and the 4-1BB costimulatory domain as well as the CD3ζ endodomain is constructed (82C2-41BB-CDζ). The variable heavy domain (VH) of 82C2 is fused with a linker sequence, using PCR-driven overlap extension, and cloned into the Sfi1 and Pme1 sites of the pUKBK vector (Heckman and Pease, Nat. Protoc. 2 (2007), 924-923; Kohli et al., Proteome Res. 11 (2012), 4075-4090). Using PCR-based cloning, the variable light domain (VL) of 82C2 is inserted into the Pme1 site after the linker, giving rise to pUKBK-FAP-ScFv. The Pme1 site at the 3'-end of the VL is preserved by using a reverse primer with 5'-ACCC extension. T2A-eGFP is amplified from Addgene plasmid #69536 by PCR and cloned into the Pm1 site of this vector. The Pme1 site at the 3'-end of the VL is preserved by using a forward primer with 5'-GTTT extension. The sequence of the CD8hinge-41BB-CD3ζ is obtained from US patent US 8,906,682 B2, synthesized by Thermo Fischer, and cloned into the Pme1 site of this vector. The Pme1 site at the 3'-end of the VL is preserved by using a forward primer with 5'-GTTT extension. The thus obtained construct, 82C2-CD8hinge-41BB-CD3ζ fused in frame to T2a and eGFP, is transferred to the pRRL lenti virus backbone (Addgene plasmid 74922), and subsequently named 82C2-41BB-CDζ.

For control experiments, a second CAR is constructed by cloning the ScFv of the FAP-binding antibody Sibrotuzumab (Sibro-41BB-CDζ). Sibrotuzumab VH and VL sequence is obtained from the US Patent Application US 2003/0103968 A1, synthesized with interconnecting linker (Thermo Fischer), and cloned into Sfi1 and Pme1 of pUKBK. The Sibrotuzumab-derived ScFV is cloned to the lenti viral plasmid 82C2-41BB-CDζ using Sfl and Pme1 sites, giving thus rise to Sibro-41BB-CDζ.

For control experiments, a third CAR with the 82C2-derived ScFv and a truncated endodomain is prepared (82C2-Δ). The sequence of the CD8hinge is obtained from US patent US 8,906,682 B2, is synthesized by Thermo Fischer, and cloned into the Pme1 of PUKBK.FAP-ScFV-T2A-eGFP. The thus obtained construct is transferred to the pRRL lenti virus backbone, and subsequently named 82C2-Δ.

Lenti viral plasmids used for CAR expression with EF1α promoter are chosen because of their high and prolonged expression in T cells compared to other routinely used promoters, as reported previously; see, Milone et al., Mol. Ther. 17 (2009), 1453-1464. For simple evaluation of CAR expression efficiency the bicistronic expression of eGFP is chosen.

All constructs are sequenced using Microsynth sequencing service.

**Example 21: 82C2-41BB-CDζ lenti virus and 82C2-CAR T-cell production**

Delivery of CARs to human T cells by lenti virus is an efficient and clinically validated procedure; see, Dotti et al., Immunol. Rev. 257 (2014), 107-126. 82C2-41BB-CDζ CAR lenti virus plasmid or control plasmid are used to produce CAR lenti virus. HEK293T cells are purchased from DSMZ (Deutsche Sammlung von Mikroorganismen und Zellkulturen) and cultivated in DMEM (Invitrogen), supplemented with 10 % FCS (Gibco) at 37°C and 5% $CO_2$. For maintenance, confluent cells are washed with PBS (Gibco), dissociated with trypsin/EDTA (Gibco), and plated at a 1:10 dilution in fresh media. HEK293 are transfected with virus and helper plasmid using 25 kDA polyethylenimine (PEI; Polysciences). 48 hours after transfection virus-containing supernatants are transferred to Falcon tubes and centrifuged for 15 min at 500 rcf to remove cellular debris. Virus-containing medium is aliquoted and stored at -80°C.

Primary T-cells are transduced with 82C2-41BB-CDζ lenti virus to produce 82C2-CAR Ts. Blood from healthy volunteer donors is obtained according to the respective regulations. T-cells are extracted from PBMC by negative selection using a pan T Cell isolation kit (Miltenyi Biotec), according to the manufacturer's instructions and T-cells proliferated as described previously; see, Carpenito et al., Proc. Natl. Acad. Sci. U S A 106 (2009), 3360-3365. Primary T-cells are transduced with 82C2-41BB-CDζ expressing lenti virus 24 hours after activation. eGFP expression is analyzed by flow cytometry approximately 24 hours after transduction. Transduction efficiency is expected to reach more than 75 %, as shown previously (Carpenito et al., Proc. Natl. Acad. Sci. U S A 106 (2009), 3360-3365). These cells are labeled hereinafter 82C2-CAR Ts.

Control CAR T cells are generated by transduction of primary T cells with Sibro-41BB-CDζ or 82C2-Δ and are expected to have similar transduction efficiency. These cells are labeled hereinafter Sibro-CAR Ts and 82C2-Δ-CAR Ts, respectively.

### Example 22: Determination of 82C2-CAR Ts specific binding to coated recombinant FAP and release of effector cytokines

To determine the effector function of 82C2-CAR Ts, cytokine release upon 82C2-CAR Ts' target engagement is measured. Release of cytokines upon binding of CAR-Ts to target epitopes, which is leading to damage of cancer cells and its environment, is one mechanism of anti-tumor effects of CAR T cells.

96-well flat bottom plates are coated with mouse anti-His antibody (Clonetech) overnight. Recombinant human FAP with 6xHis N-terminal purification tag is purchased from Sino Biologics, applied to antibody coated plates for 4 hours diluted at 1 μg/ml in PBS, and plates washed three times with PBS. 10,000 82C2-CAR Ts are plated per well on coated 96-well plates and incubated for 2 hours at 37°C. Cell medium supernatants are harvested and transferred to ELISA plates. IFNγ and TNFα ELISA kits are purchased from Thermo Scientific and used according to the manufacturer's instructions. Samples are measured on a Tecan absorbance reader. It is expected that stimulation with recombinant human FAP results in strong production of IFNγ and TNFα in 82C2-CAR Ts, as demonstrated previously; see, Tran et al., J. Exp. Med. 210 (2013), 1125-1135.

To show that cytokines are released specifically in response to 82C2-CAR T interaction with human FAP, 82C2-CAR T are plated on 96-well flat bottom plates that are not coated with FAP. It is expected that cultivation of 82C2-CAR Ts in absence of human FAP does not lead to cytokine production, *i.e.*, that cytokine production is specifically released after interaction with FAP.

To show that effector endodomain function is necessary for 82C2-CAR Ts cytokine release, 82C2-Δ-CAR Ts are plated on recombinant FAP-coated plates as described above. It is expected that 82C2- Δ-CAR Ts do not show significant production of cytokines.

### Example 23: Determination of 82C2-CAR Ts binding to human FAP-expressing cells and release of cytokines under normal pH conditions

To obtain a human FAP-expressing plasmid, PCR is done on a human FAP encoding cDNA. The PCR product is cloned between the Asc1 and Sfi1 sites of the pUKBK-C vector, thus allowing the expression of FAP from a CMV promoter and with a C-terminal HA tag (Kohli et al., Proteome Res. 11 (2012), 4075-4090). The correctness of the FAP-HA construct is verified using Microsynth sequencing service.

It was previously shown, using RT-PCR and flow cytometry, that the human lung cancer cell line A549 does not express FAP; see, Kakarla et al., Mol Ther 21 (2013), 1611-1620. A549 are purchased from ATCC and maintained in F-12K medium according to the manufacturer's instructions. 200*$10^3$ are plated per well in 24-well cell culture dishes and transfected the next day using 1 μg FAP-HA DNA and lipofectamin 2000 (Thermo Fischer) according to the manufacturer's instructions. 1 μg/ml G418 selective antibiotic (Invitrogen) is supplemented to cell culture medium one day after lipofection for selection of A549 cells transfected with the FAP plasmid. After approximately two week of maintenance in selection media, a mixed stable cell line expressing FAP is obtained, from hereinafter labeled A549-FAP. To verify stable expression of human FAP in this cell line, immunocytochemical staining with anti-HA antibody is done. 80*103 A549-FAP cells per well are plated on 4-well glass chamber slides (LabTek). 24 hours after plating, cells are fixed with 4 % paraformaldehyde (Sigma-Aldrich), incubated with anti-HA antibody (1:100; Roche) for 2 hours, incubated with secondary Cy3 anti-rat (1:250; Jackson Immuno Research), and mounted using Mowiol (Sigma). Cells are imaged using a Leica SP8 confocal microscope as described previously (Gersbacher et al., PLoS One 8 /2013), e69363). Due to the usage of the CMV promoter a strong FAP expression (*i.e.*, fluorescence signal) is expected in A549-FAP cells. Due to the selection procedure, it is expected that FAP expression (*i.e.*, fluorescence signal) is observed in more than 90 % of A549-FAP cells. The A549 parental cell line serves as negative for HA staining, which are expected to show no significant fluorescence signal.

To assess the effector functions of 82C2-CAR Ts, co-culture with A549-HA cells is prepared and cytokine secretion measured. 3,000 A549-FAP cells are plated per well in round-bottom 96-well plate in 50 μl medium. 50 μl medium containing 82C2-CAR Ts at different densities are then added to labeled A549-FAP cells to obtain 82C2-C Ts to A549-FAP cell ratios of 1:1, 10:1, 20:1, 30:1, 40:1, and 50:1. After 4 hours medium is transferred to an ELISA plates and production of IFN-γ and TNF-α measured as described previously. It is expected that co-culture of 82C2-CAR Ts with A549-FAP cells results in no (*i.e.*, none significant) production of the two measured cytokines. Additionally, it is expected that ratio of effector (82C2-CAR Ts) and target (A549-FAP) does not have an influence on the cytokine production.

In contrast, co-culture of Sibro-CAR Ts with A549-FAP cells, as described above, is expected to result in significant production of cytokines in a dose-dependent manner. Co-culture of Sibro-CAR Ts with naive A549 cells is expected to show no significant production of cytokines.

**Example 24: Determination of 82C2-CAR Ts binding to human FAP expressing cells and release of cytokines under low pH conditions**

The tumor microenvironment is documented to have an acidic environment due to the Warburg effect. The anticipated advantage of the 82C2 variable domains, compared to other documented anti FAP antibody variable domains, is selective avidity to membrane associated FAP under low pH, versus relatively lower avidity to membrane associated FAP under neutral pH.

To test a pH dependent binding of 82C2-CAR Ts, CAR Ts are co-cultured with A549-FAP cells at pH 6.3. 3,000 A549-FAP cells per well are plated in round-bottom 96-well plate in 50 μl medium (pH 6.3) together with 50 μl medium (pH 6.3) containing 82C2-CAR Ts in different densities to obtain 82C2-C Ts to A549-FAP cell ratios of 1:1, 10:1, 20:1, 30:1, 40:1, and 50:1. Supernatants are transferred to ELISA plates and cytokine production measured as described above. It is expected that co-culture of 82C2-CAR Ts with A549-FAP cells results in strong production of cytokines and that the production is dose-dependent.

To show that cytokines are released specifically in response to 82C2-CAR T interaction with human FAP, 82C2-CAR Ts are co-cultured with naive A549 cells under low pH (pH 6.3) at the previously used effector-to-target cell ratios. It is expected that co-culture of 82C2-CAR Ts and naive A549 cells does not lead to a significant production of cytokines at any given effector-to-target cell ratio.

To show that effector endodomain function is necessary for the cytokine production of 82C2-CAR Ts, 82C2-Δ-CAR Ts are co-cultured with A549-FAP cells under low pH (pH 6.3) at the previously used effector-to-target cell ratios. It is expected that co-culture of 82C2-Δ-CAR Ts and A549-FAP cells does not lead to a significant production of cytokines at any given effector-to-target cell ratio.

To monitor the anticipated advantage of 82C2-derived CAR Ts over other anti-FAP CAR Ts - which is its selective avidity to membrane associated FAP under low pH, versus relatively lower avidity to membrane associated FAP under neutral pH - a comparison of effector cytokine release of 82C2-CAR Ts and Sibro-CAR Ts at various pH conditions is done (pH range from 6.3 to 7.4 in 2.875 steps). A549-FAP cells are co-cultured with either 82C2-CAR Ts or Sibro-CAR Ts at an effector-to-target cell ratio of 20:1, as described above. It is expected that the lysis efficiency of Sibro-CAR Ts will be independent of the pH. In contrast, 82C2-CAR Ts are expected to have low (or non-significant) lysis efficiency at pH 7.4, but lysis efficiency is expected to increase with decreasing pH.

**Example 25: Determination of 82C2-CAR Ts specific cytolytic activity towards human FAP-expressing cells under different pH conditions**

To determine the cytolytic activity of 82C2-CAR Ts we use a standard $^{51}$Cr (Chromium) release assay. To label A549-FAP cells, 2*10$^6$ cells are washed and incubated with 20 μl of Chromium-51 (5 mCi/ml; Perkin Elmer) for one hour at 37°C. Cells are then washed twice with RPMI medium (Life Technologies) and 3,000 cells plated per well in round-bottom 96-well plate in 50 μl medium with pH 6.3. 50 μl medium (RPMI, pH 6.3) containing 82C2-CAR Ts in different densities are then added to labeled A549-FAP cells to obtain 82C2-C T to A549-FAP cell ratios of 1:1, 10:1, 20:1, 30:1, 40:1, and 50:1. Co-cultures are incubated for 4 hours at 37°C, supernatants harvested, and $^{51}$Cr release measured on a γ-particle counter. Lysis in % is calculated according the following formula:

$$((\text{Sample}^{51}\text{Cr release}) - (\text{Spontaneous}^{51}\text{Cr release})) / ((\text{Maximum}^{51}\text{Cr release}) - (\text{Spontaneous}^{51}\text{Cr release}))$$

Under the described low pH conditions (pH 6.3), a dose-dependent and efficient lysis of A549-FAP by 82C2-CAR Ts is expected.

To show that cytolytic activity of 82C2-CAR Ts is dependent on specific interaction with human FAP, naïve A549 are labeled with Chromium-51 and co-cultured with 82C2-CAR Ts as described above. It is expected that 82C2-CAR Ts do not lead to a significant cell lysis of naïve A549 cells. To show that effector endodomain function is necessary for the cytolytic function of 82C2-CAR Ts, 82C2-Δ-CAR Ts are co-cultured with Cromium-51-labeled A549-FAP cells under low pH (pH 6.3) as described above. It is expected that 82C2-Δ-CAR Ts do not lead to a significant cell lysis of A549-FAP cells.

To monitor the anticipated advantage of 82C2-derived CAR Ts over other anti-FAP CAR Ts - which is its selective avidity to membrane associated FAP under low pH, versus relatively lower avidity to membrane associated FAP under neutral pH - a comparison of cytolysis function of 82C2-CAR Ts and Sibro-CAR Ts at various pH conditions is done (pH range from 6.3 to 7.4 in 2.875 steps). A549-FAP cells are labeled with Chromium-51 and co-cultured with either 82C2-CAR Ts or Sibro-CAR Ts as described above. It is expected that the lysis efficiency of Sibro-CAR Ts will be independent of the pH. In contrast, 82C2-CAR Ts are expected to have low (or non-significant) lysis efficiency at pH 7.4, but lysis efficiency is expected to increase with decreasing pH.

## Claims

1. A monoclonal human B cell-derived anti-Fibroblast Activation Protein (FAP) antibody, wherein at least one of the complementarity determining regions (CDRs) and/or variable heavy ($V_H$) and/or variable light ($V_L$) chain of the antibody are encoded by a cDNA derived from an mRNA obtained from a human memory B cell which produced an anti-FAP antibody, preferably wherein the antibody is capable of binding to captured or directly coated human FAP with an EC50 of $\leq$ 0.1 $\mu$M.

2. The antibody of claim 1 or a biotechnological or synthetic derivative thereof, which is capable of binding a FAP epitope in a peptide of 15 amino acids in length, which epitope comprises or consists of the amino acid sequence NI-206-18H2 (501-IQLPKEEIKKL-511) (SEQ ID NO: 60); NI-206.82C2 (521-KMILPPQFDRSKKYP-535 (SEQ ID NO: 30); 525-PPQFDRSKKYPLLIQ-539 (SEQ ID NO: 31); 525-PPQFDRSKKYP-535 (SEQ ID NO: 32); and/or 113-YLESDYSKLWR-123 (SEQ ID NO: 61)); NI-206.59B4 (53-SYKTFFP-59 (SEQ ID NO: 33)); NI-206.22F7 (381-KDTVENAIQIT-391 (SEQ ID NO: 34)); NI-206.27E8 (169-NIYLKQR-175 (SEQ ID NO: 35)); NI-206.12G4 (481-TDQEIKILEENKELE-495 (SEQ ID NO: 36)); or NI-206.17A6 (77-VLYNIETGQSY-87 (SEQ ID NO: 37)).

3. The antibody of claim 1 or 2 or a biotechnological or synthetic derivative thereof comprising in its variable region or binding domain

   (a) at least one CDR of the $V_H$ and/or $V_L$ chain amino acid sequence depicted in any one of Figs. 1G-1K and 1A-1F;

(b) an amino acid sequence of the $V_H$ and/or $V_L$ chain amino acid sequence as depicted in Figs. 1G-1K and 1A-1F;
(c) at least one CDR consisting of an amino acid sequence resulted from a partial alteration of any one of the amino acid sequences of (a); or
(d) a $V_H$ and/or $V_L$ chain comprising an amino acid sequence resulted from a partial alteration of the amino acid sequence of (b);

preferably wherein the number of alteration in the amino acid sequence is below 50%.

**4.** The antibody of any one of claims 1 to 3 or a biotechnological or synthetic derivative thereof, which is capable of binding to transmembrane FAP.

**5.** The antibody of any one of claims 1 to 4 or a biotechnological or synthetic derivative thereof, which shows a higher avidity of binding to FAP under acidic pH as compared to neutral or physiological pH, preferably wherein the acidic pH is 6.4 or 6.8 and the physiological pH is 7.4.

**6.** The antibody of any one of claims 1 to 5 or a biotechnological or synthetic derivative thereof comprising in its variable region or binding domain

(a) at least one CDR of the $V_H$ and/or $V_L$ chain amino acid sequence depicted in any one of Fig. 1A, 1B, 1D, 1G, 1I;
(b) an amino acid sequence of the $V_H$ and/or $V_L$ chain amino acid sequence as depicted in Fig. 1A, 1B, 1D, 1G, 1I;
(c) at least one CDR consisting of an amino acid sequence resulted from a partial alteration of any one of the amino acid sequences of (a); or
(d) a $V_H$ and/or $V_L$ chain comprising an amino acid sequence resulted from a partial alteration of the amino acid sequence of (b);

preferably wherein the antibody is capable of binding a FAP epitope in a peptide of 15 amino acids in length, which epitope comprises or consists of the amino acid sequence of any one of SEQ ID NOS: 30 to 33, 35,60 and 61.

**7.** The antibody of any one of claims 1 to 6, wherein the antibody comprises a human constant region and/or an Fc region or a region equivalent to the Fc region of an immunoglobulin, preferably wherein the Fc region is an IgG Fc region, preferably wherein the antibody is a full-length IgG class antibody.

**8.** The antibody of any one of claims 1 to 7, wherein the antibody comprises a glyco-engineered Fc region and has an increased proportion of non-fucosylated oligosaccharides in the Fc region, as compared to a non-glyco-engineered antibody.

**9.** The antibody of any one of claims 1 to 8, wherein the antibody binds to FAP and at least one further epitope and/or target, preferably wherein the epitope is bispecific or multivalent; and wherein the antibody is a bispecific antibody, a trifunctional antibody, a tetrabody, a bivalent single chain fragment (ScFv), a bispecific T-cell engager (BiTE), a bispecific killer cell engager (BiKE), a dual affinity retargeting molecule (DART) or a DuoBody, preferably wherein the antibody is a bispecific antibody that binds in addition to FAP to death receptor 5 (DR5) and/or CD3.

**10.** A chimeric antigen receptor (CAR), wherein the antigen is fibroblast activation protein (FAP) and a first receptor comprises a variable region or binding domain derived from the anti-FAP antibody of any one of claims 1 to 9.

**11.** The CAR of claim 10, wherein the CAR or a cell expressing the CAR exhibits a higher avidity of binding to FAP or a cell expressing FAP on its cell surface under acidic pH as compared to neutral or physiological pH, preferably wherein the acidic pH is 6.4 or 6.8 and the physiological pH is 7.4.

**12.** The CAR of claim 10 or 11, wherein said first receptor or a second receptor comprises at least one further antigen binding domain, preferably wherein said further antigen binding domain is specific for a CD3 receptor or a death receptor, preferably wherein the death receptor is the death receptor 5 (DR5).

**13.** The CAR of any one of claims 10 to 12, comprising at an extracellular domain (receptor), a transmembrane domain and an intracellular signaling domain (endo- or cytoplasmic domain), preferably wherein the intracellular signaling domain comprises the 4-1BB costimulatory domain and/or CD28 costimulatory domains and/or the CD3ζ (zeta) endodomain.

**14.** A host cell genetically modified to express the CAR of any one claims 10 to 13, wherein the host cell optionally expresses and secretes the antibody of any one of claims 1 to 10, preferably wherein the host cell is a T cell, a cytotoxic T lymphocyte (CTL), a natural killer cell, a hematopoietic stem cell (HSC), an embryonic stem cell, or a pluripotent stem cell, preferably wherein the cell is a T cell (CAR T cell), preferably wherein the cell exhibits a higher avidity of binding to FAP or cell expressing FAP on its cell surface under acidic pH as compared to neutral or physiological pH, preferably wherein the acidic pH is 6.4 or 6.8 and the physiological pH is 7.4.

**15.** A pharmaceutical or diagnostic composition useful in the diagnosis or treatment of a disease associated with FAP comprising the antibody of any one of claims 1 to 9 or the biotechnological or synthetic derivative thereof, the CAR of any one of claims 10 to 13, or the cell of claim 14.

**A** **NI-206.82C2 VH (variable heavy chain sequence VH) (not PIMC) (SEQ ID NO: 2)**

```
FR1----------------------------CDR1---FR2-----------CDR2--------------
QVQLQESGPGLVKPSQTLSLTCAISGDSVSSNSVTWNWIRQSPSRGLEWLGRTYYRSKWYNDYAVSVKG

FR3----------------------------CDR3-----FR4--------
RITINPDTSKNQFYLQLKSVTPEDAAVYYCARDSSILYGDYWGQGTLVTVSS
```

**NI-206.82C2 VH (variable heavy chain sequence VH) (PIMC) (SEQ ID NO: 4)**

```
FR1----------------------------CDR1---FR2-----------CDR2--------------
QVQLQQSGPGLVKPSQTLSLTCAISGDSVSSNSVTWNWIRQSPSRGLEWLGRTYYRSKWYNDYAVSVKG

FR3----------------------------CDR3-----FR4--------
RITINPDTSKNQFYLQLKSVTPEDAAVYYCARDSSILYGDYWGQGTLVTVSS
```

**NI-206.82C2 VL (variable lambda light chain sequence VL) (PIMC by default) (SEQ ID NO: 6)**

```
FR1--------------------CDR1----------FR2-------------CDR2---FR3-------
QAVLTQPSSLSASPGASASLTCTLPSGINVGTYRIFWFQQKPGSPPQYLLSYKSDSDNHQGSGVPSRFS

---------------------------CDR3-----FR4-------
GSKDASANAGILLISGLQSEDEADYYCMIWHSSAWVFGGGTKLTVL
```

**B** **NI-206.59B4 VH (variable heavy chain sequence VH) (PIMC) (SEQ ID NO: 8)**

```
FR1----------------------------CDR1-FR2-----------CDR2-------------FR3
QVQLVQSGAEVKKPGASVKVSCKTSGYTFTDYYIHWVRQAPGQGLEWMGWINPNRGGTNYAQKFQGRVT

------------------------------CDR3---------FR4--------
MTRDTSIATAYMELSRLRSDDTAVYYCATASLKIAAVGTFDCWGQGTLVTVSS
```

**NI-206.59B4 VL (variable lambda light chain sequence VL) (PIMC) (SEQ ID NO: 10)**

```
FR1--------------------CDR1-------FR2------------CDR2---FR3-----------
SYELTQPPSVSVSPGQTARITCSGDALSKQYAFWFQQKPGQAPILVIYQDTKRPSGIPGRFSGSSSGTT

------------------CDR3------FR4-------
VTLTISGAQADDEADYYCQSADSSGTYVFGTGTKVTVL
```

**C** **NI-206.22F7 VH (variable heavy chain sequence VH) (not PIMC) (SEQ ID NO: 12)**

```
FR1----------------------------CDR1-FR2-----------CDR2-------------F
EVQLVETGGGVVQPGRSLRLSCAASGFSFSTHGMYWVRQPPGKGLEWVAVISYDGSDKKYADSVKGR

R3------------------------------CDR3---FR4--------
FTISRDNSKNTVYLEMSSVRAEDTALYYCFCRRDAFDLWGQGTMVTVSS
```

**NI-206.22F7 VL (variable lambda light chain sequence VL) (not PIMC) (SEQ ID NO: 14)**

```
FR1--------------------CDR1-------FR2------------CDR2---FR3-----------
SYVLTQPPSVSVSPGQTARITCSGDALPKKYAYWYQQKSGQAPVLVIYEDTKRPSGIPERFSGSSSGTM

------------------CDR3-------FR4-------
ATLTISGAQVEDEADYYCYSTDSSGNYWVFGGGTEVTVL
```

**Fig. 1**

**D NI-206.27E8 VH (variable heavy chain sequence VH) (PIMC by default) (SEQ ID NO: 16)**

```
FR1---------------------------CDR1-FR2-----------CDR2---------------F
EVQLVESGGGLVEPGGSLRLSCAASGFTFSDAWMNWVRQAPGKGLEWVGRIKTKSDGGTTDYAAPVRGR

R3------------------------------CDR3--------FR4--------
FSISRDDSKNTLFLEMNSLKTEDTAIYYCFITVIVVSSESPLDHWGQGTLVTVSS
```

**NI-206.27E8 VL (variable lambda light chain sequence VL) (PIMC by default) (SEQ ID NO: 18)**

```
FR1-------------------CDR1-------FR2------------CDR2---FR3-----------
SYELTQPPSVSVSPGQTARITCSGDELPKQYAYWYQQKPGQAPVLVIYKDRQRPSGIPERFSGSSSGTT

------------------CDR3-------FR4-------
VTLTISGVQAEDEADYYCQSAYSINTYVIFGGGTKLTVL
```

**E NI-206.12G4 VH (variable heavy chain sequence VH) (not PIMC) (SEQ ID NO: 20)**

```
FR1---------------------------CDR1-FR2-----------CDR2-------------FR3
EVQLVESGGGLVKPGGSLRLSCAASGFTFSDYYMSWIRQAPGKGLEWISYISSGSSYTNYADSVKGRFT

-----------------------------CDR3------------FR4--------
ISRDNAKKSVYLEVNGLTVEDTAVYYCARVRYGDREMATIGGFDFWGQGTLVTVSS
```

**NI-206.12G4 VL (variable lambda light chain sequence VL) (PIMC by default) (SEQ ID NO: 22)**

```
FR1-------------------CDR1-------FR2------------CDR2---FR3-----------
SYELTQPPSVSVSPGQTARITCSGDALPKQYAYWYQQSPGQAPVLVIYKDSERPSGIPERFSGSSSGTT

------------------CDR3-------FR4-------
VTLTISGVQAEDEADYYCQSADSGGTSRIFGGGTKLTVL
```

**F NI-206.17A6 VH (variable heavy chain sequence VH) (PIMC by default) (SEQ ID NO: 24)**

```
FR1---------------------------CDR1-FR2-----------CDR2------------FR3-
QVQLQESGPGLVRSTETLSLTCLVSGDSINSHYWSWLRQSPGRGLEWIGYIYYTGPTNYNPSLKSRVSI

----------------------------CDR3------------FR4--------
SLGTSKDQFSLKLSSVTAADTARYYCARNKVFWRGSDFYYYMDVWGKGTTVTVSS
```

**NI-206.17A6 VK (variable kappa light chain sequence VK) (not PIMC) (SEQ ID NO: 26)**

```
FR1--------------------CDR1--------FR2------------CDR2---FR3---------
EIVLTQSPGTLSLSLGERATLSCRASQSLANNYLAWYQQKPGQAPRLLMYDASTRATGIPDRFSGSGSG

--------------------CDR3-------FR4--------
TDFTLTISRLEPEDFAVYYCQQFVTSHHMYIFGQGTKVEIK
```

## Fig. 1 (continued)

**G NI-206.18H2 VH (variable heavy chain sequence VH) (not PIMC) (SEQ ID NO: 41)**

```
FR1---------------------------CDR1-FR2------------CDR2------------FR3
QVQLQESGPRLVKPSETLSLTCTVSGYSISNGYYWGWIRQPPGQGLEWIASVWHSGNTYHNPSLKSRVT

------------------------------CDR3--------FR4--------
ILVDTLKNQFSLNLKSVTAADTALYYCARSYTRNDVGAFETWGQGTMVTVSS
```

**NI-206. 18H2 VL (variable lambda light chain sequence VL) (PIMC by default) (SEQ ID NO: 43)**

```
FR1--------------------CDR1-------FR2------------CDR2---FR3-----------
SYELTQPPSVSVSPGQTARIACSGDELPKRYAYWYQQKSGQAPVLVMYEDTKRPSGIPERLSGSSSGTV

--------------------CDR3--------FR4------
TTLTISGAQVEDEADYYCYSTAPSGNHTFVFGTGTRVTVL
```

**H NI-206.20A8 VH (variable heavy chain sequence VH) (PIMC by default) (SEQ ID NO: 45)**

```
FR1---------------------------CDR1-FR2-----------CDR2--------------
EVQLVESGGGLVQPGGSLKLSCAGSGLDLSASAVHWVRQASGKGLEWIGRIRSKPNHYATTYLASVRG

FR3-----------------------------CDR3---FR4--------
RFILSRDDSENTAYLQMNSLRTEDTAVYYCRIGILNSDHWGRGTLVTVSS
```

**NI-206.20A8 VL (variable lambda light chain sequence VL) (PIMC by default) (SEQ ID NO: 47)**

```
FR1--------------------CDR1---------FR2------------CDR2---FR3---------
QSVLTQPPSASGTPGQTVIISCSGSSSNLGRKTLNWYQQLPGAAPKLLIFKNDQRASGVPDRFSASKSG

---------------------CDR3--------FR4------
TSASLTISGLQSDDEADYYCGTWDDSLDNWVFGGGTKVTVL
```

**I NI-206.6D3 VH (variable heavy chain sequence VH) (PIMC by default) (SEQ ID NO: 49)**

```
FR1---------------------------CDR1-FR2-----------CDR2---------------
EVQLVESGGGLVKPGESLRLSCAASGFTFSKAWMNWVRQGPGKGLEWVGRVKSKTDGGTTDYAAPVKG

FR3------------------------------CDR3-------------FR4--------
RFTISRDDSKDTVFLQMNSLKTEDTAVYYCSIQFIVVGDRGRDDQYMDVWGKGTTVTVSS
```

**NI-206.6D3 VL (variable lambda light chain sequence VL) (not PIMC) (SEQ ID NO: 51)**

```
FR1--------------------CDR1-------FR2------------CDR2---FR3---------
SYVLTQPPSVSVSPGQTARITCSGDALPKQYAFWYQQKPGQAPVIMIYKDNQRPSGIPERFSGSSSGTT

------------------CDR3-------FR4-------
VTLTISGVQTEDEADYYCQSADSTNTHVVFGGGTKLTVL
```

# Fig. 1 (continued)

**J NI-206.14C5 VH (variable heavy chain sequence VH) (PIMC by default) (SEQ ID NO: 53)**

```
FR1---------------------------CDR1-FR2------------CDR2------------FR3
QVQLQESGPGLVKPSETLSLTCTVSNGSINNNYWSWLRQPPGKGLQWIGYVYYSGSAKYNPSLQSRVSL

----------------------------CDR3-----------FR4--------
SVDRSKNQFSLELSSVTAADTAVYYCARTYCSGRDTCFYFFDNWGQGTLVTVSS
```

**NI-206. 14C5 VL (variable lambda light chain sequence VL) (PIMC by default) (SEQ ID NO: 55)**

```
FR1-------------------CDR1----------FR2------------CDR2---FR3--------
QSALTQPASVSGSPRQSLTISCTGTMSDVGRYDLVSWYQQHPGKAPNVIIYAVTKRPSGVSDRFSGSKS

---------------------CDR3------FR4-------
GTTASLTISGLQAEDEAYYYCCSYATVNSWLFGGGTKVTVL
```

**K NI-206.34C11 VH (variable heavy chain sequence VH) (not PIMC) (SEQ ID NO: 57)**

```
FR1---------------------------CDR1-FR2------------CDR2-------------FR3
EVQLVETGGGLVQPGGSLRLACAASGFTFSSYAMSWVRQAPGKGLEWVSGISDSGGSTYYADAVKGRF

------------------------------CDR3----FR4--------
TISKDNSKNTLYLQMNSLRAEDTAVYYCARVDSNSMDVWGQGTTVTVSS
```

**NI-206. 34C11 VL (variable lambda light chain sequence VL) (PIMC by default) (SEQ ID NO: 59)**

```
FR1-------------------CDR1---------FR2------------CDR2---FR3---------
QSVLTQPPSLSAAPGQKVTISCSGSSSNIGNNYVSWYQQLPGTAPKLLIYNNDKRPSGISDRFSGSKSG

--------------------CDR3-------FR4-------
TSATLGITGLQTGDEADYYCGTWDRSLSGRVFGGGTKLTVL
```

**Fig. 1 (continued)**

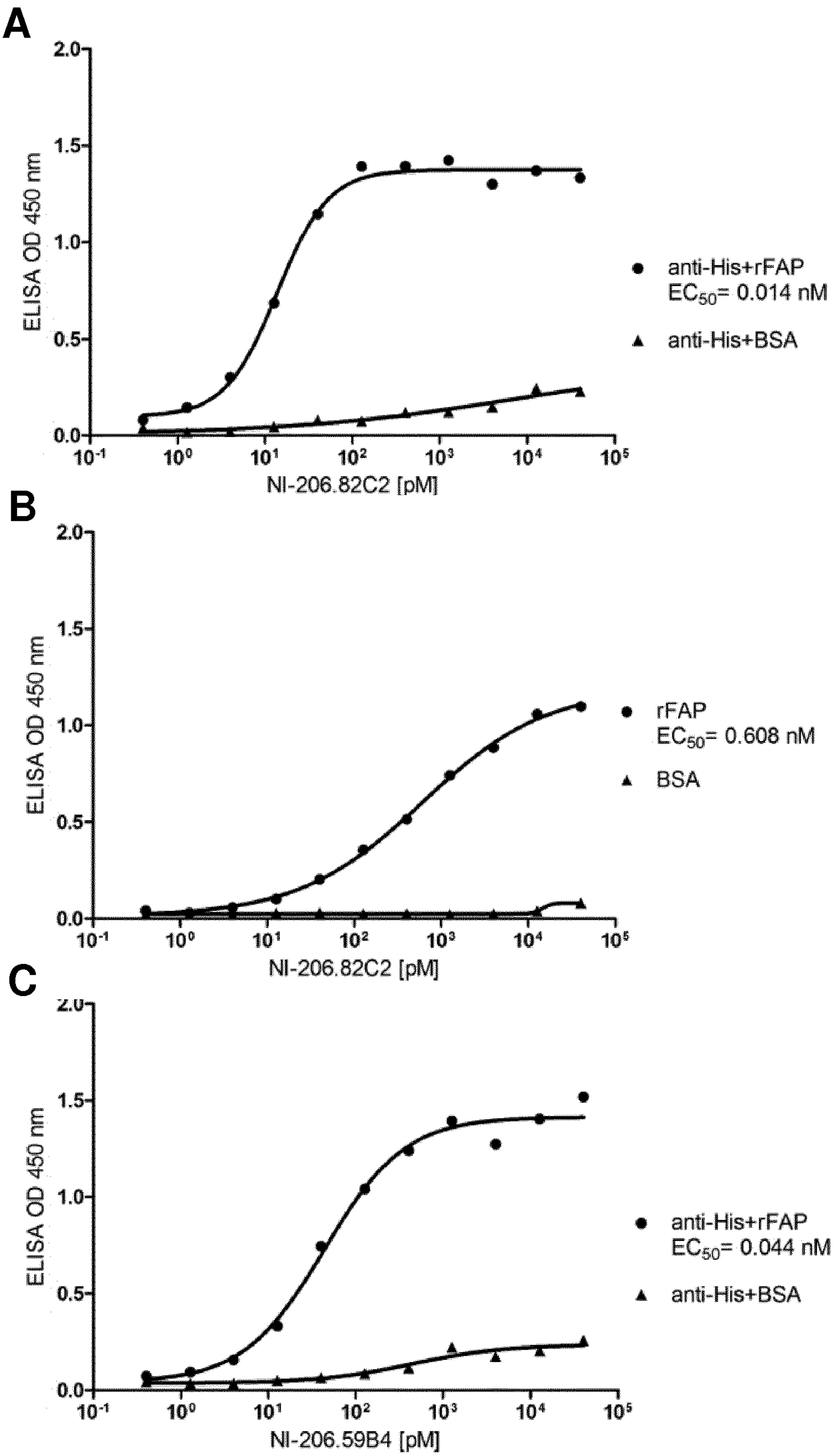
A
2.0
1.5
1.0
0.5
0.0
ELISA OD 450 nm
$10^{-1}$ $10^{0}$ $10^{1}$ $10^{2}$ $10^{3}$ $10^{4}$ $10^{5}$
NI-206.82C2 [pM]
anti-His+rFAP
$EC_{50}$= 0.014 nM
anti-His+BSA
B
2.0
1.5
1.0
0.5
0.0
ELISA OD 450 nm
$10^{-1}$ $10^{0}$ $10^{1}$ $10^{2}$ $10^{3}$ $10^{4}$ $10^{5}$
NI-206.82C2 [pM]
rFAP
$EC_{50}$= 0.608 nM
BSA
C
2.0
1.5
1.0
0.5
0.0
ELISA OD 450 nm
$10^{-1}$ $10^{0}$ $10^{1}$ $10^{2}$ $10^{3}$ $10^{4}$ $10^{5}$
NI-206.59B4 [pM]
anti-His+rFAP
$EC_{50}$= 0.044 nM
anti-His+BSA

**Fig. 2**

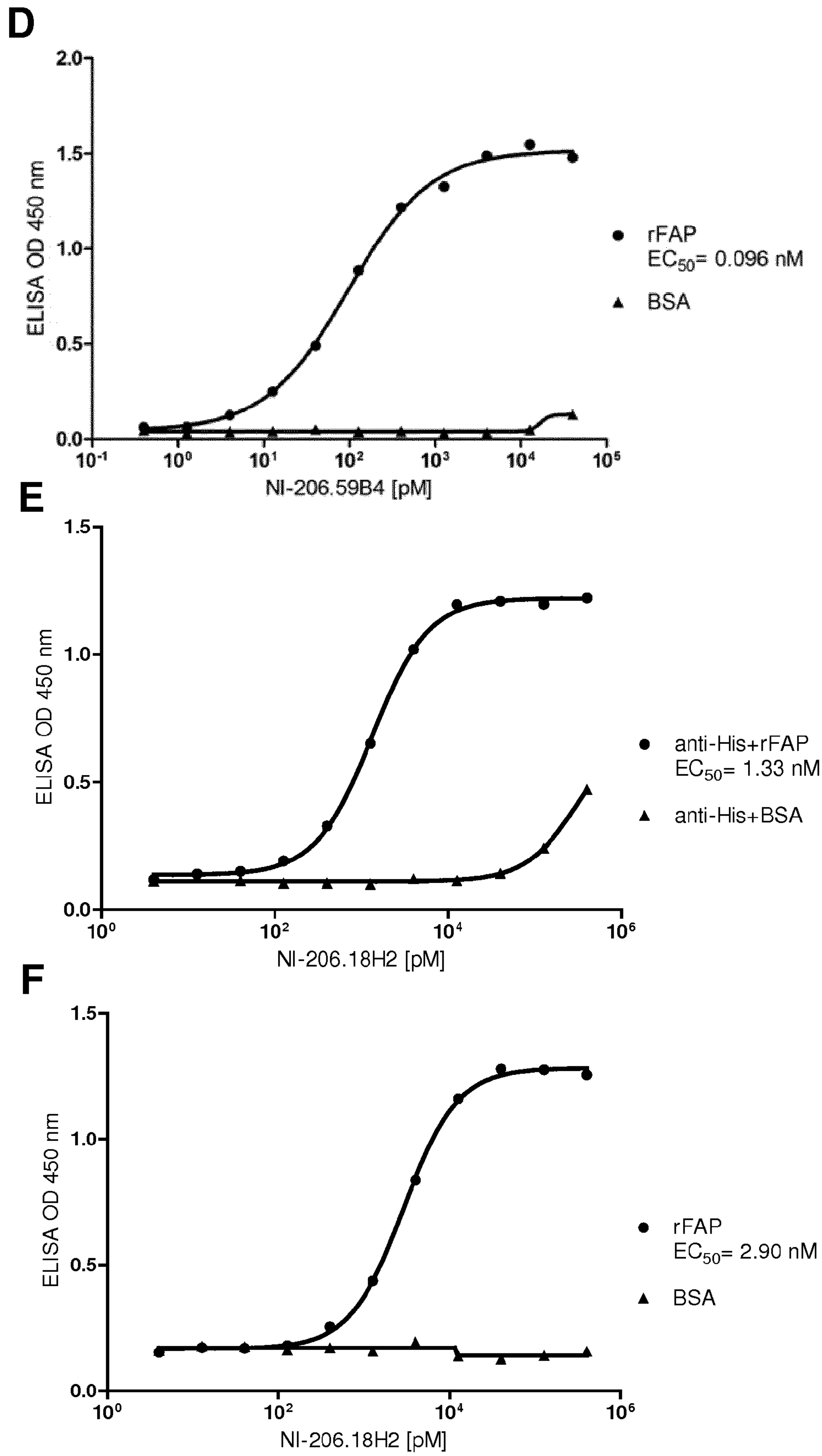


Fig. 2 (continued)

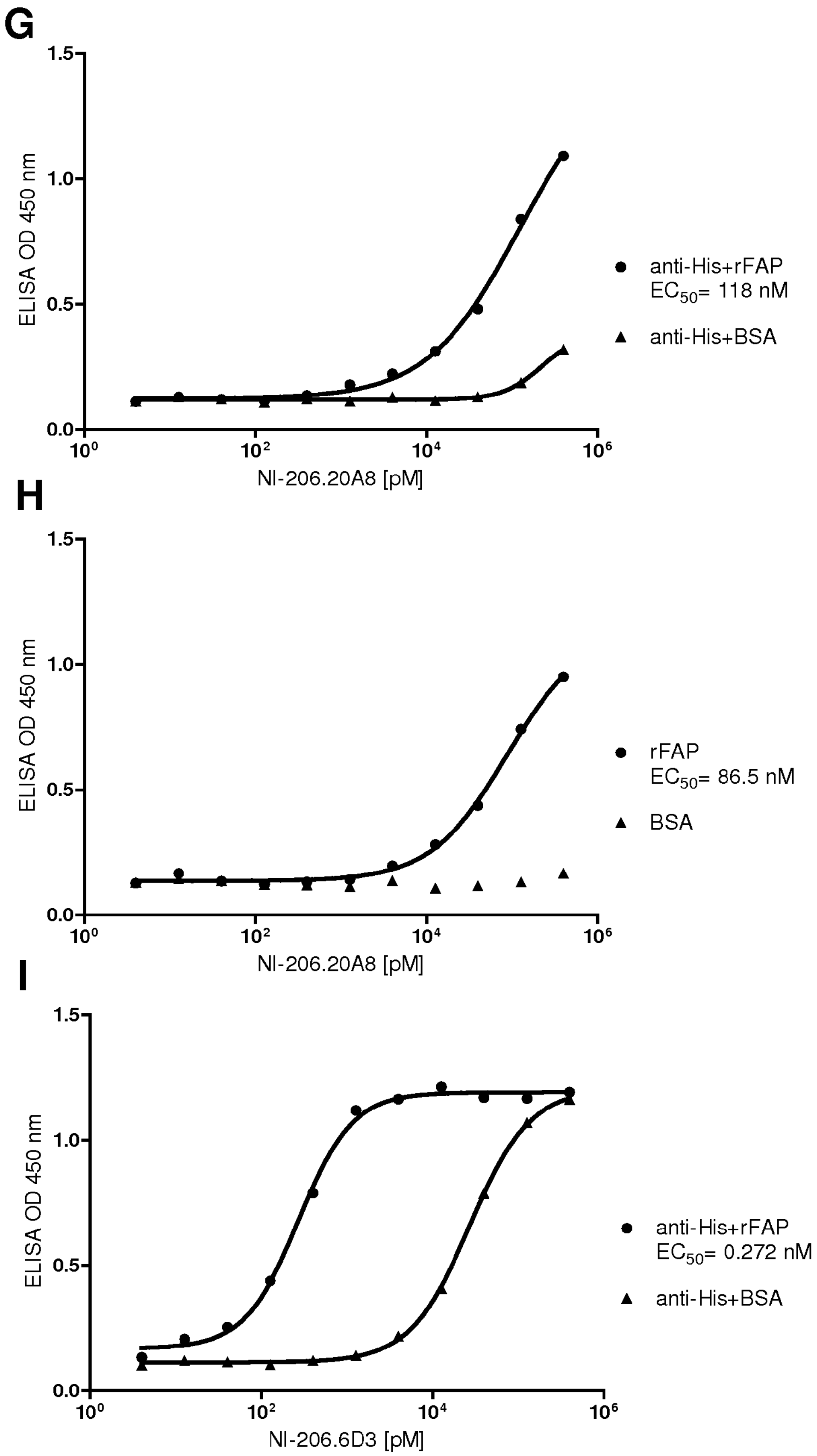


**Fig. 2 (continued)**

J

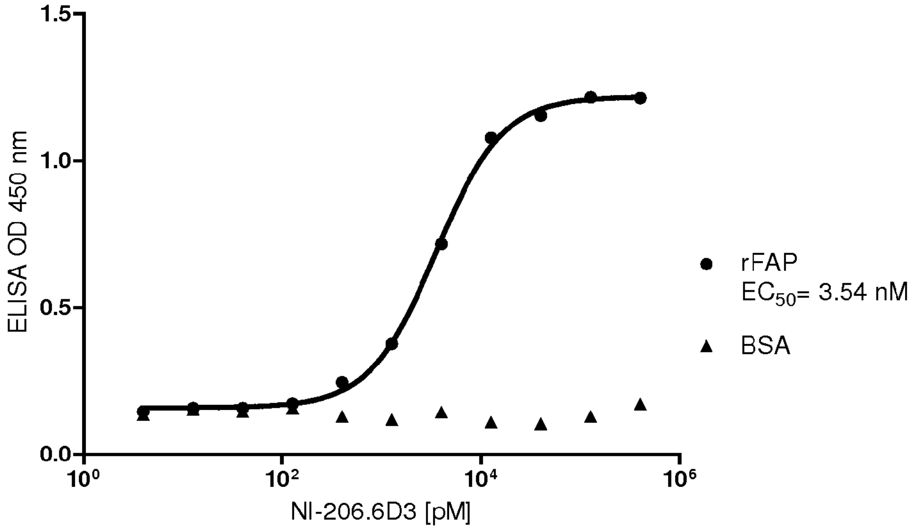


K

| Antibody | sFAP $EC_{50}$ (nM) | FAP $EC_{50}$ (nM) | cFAP $EC_{50}$ (nM) |
|---|---|---|---|
| NI-206.82C2 | 0.014 | 0.61 | N/A |
| NI-206.59B4 | 0.044 | 0.096 | N/A |
| NI-206.22F7 | N/A | N/A | 0.12 |
| NI-206.27E8 | 3.36 | 0.33 | N/A |
| NI-206.12G4 | N/T | 1.4 | N/A |
| NI-206.17A6 | 50.2 | 10.5 | N/A |
| NI-206.18H2 | 0.272 | 3.54 | N/A |
| NI-206.20A8 | 1.33 | 2.90 | N/A |
| NI-206.6D3 | 118 | 86.5 | N/A |

Fig. 2 (continued)

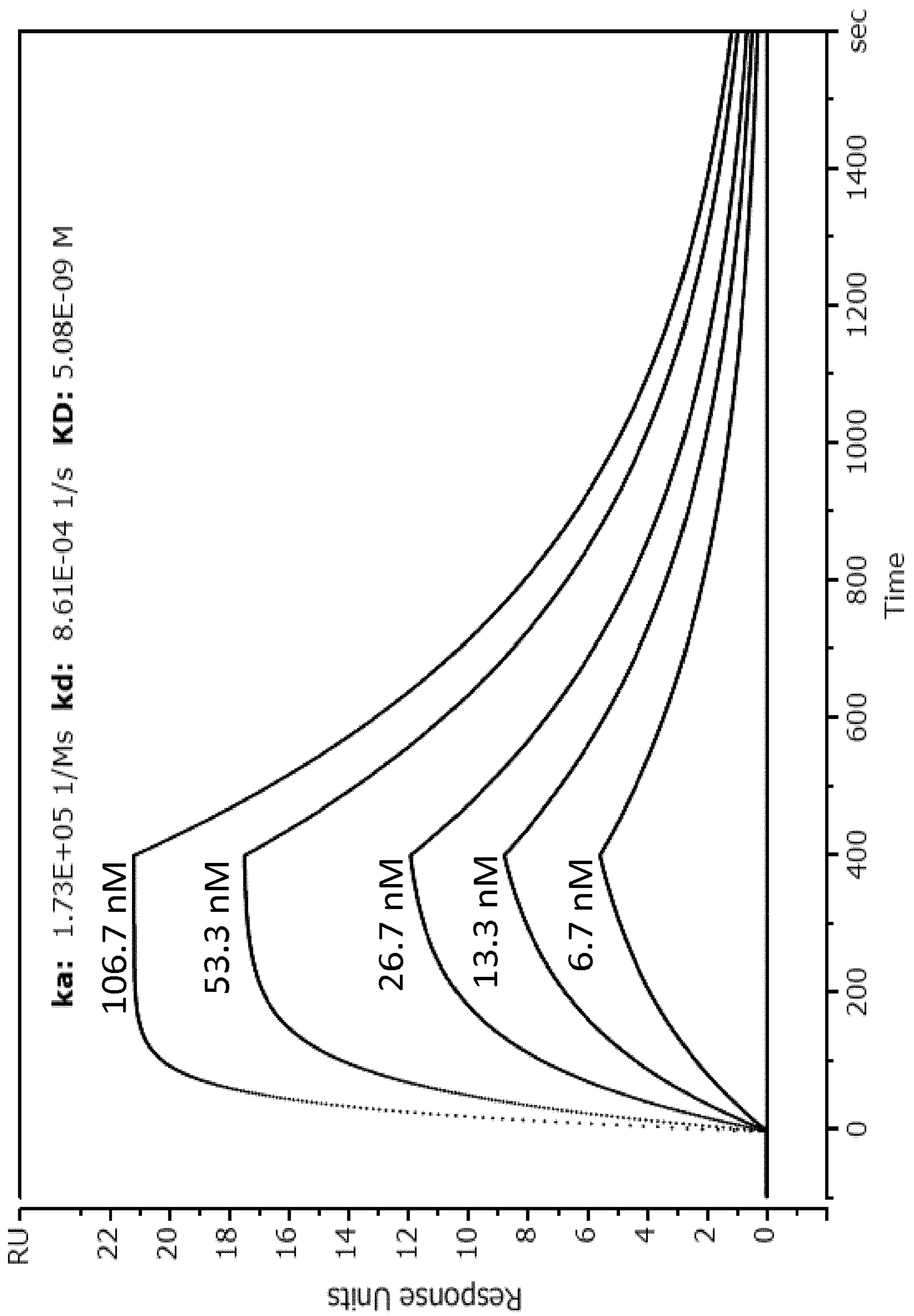
ka: 1.73E+05 1/Ms kd: 8.61E-04 1/s KD: 5.08E-09 M
106.7 nM
53.3 nM
26.7 nM
13.3 nM
6.7 nM
RU
Response Units
22
20
18
16
14
12
10
8
6
4
2
0
0
200
400
600
800
1000
1200
1400
sec
Time

**Fig. 3**

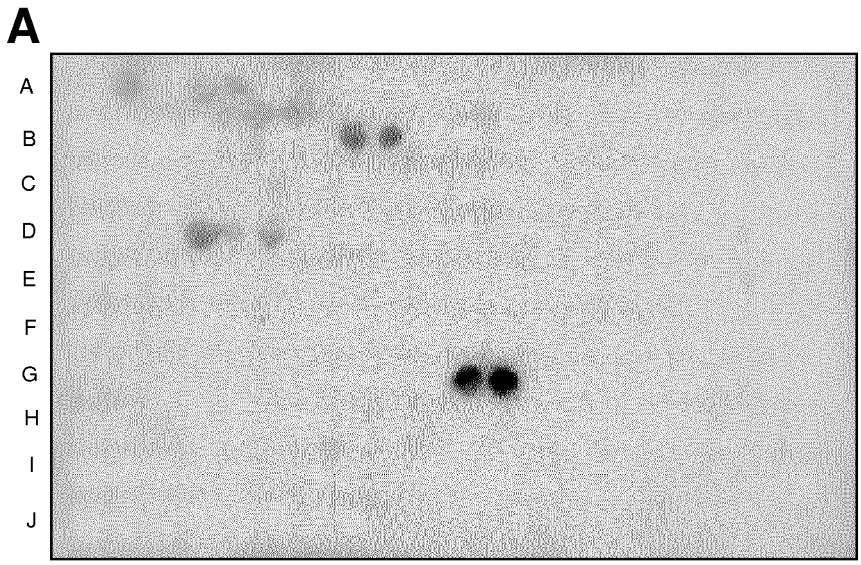


NI-206.82C2 plus secondary detection antibody

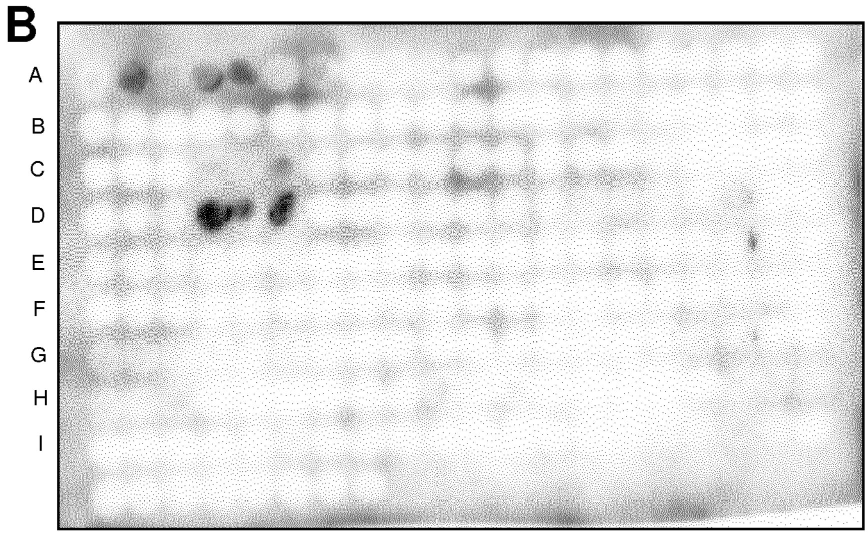


Secondary detection antibody only

Fig. 4

**C**

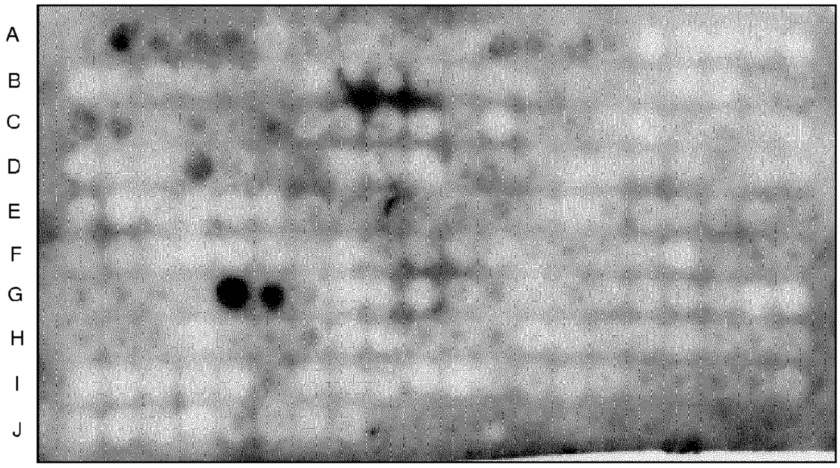


NI-206.18H2 plus secondary detection antibody

**D**

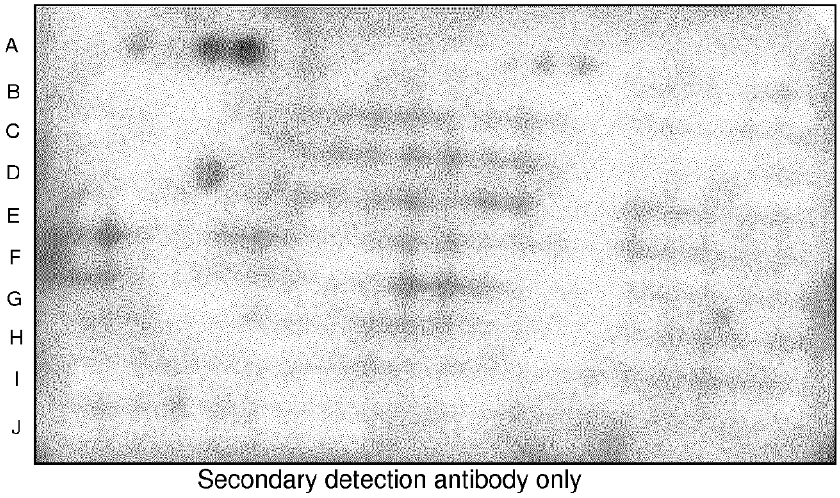


Secondary detection antibody only

**E**

| Antibody | Epitope |
|---|---|
| NI-206.82C2 | 525-PPQFDRSKKYP-535 (SEQ ID NO: 32)<br>113-YLESDYSKLWR-123 (SEQ ID NO: 61) |
| NI-206.59B4 | 53-SYKTFFP-59 (SEQ ID NO: 33) |
| NI-206.22F7 | 381-KDTVENAIQIT-391 (SEQ ID NO: 34) |
| NI-206.27E8 | 169-NIYLKQR-175 (SEQ ID NO: 35) |
| NI-206.12G4 | 481-TDQEIKILEENKELE-495 (SEQ ID NO: 36) |
| NI-206.17A6 | 77-VLYNIETGQSY-87 (SEQ ID NO: 37) |
| NI-206.18H2 | 501-IQLPKEEIKKL-511 (SEQ ID NO: 60) |

**Fig. 4 (continued)**

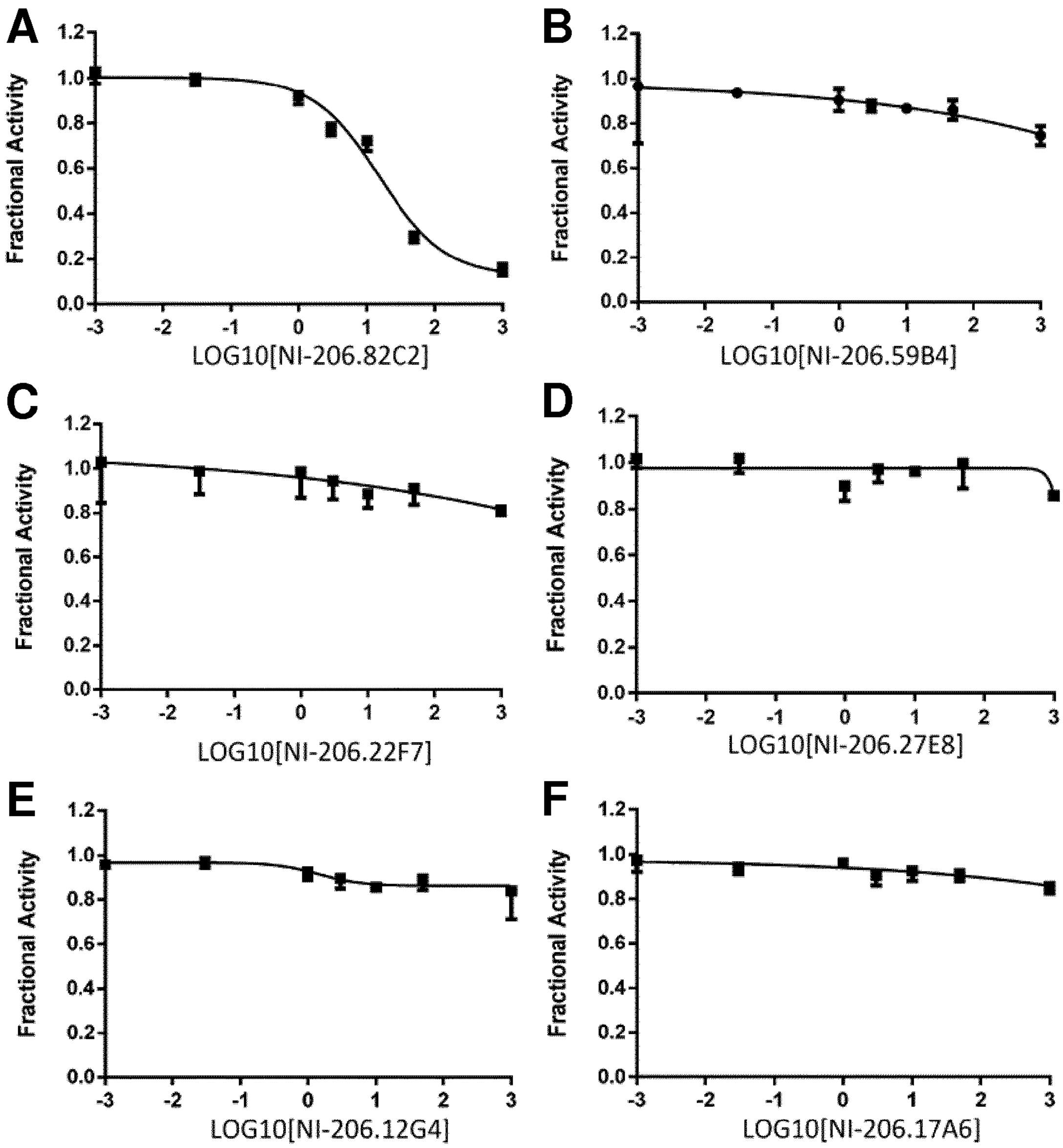


G

| Antibody | Inhibition ($IC_{50}$) nM |
|---|---|
| NI-206.82C2 | 7,7 nM |
| NI-206.59B4 | Not inhibitory |
| NI-206.22F7 | Not inhibitory |
| NI-206.27E8 | Not inhibitory |
| NI-206.12G4 | Not inhibitory |
| NI-206.17A6 | Not inhibitory |

**Fig. 5**

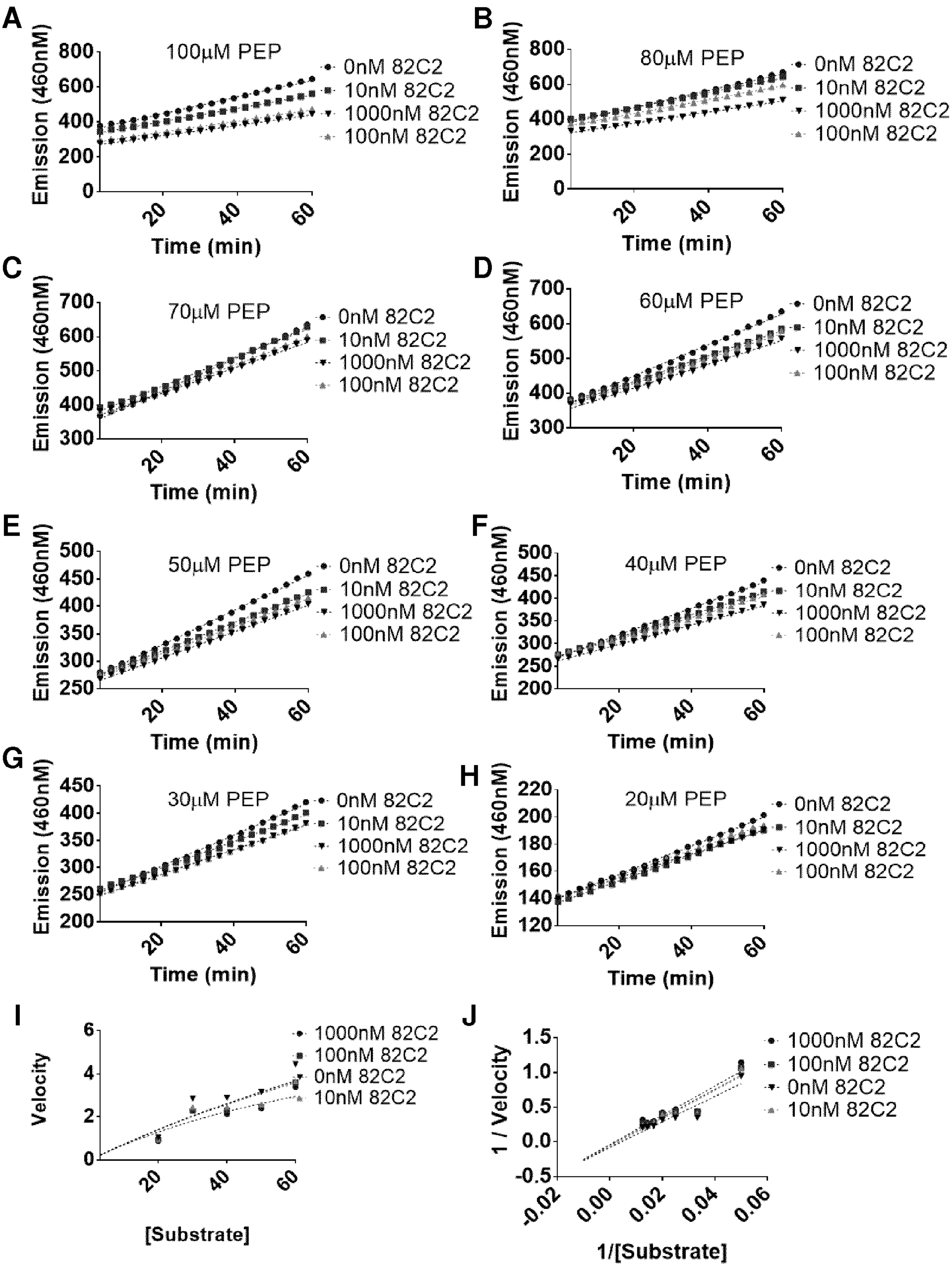
A
100μM PEP
B
80μM PEP
C
70μM PEP
D
60μM PEP
E
50μM PEP
F
40μM PEP
G
30μM PEP
H
20μM PEP
0nM 82C2
10nM 82C2
1000nM 82C2
100nM 82C2
Emission (460nM)
Time (min)
I
Velocity
[Substrate]
J
1 / Velocity
1/[Substrate]

Fig. 6

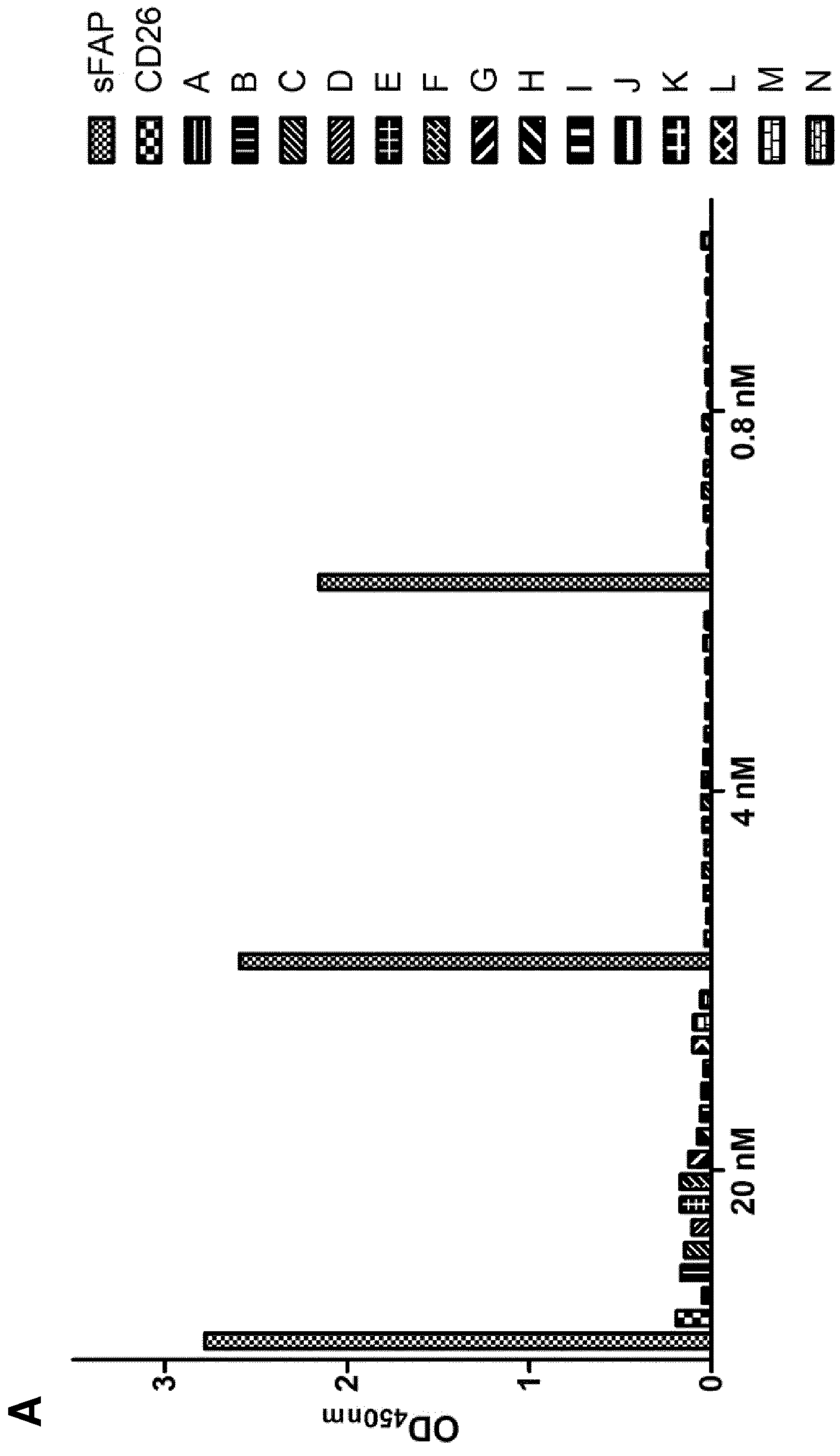


Fig. 7

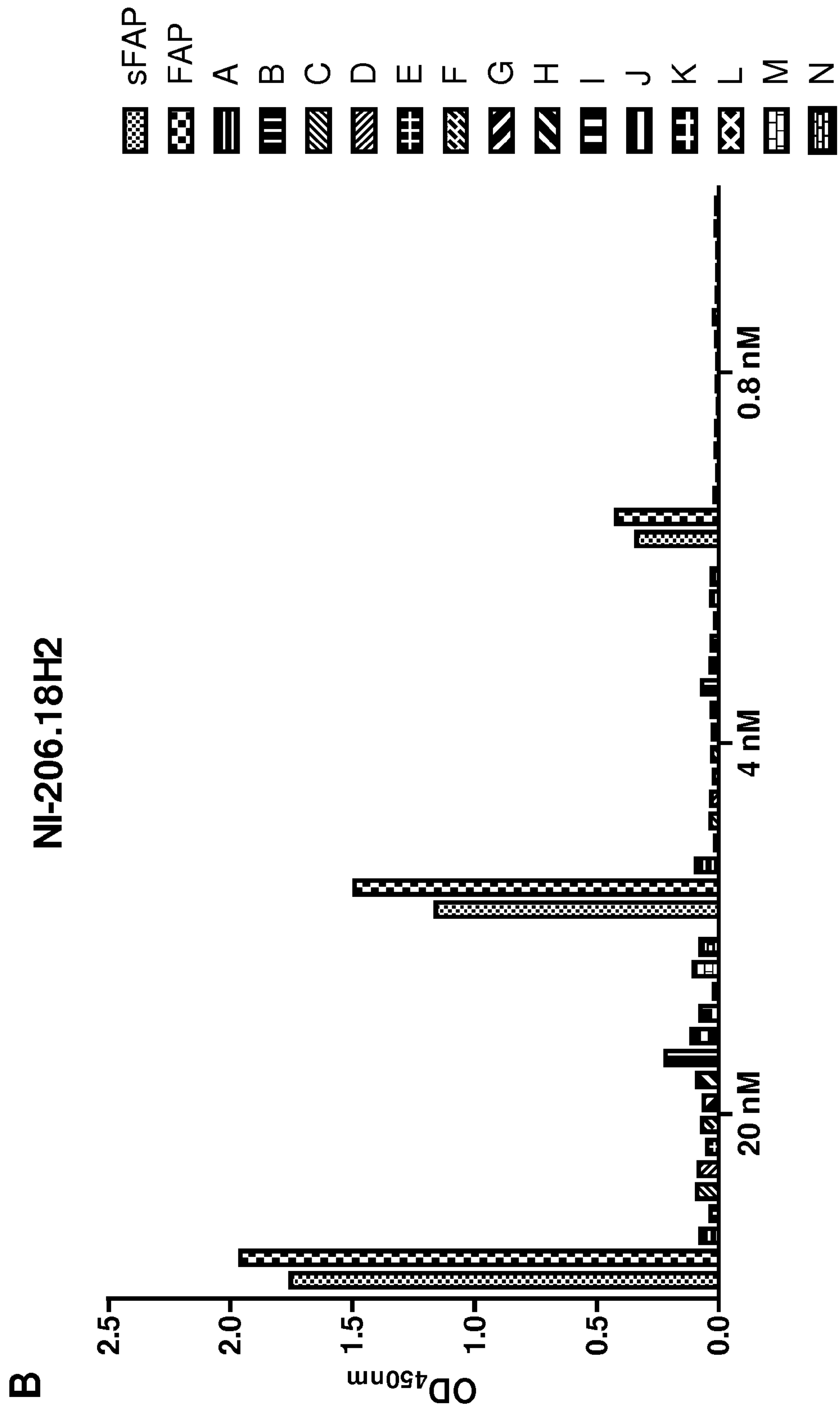


Fig. 7 (continued)

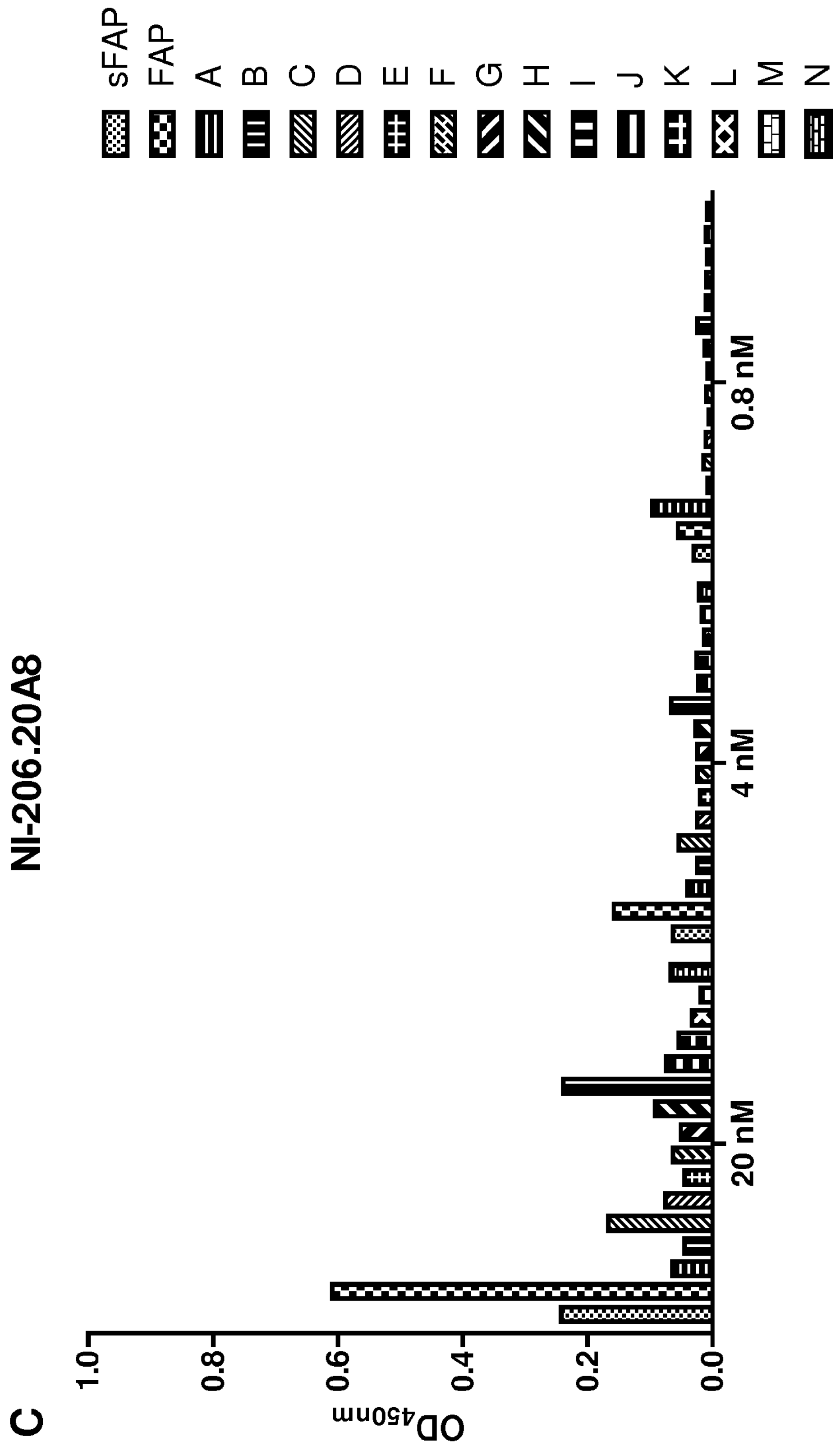


**Fig. 7 (continued)**

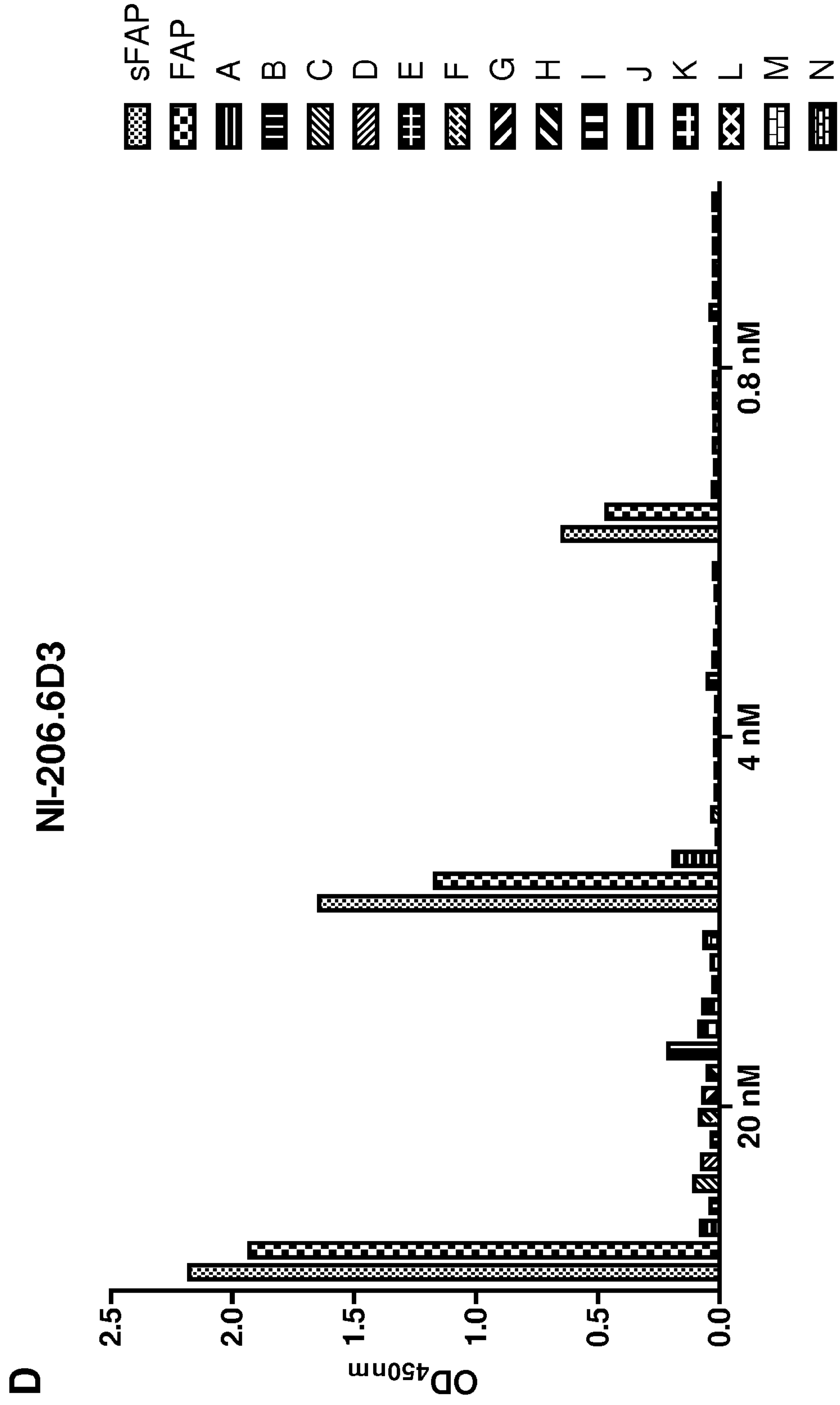


Fig. 7 (continued)

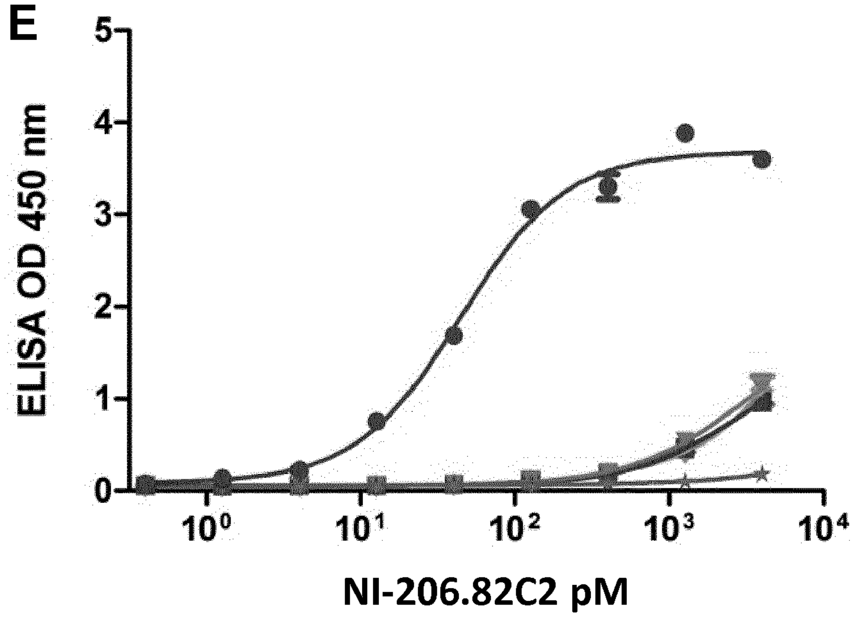


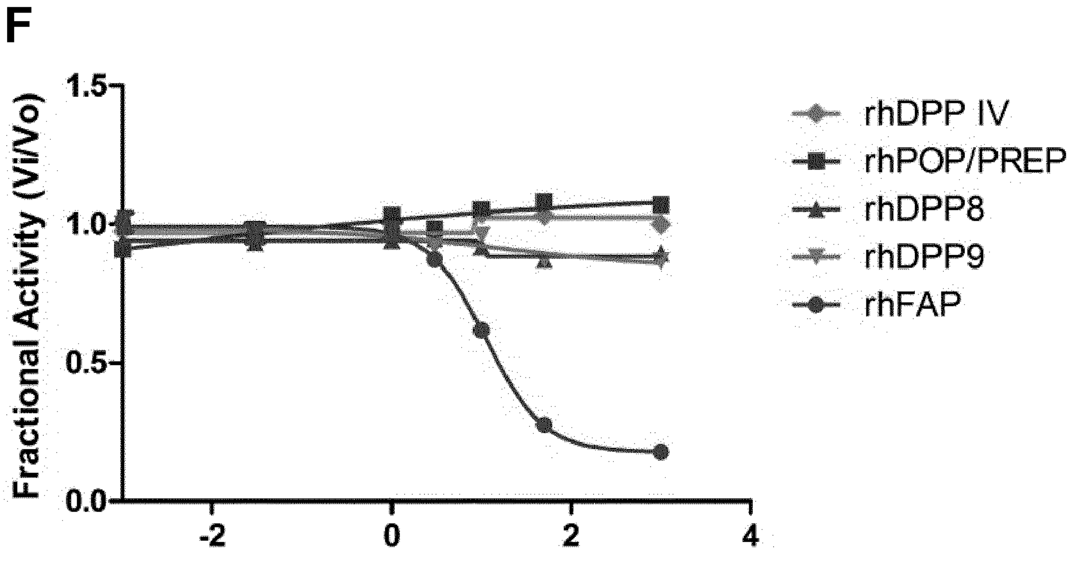


Fig. 7 (continued)

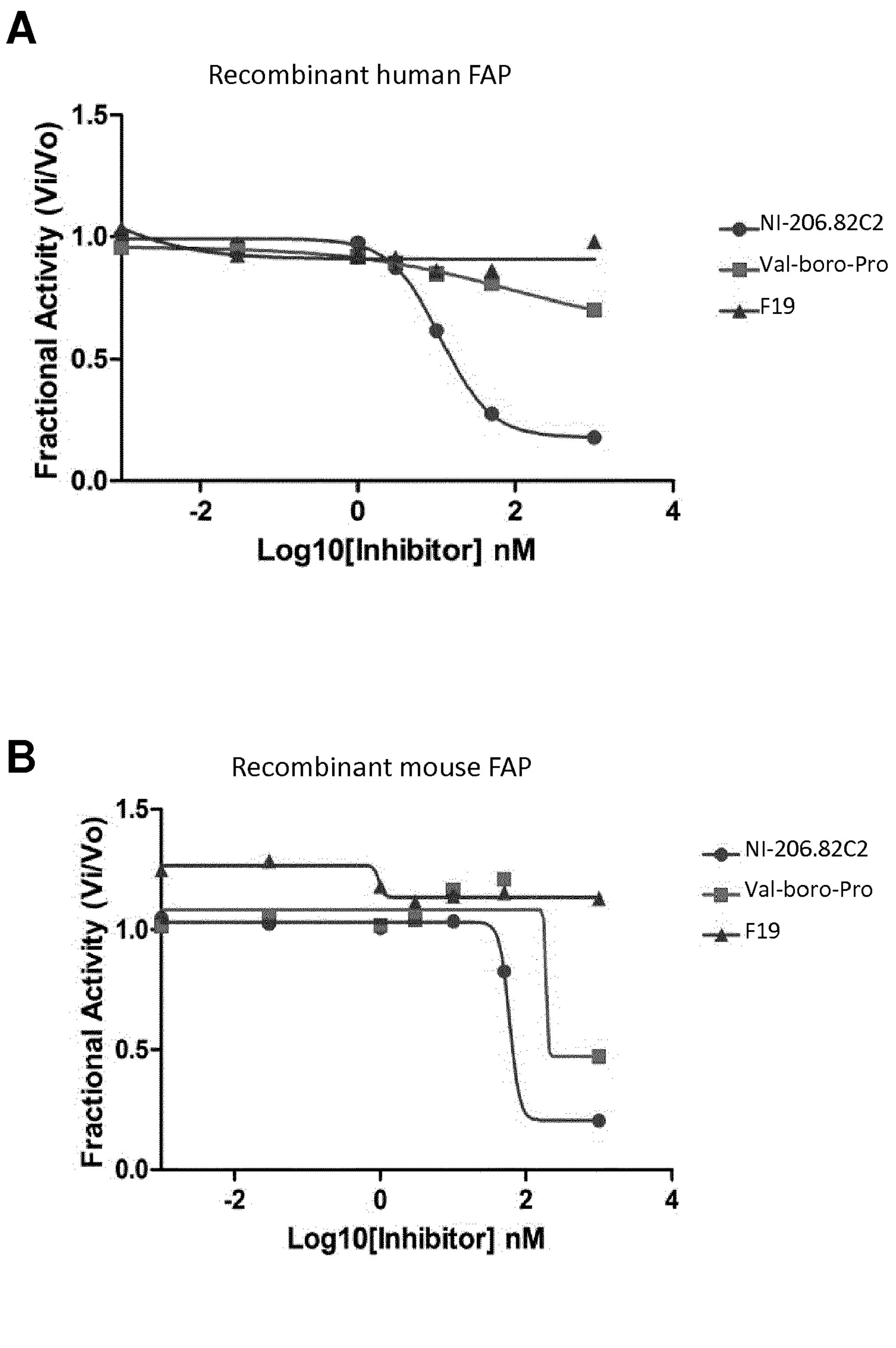
A
Recombinant human FAP
Fractional Activity (Vi/Vo)
1.5
1.0
0.5
0.0
-2
0
2
4
Log10[Inhibitor] nM
NI-206.82C2
Val-boro-Pro
F19
B
Recombinant mouse FAP
Fractional Activity (Vi/Vo)
1.5
1.0
0.5
0.0
-2
0
2
4
Log10[Inhibitor] nM
NI-206.82C2
Val-boro-Pro
F19

Fig. 8

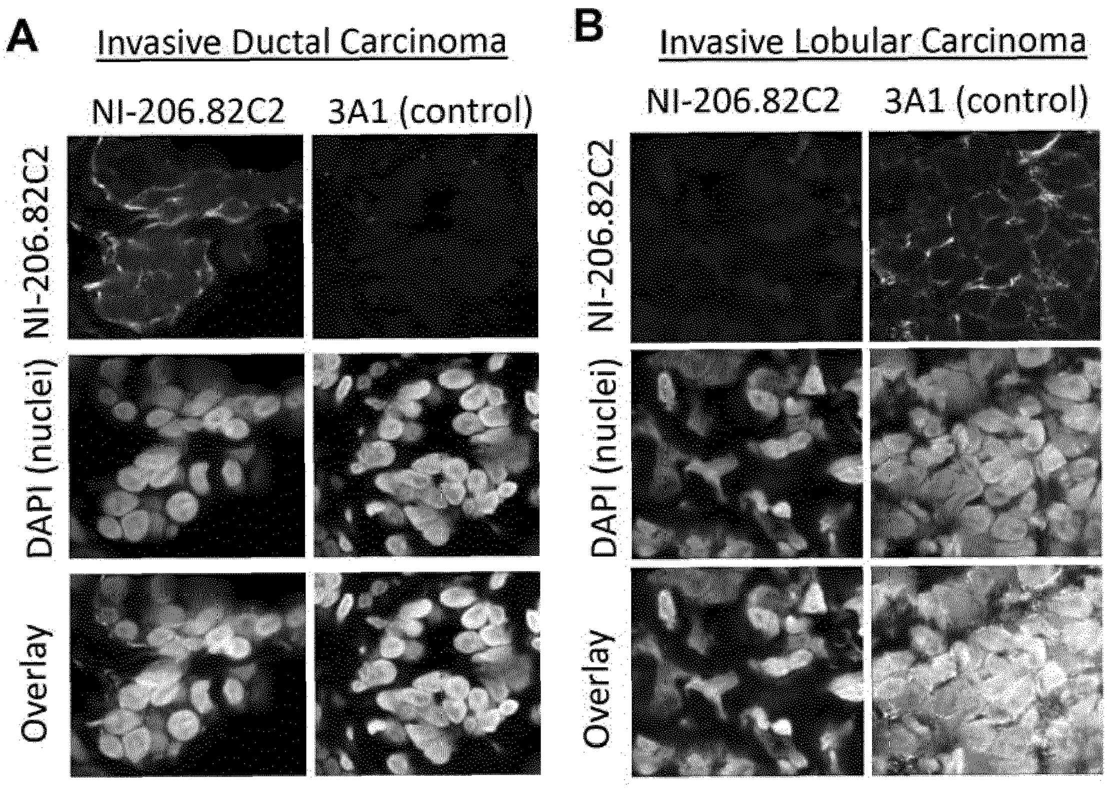
A
Invasive Ductal Carcinoma
NI-206.82C2
3A1 (control)
NI-206.82C2
DAPI (nuclei)
Overlay
B
Invasive Lobular Carcinoma
NI-206.82C2
3A1 (control)
NI-206.82C2
DAPI (nuclei)
Overlay

Fig. 9

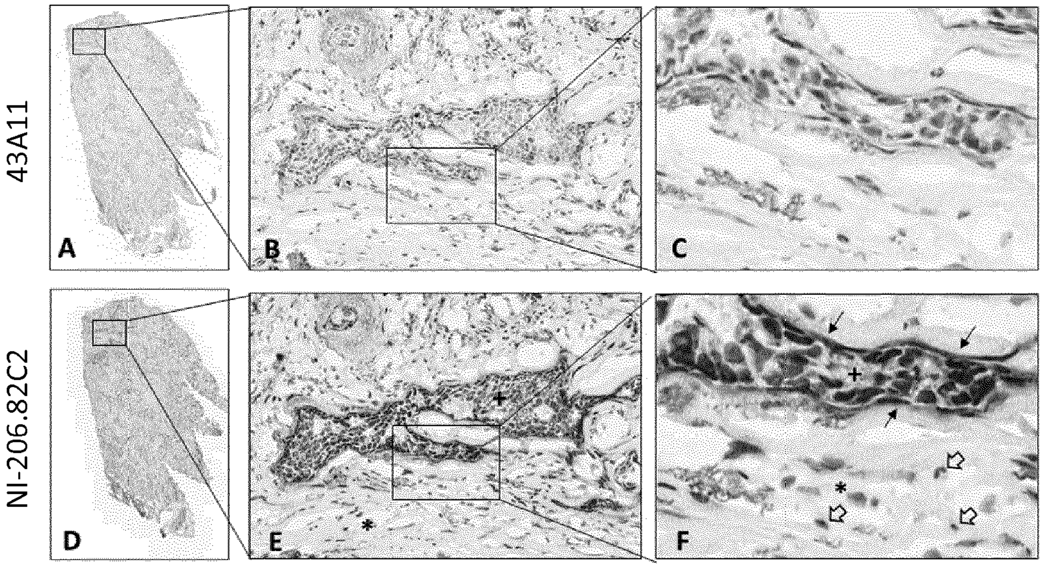


Fig. 10

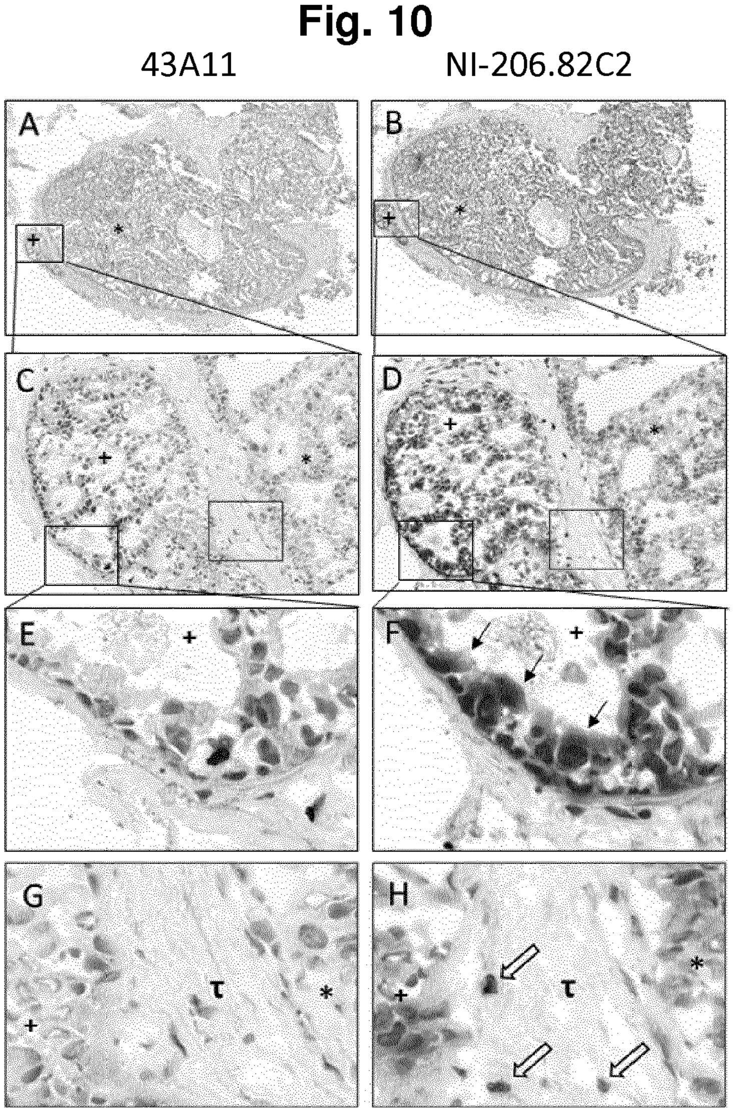


Fig. 11

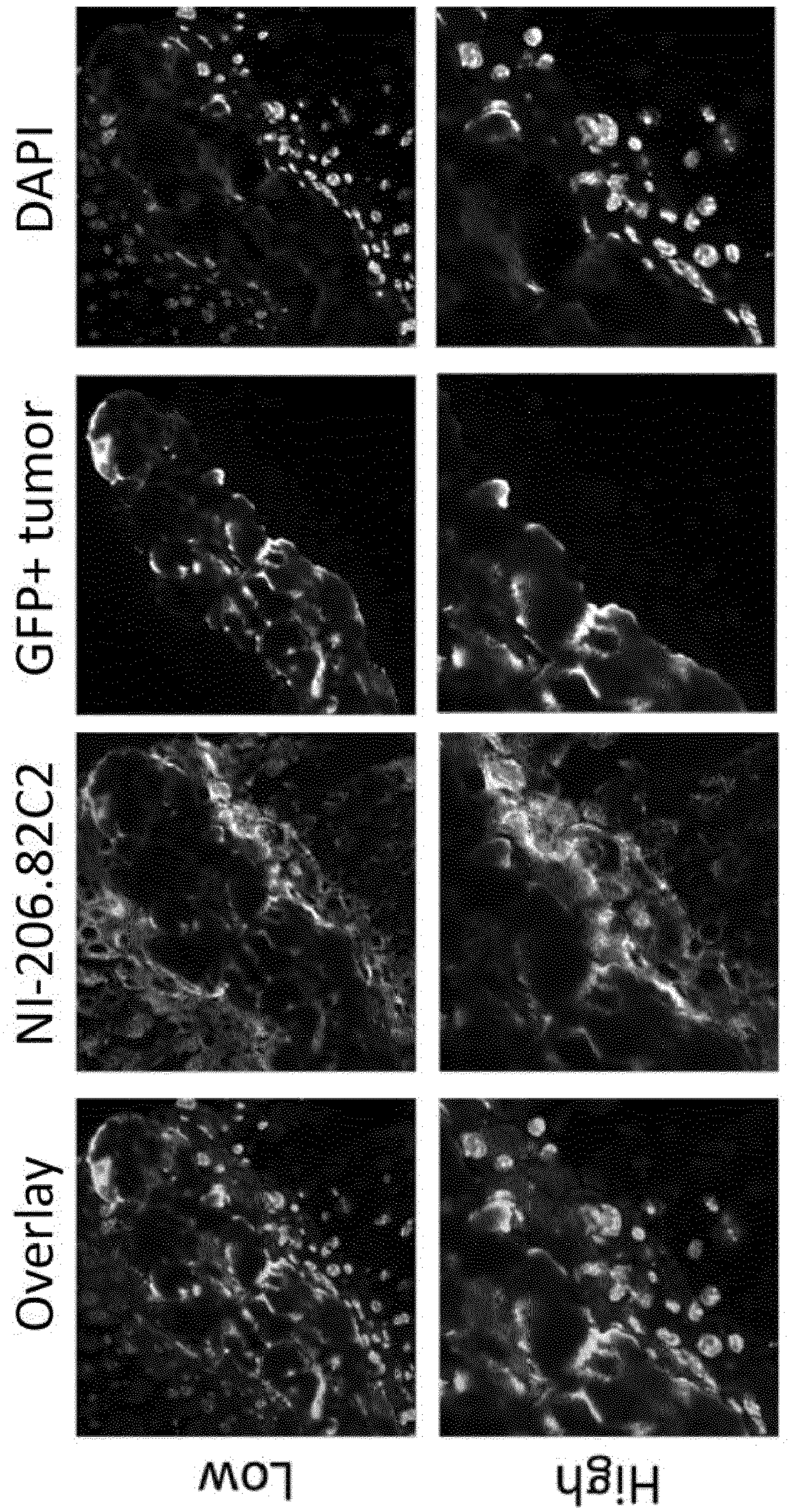


Fig. 12

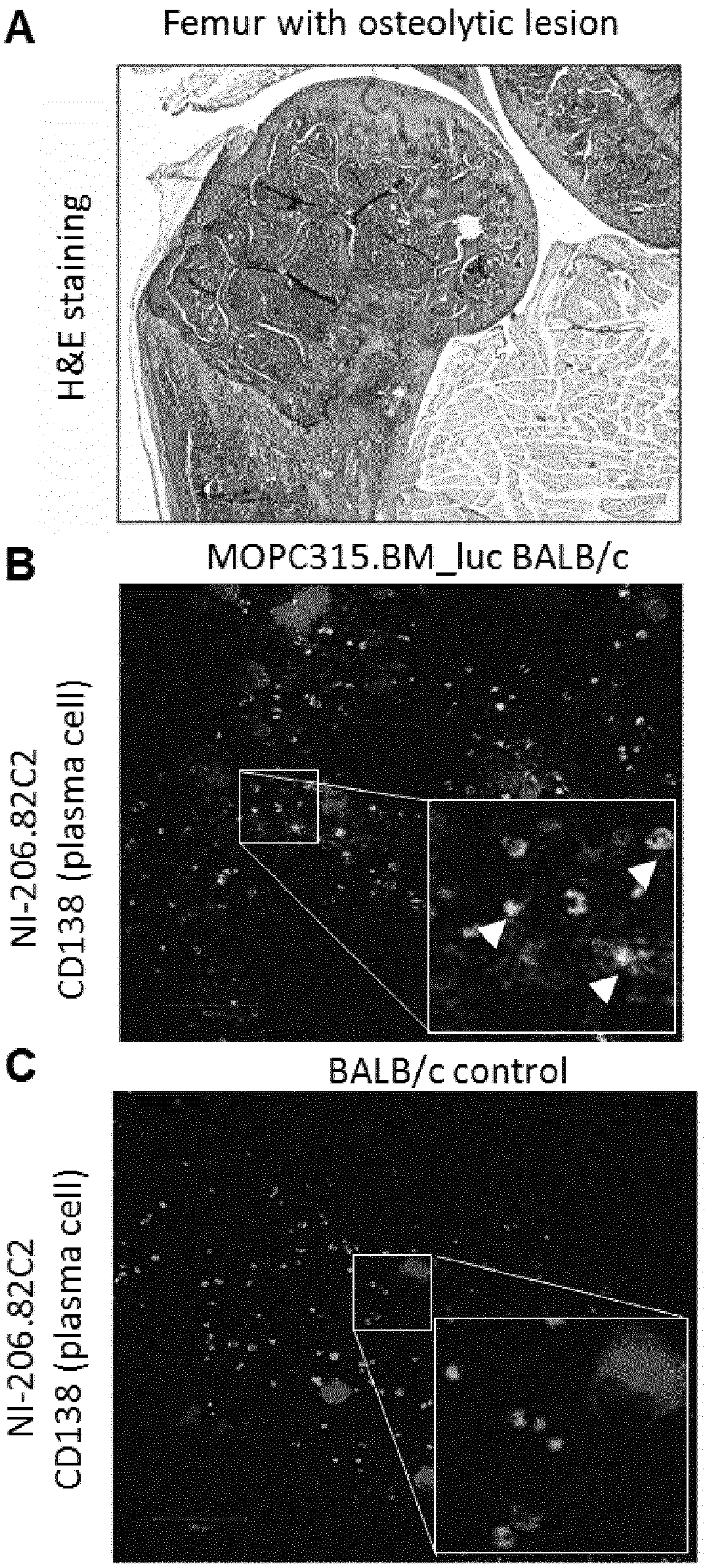
A
Femur with osteolytic lesion
H&E staining
B
MOPC315.BM_luc BALB/c
NI-206.82C2
CD138 (plasma cell)
C
BALB/c control
NI-206.82C2
CD138 (plasma cell)

Fig. 13

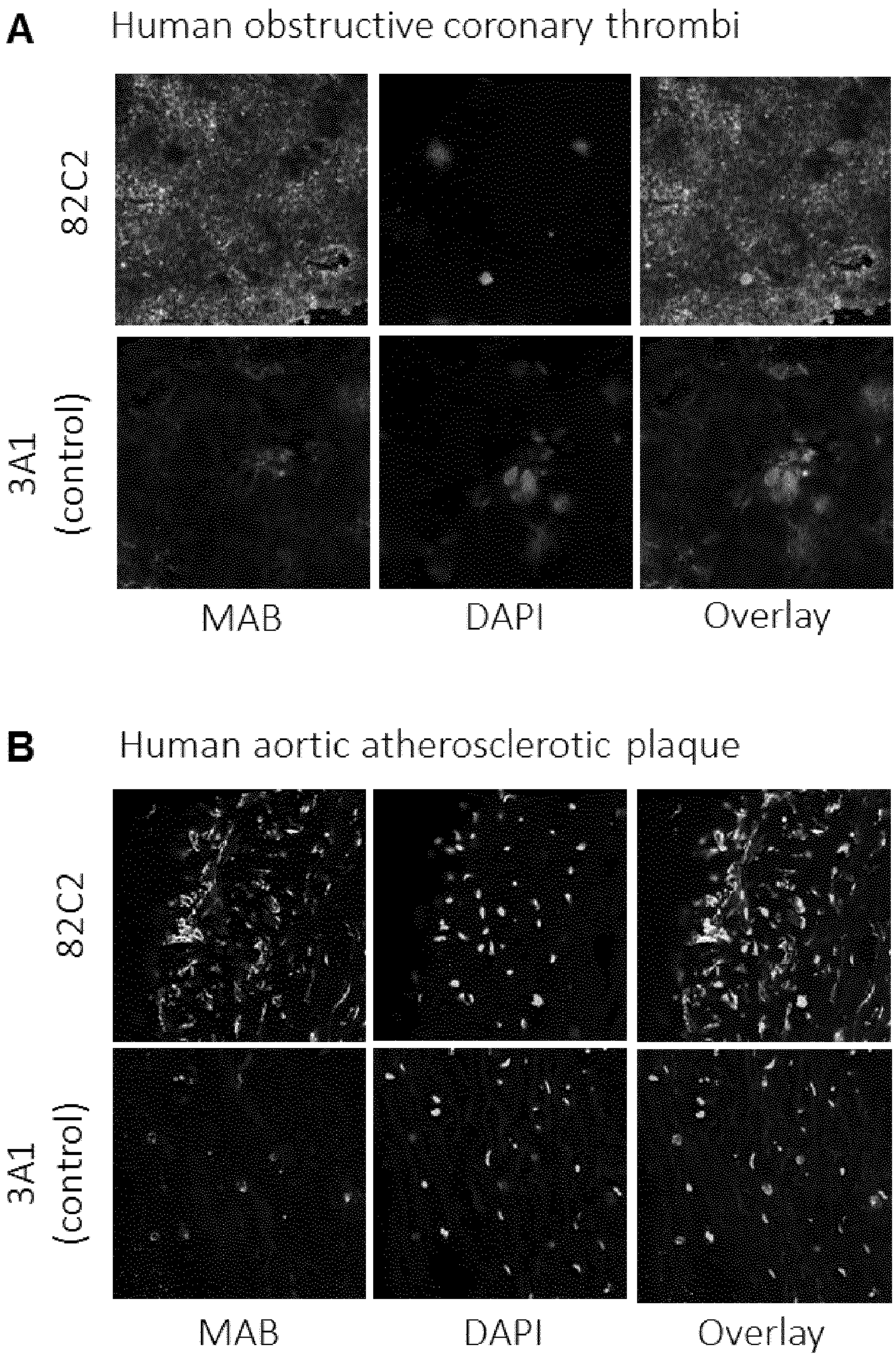
A
Human obstructive coronary thrombi
82C2
3A1
(control)
MAB
DAPI
Overlay
B
Human aortic atherosclerotic plaque
82C2
3A1
(control)
MAB
DAPI
Overlay

Fig. 14

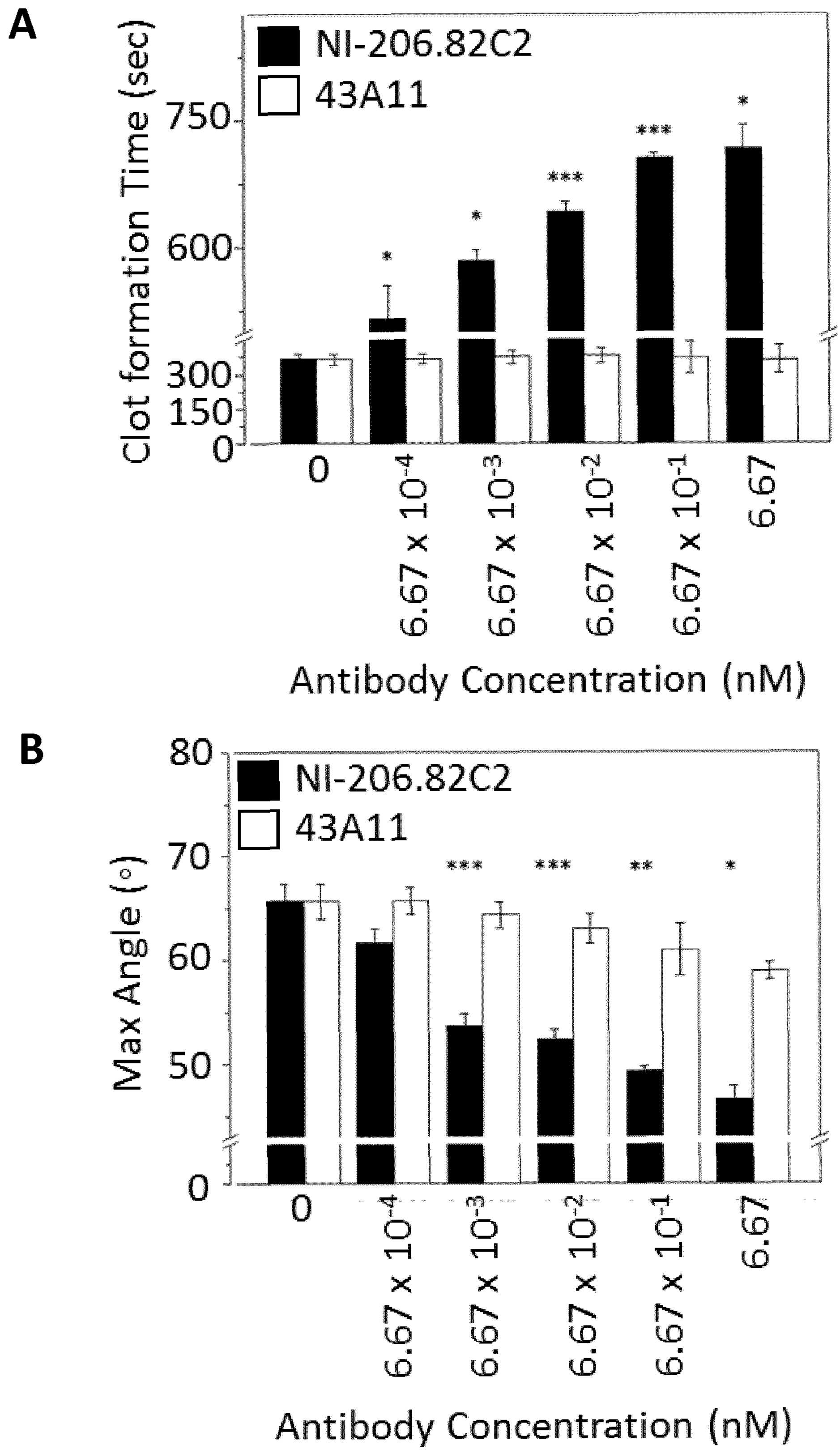
A
NI-206.82C2
43A11
Clot formation Time (sec)
750
600
300
150
0
*
*
***
***
*
0
6.67 x 10-4
6.67 x 10-3
6.67 x 10-2
6.67 x 10-1
6.67
Antibody Concentration (nM)
B
NI-206.82C2
43A11
Max Angle (∘)
80
70
60
50
0
***
***
**
*
0
6.67 x 10-4
6.67 x 10-3
6.67 x 10-2
6.67 x 10-1
6.67
Antibody Concentration (nM)

Fig. 15

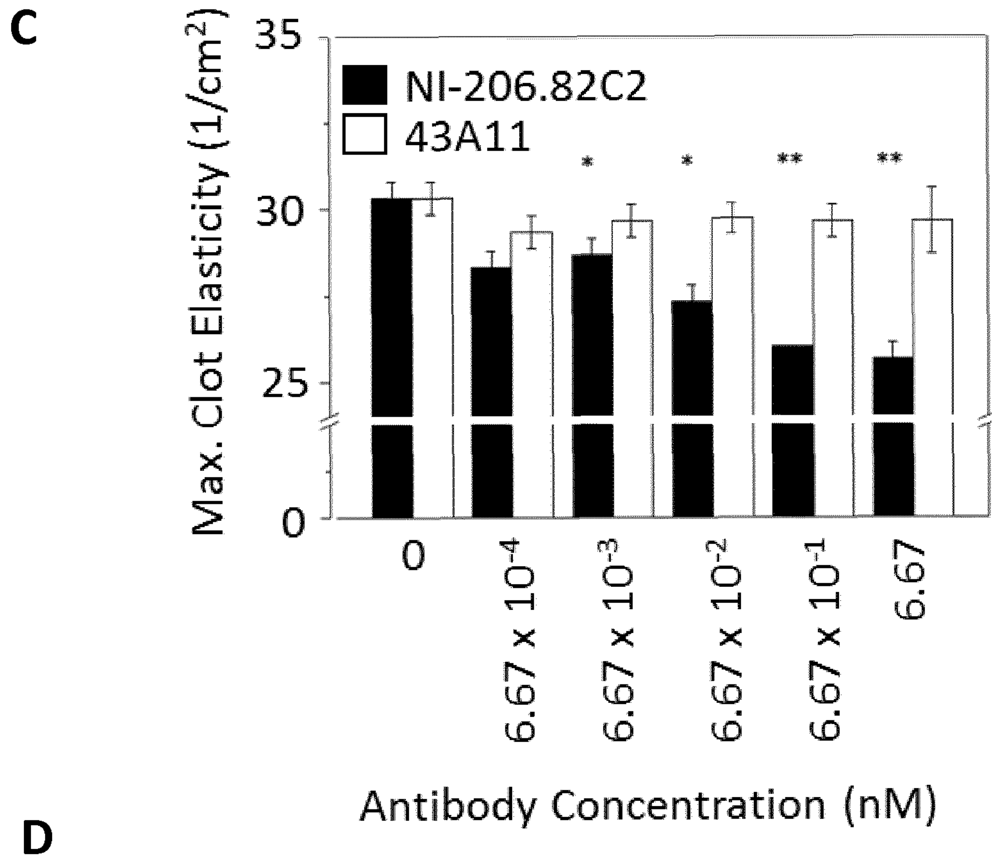


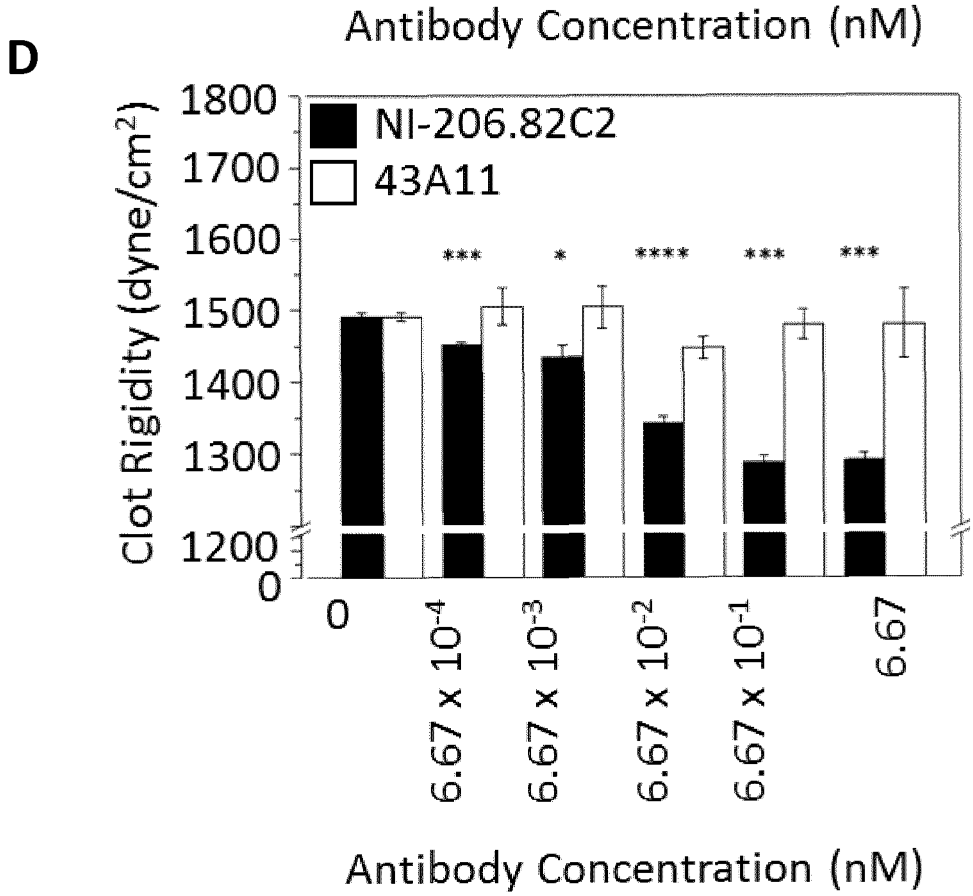


Fig. 15 (continued)

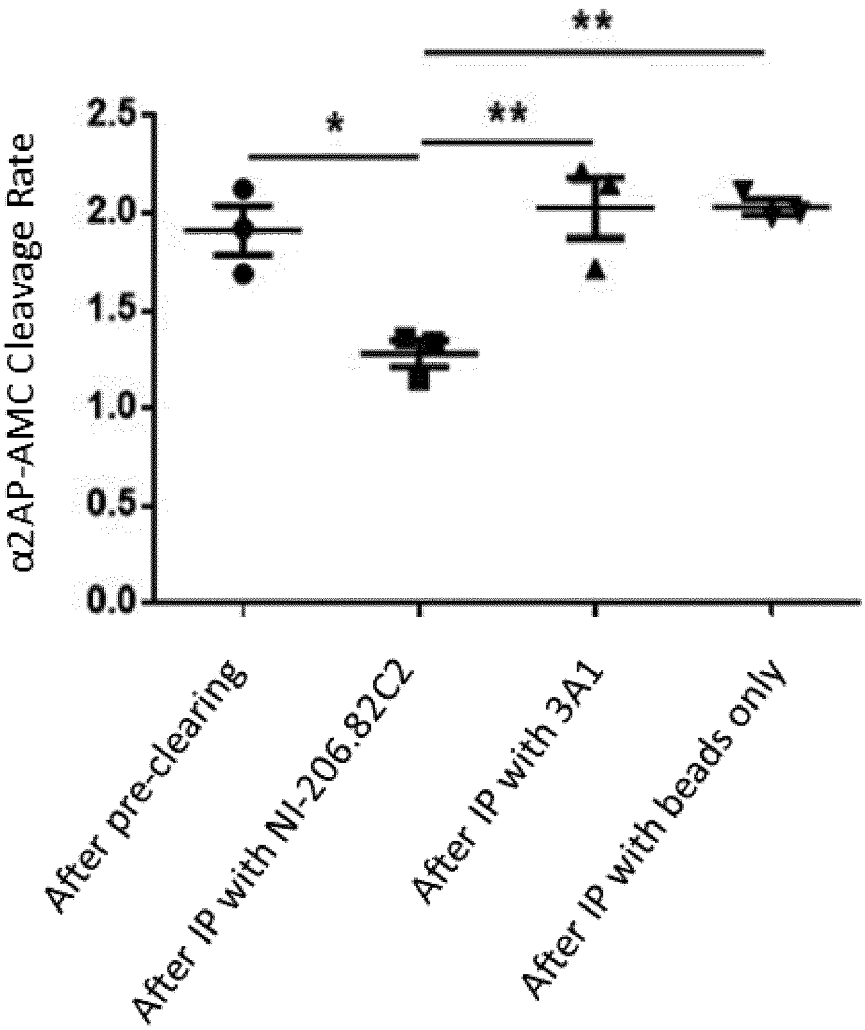

**
*
**
2.5
2.0
1.5
1.0
0.5
0.0
α2AP-AMC Cleavage Rate
After pre-clearing
After IP with NI-206.82C2
After IP with 3A1
After IP with beads only


**Fig. 16**

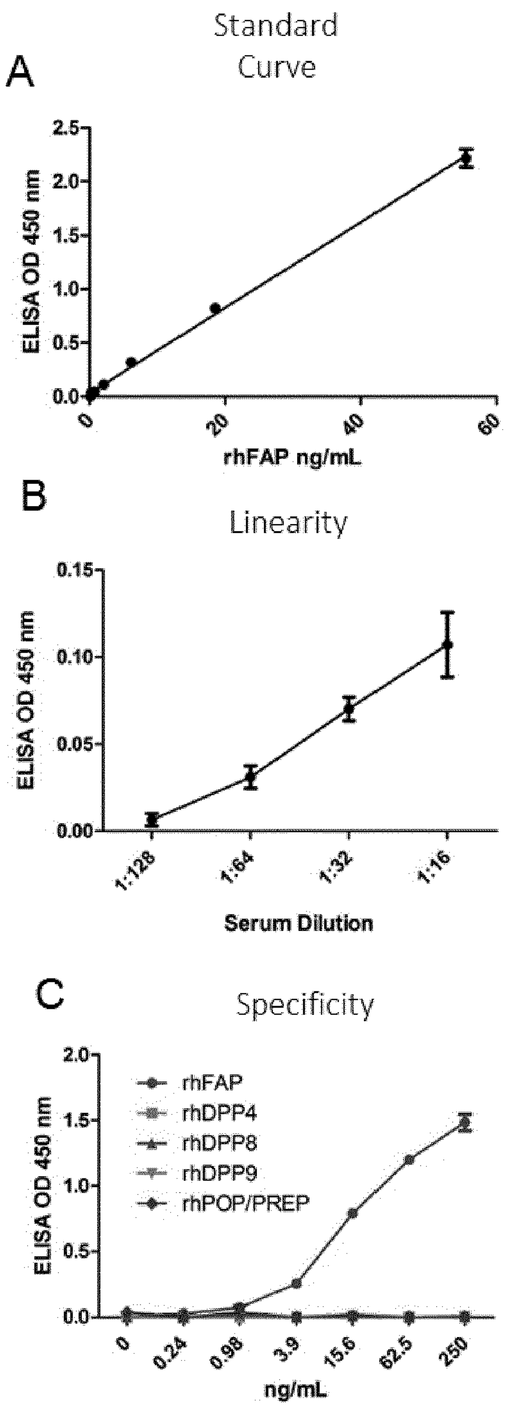


- Inter-Assay Coefficient of Variance = 8.56%
- Intra-Assay Coefficient of Variance = 8.63%

**Fig. 17**

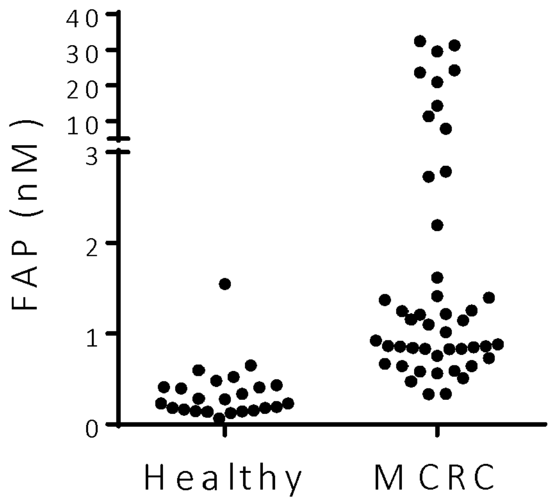


**Fig. 18**

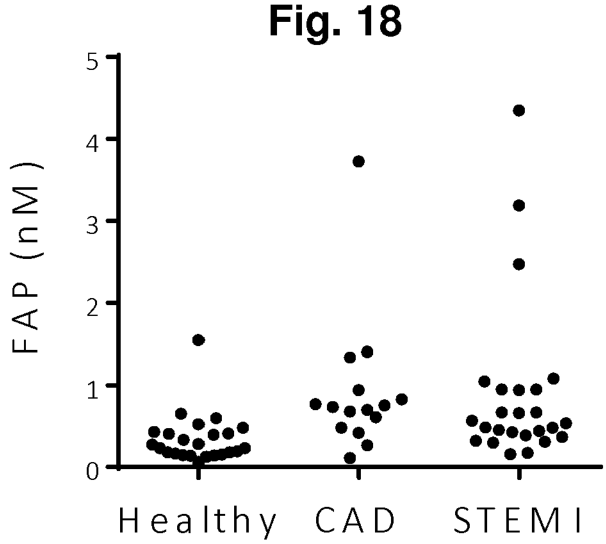


**Fig. 19**

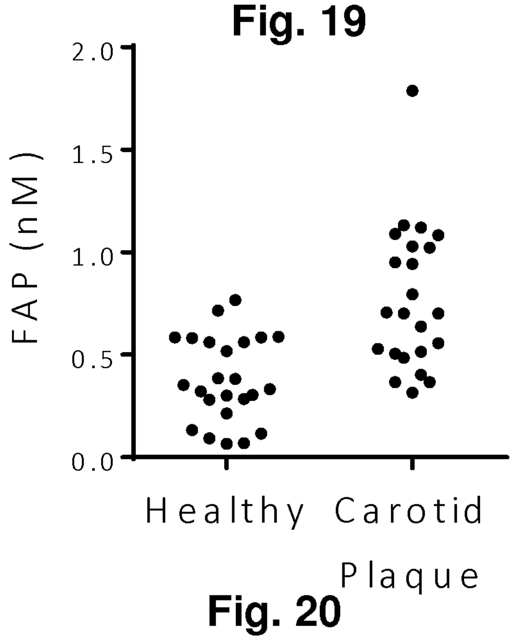


**Fig. 20**

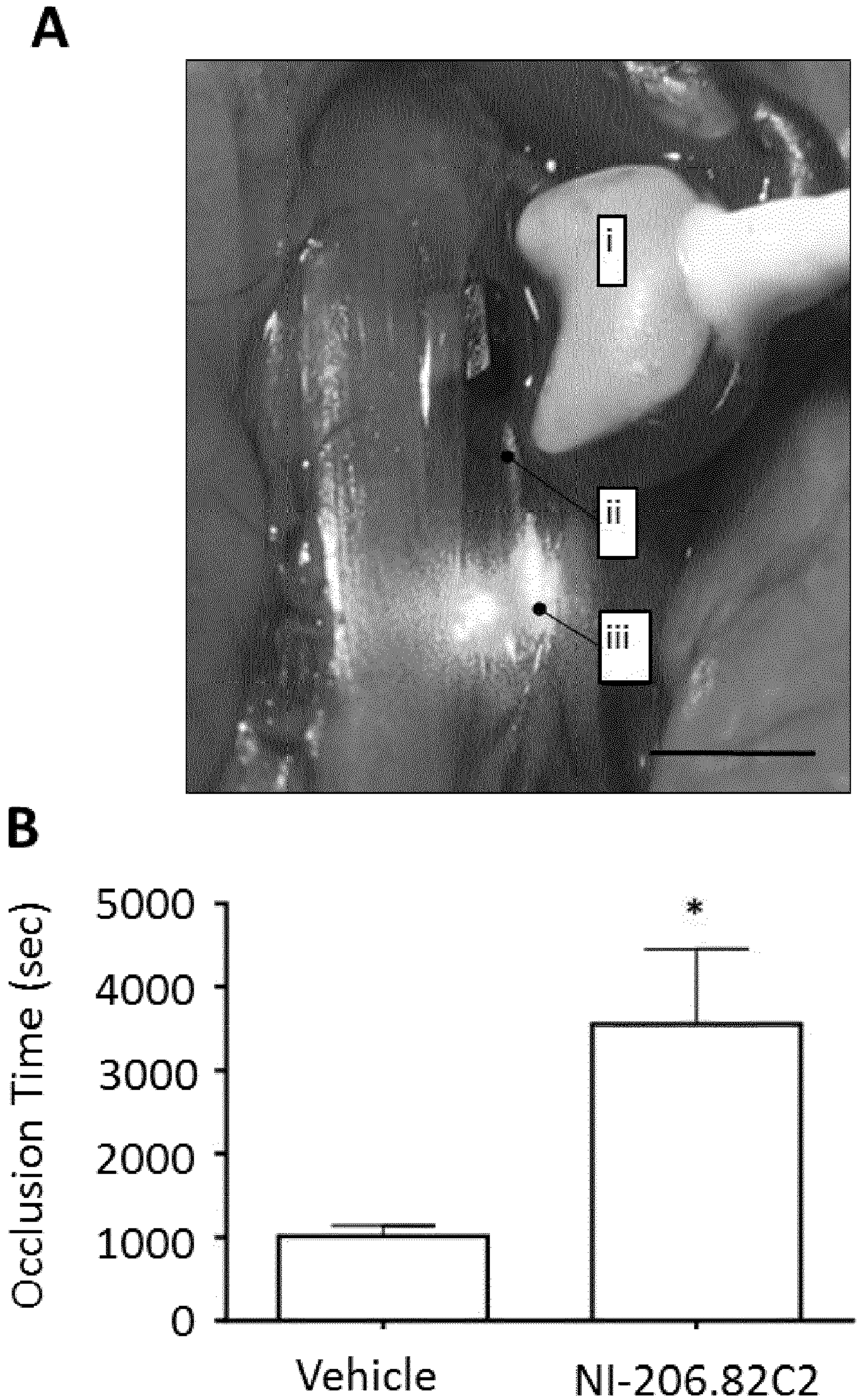
A
i
ii
iii
B
5000
4000
3000
2000
1000
0
Occlusion Time (sec)
*
Vehicle
NI-206.82C2

Fig. 21

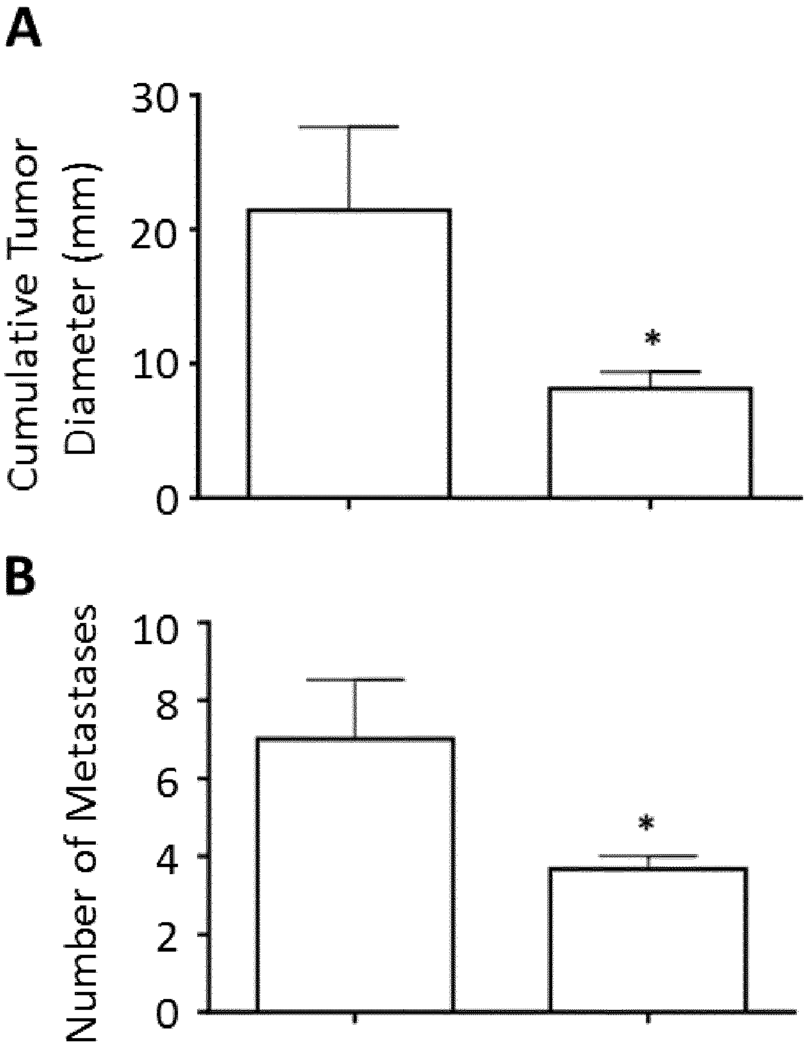
A
30
20
10
0
Cumulative Tumor
Diameter (mm)
*
B
10
8
6
4
2
0
Number of Metastases
*

Fig. 22

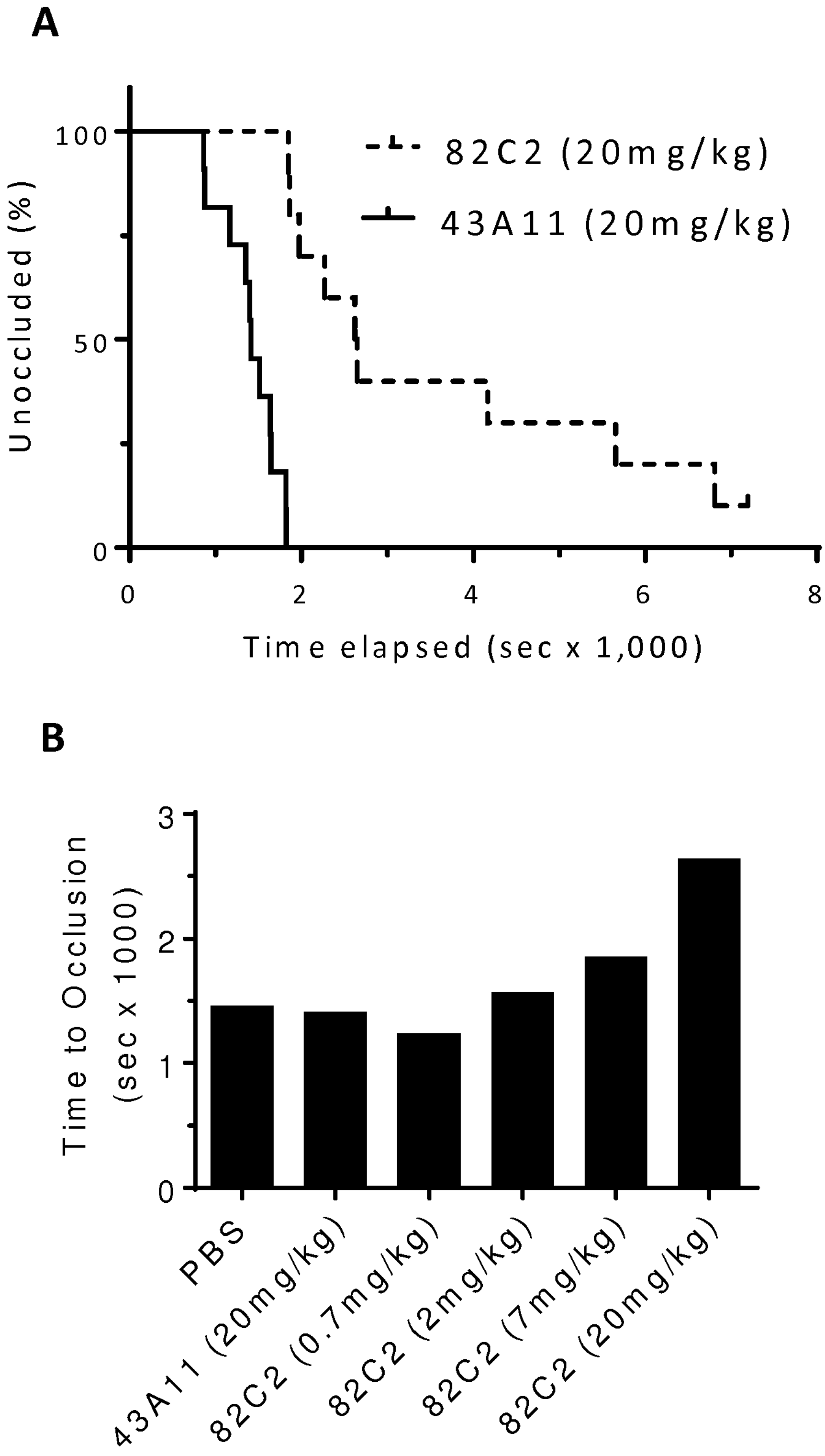
A
100
50
0
Unoccluded (%)
82C2 (20mg/kg)
43A11 (20mg/kg)
0
2
4
6
8
Time elapsed (sec x 1,000)
B
3
2
1
0
Time to Occlusion (sec x 1000)
PBS
43A11 (20mg/kg)
82C2 (0.7mg/kg)
82C2 (2mg/kg)
82C2 (7mg/kg)
82C2 (20mg/kg)

**Fig. 23**

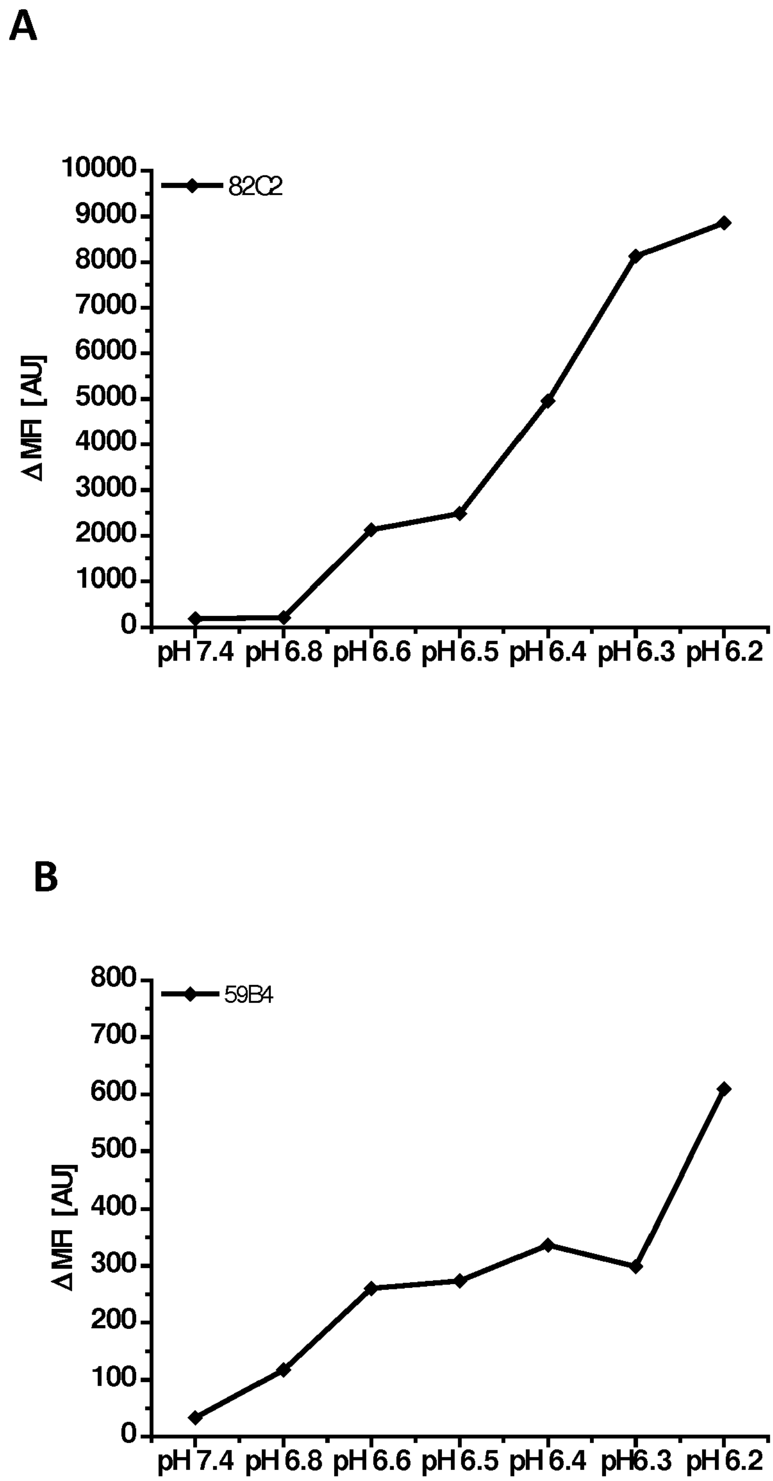
A
82C2
ΔMFI [AU]
10000
9000
8000
7000
6000
5000
4000
3000
2000
1000
0
pH 7.4
pH 6.8
pH 6.6
pH 6.5
pH 6.4
pH 6.3
pH 6.2
B
59B4
ΔMFI [AU]
800
700
600
500
400
300
200
100
0
pH 7.4
pH 6.8
pH 6.6
pH 6.5
pH 6.4
pH 6.3
pH 6.2

Fig. 24

C

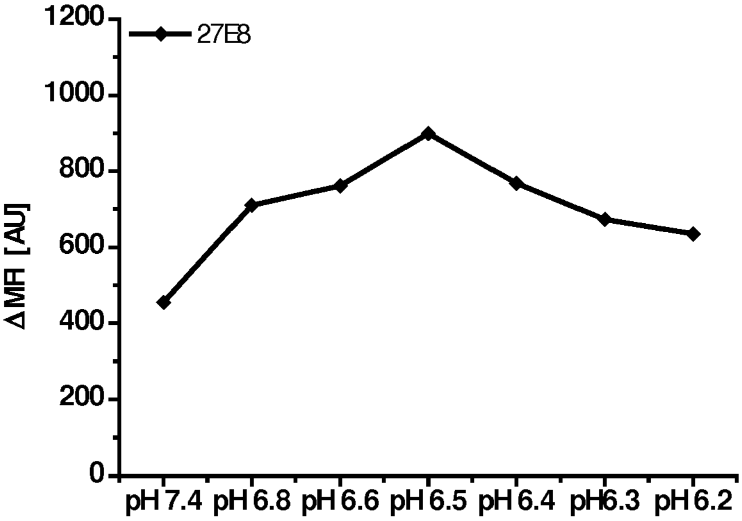


D

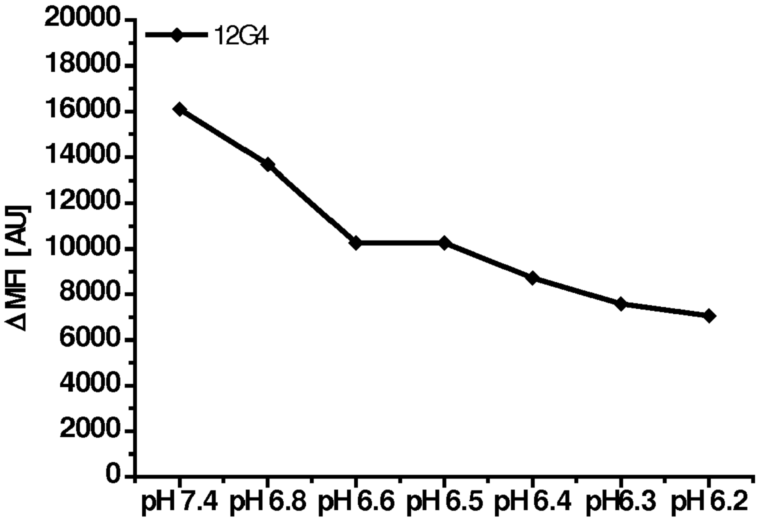


Fig. 24 (continued)

E

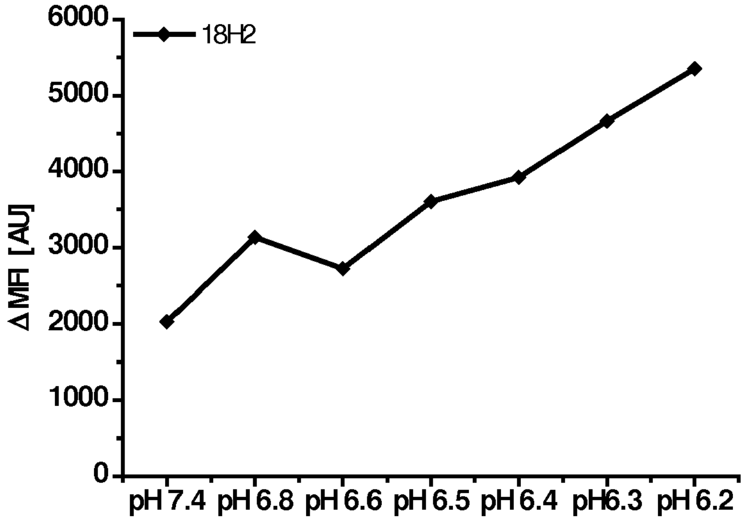


F

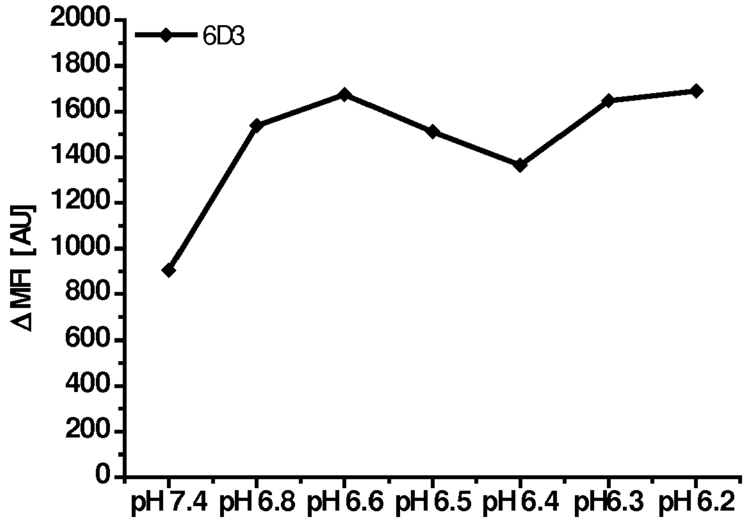


Fig. 24 (continued)

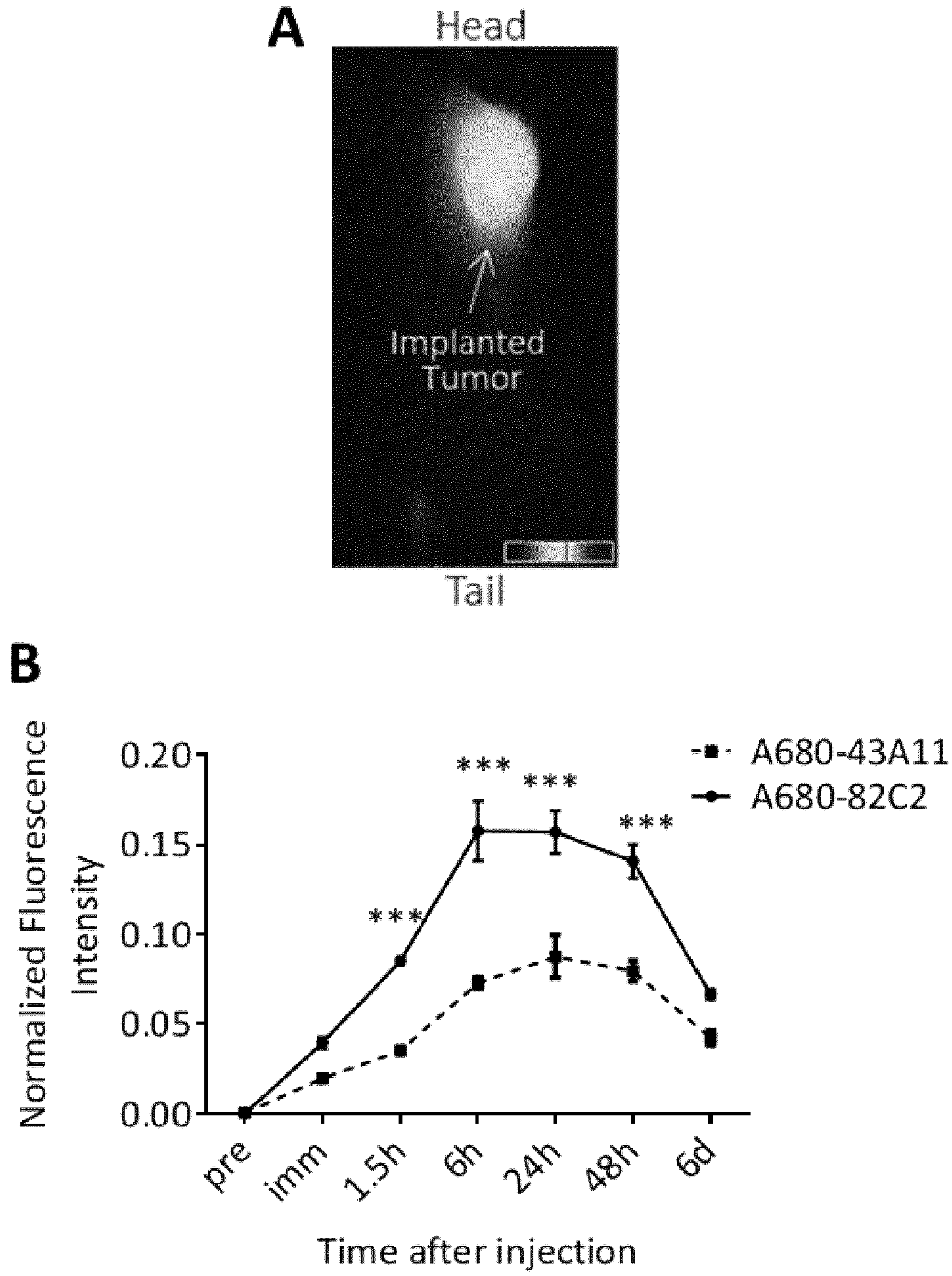
A
Head
Implanted
Tumor
Tail
B
Normalized Fluorescence
Intensity
0.20
0.15
0.10
0.05
0.00
***
***
***
***
A680-43A11
A680-82C2
pre
imm
1.5h
6h
24h
48h
6d
Time after injection

**Fig. 25**

K M I L P P Q | F D R S K | K Y P L L I Q

consensus: F D R S K

Fig. 26

521 526 531 536

K M I L **P P Q F D R S K K Y P** L L I Q

Fig. 27

Europäisches Patentamt
European Patent Office
Office européen des brevets

# EUROPEAN SEARCH REPORT

Application Number
EP 17 18 1249

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2014/055442 A2 (UNIV PENNSYLVANIA [US]; WISTAR INST [US]; JUNE CARL H [US]; PURE ELLEN) 10 April 2014 (2014-04-10)<br>* the whole document *<br>----- | 1-15 | INV.<br>C07K16/40<br>A61K39/00<br>C07K14/725<br>C07K16/30 |
| Y,D | PETRA C SCHUBERTH ET AL: "Treatment of malignant pleural mesothelioma by fibroblast activation protein-specific re-directed T cells",<br>JOURNAL OF TRANSLATIONAL MEDICINE, BIOMED CENTRAL, LONDON, GB,<br>vol. 11, no. 1,<br>12 August 2013 (2013-08-12), page 187,<br>XP021160242,<br>ISSN: 1479-5876, DOI: 10.1186/1479-5876-11-187<br>* the whole document *<br>----- | 1-15 | |
| Y,D | TRAN ERIC ET AL: "Immune targeting of fibroblast activation protein triggers recognition of multipotent bone marrow stromal cells and cachexia.",<br>THE JOURNAL OF EXPERIMENTAL MEDICINE 3 JUN 2013,<br>vol. 210, no. 6, 3 June 2013 (2013-06-03), pages 1125-1135, XP002764896,<br>ISSN: 1540-9538<br>* the whole document *<br>-----<br>-/-- | 1-15 | TECHNICAL FIELDS SEARCHED (IPC)<br>C07K<br>A61K |

The present search report has been drawn up for all claims

1

| Place of search | Date of completion of the search | Examiner |
|---|---|---|
| The Hague | 2 October 2017 | Hix, Rebecca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 18 1249

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | L.-C. S. WANG ET AL: "Targeting Fibroblast Activation Protein in Tumor Stroma with Chimeric Antigen Receptor T Cells Can Inhibit Tumor Growth and Augment Host Immunity without Severe Toxicity", CANCER IMMUNOLOGY RESEARCH, vol. 2, no. 2, 1 February 2014 (2014-02-01), pages 154-166, XP055324766, US ISSN: 2326-6066, DOI: 10.1158/2326-6066.CIR-13-0027 * the whole document * ----- | 1-15 | |
| Y | SUNITHA KAKARLA ET AL: "Antitumor Effects of Chimeric Receptor Engineered Human T Cells Directed to Tumor Stroma", MOLECULAR THERAPY, vol. 21, no. 8, 4 June 2013 (2013-06-04), pages 1611-1620, XP055324842, GB ISSN: 1525-0016, DOI: 10.1038/mt.2013.110 * the whole document * ----- | 1-15 | TECHNICAL FIELDS SEARCHED (IPC) |
| Y,D | ULF PETRAUSCH ET AL: "Re-directed T cells for the treatment of fibroblast activation protein (FAP)-positive malignant pleural mesothelioma (FAPME-1)", BMC CANCER, BIOMED CENTRAL, LONDON, GB, vol. 12, no. 1, 22 December 2012 (2012-12-22), page 615, XP021141216, ISSN: 1471-2407, DOI: 10.1186/1471-2407-12-615 * the whole document * ----- | 1-15 | |
| | -/-- | | |

1 The present search report has been drawn up for all claims

| Place of search | Date of completion of the search | Examiner |
|---|---|---|
| The Hague | 2 October 2017 | Hix, Rebecca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 18 1249

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A,D | SCOTT ANDREW M ET AL: "A Phase I dose-escalation study of sibrotuzumab in patients with advanced or metastatic fibroblast activation protein-positive cancer", CLINICAL CANCER RESEARCH, THE AMERICAN ASSOCIATION FOR CANCER RESEARCH, US, vol. 9, no. 5, 1 May 2003 (2003-05-01), pages 1639-1647, XP002444912, ISSN: 1078-0432 * the whole document * ----- | 1-15 | |
| A | HOFHEINZ R-D ET AL: "STROMAL ANTIGEN TARGETING BY A HUMANISED MONOCLONAL ANTIBODY: AN EARLY PHASE II TRIAL OF SIBROTUZUMAB IN PATIENTS WITH METASTATIC COLORECTAL CANCER", ONKOLOGIE, KARGER, FREIBURG, DE, vol. 26, no. 1, 1 January 2003 (2003-01-01), pages 44-48, XP008049689, ISSN: 0378-584X, DOI: 10.1159/000069863 * the whole document * ----- | 1-15 | TECHNICAL FIELDS SEARCHED (IPC) |
| A,D | WELT ET AL: "Antibody targeting in metastatic colon cancer: a phase I study of monoclonal antibody F19 against a cell-surface protein of reactive tumor stromal fibroblasts", JOURNAL OF CLINICAL ONCOLOGY, AMERICAN SOCIETY OF CLINICAL ONCOLOGY, US, vol. 12, no. 6, 1 June 1994 (1994-06-01), pages 1193-1203, XP002088696, ISSN: 0732-183X * the whole document * ----- -/-- | 1-15 | |

1

The present search report has been drawn up for all claims

| Place of search | Date of completion of the search | Examiner |
|---|---|---|
| The Hague | 2 October 2017 | Hix, Rebecca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 18 1249

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | KALYANI NARRA ET AL: "Phase II trial of single agent Val-boroPro (talabostat) inhibiting fibroblast activation protein in patients with metastatic colorectal cancer", CANCER BIOLOGY & THERAPY, vol. 6, no. 11, 1 November 2007 (2007-11-01), pages 1691-1699, XP055324844, US ISSN: 1538-4047, DOI: 10.4161/cbt.6.11.4874 * the whole document * ----- | 1-15 | |
| A | EAGER ROBERT M ET AL: "Phase II assessment of talabostat and cisplatin in second-line stage IV melanoma", BMC CANCER, BIOMED CENTRAL, LONDON, GB, vol. 9, no. 1, 30 July 2009 (2009-07-30), page 263, XP021057641, ISSN: 1471-2407, DOI: 10.1186/1471-2407-9-263 * the whole document * ----- | 1-15 | TECHNICAL FIELDS SEARCHED (IPC) |
| A,D | WO 2007/077173 A1 (BOEHRINGER INGELHEIM INT [DE]; ADOLF GUENTHER [AT]; OSTERMANN ELINBORG) 12 July 2007 (2007-07-12) * the whole document * ----- | 1-15 | |

The present search report has been drawn up for all claims

1

| Place of search | Date of completion of the search | Examiner |
|---|---|---|
| The Hague | 2 October 2017 | Hix, Rebecca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 18 1249

This annex lists the patent family members relating to the patent documents cited in the above-mentioned European search report.
The members are as contained in the European Patent Office EDP file on
The European Patent Office is in no way liable for these particulars which are merely given for the purpose of information.

02-10-2017

| Patent document cited in search report | Publication date | Patent family member(s) | Publication date |
|---|---|---|---|
| WO 2014055442 A2 | 10-04-2014 | AR 092745 A1 | 29-04-2015 |
| | | EP 2904106 A2 | 12-08-2015 |
| | | TW 201414837 A | 16-04-2014 |
| | | US 2014099340 A1 | 10-04-2014 |
| | | US 2016326265 A1 | 10-11-2016 |
| | | UY 35063 A | 31-07-2014 |
| | | WO 2014055442 A2 | 10-04-2014 |
| WO 2007077173 A1 | 12-07-2007 | AT 478094 T | 15-09-2010 |
| | | CA 2634989 A1 | 12-07-2007 |
| | | EP 1806365 A1 | 11-07-2007 |
| | | EP 2004696 A1 | 24-12-2008 |
| | | ES 2348556 T3 | 09-12-2010 |
| | | JP 2009522329 A | 11-06-2009 |
| | | US 2009304718 A1 | 10-12-2009 |
| | | WO 2007077173 A1 | 12-07-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9305804 A
- WO 9957151 A
- WO 0168708 A
- WO 2007077173 A
- WO 2012020006 A
- WO 2014055442 A2
- WO 2011040972 A
- WO 2014161845 A
- WO 2013026837 A
- EP 10771102 A1
- WO 2012025633 A
- WO 2004072240 A
- US 20110144037 A1
- WO 2009074275 A
- WO 2010127782 A
- WO 2011059836 A
- US 5892019 A
- US 5939598 A, Kucherlapati
- WO 2014055442 A
- WO 2010121140 A
- WO 2012049570 A
- WO 8909622 A
- EP 0239400 A1
- WO 9007861 A
- WO 9110741 A
- WO 9402602 A
- WO 9634096 A
- WO 9633735 A
- WO 8809344 A
- WO 0030680 A
- WO 2005018572 A
- WO 9852976 A
- WO 0034317 A
- WO 02060955 A
- WO 02096948 A2
- US 5837821 A
- WO 9409817 A
- WO 8605807 A
- WO 8901036 A
- US 5122464 A
- US 5736137 A
- US 5658570 A
- US 6413777 B
- US 20030157641 A1
- US 6193980 B
- WO 02096948 A
- US 20020123057 A1
- US 5399346 A
- US 5580859 A
- US 5589466 A
- US 20040014645 A1
- US 20050052630 A1
- US 20050070841 A1
- US 20040059285 A1
- US 20040092907 A1
- US 6678556 B
- US 7171264 B
- US 7173116 B
- US 6567694 B
- US 6516223 B
- US 5993434 A
- US 6181964 B
- US 6241701 B
- US 6233482 B
- US 20070128708 A1
- US 6352694 B
- US 6534055 B
- US 6905680 B
- US 6692964 B
- US 5858358 A
- US 6887466 B
- US 6905681 B
- US 7144575 B
- US 7067318 B
- US 7172869 B
- US 7232566 B
- US 7175843 B
- US 5883223 A
- US 6905874 B
- US 6797514 B
- US 6867041 B
- US 20060121005 A1
- WO 9208495 A
- WO 9114438 A
- WO 8912624 A
- US 5314995 A
- EP 0396387 A
- US 5116964 A
- US 5225538 A
- US 4741900 A
- WO 2012088157 A
- US 5199942 A
- WO 2008081008 A
- US 8906682 B2
- US 20030103968 A1

## Non-patent literature cited in the description


- **YU et al.** *FEBS J.,* 2010, vol. 277, 1126-1144
- **SCANLAN et al.** *Proc. Natl. Acad. Sci. USA,* 1994, vol. 91, 5657-5661
- **SUN et al.** *Protein Expr. Purif.,* 2002, vol. 24, 274-281
- **NIEDERMEYER et al.** *Int. J. Cancer,* 1997, vol. 71, 383-389
- **NIEDERMEYER et al.** *Eur. J. Biochem.,* 1998, vol. 254, 650-654
- **RETTIG et al.** *Proc. Natl. Acad. Sci. USA,* 1988, vol. 85, 3110-3114
- **GARIN-CHESA et al.** *Proc. Natl. Acad. Sci. USA,* 1990, vol. 87, 7235-7239
- **JIN et al.** *Anticancer Res.,* 2003, vol. 23, 3195-3198
- **MERSMANN et al.** *Int. J. Cancer,* 2001, vol. 92, 240-10 248
- **SCHMIDT et al.** *Eur. J. Biochem.,* 2001, vol. 268, 1730-1738
- **WUEST et al.** *J. Biotech.,* 2001, vol. 92, 159-168
- **BRACKS et al.** *Mol. Med,* 2001, vol. 7, 461-469
- **OSTERMANN et al.** *Clin. Cancer Res.,* 2008, vol. 14, 4584-4592
- **WELT et al.** *J. Clin. Oncol.,* 1994, vol. 12, 1193-1203
- **SCOTT et al.** *Clin. Cancer Res.,* 2003, vol. 9, 1639-1647
- **HOFHEINZ et al.** *Onkologie,* 2003, vol. 26, 44-48
- **ZHANG et al.** *FASEB J.,* 2013, vol. 27, 581-589
- **CUNNINGHAM.** *Expert Opinion on Investigational Drugs,* 2007, vol. 16, 1459-1465
- **NARRA et al.** *Cancer Biology & Therapy,* 2007, vol. 6, 1691-1699
- **KELLY et al.** *Journal of the American Chemical Society,* 1993, vol. 115, 12637-12638
- **LEE et al.** *Journal of Thrombosis and Haemostasis,* 2011, vol. 9, 987-996
- **UPRICHARD ; JONES.** *ASH Annual Meeting,* 2004, 4215
- **LANKAS et al.** *Diabetes,* 2005, vol. 54, 2988-2994
- **NAUCK et al.** *Diabetes Care.,* 17 April 2014
- **SANTOS et al.** *Journal of Clinical Investigation,* 2009, vol. 119, 3613-3625
- **LEE et al.** *Journal of Thrombosis and Haemostasis,* 2011, vol. 9, 1268-1269
- **JANSEN et al.** *J. Med. Chem.,* 2014, vol. 57, 3053-3074
- **JANSEN et al.** *Journal of Medicinal Chemistry,* 2014, vol. 57, 3053-3074
- **MAUS ; JUNE.** *Clin. Cancer Res.,* 2016, vol. 22, 1875-1884
- **ZHANG et al.** *Cancer Transl. Med.,* 2015, vol. 1, 43-49
- **DAI et al.** *JNCI J. Natl. Cancer Inst.,* 2016, vol. 108
- **RODGERS et al.** *Proc. Natl. Acad. Sci. USA,* 2016, vol. 113, E459-68
- **TRAN et al.** *J. Exp. Med.,* 2013, vol. 210, 1125-1135
- **SCHUBERTH.** *J. Transl. Med.,* 2013, vol. 11, 187
- **KAKARLA et al.** *Mol. Ther.,* 2013, vol. 21, 1611-1620
- **WANG et al.** *Cancer Immunol. Res.,* 2014, vol. 2, 154-166
- **FISCHER et al.** *Clin. Cancer Res.,* 2012, vol. 15, 6208-6018
- **CHAMES et al.** *British Journal of Pharmacology,* 2009, vol. 157, 220-233
- **VUGMEYSTER et al.** *World J. Biol. Chem.,* 2012, vol. 3, 73-92
- **GILLIES et al.** *Am. J. Physiol.,* 1994, vol. 267 (1), C195-203
- **STUBBS et al.** *Mol. Med. Today,* 2000, vol. 6, 15-19
- **SCHORNACK et al.** *Neoplasia,* 2003, vol. 5, 135-145
- **LIU et al.** *Cancer Biology & Therapy,* 2012, vol. 13, 123-129
- **NICAISE et al.** *Protein Science,* 2004, vol. 13, 1882-1891
- **HOSSE et al.** *Protein Science,* 2006, vol. 15, 14-27
- **LAWSON.** *Nature Reviews Drug Discovery,* 2012, vol. 11, 519-525
- **HORNIG et al.** *J. Immunother.,* 2012, vol. 35, 418-429
- **EISSLER et al.** *Cancer Res.,* 2012, vol. 72, 3958-66
- **HUDSON et al.** *Nat. Med.,* 2003, vol. 9, 129-134
- **TODOROVSKA et al.** *J. Immunol. Methods.,* 2001, vol. 248, 47-66
- **MOORE et al.** *Blood,* 2001, vol. 117, 4542-5451
- **KAST et al.** *Leukemia,* 2002, vol. 16, 970-971
- **BUONAGURO et al.** *Clin. Vac. Immunol.,* 2011, vol. 18, 23-34
- **LEE et al.** *Blood,* 2006, vol. 107, 1397-1404
- **JAVIDROOZI et al.** *Disease Markers,* 2012, vol. 32, 309-320
- **TILLMANNS et al.** *International Journal of Cardiology,* 2013, vol. 168, 3926-3931
- **KEANE et al.** *FEBS Open Bio,* 2014, vol. 4, 43-54
- **PINEIRO-SANCHEZ et al.** *J. Biol. Chem.,* 1997, vol. 12, 7595-7601

- Oxford Dictionary of Biochemistry and Molecular Biology. Oxford University Press, 1997
- **ESTRELLA et al.** *Cancer Res.,* 2013, vol. 73, 1524-1535
- The Atlas of Protein Sequence and Structure. National Biomedical Research Foundation, 1978, vol. 5
- **ARGOS.** *EMBO J.,* 1989, vol. 8, 779-785
- **KARLIN ; ALTSCHUL.** *Proc. Natl. Acad. Sci USA,* 1993, vol. 90, 5873-5877
- **ALTSCHUL et al.** *J. Mol. Biol.,* 1990, vol. 215, 403-410
- **HARLOW et al.** Antibodies: A Laboratory Manual. Cold Spring Harbor Laboratory Press, 1988
- **KABAT, E. et al.** Sequences of Proteins of Immunological Interest. U.S. Department of Health and Human Services, 1983
- **CHOTHIA ; LESK.** *J. Mol. Biol.,* 1987, vol. 196, 901-917
- **KABAT et al.** Sequences of Proteins of Immunological Interest. U.S. Dept. of Health and Human Services, 1983
- **KABAT et al.** Sequence of Proteins of Immunological Interest. U.S. Dept. of Health and Human Services, 1983
- **ROUX et al.** *J. Immunol.,* 1998, vol. 161, 4083-4090
- **DAI et al.** *JNCI J. Natl. Cancer. Inst.,* 2016, vol. 108, 1-15
- **HARLOW et al.** Antibodies: A Laboratory Manual. Cold Spring Harbor Laboratory Press, 1988, 27-28
- Antibody-Antigen Interactions. **BERZOFSKY et al.** Fundamental Immunology. Raven Press, 1984
- **KUBY.** Janis Immunology. W. H. Freeman and Company, 1992
- Remington: The Science and Practice of Pharmacy. University of Sciences in Philadelphia, 2000
- **ROBINSON et al.** Vaccine Protocols. Humana Press, 2003
- **BANGA.** Therapeutic Peptides and Proteins: Formulation, Processing, and Delivery Systems. Taylor and Francis, 2006
- **O'HAGAN et al.** *Nature Reviews, Drug Discovery,* 2003, vol. 2 (9), 727-735
- Remington's Pharmaceutical Sciences. Mace Publishing Company, 1985
- **LANGER.** *Science,* 1990, vol. 249, 1527-1533
- **GAVEL et al.** *Protein Engineering,* 1990, vol. 3, 433-442
- **HELENIUS et al.** *Annu. Rev. Biochem.,* 2004, vol. 73, 1019-1049
- **STAHLI et al.** *Methods in Enzymology,* 1983, vol. 9, 242-253
- **KIRKLAND et al.** *J. Immunol.,* 1986, vol. 137, 3614-3619
- **CHEUNG et al.** *Virology,* 1990, vol. 176, 546-552
- **HARLOW ; LANE.** Antibodies, A Laboratory Manual. Cold Spring Harbor Press, 1988
- **MOREL et al.** *Molec. Immunol.,* 1988, vol. 25, 7-15
- **MOLDENHAUER et al.** *Scand. J. Immunol.,* 1990, vol. 32, 77-82
- **HARLOW ; LANE.** Antibodies, A Laboratory Manual. CSH Press, 1988
- **SCHIER.** *Human Antibodies Hybridomas,* 1996, vol. 7, 97-105
- **MALMBORG.** *J. Immunol. Methods,* 1995, vol. 183, 7-13
- **SAMBROOK.** Molecular Cloning A Laboratory Manual. Cold Spring Harbor Laboratory, 1989
- **AUSUBEL.** Current Protocols in Molecular Biology. Green Publishing Associates and Wiley Interscience, 1994
- **GILLILAND et al.** *Tissue Antigens,* 1996, vol. 47, 1-20
- **DOENECKE et al.** *Leukemia,* 1997, vol. 11, 1787-1792
- **RIECHMANN et al.** *Nature,* 1988, vol. 332, 323-327
- **HAMMERLING et al.** Monoclonal Antibodies and T-Cell Hybridomas. Elsevier, 1981, 563-681
- **KOHLER et al.** *Nature,* 1975, vol. 256, 495
- **GODING.** Monoclonal Antibodies: Principles and Practice. Academic Press, 1986, 59-103
- Current Protocols in Immunology. Green Publishing Associates and Wiley-Interscience, John Wiley and Sons, 1991
- **GROSS ; ESHHAR.** Therapeutic Potential of T Cell Chimeric Antigen Receptors (CARs) in Cancer Treatment: Counteracting Off-Tumor Toxicities for Safe CAR T Cell Therapy. *Annual Review of Pharmacology and Toxicology,* 2016, vol. 56, 59-83
- **ZHANG et al.** New Strategies for the Treatment of Solid Tumors with CAR-T Cells. *Int. J. Biol. Sci.,* 2016, vol. 12, 718-729
- **SCHUBERTH et al.** *J. Transl. Med.,* 2013, vol. 11, 187
- **SMITH ; WATERMAN.** *Advances in Applied Mathematics,* 1981, vol. 2, 482-489
- **KUTMEIER et al.** *BioTechniques,* 1994, vol. 17, 242
- **SAMBROOK et al.** Molecular Cloning, A Laboratory Manual. Cold Spring Harbor Laboratory, 1990
- Current Protocols in Molecular Biology. John Wiley & Sons, 1998
- **UI-TEI et al.** *FEBS Letters,* 2000, vol. 479, 79-82
- **PROUDFOOT.** *Nature,* 1986, vol. 322, 52
- **KOHLER.** *Proc. Natl. Acad. Sci. USA,* 1980, vol. 77, 2197

- **FOECKING et al.** *Gene,* 1986, vol. 45, 101
- **COCKETT et al.** *Bio/Technology,* 1990, vol. 8, 2
- **WIGLER et al.** *Cell,* 1977, vol. 11, 223
- **SZYBALSKA ; SZYBALSKI.** *Proc. Natl. Acad. Sci. USA,* 1992, vol. 48, 202
- **LOWY et al.** *Cell,* 1980, vol. 22, 817
- **WIGLER et al.** *Natl. Acad. Sci. USA,* 1980, vol. 77, 357
- **O'HARE et al.** *Proc. Natl. Acad. Sci. USA,* 1981, vol. 78, 1527
- **MULLIGAN ; BERG.** *Proc. Natl. Acad. Sci. USA,* 1981, vol. 78, 2072
- **GOLDSPIEL et al.** *Clinical Pharmacy,* 1993, vol. 12, 488-505
- **WU ; WU.** *Biotherapy,* 1991, vol. 3, 87-95
- **TOLSTOSHEV.** *Ann. Rev. Pharmacol. Toxicol.,* 1993, vol. 32, 573-596
- **MULLIGAN.** *Science,* 1993, vol. 260, 926-932
- **MORGAN ; ANDERSON.** *Ann. Rev. Biochem.,* 1993, vol. 62, 191-217
- *TIB TECH,* 1993, vol. 11, 155-215
- **SANTERRE et al.** *Gene,* 1984, vol. 30, 147
- Current Protocols in Molecular Biology. John Wiley & Sons, 1993
- **KRIEGLER.** Gene Transfer and Expression, A Laboratory Manual. Stockton Press, 1990
- Current Protocols in Human Genetics. John Wiley & Sons, 1994
- **COLBERRE-GARAPIN et al.** *J. Mol. Biol.,* 1981, vol. 150, 1
- DNA cloning. Academic Press, 1987, vol. 3
- **CROUSE et al.** *Mol. Cell. Biol.,* 1983, vol. 3, 257
- **RUTHER et al.** *EMBO J.,* 1983, vol. 2, 1791
- **INOUYE ; INOUYE.** *Nucleic Acids Res.,* 1985, vol. 13, 3101-3109
- **VAN HEEKE ; SCHUSTER.** *J. Biol. Chem.,* 1989, vol. 24, 5503-5509
- **STINCHCOMB et al.** *Nature,* 1979, vol. 282, 39
- **KINGSMAN et al.** *Gene,* 1979, vol. 7, 141
- **TSCHEMPER et al.** *Gene,* 1980, vol. 10, 157
- **JONES.** *Genetics,* 1977, vol. 85, 12
- **SCOPES.** Protein Purification. Springer Verlag, 1982
- **MILONE et al.** *Mol. Ther.,* 2009, vol. 17, 1453-1464
- **SCHUTSKY et al.** *Oncotarget,* 2015, vol. 6, 28911-28928
- **BERG et al.** *Transplant Proc.,* 1998, vol. 30, 3975-3977
- **HAANEN et al.** *J. Exp. Med.,* 1999, vol. 190, 13191328
- **GARLAND et al.** *J. Immunol. Meth.,* 1999, vol. 227, 53-63
- **T. E. CREIGHTON.** Proteins - Structure And Molecular Properties. W. H. Freeman and Company, 1993
- Posttranslational Covalent Modification Of Proteins. Academic Press, 1983, 1-12
- **SEIFTER et al.** *Meth. Enzymol.,* 1990, vol. 182, 626-646
- **RATTAN et al.** *Ann. NY Acad. Sci.,* 1992, vol. 663, 48-62
- **GASCOIGNE et al.** *Proc. Natl. Acad. Sci. USA,* 1987, vol. 84, 2936-2940
- **CAPON et al.** *Nature,* 1989, vol. 337, 525-531
- **TRAUNECKER et al.** *Nature,* 1989, vol. 339, 68-70
- **ZETTMEISSL et al.** *DNA Cell Biol. USA,* 1990, vol. 9, 347-353
- **BYRN et al.** *Nature,* 1990, vol. 344, 667-670
- **WATSON et al.** *J. Cell. Biol.,* 1990, vol. 110, 2221-2229
- **WATSON et al.** *Nature,* 1991, vol. 349, 164-167
- **ARUFFO et al.** *Cell,* 1990, vol. 61, 1303-1313
- **LINSLEY et al.** *J. Exp. Med.,* 1991, vol. 173, 721-730
- **LISLEY et al.** *J. Exp. Med.,* 1991, vol. 174, 561-569
- **STAMENKOVIC et al.** *Cell,* 1991, vol. 66, 1133-1144
- **ASHKENAZI et al.** *Proc. Natl. Acad. Sci. USA,* 1991, vol. 88, 10535-10539
- **LESSLAUER et al.** *Eur. J. Immunol.,* 1991, vol. 27, 2883-2886
- **PEPPEL et al.** *J. Exp. Med.,* 1991, vol. 174, 1483-1489
- **RIDGWAY ; GORMAN.** *J. Cell. Biol.,* 1991, vol. 115
- **LEONG et al.** *Cytokine,* 2001, vol. 16, 106-119
- *Adv. in Drug Deliv. Rev.,* 2002, vol. 54, 531
- **WEIR et al.** *Biochem. Soc. Transactions,* 2002, vol. 30, 512
- **GENTZ et al.** *Proc. Natl. Acad. Sci. USA,* 1989, vol. 86, 821-824
- **WILSON et al.** *Cell,* 1984, vol. 37, 767
- **BILL BRIZZARD.** *BioTechniques,* 2008, vol. 44, 693-695
- **BYERS.** *Seminars Cell. Biol.,* 1991, vol. 2, 59-70
- **FANGER.** *Immunol. Today,* 1991, vol. 12, 51-54
- **RONCA et al.** *Immunobiology,* 2009, vol. 214, 800-810
- The Enzyme Linked Immunosorbent Assay (ELISA). **VOLLER, A.** Microbiological Associates Quarterly Publication. Diagnostic Horizons, 1978, vol. 2, 1-7
- **VOLLER et al.** *J. Clin. Pathol.,* 1978, vol. 31, 507-520

- **BUTLER.** *Meth. Enzymol.,* 1981, vol. 73, 482-523
- Enzyme Immunoassay. CRC Press, 1980
- Enzyme Immunoassay. Kgaku Shoin, 1981
- **WEINTRAUB, B.** Principles of Radioimmunoassays, Seventh Training Course on Radioligand Assay Techniques. The Endocrine Society, March 1986
- Monoclonal Antibodies For Immunotargeting Of Drugs In Cancer Therapy. **ARNON et al.** Monoclonal Antibodies And Cancer Therapy. Alan R. Liss, Inc, 1985, 243-56
- Antibodies For Drug Delivery. **HELLSTROM et al.** Controlled Drug Delivery. Marcel Dekker, Inc, 1987, 623-53
- Antibody Carriers Of Cytotoxic Agents In Cancer Therapy: A Review. **THORPE et al.** Monoclonal Antibodies '84: Biological And Clinical Applications. 1985, 475-506
- Analysis, Results, And Future Prospective Of The Therapeutic Use Of Radiolabeled Antibody In Cancer Therapy. Monoclonal Antibodies For Cancer Detection And Therapy. Academic Press, 1985, 303-16
- **THORPE et al.** The Preparation And Cytotoxic Properties Of Antibody-Toxin Conjugates. *Immunol. Rev.,* 1982, vol. 62, 119-158
- **LEONG et al.** *Cytokine,* 2001, vol. 16
- **MILLER ; MESSER.** *Molecular Therapy,* 2005, vol. 12, 394-401
- **MULLER et al.** *Expert Opin. Biol. Ther.,* 2005, 237-241
- **NOGUCHI et al.** *Acta Med. Okayama,* 2006, vol. 60
- **KUSNEZOW et al.** *Mol. Cell Proteomics,* 2006, vol. 5, 1681-1696
- **ROSENBERG et al.** *New Eng. J. Med,* 1988, vol. 319, 1676
- **LIU et al.** *Cell,* 1991, vol. 66, 807-815
- **HENDERSON et al.** *Immun.,* 1991, vol. 73, 316-321
- **BIERER et al.** *Curr. Opin. Immun.,* 1993, vol. 5, 763-773
- **ERTL et al.** *Cancer Res,* 2011, vol. 71, 3175-3181
- **JUNGHANS.** *Journal of Translational Medicine,* 2010, vol. 8, 55
- **BERKS.** *TIBTECH,* 1994, vol. 12, 352-364
- **SMITH et al.** *Nat. Protoc.,* 2009, vol. 4, 372-384
- **MARKS et al.** *Mol. Biol.,* 1991, vol. 222, 581-597
- **DE HAARD et al.** *J. Biol. Chem.,* 1999, vol. 26, 18218-18230
- **TABRIZI et al.** Development of Antibody-Based Therapeutics. 218-219
- *Science,* 19 September 1986
- *J. Exp. Med.,* 2013, vol. 210 (6), 1125-1135
- **HECKMAN ; PEASE.** *Nat. Protoc.,* 2007, vol. 2, 924-923
- **KOHLI et al.** *Proteome Res.,* 2012, vol. 11, 4075-4090
- **DOTTI et al.** *Immunol. Rev.,* 2014, vol. 257, 107-126
- **CARPENITO et al.** *Proc. Natl. Acad. Sci. U S A,* 2009, vol. 106, 3360-3365
- **KAKARLA et al.** *Mol Ther,* 2013, vol. 21, 1611-1620
- **GERSBACHER et al.** *PLoS One,* 2013, vol. 8, e69363